# EUROPEAN PATENT SPECIFICATION

(11) **EP 1 747 210 B1**
(45) Date of publication and mention of the grant of the patent: **30.06.2010**
(21) Application number: 05718521.7
(22) Date of filing: 19.04.2005
(51) Int. Cl.: C07D 309/06, C07D 405/12, C07D 309/08, C07D 295/12, C07D 405/06, C07D 405/14, C07D 295/14, C07D 417/04, C07D 417/06, A61K 31/55, A61P 25/06

(54) **3- OR 4-MONOSUBSTITUTED PHENOL AND THIOPHENOL DERIVATIVES USEFUL AS H3 LIGANDS**
ALS H3-LIGANDEN GEEIGNETE 3- ODER 4-MONOSUBSTITUIERTE PHENOL- UND THIOPHENOL-DERIVATE
DÉRIVÉS DE PHÉNOL 3- OU 4-MONOSUBSTITUÉS UTILES COMME LIGANDS DE H3

(30) Priority: 07.05.2004 EP 04291187; 04.03.2005 GB 0504564
(43) Date of publication of application: 31.01.2007
(73) Proprietor: Warner-Lambert Company LLC, New York, NY 10017 (US)
(72) Inventor: BERNARDELLI, P., Pfizer Global Res. and Develop., Kent CT13 9NJ (GB); CRONIN, A.M., Pfizer Global Res. andDevelopment, Kent CT13 9NJ (GB); DENIS, A., Pfizer Global Res. and Development, Kent CT13 9NJ (GB); DENTON, S.M., Pfizer Global Res. andDevelopment, Kent CT13 9NJ (GB); JACOBELLI, H., Pfizer Global Res. and Development, Kent CT13 9NJ (GB); KEMP, M.I., Pfizer Global Res. and Development, Kent CT13 9NJ (GB); LORTHIOIS, E., Pfizer Global Res. and Development, Kent CT13 9NJ (GB); ROUSSEAU, F., Pfizer Global Res. and Development, Kent CT13 9NJ (GB); SERRADEIL-CIVIT, D., Pfizer Global Res. & Develop., Kent CT13 9N (GB); VERGNE, F., Pfizer Global Res. and Development, Kent CT13 9NJ (GB)
(74) Representative: Hodge, Emma Jane
(86) International application number: PCT/IB2005/001114
(87) International publication number: WO 2005/108384

(56) References cited:
- EP-A- 0 269 363
- WO-A-02/06223
- WO-A-02/12190
- WO-A-02/076925
- DE-A1- 3 024 836
- DATABASE BEILSTEIN BEILSTEIN INSTITUTE FOR ORGANIC CHEMISTRY, FRANKFURT-MAIN, DE; XP002300332 & Z. CHEM., vol. 20, no. 8, 1980, pages 298-299,

## Description

The present invention relates to 3- or 4-monosubstituted phenol and thiophenol derivatives of general formula : 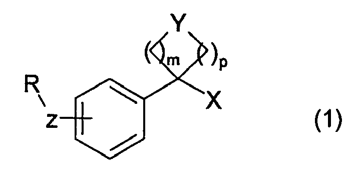 in which R, X, Y, Z, m and p have the meanings indicated below,
and to processes for the preparation of, intermediates used in the preparation of, compositions containing and the uses of, such derivatives.

Histamine H₃ receptors are found inter alia on presynaptic terminals of peripheral nerves, where they modulate autonomic neurotransmission and modulate a variety of end organ responses under control of the autonomic nervous system. They are also heteroreceptors, modulating the release of numerous other neurotransmitters such as dopamine, glutamate, noradrenaline, serotonin, GABA, acetylcholine, some peptides and co-transmitters.

Recently numerous histamine H₃ receptor ligands have been developed. An overview of the current advance in H₃ ligand research and patenting is given in Expert Opin. Ther. Patents (2003) 13(6). Examples of Histamine H₃ receptor ligands can be found in WO02/76925, WO00/06254, WO02/12190, WO02/12214 and WO02/06223

H₃ receptor ligands are believed to be suitable for the treatment of various diseases including both disorders of the central nervous system and inflammatory disorders. Examples of diseases where treatment with H₃ ligands is believed to be useful are inflammatory bowel disease, Crohn's disease, colitis ulcerosa, sleep disorders, migraine, dyskinesia, stress-induced anxiety, psychotic disorders, epilepsy, Cognition deficiency diseases such as Alzheimer's disease or mild coginitive impairment, depression, mood disorders, schizophrenia, anxiety disorders, attention-deficit hyperactivity disorder (ADHD), psychotic disorders, obesity, dizziness, epilepsy, motion sickness, vertigo, respiratory diseases such as adult respiratory distress syndrome, acute respiratory distress syndrome, bronchitis, chronic bronchitis, chronic obstructive pulmonary disease, cystic fibrosis, asthma, emphysema, rhinitis, chronic sinusitis, allergy, allergy-induced airway responses, allergic rhinitis, viral rhinitis, non-allergic rhinitis, perennial and seasonal rhinitis, nasal congestion and allergic congestion.

Although H₃ ligands are known there is still a need to provide new H₃ ligands that are good drug candidates. In particular, preferred compounds should bind potently to the histamine H₃ receptor whilst showing little affinity for other receptors. They should be well absorbed from the gastrointestinal tract, be metabolically stable and possess favourable pharmacokinetic properties. They should be non-toxic and demonstrate few side-effects.

In this context, the present invention concerns new substituted phenol and thiophenol derivatives of general formula (1) : 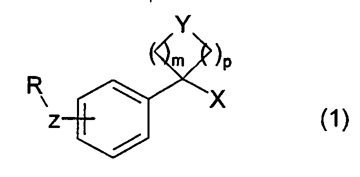 or a pharmaceutically acceptable salt and/or thereof wherein :
the substituent of formula -Z-R is in the meta or para position of the phenyl group;
X is selected from -CN, -CH₂OH, -CH₂-O-(C₁-C₄)alkyl, -C(O)OH, -C(O)O(C₁-C₄)alkyl, -CH₂-NR¹R², - C(O)NR³R⁴, -CH₂-O-het², -CH₂-het¹ and het¹, the group het¹ in both -CH₂-het¹ and het¹ being optionally substituted by one or two substituents independently selected from halo, cyano, (C₁-C₄)alkyl, -S-(C₁-C₄)alkyl and (C₁-C₄)alkoxy;
R¹ is hydrogen or (C₁-C₄)alkyl optionally substituted by (C₃-C₆)cycloalkyl;
R² is selected from the group consisting of :
   hydrogen,
   (C₁-C₆)alkyl optionally substituted by one or two substituents independently selected from (C₃-C₆)cycloalkyl, hydroxy, -S-(C₁-C₄)alkyl, -O-(C₁-C₄)alkyl, -SO₂-(C₁-C₄)alkyl, -SO-(C₁-C₄)alkyl, halo, het¹, amino, (C₁-C₄)alkylamino [(C₁-C₄)alkyl]₂amino and phenyl, said phenyl being optionally substituted by one or two substituents independently selected from halo, hydroxy, cyano, (C₁-C₄)alkyl and (C₁-C₄)alkoxy, (C₃-C₆)cycloalkyl,
   het², optionally substituted by one or two substituents independently selected from halo, cyano, (C₁-C₄)alkyl, NH₂ and (C₁-C₄)alkoxy, -SO₂-R⁵ wherein R⁵ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionally substituted by one or two substituents independently selected from halo, cyano, (C₁-C₄)alkyl and (C₁-C₄)alkoxy, and
   -C(O)-R⁶ wherein R⁶ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionaly substituted by one or two substituents independently selected from halo, cyano, (C₁-C₄)alkyl and (C₁-C₄)alkoxy;
or R¹ and R² form together with the N atom to which they are attached a 3-, 4-, 5-, 6- or 7-membered saturated heterocycle wherein one C atom may be replaced by N, O, S, SO or SO₂ and wherein said
   saturated heterocycle is optionally substituted by one or two groups independently selected from hydroxy, halo, =O, (C₁-C₄)alkyl, -(C₁-C₄)alkyl(C₃-C₆)cycloalkyl, (C₁-C₄)alkoxy, hydroxy(C₁-C₄)alkyl, (C₁-C₄)alkoxy(C₁-C₄)alkyl, -SO₂(C₁-C₄)alkyl, -C(O)(C₁-C₄)alkyl, [(C₁-C₄)alkyl]₂amino, amino, (C₁-C₄)alkylamino, -C(O)NH₂, C(O)O(C₁-C₄)alkyl and pyrrolidinone;
R³ and R⁴ are each independently selected from hydrogen, (C₃-C₆)cycloalkyl, and (C₁-C₄)alkyl, said (C₃-C₆)cycloalkyl and (C₁-C₄)alkyl being optionally substituted by amino, (C₁-C₄)alkylamino, [(C₁-C₄)alkyl]₂amino or (C₃-C₆)cycloalkyl, or R³ and R⁴ form together with the N atom to which they are attached a 4-, 5-, 6- or 7-membered saturated heterocycle wherein one C atom may be replaced by N or O and wherein said saturated heterocycle is optionally substituted by (C₁-C₄)alkyl, [(C₁-C₄)alkyl]₂amino, amino, (C₁-C₄)alkylamino, or -C(O)(C₁-C₄)alkyl, said -C(O)(C₁-C₄)alkyl being optionally substituted by methoxy or ethoxy,
Y is selected from CH₂, CH(OH), O, C=O and N, said N being substituted by H, (C₁-C₄)alkyl, C(O)(C₁-C₄)alkyl or (C₁-C₄)alkoxy(C₁-C₄)alkyl;
Z is selected from O, S, SO and SO₂;
m and p are both integers which are independently 1, 2 or 3, with the condition that m+p is equal to or less than 4 so that the ring formed by : 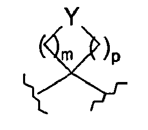 is a 4-, 5- or 6-membered ring;
and R is either a group of formula : 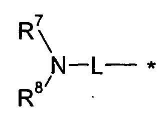 wherein * represents the attachment point to Z, L is a straight chain or branched (C₂-C₆)alkylene and R⁷ and R⁸ are each independently selected from hydrogen, (C₁-C₆)alkyl, (C₃-C₆)cycloalkyl, hydroxy(C₁-C₆)alkyl or R⁷ and R⁸ form together with the N atom to which they are attached a 4-, 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂, halo, amino, (C₁-C₄)alkylamino and [(C₁-C₄)alkyl]₂amino,
   or R is a group of formula: 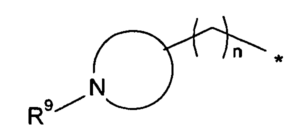 wherein * represents the attachment point to Z, the N-containing ring is a 4- to 7-membered saturated heterocycle, n is an integer equal to 0, 1 or 2, and R⁹ represents a substituent selected from hydrogen, (C₁-C₄)alkyl, hydroxy(C₁-C₆ alkyl) and (C₃-C₆)cycloalkyl;
het¹ is selected from monocyclic or bicyclic heteroaromatic groups having 5 to 10 ring members, which contain 1, 2, 3 or 4 heteroatom(s) selected from nitrogen, oxygen and sulphur and het² is selected from monocyclic or bicyclic heteroaromatic groups having 5 to 10 ring members, which contain 1, 2, 3 or 4 heteroatom(s) selected from nitrogen, oxygen and sulphur.

It has been found that these compounds are H3 ligands and are thus particularly useful for the treatment of H3-related diseases such as neurologic disorders, or inflammatory, respiratory and allergic diseases, disorders and conditions.

In the present description the following definitions are used, unless otherwise specified.
"halo" denotes a halogen atom selected from the group consisting of fluoro, chloro, bromo and iodo. "(C₁-Cₓ)alkyl" denotes a saturated, straight-chain or branched hydrocarbon group having from 1 to x carbon atoms and includes for example (when x= 4) methyl, ethyl, propyl, i-propyl, n-butyl, i-butyl, sec-butyl and t-butyl and further (when x=6) pentyl, I-pentyl, n-pentyl and hexyl. This also applies if the alkyl group carries substituents or is a substituent for another group, e.g. in -S-(C₁-C₄)alkyl, -O-(C₁-C₄)alkyl, - SO₂-(C₁-C₄)alkyl, -SO-(C₁-C₄)alkyl, -CH₂-O-(C₁-C₄)alkyl, -C(O)O(C₁-C₄)alkyl, (C₁-C₄)alkylamino, [(C₁-C₄)alkyl]₂amino, -(C₁-C₄)alkyl-phenyl, -(C₁-C₄)alkyl(C₃-C₆)cycloalkyl, hydroxy(C₁-C₄)alkyl, (C₁-C₄)alkoxy(C₁-C₄)alkyl, -C(O)(C₁-C₄)alkyl.
"(C₁-C₄)alkoxy" denotes straight-chain and branched alkoxy groups and includes for example methoxy, ethoxy, n-propoxy, i-propoxy, n-butoxy, i-butoxy, sec-butoxy and t-butoxy.
"(C₂-C₆)alkylene" denotes a divalent radical derived from straight-chain or branched alkane containing from 2 to 6 carbon atoms. Examples of (C₂-C₆)alkylene radicals are methylene, ethylene (1,2-ethylene or 1,1-ethylene), trimethylene (1,3-propylene), tetramethylene (1,4-butylene), pentamethylene and hexamethylene.
"hydroxy(C₁-C₄)alkyl" radicals are alkyl radicals substituted by hydroxy. They can contain 1 or several hydroxy substituents, if not stated otherwise. Examples of suitable hydroxy(C₁-C₄)alkyl radicals are hydroxymethyl, 1-hydroxyethyl or 2-hydroxyethyl.
"(C₃-C₆)cycloalkyl" denotes a saturated monocyclic carbocyclic group having 3 to 6 carbon atoms and includes for example cyclopropyl, cyclobutyl, cyclopentyl and cyclohexyl.

In the case where the (C₁-Cₓ)alkyl radicals are substituted by halo, such radical can contain 1 or several halogen atoms, if not stated otherwise. Said halo is preferably a fluoro, a chloro, a bromo or a iodo, in particular fluoro or chloro. For example in a fluoro-substituted alkyl radical, a methyl group can be present as a difluoromethyl or a trifluoromethyl group.

"saturated heterocycle" denotes a saturated monocyclic group having 4 to 7 ring members, which contains 1 nitrogen atom and optionally a further heteroatom selected from nitrogen, oxygen and sulphur. Examples of saturated heterocycles are azetidinyl, pyrrolidinyl, piperidinyl, morpholinyl, piperazinyl, oxazepanyl and azepanyl.
het¹ and het² are monocyclic or bicyclic heteroaromatic groups having 5 to 10 ring members, which contain 1, 2, 3 or 4 heteroatom(s) selected from nitrogen, oxygen and sulphur. In particular the heteroaromatic group contains either (a) 1 to 4 nitrogen atoms, (b) one oxygen atom or one sulfur atom or (c) 1 oxygen atom or 1 sulfur atom and 1 or 2 nitrogen atoms. het² is preferably C-linked, which means that the group is linked to the adjacent atom by a ring carbon atom. het¹ can be C-linked or N-linked. The heteroaryl group can be unsubstituted, monosubstituted or disubstituted, as indicated in the definition of X and R² hereabove for general formula (1) according to the present invention. Examples of heteroaryl groups include, but are not limited to thiophenyl, furanyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyranyl, pyridinyl, pyrazinyl, pyrimidinyl, pyridazinyl, triazinyl, thiadiazinyl, isobenzofuranyl, benzofuranyl, chromenyl, indolizinyl, isoindolyl, indolyl, indazolyl, purinyl, quinolinyl, isoquinolyl, cinnolinyl, phthalazinyl, naphthyridinyl, quinazolinyl, quinoxalinyl, benzoxazolyl, benzothiazolyl, benzimidazolyl, benzofuranyl, benzothiophenyl, pyrrolopyrazinyl, pyrrolopyridinyl, and imidazopyridinyl. Preferred definitions for het¹ and het² will follow hereafter.

In the general formula (1) according to the present invention, when a radical is mono- or poly-substituted, said substituent(s) can be located at any desired position(s), unless otherwise stated. Also, when a radical is polysubstituted, said substituents can be identical or different, unless otherwise stated.

According to a preferred aspect of the invention, X is selected from -CN, -CH₂OH, -C(O)OH, -CH₂-NR¹R², -C(O)NR³R⁴, -CH₂-het¹ and het¹, het¹ in both -CH₂-het¹ and het¹ being optionally substituted once or twice by (C₁-C₄)alkyl, more preferably X is selected from -CH₂-NR¹R², -CONR³R⁴, -CH₂-het¹ and het¹, het¹ in both -CH₂-het¹ and het¹ being optionally substituted once or twice by (C₁-C₄)alkyl, wherein R¹, R², R³, R⁴ and het¹ are as previously defined.

het¹ is preferably selected from a 5 or 6 membered monocyclic heteroaromatic group, or a 9 membered bicyclic heteroaromatic group, each heteroaromatic group containing 1 to 3 nitrogen atoms, or 1 to 2 nitrogen atoms and 1 oxygen atom, or 1 nitrogen atom and 1 sulphur atom, and each heteroaromatic group being optionally substituted once or twice by (C₁-C₄)alkyl, and preferably, optionally substituted once or twice by (C₁-C₂)alkyl.

More preferably, X is thiazolyl, benzimidazolylmethyl-, pyridinyl, oxazolyl, imidazopyridinylmethyl-, pyrimidinyl, imidazolyl, imidazolylmethyl- or triazolylmethyl-, said thiazolyl, benzimidazolylmethyl-, pyridinyl, oxazolyl, imidazopyridinylmethyl-, pyrimidinyl, imidazolyl, imidazolylmethyl- and triazolylmethyl- each being optionally substituted with one methyl group.

R¹ is preferably hydrogen, methyl or ethyl.

R² is preferably selected from the group consisting of
hydrogen,
(C₁-C₆)alkyl optionally substituted by one or two substituents independently selected from -S-(C₁-C₄)alkyl, -O-(C₁-C₄)alkyl, -SO₂-(C₁-C₄)alkyl, and phenyl, said phenyl being optionally substituted by one or two substituents independently selected from halo, hydroxy, cyano, (C₁-C₄)alkyl and (C₁-C₄)alkoxy,
(C₃-C₆)cycloalkyl,
het² optionally substituted by one or two substituents independently selected from halo, cyano, (C₁-C₄)alkyl and (C₁-C₄)alkoxy, wherein het² is defined as above,
-SO₂-R⁵ wherein R⁵ is selected from the group consisting of (C₁-C₄)alkyl, [(C₁-C₄)alkyl]₂amino, phenyl, and -(C₁-C₄)alkyl-phenyl, wherein said phenyl is optionally substituted by 1 substituent independently selected from halo and cyano and
-C(O)-R⁶ wherein R⁶ is selected from the group consisting of (C₁-C₄)alkyl, ((C₁-C₄)alkyl]₂amino, amino, and -(C₁-C₄)alkyl-phenyl, said phenyl being optionally substituted by one or two substituents independently selected from halo, cyano, (C₁-C₄)alkyl and (C₁-C₄)alkoxy.

More preferably R² is selected from the group consisting of
(C₁-C₃)alkyl optionally substituted by -O-(C₁-C₃)alkyl, preferably methoxy,
(C₃-C₅)cycloalkyl,
het², wherein het² is preferably selected from the group consisting of 5 or 6 membered monocyclic heteroaromatic ring systems containing 1 to 2 nitrogen atoms or 1 nitrogen atom and 1 oxygen atom or 1 nitrogen atom and 1 sulphur atom, said het² being optionally substituted by one or two substituents independently selected from halo, cyano, (C₁-C₄)alkyl and (C₁-C₄)alkoxy, preferably (C₁-C₄)alkyl,
SO₂-R⁵ wherein R⁵ is selected from the group consisting of (C₁-C₄)alkyl, [(C₁-C₄)alkyl]₂amino, phenyl, and -(C₁-C₄)alkyl-phenyl, wherein said phenyl is optionally substituted by 1 substituent independently selected from halo and cyano and wherein R⁵ is preferably (C₁-C₄)alkyl,
C(O)-R⁶ wherein R⁶ is selected from the group consisting of (C₁-C₄)alkyl, ((C₁-C₄)alkyl]₂amino, amino, and -(C₁-C₄)alkyl-phenyl, said phenyl being optionally substituted by one or two substituents independently selected from halo, cyano, (C₁-C₄)alkyl and (C₁-C₄)alkoxy, and wherein R6 is preferably (C₁-C₄)alkyl.

In another aspect of the invention, R² is preferably (C₁-C₃)alkyl optionally substituted by methoxy.
het² is preferably selected from the group consisting of 5 or 6 membered monocyclic heteroaromatic ring systems containing 1 or 2 nitrogen atoms.

In another aspect of the invention, R² is preferably a pyridazinyl group.

According to a further preferred aspect of the invention R¹ and R² form together with the N atom to which they are attached a 4-, 5-, 6- or 7-membered saturated heterocycle wherein one C atom may be replaced by N, O, S, SO or SO₂ and wherein said saturated heterocycle is optionally substituted by one or two groups independently selected from hydroxy, halo, =O, (C₁-C₄)alkyl, -(C₁-C₄)alkyl(C₃-C₆)cycloalkyl, (C₁-C₄)alkoxy, hydroxy(C₁-C₄)alkyl, (C₁-C₄)alkoxy(C₁-C₄)alkyl, -SO₂(C₁-C₄)alkyl, -C(O)(C₁-C₄)alkyl, [(C₁-C₄)alkyl]₂amino, -C(O)NH₂, C(O)O(C₁-C₄)alkyl and pyrrolidinone, more preferably R¹ and R² form together with the N atom to which they are attached a 5- or 6-membered saturated heterocycle selected from 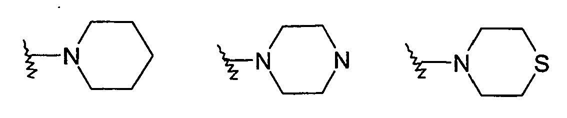 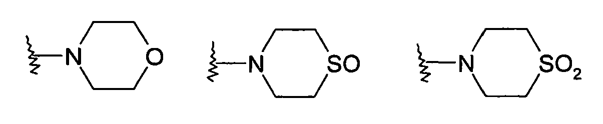 or 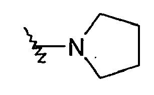 optionally substituted by one or two groups independently selected from hydroxy, fluoro, hydroxymethyl, methoxymethyl, SO₂(C₁-C₄)alkyl, -C(O)(C₁-C₄)alkyl, -CONH₂, and pyrrolidinone.

Even more preferably, R¹ and R² form together with the N atom to which they are attached a morpholinyl group.

Preferably R³ and R⁴ are each independently selected from hydrogen and (C₁-C₄)alkyl or R³ and R⁴ form together with the N atom to which they are attached a 4, 5 or 6 membered saturated heterocycle wherein one C atom may be replaced by N or O and wherein said saturated heterocycle is optionally substituted by (C₁-C₄)alkyl.

More preferably, R³ and R⁴ are selected independently from hydrogen, methyl and ethyl, or R³ and R⁴ form together with the N atom to which they are attached a pyrrolidinyl, piperidinyl, piperazinyl or azetidinyl ring, (each pyrrolidinyl, piperidinyl, piperazinyl and azetidinyl ring being optionally methyl substituted).

Preferably, Y is selected from CH₂, CH(OH), O and C=O, more preferably Y is O.

Preferably, Z is O

Preferably m is equal to 1 or 2 and p is equal to 2, more preferably m and p are both 2.

In one preferred aspect of the invention R is a group of formula : 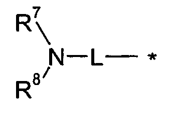 wherein * represents the attachment point to Z, L is a (C₂-C₅)alkylene and R⁷ and R⁸ are each independently selected from hydrogen, (C₁-C₆)alkyl, (C₃-C₆)cycloalkyl, hydroxy(C₁-C₆)alkyl or R⁷ and R⁸ form together with the N atom to which they are attached a 4-, 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂ and halo. Examples of the 4-, 5-, 6- or 7-membered saturated heterocycle are : 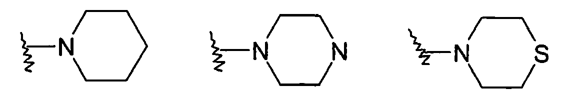 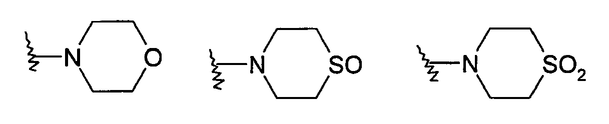 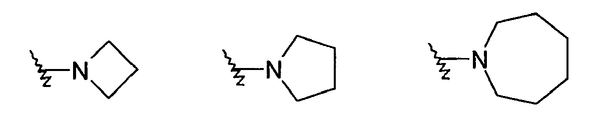 or

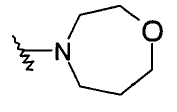

Even more preferably, R⁷ and R⁸ form together with the N atom to which they are attached a morpholinyl or oxazepanyl group.

In another preferred aspect, R⁷ and R⁸ form together with the N atom to which they are attached a 4-, 5- or 6-membered saturated heterocycle, optionally substituted by one or two (C₁-C₄)alkyl, preferably methyl. In another more preferred aspect, the saturated heterocycle is a pyrrolidinyl group, optionally substituted by one or two methyl groups.

Preferably, L is propylene.

According to another preferred aspect of the invention R is a group of formula: 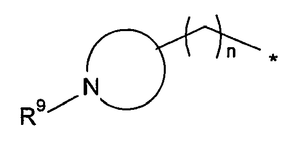 wherein * represents the attachment point to Z, the N-containing ring is a 4- or 6-membered saturated heterocycle, n is an integer equal to 0 or 1, and R⁹ represents a substituent selected from hydrogen, C₁-C₄)alkyl and (C₃-C₆)cycloalkyl.

Preferably, R⁹ is isopropyl or cyclobutyl.

When one of the groups in the compound of formula (1) is substituted by halo, generally fluoro or chloro, in particular fluoro is preferred.

Particularly preferred compounds of the invention include those in which each variable in formula (1) is selected from the suitable and/or preferred groups for each variable. Even more preferable compounds of the invention include those where each variable in formula (1) is selected from the more preferred or most preferred groups for each variable.

According to a particular aspect of the invention the following compounds are excluded from the invention: compounds of formula (1), wherein Y is O, Z is O, R is a group of formula : 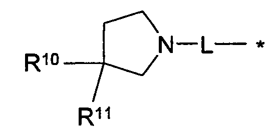 wherein R¹⁰ is hydrogen or (C₁-C₄)alkyl, R¹¹ is (C₁-C₄)alkoxy, hydroxy or N((C₁-C₄)alkyl)₂ and X is -CN, - CH₂OH, -CH₂-O-(C₁-C₄)alkyl, -C(O)OH, -C(O)O(C₁-C₄)alkyl, -CONR³R⁴ or CH₂NR¹R² wherein R¹ is hydrogen or a (C₁-C₄)alkyl group and R² is either hydrogen, (C₁-C₄)alkyl (optionally substituted by phenyl) or -C(O)(C₁-C₄)alkyl.

In another emdodiment of the present invention, there is provided a compound of formula (1) selected from:
3-(4-{4-[(dimethylamino)methyl]tetrahydro-2H-pyran-4-yl}phenoxy)-N,N-dimethylpropan-1-amine
1-isopropyl-4-{4-[4-(4-methyl-1,3-thiazol-2-yl)tetrahydro-2H-pyran-4-yl]phenoxy}piperidine
4-methyl-2-{4-[4-(4-pyrrolidin-1-ylbutoxy) phenyl]tetrahydro-2H-pyran-4-yl}-1,3-thiazole
2-{4-[4-(4-pyrrolidin-1-ylbutoxy)phenyl]tetrahydro-2H-pyran-4-yl}-1,3-thiazole
4-(4-{3-[ethyl(methyl)amino]propoxy}phenyl)tetrahydro-2H-pyran-4-carbonitrile
4-[4-(4-pyrrolidin-1-ylbutoxy)phenyl]tetrahydro-2H-pyran-4-carboxamide
1-({4-[4-(4-pyrrolidin-1-ylbutoxy)phenyl]tetrahydro-2H-pyran-4-yl}carbonyl)pyrrolidine
N-ethyl-N',N'-dimethyl-N-((4-[4-(3-pyrrolidin-1-ylpropoxy)phenyl]tetrahydro-2H-pyran-4-yl)methyl)ethane-1,2-diamine
1-(4-{4-[4-(azetidin-1-ylcarbonyl)tetrahydro-2H-pyran-4-yl]phenoxy}butyl)pyrrolidine
N,N-dimethyl-4-[4-(4-pyrrolidin-1-ylbutoxy)phenyl]tetrahydro-2H-pyran-4-carboxamide
N-methyl-1-pyridin-2-yl-N-({4-[4-(3-pyrrolidin-1-ylpropoxy)phenyl]tetrahydro-2H-pyran-4-yl}methyl)methanamine
1-cyclohexyl-N-methyl-N-({4-[4-(3-pyrrolidin-1-ylpropoxy)phenyl]tetrahydro-2H-pyran-4-yl}methyl)methanamine
N, N-dimethyl-1-({4-[4-(3-pyrrolidin-1-ylpropoxy)phenyl]tetrahydro-2H-pyran-4-yl}carbonyl)azetidin-3-amine
N,N,N'-trimethyl-N'-({4-[4-(3-pyrrolidin-1-ylpropoxy)phenyl]tetrahydro-2H-pyran-4-yl}methyl)ethane-1,2-diamine
N,N-dimethyl-1-({4-[4-(3-pyrrolidin-1-ylpropoxy)phenyl]tetrahydro-2H-pyran-4-yl}methyl)azetidin-3-amine
N-(3-{4-[4-(aminomethyl)tetrahydro-2H-pyran-4-yl]phenoxy}propyl)cyclobutanamine
3-{4-[4-(aminomethyl)tetrahydro-2H-pyran-4-yl]phenoxy}-N-ethyl-N-methylpropan-1-amine
N-cyclobutyl-4-[4-(3-pyrrolidin-1-ylpropoxy)phenyl]tetrahydro-2H-pyran-4-carboxamide
N-cyclopentyl-4-[4-(3-pyrrolidin-1-ylpropoxy)phenyl]tetrahydro-2H-pyran-4-carboxamide
N-(cyclopropylmethyl)-4-[4-(3-pyrrolidin-1-ylpropoxy)phenyl]tetrahydro-2H-pyran-4-carboxamide
N-cyclohexyl-4-[4-(3-pyrrolidin-1-ylpropoxy)phenyl]tetrahydro-2H-pyran-4-carboxamide
1-(4-{4-[(2-pyrrolidin-1-ylethyl)thio]phenyl}tetrahydro-2H-pyran-4-yl)methanamine
N,N-dimethyl-1-(4-{4-[(2-pyrrolidin-1-ylethyl)thio]phenyl}tetrahydro-2H-pyran-4-yl)methanamine
1-[3-(4-{4-[(dimethylamino)methyl]tetrahydro-2H-pyran-4-yl}phenoxy)propyl]pyrrolidin-3-ol
1-[4-(4-{3-[(2R,6S)-2,6-dimethylmorpholin-4-yl]propoxy}phenyl)tetrahydro-2H-pyran-4-yl]-N,N-dimethylmethanamine
1-[(4-{4-[(3-pyrrolidin-1-ylpropyl)thio]phenyl}tetrahydro-2H-pyran-4-yl)carbonyl]piperidine
1-[3-({4-[4-(pyrrolidin-1-ylcarbonyl)tetrahydro-2H-pyran-4-yl]phenyl}thio)propyl]pyrrolidine
(4-{4-[(3-pyrrolidin-1-ylpropyl)thio]phenyl}tetrahydro-2H-pyran-4-yl)methanol
N-ethyl-N-({4-[4-(3-thiomorpholin-4-ylpropoxy)phenyl]tetrahydro-2H-pyran-4-yl}methyl)acetamide
N,N-dimethyl-1-[4-(4-{3-[(3R)-3-methylmorpholin-4-yl]propoxy}phenyl)tetrahydro-2H-pyran-4-yl]methanamine
N-[(4-{4-[(3-pyrrolidin-1-ylpropyl)thio]phenyl}tetrahydro-2H-pyran-4-yl)methyl]acetamide
N-[(4-{4-[(3-pyrrolidin-1-ylpropyl)thio]phenyl}tetrahydro-2H-pyran-4-yl)methyl]ethanamine
N-methyl-N-[(4-{4-[(3-pyrrolidin-1-ylpropyl)thio]phenyl}tetrahydro-2H-pyran-4-yl)methyl]ethanamine
N-ethyl-N-[(4-{4-[(3-pyrrolidin-1-ylpropyl)thio]phenyl}tetrahydro-2H-pyran-4-yl)methyl]acetamide
N-methyl-N-({4-[4-(3-thiomorpholin-4-ylpropoxy)phenyl]tetrahydro-2H-pyran-4-yl}methyl)ethanamine
N-ethyl-N-({4-[4-(3-thiomorpholin-4-ylpro poxy)phenyl]tetrahydro-2H-pyran-4-yl}methyl)ethanamine
4-(3-{4-[4-(pyrrolidin-1-ylcarbonyl)tetrahydro-2H-pyran-4-yl]phenoxy}propyl)thiomorpholine
N-ethyl-N-[(4-{4-[(3-pyrrolidin-1-ylpropyl)thio]phenyl}tetrahydro-2H-pyran-4-yl)methyl]ethanamine
N-({4-[4-(3-thiomorpholin-4-ylpropoxy)phenyl]tetrahydro-2H-pyran-4-yl}methyl)pyridin-2-amine
4-[(4-{4-[(1-isopropylpiperidin-4-yl)oxy]phenyl}tetrahydro-2H-pyran-4-yl)methoxy]pyridine
4-[3-(4-{1-[(4-methylpiperazin-1-yl)carbonyl]cyclohexyl}phenoxy)propyl]morpholine
4-(3-{4-[1-(piperazin-1-ylcarbonyl)cyclohexyl]phenoxy}propyl)morpholine
(4-{4-[(1-isopropylazetidin-3-yl)methoxy]phenyl}tetrahydro-2H-pyran-4-yl)methanol
4-[3-(4-{4-[(4-methylpiperazin-1-yl)carbonyl]tetrahydro-2H-pyran-4-yl}phenoxy)propyl]morpholine
4-(3-{4-[4-(piperazin-1-ylcarbonyl)tetrahydro-2H-pyran-4-yl]phenoxy}propyl)morpholine
4-[(4-{4-[(1-isopropylazetidin-3-yl)methoxy]phenyl}tetrahydro-2H-pyran-4-yl)methoxy]pyridine
4-[(4-{4-[(1-isopropylazetidin-3-yl)m ethoxy]phenyl}tetrahydro-2H-pyran-4-yl)carbonyl]morpholine
4-{4-[4-(1H-imidazol-1-ylmethyl)tetrahydro-2H-pyran-4-yl]phenoxy}-1-isopropylpiperidine
4-[4-(1-methyl-3-pyrrolidin-1-ylpropoxy)phenyl]tetrahydro-2H-pyran-4-carbonitrile
4-(4-{3-[(3R)-3-hydroxypyrrolidin-1-yl]propoxy}phenyl)tetrahydro-2H-pyran-4-carbonitrile
4-{4-[(1-ethylpiperidin-4-yl)oxy]phenyl}tetrahydro-2H-pyran-4-carbonitrile
4-{4-[(1-propylpiperidin-4-yl)oxy]phenyl}tetrahydro-2H-pyran-4-carbonitrile
(3R)-N,N-dimethyl-1-[3-(4-{4-[(methylamino)methyl]tetrahydro-2H-pyran-4-yl}phenoxy)propyl]pyrrolidin-3-amine
N-{[4-(4-{3-[(3R)-3-(dimethylamino)pyrrolidin-1-yl]propoxy}phenyl)tetrahydro-2H-pyran-4-yl]methyl}-N-methylmethanesulfonamide
(3R)-1-{3-[4-(4-{[(2-methoxyethyl)(methyl)amino]methyl}tetrahydro-2H-pyran-4-yl)phenoxy]propyl}-N,N-dimethylpyrrolidin-3-amine
N-{[4-(4-{3-[(3R)-3-(dimethylamino)pyrrolidin-1-yl]propoxy}phenyl)tetrahydro-2H-pyran-4-yl]methyl}-N,N',N'-trimethylurea
(3R)-N,N-dimethyl-1-(3-{4-[4-(1,3-thiazol-2-yl)tetrahydro-2H-pyran-4-yl]phenoxy}propyl)pyrrolidin-3-amine
(3R)-N,N-dimethyl-1-(3-{4-[4-(morpholin-4-ylcarbonyl)tetrahydro-2H-pyran-4-yl]phenoxy}propyl)pyrrolidin-3-amine
N-{[4-(4-{3-[(3R)-3-(dimethylamino)pyrrolidin-1-yl]propoxy}phenyl)tetrahydro-2H-pyran-4-yl]methyl}-N-methylpyrimidin-2-amine
(3R)-N,N-dimethyl-1-(3-{4-[4-(morpholin-4-ylmethyl)tetrahydro-2H-pyran-4-yl]phenoxy}propyl)pyrrolidin-3-amine
1-isopropyl-4-{4-[4-(piperidin-1-ylcarbonyl)tetrahydro-2H-pyran-4-yl]phenoxy}piperidine
4-[(4-{4-[(1-isopropylpiperidin-4-yl)oxy]phenyl}tetrahydro-2H-pyran-4-yl)carbonyl]morpholine
N-isopropyl-4-{4-[(1-isopropylpiperidin-4-yl)oxy]phenyl}-N-methyltetrahydro-2H-pyran-4-carboxamide
N*4*-{4-[4-(3-Pyrrolidin-1-yl-propoxy)-phenyl]-tetrahydro-pyran-4-ylmethyl}-pyridine-3,4-diamine
N*2*-{4-[4-(3-Pyrrolidin-1-yl-propoxy)-phenyl]-tetrahydro-pyran-4-ylmethyl}-pyridine-2,3-diamine

Particularly preferred compounds of formula (1) are as described in the Examples section hereafter. Where the salt form is obtained in the Examples, a compound of the present invention includes the free base thereof, for example:
{4-[4-(3-Pyrrolidin-1-ylpropoxy)phenyl]tetrahydropyran-4-yl} methylamine,
{4-[4-(3-Piperidin-1-ylpropoxy)phenyl]tetrahydropyran-4-yl} methylamine, and
Dimethyl-{4-[4-(3-piperidin-1-ylpropoxy)phenyl]tetrahydro-pyran-4-ylmethyl}amine.

In another embodiment of the invention, particularly preferred examples are:
{4-[4-(1-Isopropylpiperidin-4-yloxy)phenyl]tetrahydropyran-4-ylmethyl}dimethyl-amine
Dimethyl-(4-[4-(4-pyrrolidin-1-ylbutoxy)phenyl]tetrahydro-pyran-4-ylmethyl)amine
{4-[4-(3-Pyrrolidin-1-ylpropoxy)phenyl]tetrahydropyran-4-yl}methylamine
Dimethyl-{4-[4-(3-pyrrolidin-1-ylpropoxy)phenyl]-tetrahydropyran-4-ylmethyl}amine
{4-[4-(3-Piperidin-1-ylpropoxy)phenyl]tetrahydropyran-4-yl} methylamine
Dimethyl-(4-[4-(3-piperidin-1-ylpropoxy)phenyl]tetra-hydropyran-4-ylmethyl)amine
1-Methyl-4-{4-[4-(3-pyrrolidin-1-yl-propoxy)-phenyl]-tetrahydro-pyran-4-ylmethyl}-piperazine
(R)-2-Methoxymethyl-1-{4-[4-(3-pyrrolidin-1-yl-propoxy)-phenyl]-tetrahydro-pyran-4-ylmethyl}-pyrrolidine
(S)-2-Methoxymethyl-1-{4-[4-(3-pyrrolidin-1-ylpropoxy)-phenyl]tetrahydropyran-4-ylmethyl}pyrrolidine 1-{4-[4-(3-Pyrrolidin-1-ylpropoxy)phenyl]tetrahydropyran-4-ylmethyl}piperidine
methyl-{4-[4-(3-pyrrolidin-1-ylpropoxy)phenyl]-tetrahydropyran-4-ylmethyl}amine
isopropyl-methyl-{4-[4-(3-pyrrolidin-1-ylpropoxy)phenyl]-tetrahydropyran-4-ylmethyl}amine
1-{4-[4-(3-Pyrrolidin-1-ylpropoxy)phenyl]tetrahydropyran-4-ylmethyl}pyrrolidine
4-{4-[4-(3-Pyrrolidin-1-ylpropoxy)phenyl]tetrahydropyran-4-ylmethyl}-morpholine
(2-methoxyethyl)-methyl-{4-[4-(3-pyrrolidin-1-ylpropoxy)-phenyl]tetrahydropyran-4-ylmethyl}amine
4-[4-(1-Isopropylpiperidin-4-yloxy)phenyl]tetrahydropyran-4-carbonitrile
4-[4-(3-Pyrrolidin-1-ylpropoxy)phenyl]tetrahydro-2H-pyran-4-carboxylic acid amide
*N*-methyl-1-{1-[4-(3-pyrrolidin-1-ylpropoxy)phenyl]-cyclohexyl}methanamine
*N*-ethyl-*N*-({4-[4-(3-pyrrolidin-1-ylpropoxy)phenyl]tetrahydro-2*H*-pyran-4-yl}methyl)ethanamine
*N*-({4-[4-(3-pyrrolidin-1-ylpropoxy)phenyl]tetrahydro-2*H*-pyran-4-yl}methyl)ethanamine
*N*-methyl-*N*-({4-[4-(3-pyrrolidin-1-ylpropoxy)phenyl]-tetrahydro-2*H*-pyran-4-yl}methyl)ethanamine
4-{4-[(1-cyclobutyl-4-piperidinyl)oxy]phenyl}tetrahydro-2*H*-pyran-4-carbonitrile
4-{4-[(1-isopropylpiperidin-4-yl)oxy]phenyl}tetrahydro-2*H*-pyran-4-carboxamide
4-{4-[(1-isopropylpiperidin-4-yl)oxy]phenyl]-*N,N*-dimethyltetrahydro-2*H*-pyran-4-carboxamide
4-{4-[(1-isopropylpiperidin-4-yl)oxy]phenyl]-*N,N*-diethyltetra-hydro-2*H*-pyran-4-carboxamide
4-{4-[(1-isopropylpiperidin-4-yl)oxy]phenyl)tetrahydro-2*H*-pyran-4-yl]carbonyl}pyrrolidine
4-methyl-2-[4-(4-(3-pyrrolidin-1-ylpropoxy)phenyl)tetrahydro-2*H*-pyran-4-yl]-1,3-thiazole
2-[4-(4-(3-pyrrolidin-1-ylpropoxy)phenyl)tetrahydro-2*H*-pyran-4-yl]-1,3-thiazole
*N*-[(4-{4-[(1-isopropylpiperidin-4-yl)oxy]phenyl}tetrahydro-2*H*-pyran-4-yl)methyl]-*N*-methylamine
*N*-[(4-{4-[(1-isopropylpiperidin-4-yl)oxy]phenyl}tetrahydro-2*H*-pyran-4-yl)methyl]-*N*-ethylamine
*N*-{[4-(4-[(1-isopropylpiperidin-4-yl)oxy]phenyl)tetrahydro-2*H*-pyran-4-yl]methyl}pyrimidin-2-amine and
*N*-{[4-(4-(3-Pyrrolidin-1-ylpropoxy)phenyl)tetrahydro-2*H*-pyran-4-yl]methyl}pyrimidin-2-amine.
1-(4-{4-[(3-cyclopentylazetidin-3-yl)methoxy]phenyl}tetrahydro-2H-pyran-4-yl)-N,N-dimethylmethanamine
3-({4-[4-(3-pyrrolidin-1-ylpropoxy)phenyl]tetrahydro-2H-pyran-4-yl}methyl)-3H-imidazo[4,5-b]pyridine
2-{[methyl({4-[4-(3-pyrrolidin-1-ylpropoxy)phenyl]tetrahydro-2H-pyran-4-yl}methyl)amino]methyl}phenol
N-methyl-N-({4-[4-(3-pyrrolidin-1-ylpropoxy)phenyl]tetrahydro-2H-pyran-4-yl}methyl)cyclopentanamine
2-[methyl({4-[4-(3-pyrrolidin-1-ylpropoxy)phenyl]tetrahydro-2H-pyran-4-yl}methyl)amino]ethanol
N-methyl-N-({4-[4-(3-pyrrolidin-1-ylpropoxy)phenyl]tetrahydro-2H-pyran-4-yl}methyl)cyclopropanamine
1-(3-{4-[4-(aziridin-1-ylmethyl)tetrahydro-2H-pyran-4-yl]phenoxy}propyl)pyrrolidine
N-({4-[4-(3-thiomorpholin-4-ylpropoxy)phenyl]tetrahydro-2H-pyran-4-yl}methyl)ethanamine
1-(4-{4-[(1-cyclobutylpiperidin-4-yl)oxy]phenyl}tetrahydro-2H-pyran-4-yl)-N,N-dimethylmethanamine
N,N-dimethyl-1-(4-{4-[(3-pyrrolidin-1-ylpropyl)thio]phenyl}tetrahydro-2H-pyran-4-yl)methanamine
N-methyl-N-({4-[4-(3-pyrrolidin-1-ylpropoxy)phenyl]tetrahydro-2H-pyran-4-yl}methyl)cyclohexanamine
1-[3-({4-[4-(pyrrolidin-1-ylmethyl)tetrahydro-2H-pyran-4-yl]phenyl}thio)propyl]pyrrolidine
1-({1-[4-(3-pyrrolidin-1-ylpropoxy)phenyl]cyclohexyl}methyl)piperidin-4-ol
1-({4-[4-(3-pyrrolidin-1-ylpropoxy)phenyl]tetrahydro-2H-pyran-4-yl}methyl)-1H-benzimidazole
4-{4-[(1-cyclobutylpiperidin-4-yl)oxy]phenyl}tetrahydro-2H-pyran-4-carboxamide
N-methyl-N-({4-[4-(3-pyrrolidin-1-ylpropoxy)phenyl]tetrahydro-2H-pyran-4-yl}methyl) hexan-1-am ine
1-cyclopropyl-N-(cyclopropylmethyl)-N-({4-[4-(3-pyrrolidin-1-ylpropoxy)phenyl]tetrahydro-2H-pyran-4-yl}methyl)methanamine
N-[2-(dimethylamino)ethyl]-N-ethyl-4-[4-(3-pyrrolidin-1-ylpropoxy)phenyl]tetrahydro-2H-pyran-4-carboxamide
N-methyl-N-({4-[4-(3-pyrrolidin-1-ylpropoxy)phenyl]tetrahydro-2H-pyran-4-yl}methyl)butan-1-amine
4-{4-[4-(4,5-dimethyl-1H-imidazol-2-yl)tetrahydro-2H-pyran-4-yl]phenoxy}-1-isopropylpiperidine
3-{[methyl({4-[4-(3-pyrrolidin-1-ylpropoxy)phenyl]tetrahydro-2H-pyran-4-yl}methyl)amino]methyl}phenol
4-[(4-{4-[(1-cyclopentylazetidin-3-yl)methoxy]phenyl}tetrahydro-2H-pyran-4-yl)methyl]morpholine
N-methyl-N-({4-[4-(3-pyrrolidin-1-ylpropoxy)phenyl]tetrahydro-2H-pyran-4-yl}methyl)pentan-1-amine
4-{4-[(1-cyclobutylpiperidin-4-yl)oxy]phenyl}-N,N-dimethyltetrahydro-2H-pyran-4-carboxamide
N,N-dimethyl-1-(4-{4-[3-(1,4-oxazepan-4-yl)propoxy]phenyl}tetrahydro-2H-pyran-4-yl)methanamine
N,3,3-trimethyl-N-({4-[4-(3-pyrrolidin-1-ylpropoxy)phenyl]tetrahydro-2H-pyran-4-yl}methyl)butan-1-amine
4-{4-[(1-cyclobutylpiperidin-4-yl)oxy]phenyl}-N-isopropyltetrahydro-2H-pyran-4-carboxamide
1-methyl-4-({4-[4-(3-pyrrolidin-1-ylpropoxy)phenyl]tetrahydro-2H-pyran-4-yl}methyl)-1,4-diazepane
1-cyclobutyl-4-{4-[4-(1,3-thiazol-2-yl)tetrahydro-2H-pyran-4-yl]phenoxy}piperidine
N-[(4-{4-[(1-cyclobutylpiperidin-4-yl)oxy]phenyl}tetrahydro-2H-pyran-4-yl)methyl]pyridin-2-amine
N-methyl-1-{4-[4-(3-morpholin-4-ylpropoxy)phenyl]tetrahydro-2H-pyran-4-yl}methanamine
1-[(4-{4-[(1-isopropylpiperidin-4-yl)oxy]phenyl}tetrahydro-2H-pyran-4-yl)carbonyl]-4-methylpiperazine
1-cyclopentyl-N-methyl-N-({4-[4-(3-pyrrolidin-1-ylpropoxy)phenyl]tetrahydro-2H-pyran-4-yl}methyl)methanamine
4-{4-[(1-cyclobutylpiperidin-4-yl)oxy]phenyl}-N-ethyltetrahydro-2H-pyran-4-carboxamide
N-[(4-{4-[(1-cyclobutylpiperidin-4-yl)oxy]phenyl}tetrahydro-2H-pyran-4-yl)methyl]pyrimidin-2-amine
N,N-diethyl-4-[4-(3-pyrrolidin-1-ylpropoxy)phenyl]tetrahydro-2H-pyran-4-carboxamide
4-[(4-{4-[(1-cyclobutylazetidin-3-yl)methoxy]phenyl}tetrahydro-2H-pyran-4-yl)methyl]morpholine
4-{4-[(1-cyclobutylpiperidin-4-yl)oxy]phenyl}-N-methyltetrahydro-2H-pyran-4-carboxamide
1-cyclobutyl-4-{4-[4-(4-methyl-1,3-thiazol-2-yl)tetrahydro-2H-pyran-4-yl]phenoxy}piperidine
4-[(4-{4-[(1-isopropylazetidin-3-yl)methoxy]phenyl}tetrahydro-2H-pyran-4-yl)methyl]morpholine
1-isopropyl-4-{4-[4-(1,3-thiazol-2-yl)tetrahydro-2H-pyran-4-yl]phenoxy}piperidine
1-(4-{4-[(1-cyclobutylazetidin-3-yl)methoxy]phenyl}tetrahydro-2H-pyran-4-yl)methanamine
1-ethyl-4-({4-[4-(3-pyrrolidin-1-ylpropoxy)phenyl]tetrahydro-2H-pyran-4-yl}carbonyl)piperazine
N-ethyl-N-methyl-4-[4-(3-pyrrolidin-1-ylpropoxy)phenyl]tetrahydro-2H-pyran-4-carboxamide
1-(4-{4-[(1-isopropylazetidin-3-yl)methoxy]phenyl}tetrahydro-2H-pyran-4-yl)methanamine
1-[(4-{4-[(1-isopropylpiperidin-4-yl)oxy]phenyl}tetrahydro-2H-pyran-4-yl)methyl]-4-methylpiperazine
1-(cyclopropylmethyl)-4-({4-[4-(3-pyrrolidin-1-ylpropoxy)phenyl]tetrahydro-2H-pyran-4-yl}methyl)piperazine
N,N-dimethyl-1-{4-[4-(3-morpholin-4-ylpropoxy)phenyl]tetrahydro-2H-pyran-4-yl}methanamine
N-[2-(dimethylamino)ethyl]-N-methyl-4-[4-(3-pyrrolidin-1-ylpropoxy)phenyl]tetrahydro-2H-pyran-4-carboxamide
1-({4-[4-(3-pyrroiidin-1-ylpropoxy)phenyl]tetrahydro-2H-pyran-4-yl}carbonyl)piperazine
1-ethyl-4-({4-[4-(3-pyrrolidin-1-ylpropoxy)phenyl]tetrahydro-2H-pyran-4-yl}methyl)piperazine
4-[(4-{4-[(1-cyclobutylpiperidin-4-yl)oxy]phenyl}tetrahydro-2H-pyran-4-yl)carbonyl]morpholine
N-[(4-{4-[(1-cyclobutylpiperidin-4-yl)oxy]phenyl}tetrahydro-2H-pyran-4-yl)methyl]pyridin-3-amine
1-methyl-4-({1-[4-(3-pyrrolidin-1-ylpropoxy)phenyl]cyclohexyl}carbonyl)piperazine
1-({4-[4-(3-pyrrolidin-1-ylpropoxy)phenyl]tetrahydro-2H-pyran-4-yl}methyl)-1H-imidazole
N-[(4-{4-[(1-isopropylpiperidin-4-yl)oxy]phenyl}tetrahydro-2H-pyran-4-yl)methyl]pyridin-2-amine
1-isopropyl-4-({4-[4-(3-pyrrolidin-1-ylpropoxy)phenyl]tetrahydro-2H-pyran-4-yl}methyl)piperazine
1-({4-[4-(3-pyrrolidin-1-ylpropoxy)phenyl]tetrahydro-2H-pyran-4-yl}methyl)-1H-imidazo[4,5-c]pyridine
N-({4-[4-(3-piperidin-1-ylpropoxy)phenyl]tetrahydro-2H-pyran-4-yl}methyl)pyridin-2-amine
1-propyl-4-({4-[4-(3-pyrrolidin-1-ylpropoxy)phenyl]tetrahydro-2H-pyran-4-yl}carbonyl)piperazine
N-({4-[4-(3-pyrrolidin-1-ylpropoxy)phenyl]tetrahydro-2H-pyran-4-yl}methyl)pyridazin-4-amine
N-ethyl-4-{4-[(1-isopropylpiperidin-4-yl)oxy]phenyl}-N-methyltetrahydro-2H-pyran-4-carboxamide
1-methyl-4-({4-[4-(3-pyrrolidin-1-ylpropoxy)phenyl]tetrahydro-2H-pyran-4-yl}carbonyl)piperazine
1-propyl-4-({4-[4-(3-pyrrolidin-1-ylpropoxy)phenyl]tetrahydro-2H-pyran-4-yl}methyl)piperazine
N-({4-[4-(3-pyrrolidin-1-ylpropoxy)phenyl]tetrahydro-2H-pyran-4-yl}methyl)pyridin-2-amine
4-{4-[4-(azetidin-1-ylcarbonyl)tetrahydro-2H-pyran-4-yl]phenoxy}-1-isopropylpiperidine
1-(2-methoxyethyl)-4-({4-[4-(3-pyrrolidin-1-ylpropoxy)phenyl]tetrahydro-2H-pyran-4-yl}methyl)piperazine
2-{4-[4-(3-pyrrolidin-1-ylpropoxy)phenyl]tetrahydro-2H-pyran-4-yl}pyridine
N-[(4-{4-[(1-isopropylpiperidin-4-yl)oxy]phenyl}tetrahydro-2H-pyran-4-yl)methyl]pyridazin-4-amine
N-[(4-{4-[(1-isopropylazetidin-3-yl)methoxy]phenyl}tetrahydro-2H-pyran-4-yl)methyl]pyridin-2-amine
4-[(4-{4-[(1-isopropylpiperidin-4-yl)oxy]phenyl}tetrahydro-2H-pyran-4-yl)methyl]morpholine
1-methyl-4-({4-[4-(3-pyrrolidin-1-ylpropoxy)phenyl]tetrahydro-2H-pyran-4-yl}carbonyl)-1,4-diazepane
4-(3-{4-[4-(morpholin-4-ylmethyl)tetrahydro-2H-pyran-4-yl]phenoxy}propyl)-1,4-oxazepane
1-({4-[4-(3-pyrrolidin-1-ylpropoxy)phenyl]tetrahydro-2H-pyran-4-yl}carbonyl)piperidine
1-methyl-4-[4-(3-pyrrolidin-1-ylpropoxy)phenyl]piperidine-4-carbonitrile
1-(3-{4-[4-(pyrrolidin-1-ylcarbonyl)tetrahydro-2H-pyran-4-yl]phenoxy}propyl)pyrrolidine
2-(methylthio)-1-({4-[4-(3-pyrrolidin-1-ylpropoxy)phenyl]tetrahydro-2H-pyran-4-yl}methyl)-1H-imidazole
4-[4-(3-pyrrolidin-1-ylpropoxy)phenyl]piperidine-4-carbonitrile
N-[(4-{4-[(1-isopropylpiperidin-4-yl)oxy]phenyl}tetrahydro-2H-pyran-4-yl)methyl]pyridin-3-amine
6-methyl-N-({4-[4-(3-pyrrolidin-1-ylpropoxy)phenyl]tetrahydro-2H-pyran-4-yl}methyl)pyridin-3-amine
N-[(4-{4-[(1-ethylpiperidin-4-yl)oxy]phenyl}tetrahydro-2H-pyran-4-yl)methyl]pyridin-2-amine
N,N-dimethyl-4-[4-(3-pyrrolidin-1-ylpropoxy)phenyl]tetrahydro-2H-pyran-4-carboxamide
4-({4-[4-(3-pyrrolidin-1-ylpropoxy)phenyl]tetrahydro-2H-pyran-4-yl}methyl)-4H-1,2,4-triazole
1-({1-[4-(3-pyrrolidin-1-ylpropoxy)phenyl]cyclohexyl}methyl)piperazine
1-isopropyl-4-[4-(3-pyrrolidin-1-ylpropoxy)phenyl]piperidine-4-carbonitrile
5-{4-[4-(3-pyrrolidin-1-ylpropoxy)phenyl]tetrahydro-2H-pyran-4-yl}-1,3-oxazole
1-acetyl-4-({1-[4-(3-pyrrolidin-1-ylpropoxy)phenyl]cyclohexyl}methyl)piperazine
4-({4-[4-(3-pyrrolidin-1-ylpropoxy)phenyl]tetrahydro-2H-pyran-4-yl}methoxy)pyridine
1-acetyl-4-({4-[4-(3-pyrrolidin-1-ylpropoxy)phenyl]tetrahydro-2H-pyran-4-yl}methyl)piperazine
N-({4-[4-(4-pyrrolidin-1-ylbutoxy)phenyl]tetrahydro-2H-pyran-4-yl}methyl)pyridin-2-amine
1-[3-(4-{4-[(dimethylamino)methyl]tetrahydro-2H-pyran-4-yl}phenoxy)propyl]-N,N-dimethylazetidin-3-amine
1-(3-{4-[4-(azetidin-1-ylcarbonyl)tetrahydro-2H-pyran-4-yl]phenoxy}propyl)pyrrolidine
N-[(4-{4-[(1-cyclobutylazetidin-3-yl)methoxy]phenyl}tetrahydro-2H-pyran-4-yl)methyl]pyridin-2-amine
N-{[4-(4-{3-[cyclobutyl(methyl)amino]propoxy}phenyl)tetrahydro-2H-pyran-4-yl]methyl}pyridin-2-amine
N-({4-[4-(3-pyrrolidin-1-ylpropoxy)phenyl]tetrahydro-2H-pyran-4-yl}methyl)pyridin-3-amine
4-(3-{4-[4-(morpholin-4-ylmethyl)tetrahydro-2H-pyran-4-yl]phenoxy}propyl)morpholine
N,N-dimethyl-1-(4-{4-[3-(4-methyl-1,4-diazepan-1-yl)propoxy]phenyl}tetrahydro-2H-pyran-4-yl)methanamine
1-({4-[4-(3-pyrrolidin-1-ylpropoxy)phenyl]tetrahydro-2H-pyran-4-yl}methyl)-1H-imidazo[4,5-b]pyridine
4-({4-[4-(3-azetidin-1-ylpropoxy)phenyl]tetrahydro-2H-pyran-4-yl}methyl)morpholine
2-{4-[4-(3-pyrrolidin-1-ylpropoxy)phenyl]tetrahydro-2H-pyran-4-yl}-1H-imidazole
4-(3-{4-[4-(piperazin-1-ylcarbonyl)tetrahydro-2H-pyran-4-yl]phenoxy}propyl)-1,4-oxazepane
4-[3-(4-{4-[(4-methylpiperazin-1-yl)carbonyl]tetrahydro-2H-pyran-4-yl}phenoxy)propyl]-1,4-oxazepane
4-{4-[4-(3-pyrrolidin-1-ylpropoxy)phenyl]tetrahydro-2H-pyran-4-yl}pyrimidine
4-methyl-1-({4-[4-(3-pyrrolidin-1-ylpropoxy)phenyl]tetrahydro-2H-pyran-4-yl}methyl)-1H-imidazole
4-{4-[4-(3-pyrrolidin-1-ylpropoxy)phenyl]tetrahydro-2H-pyran-4-yl}-1H-imidazole
4-[4-(4-{3-[ethyl(methyl)amino]propoxy}phenyl)tetrahydro-2H-pyran-4-ylmethyl]-morpholine
Another embodiment of the invention is an intermediate as described herein.

Pharmaceutically acceptable salts of the compounds of formula (1) include the acid addition and base salts thereof.

Suitable acid addition salts are formed from acids which form non-toxic salts. Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (1) may be prepared by one or more of three methods:
(i) by reacting the compound of formula (1) with the desired acid or base;
(ii) by removing an acid- or base-labile protecting group from a suitable precursor of the compound of formula (1) or by ring-opening a suitable cyclic precursor, for example, a lactone or lactam, using the desired acid or base; or
(iii) by converting one salt of the compound of formula (1) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in both unsolvated and solvated forms. The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionised, partially ionised, or non-ionised. For a review of such complexes, see J Pharm Sci, 64 (8), 1269-1288 by Haleblian (August 1975).

Hereinafter all references to compounds of formula (1) include references to salts, solvates and complexes thereof and to solvate and complexes of salts thereof.

The compounds of the invention may also exist as polymorphs and crystal habits thereof, prodrugs and isomers (including optical, geometric and tautomeric isomers) as hereinafter defined and isotopically-labeled compounds of formula (1). Certain compounds of formula (1) may themselves act as prodrugs of other compounds of formula (1).

Metabolites of compounds of formula (1), may also be formed *in vivo* upon administration of the drug. Some examples of metabolites include :
(i) where the compound of formula (1) contains a methyl group, an hydroxymethyl derivative thereof (-CH₃ → -CH₂OH):
(ii) where the compound of formula (1) contains an alkoxy group, an hydroxy derivative thereof (-OR → -OH);
(iii) where the compound of formula (1) contains a tertiary amino group, a secondary amino derivative thereof (-NR^{a}R^{b} → -NHR^{a} or -NHR^{b});
(iv) where the compound of formula (1) contains a secondary amino group, a primary derivative thereof (-NHR⁸ → -NH₂);
(v) where the compound of formula (1) contains a phenyl moiety, a phenol derivative thereof (-Ph → - PhOH); and
(vi) where the compound of formula (1) contains an amide group, a carboxylic acid derivative thereof (-CONR^{c}R^{d} → COOH).

Compounds of formula (1) containing one or more asymmetric carbon atoms_can exist as two or more stereoisomers. Where structural isomers are interconvertible *via* a low energy barrier, tautomeric isomerism ('tautomerism') can occur. This can take the form of proton tautomerism in compounds of formula (1) containing, for example, an imino, keto, or oxime group, or so-called valence tautomerism In compounds which contain an aromatic moiety. It follows that a single compound may exhibit more than one type of isomerism.

The compounds of the present invention may also exist as stereoisomers, geometric isomers and tautomeric forms and may exhibit more than one type of isomerism, and mixtures of one or more thereof. Acid addition or base salts wherein the counterion is optically active, for example, *d*-lactate or *I*-lysine, or racemic, for example, *di*-tartrate or *di-*arginine may also be formed.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (1) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

Stereoisomeric conglomerates may be separated by conventional techniques known to those skilled in the art - see, for example, "Stereochemistry of Organic Compounds" by E. L. Eliel (Wiley, New York, 1994).

The compounds of the present invention may also exist as pharmaceutically acceptable isotopically-labelled compounds of formula (1) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C,¹³C and ¹⁴C, chlorine, such as ³⁶Cl, fluorine, such as ¹⁸F, iodine, such as ¹²³I and ¹²⁵I, nitrogen, such as ¹³N and ¹⁶N, oxygen, such as ¹⁵O, ¹⁷O and ¹⁸O, phosphorus, such as ³²P, and sulphur, such as ³⁵S.

Certain isotopically-labelled compounds, for example those incorporating a radioactive isotope, are useful in drug and/or substrate tissue distribution studies. The radioactive isotopes tritium, *i.e*. ³H, and carbon-14, *i.e*. ¹⁴C, are particularly useful for this purpose in view of their ease of incorporation and ready means of detection.

Substitution with heavier isotopes such as deuterium, *i.e*. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Position Emission Topography (PET) studies for examining substrate receptor occupancy.

Isotopically-labeled compounds can generally be prepared by conventional techniques known to those skilled in the art or by processes analogous to those described in the accompanying Examples and Preparations using an appropriate isotopically-labeled reagents in place of the non-labeled reagent previously employed.

Pharmaceutically acceptable solvates in accordance with the invention include those wherein the solvent of crystallization may be isotopically substituted, e.g. D₂O, d₆-acetone, d₆-DMSO.

The phenol and thiophenol derivatives of formula (1) can be prepared using conventional procedures such as by the following illustrative methods in which R, X, Y, Z, m, p, R¹, R², R³, R⁴, R⁵, R⁶, R⁷, R⁸ and R⁹ are as previously defined unless otherwise stated.

The compounds of formula (1), wherein X is -CH₂NR¹R², may be prepared from compounds of formula (2) 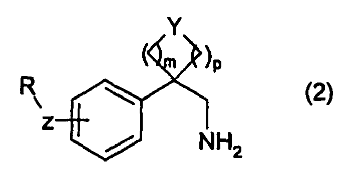 using standard techniques for functional group interconversion known to those skilled in the art, for example as described in Comprehensive Organic Transformations, R.C. Larock, 1st Edition, 1989 VCH Publishers Inc.

These techniques include:
1) Reductive alkylation with aldehydes or ketones with a reducing agent (e.g. formic acid or NaHB(OAc)₃) in a suitable solvent (e.g. dichloromethane, tetrahydrofuran), optionally with the addition of acetic acid, at between room temperature (about 20°C) and reflux;
2) Alkylations with alkyl halides and a base (e.g. potassium carbonate, potassium *tert*-butoxide) in a suitable solvent (e.g. acetonitrile, tetrahydrofuran) at between room temperature (about 20°C) and reflux;
3) Sulphonylation with a sulphonyl chloride and a base (e.g. triethylamine, N-ethyldiisopropylamine) in a suitable solvent (e.g. dichloromethane) at between room temperature and reflux;
4) Heteroarylation with a haloheteroaromatic or heteroaromatic sulphonic acid compound and a base (e.g. potassium carbonate) in a suitable solvent (e.g. acetonitrile) at between room temperature (about 20°C) and reflux. Alternatively this reaction may be performed under microwave radiation, alternatively using N-ethyldiisopropylamine as base, optionally in a suitable solvent (e.g.N-methyl pyrrolidinone, acetonitrile).
5) Urea formation with a dialkylcarbamoyl chloride or a cyanate salt, optionally in the presence of a base (e.g. triethylamine) or an acid (e.g. acetic acid) in a suitable solvent (e.g. dichloromethane or water) at between room temperature and reflux;
6) Sulphonyl urea formation with a dialkylsulphamoyl chloride and a base (e.g. triethylamine) in a suitable solvent (e.g. dichloromethane) at between room temperature and reflux;
7) Acylation with an activated acid (e.g. an acyl chloride or anhydride) and a base (e.g. triethylamine) in a suitable solvent (e.g. dichloromethane) at between room temperature and reflux.
8) By palladium catalysed cross-coupling with a haloheteroaromatic compound, using a suitable palladium source (e.g. tris(dibenzylideneacetone)palladium (0)), optionally in the presence of a chelating ligand (e.g. BINAP) and in the presence of a suitable base (e.g. sodium *tert*-butoxide) in a solvent (e.g. toluene), at between room temperature and reflux.

These transformations can be performed sequentially to prepare compounds in which R¹ and R² are as here above defined.

The compounds of formula (2) may be prepared by reduction of the corresponding cyano derivatives of formula (3) : 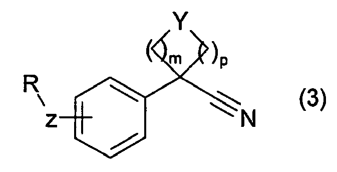 with a suitable reducing agent (e.g. LiAlH₄ or hydrogen gas in the presence of a catalyst such as PtO₂) in a suitable solvent (e.g. Et₂O/dichloromethane, tetrahydrofuran or isopropanol) at between room temperature and reflux.

The compounds of formula (3) above correspond actually to the compounds of formula (1) wherein X is a CN group.

The compounds of formula (3) wherein Z is O may be prepared by alkylation of the corresponding hydroxy derivatives of formula (4) : 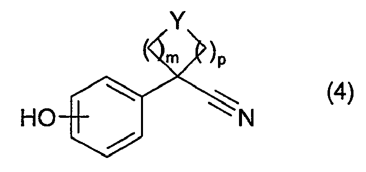 with a derivative of formula R-R^{LG} wherein R^{LG} is a leaving group such as halo, mesylate or tosylate, in the presence of a base (e.g. potassium carbonate) and optionally in the presence of an additive (e.g. potassium iodide) in a suitable solvent (e.g. dimethylformamide) at between room temperature and reflux.

When R is a group of formula: 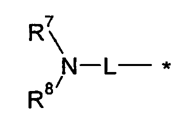 as previously defined, then the derivative of formula R-R^{LG} may be prepared by alkylation of the corresponding amino derivative of formula NHR⁷R⁸ with a derivative of formula R^{LG}-L-R^{LG} and a base (e.g. NaOH, Na₂CO₃, K₂CO₃, Cs₂C0₃), optionally in the presence of an additive (e.g. potassium iodide) in a suitable solvent (e.g. N,N-dimethylformamide, acetonitrile, acetone/H₂O) at between room temperature and reflux. The amino derivative of formula NHR⁷R^{B} is either commercial or made using procedures known to the skilled person.

When R is a group of formula : 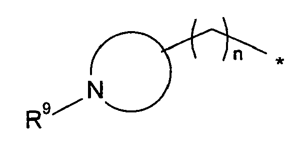 as previously defined, then the derivative of formula R-R^{LG} is either commercial or may be prepared using literature procedures well-known to the skilled person.

The compounds of formula (4) may be prepared by deprotection of the corresponding derivatives of formula (5) wherein Z is O: 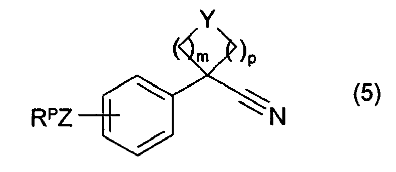 wherein R^{P} is a protecting group (e.g. methyl, deprotected with BBr₃ in dichloromethane at between 0°C and room temperature).

The compounds of formula (5) are either commercial or may be prepared by double alkylation of the compounds of formula (6) : 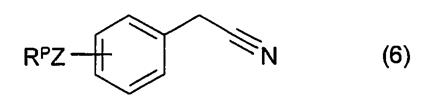 wherein R^{P} is as previously defined,
with the compounds of formula (7) : 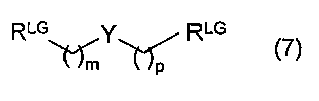 wherein R^{LG} is as previously defined, and a base (e.g. NaH), optionally in the presence of an additive (e.g. potassium iodide) in a suitable solvent (e.g. N,N-dimethylformamide, N-methyl pyrrolidinone) at between room temperature and reflux.

The compounds of formula (6) and (7) are each either commercially available or made using literature procedures well-known to the skilled person.

Compounds of formula (3), wherein Z represents S may be prepared from compounds of formula (5) wherein Z represents S and R^{P} is Me by a Pummerer rearrangement, followed by alkylation of the resulting intermediate with a derivative of formula R-R^{LG}. The reaction is achieved by treatment of the compound of formula (5) with a suitable oxidant (e.g.m-CPBA) in a suitable solvent (e.g. dichloromethane) at 0°C to provide the corresponding sulphide. Treatment of this intermediate with trifluoroacetic anhydride in the presence of a suitable base (e.g. 2,6-lutidine) in a suitable solvent (e.g. acetonitrile) at about -15°C, followed by reaction with R-R^{LG}, in the presence of a base (e.g. triethylamine, potassium carbonate) in a suitable solvent (e.g. N,N-dimethylformamide) at between 0°C and room temperature provides the compound of formula (3).

Alternatively, the compounds of formula (3) wherein Z is O may be prepared by alkylation of the compounds of formula (4) with the alcohol derivative of formula ROH, which is either commercial or made using literature procedures well-known to the skilled person, using Mitsunobu reagents such as PPh₃ and DIAD in a suitable solvent (e.g. THF) at between 0°C and reflux.

Persons skilled in the art will appreciate that, in order to obtain compounds of formula (1) in an alternative or more convenient manner, the individual process steps mentioned in this section may be performed in a different order. For example compounds of formula (1) wherein Z is O and X is -CH₂NR¹R², may be prepared by alkylation of the compounds of formula (8) : 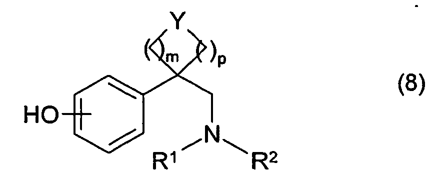 with a derivative of formula R-R^{LG} or ROH as previously defined, using conditions analogous to those decribed for the preparation of compounds of formula (3).

The compounds of formula (8) may be prepared by deprotection of the compounds of formula (9) : 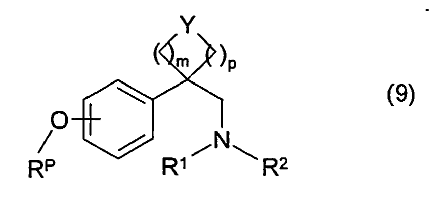 wherein R^{P} is as previously defined,
using conditions appropriate for the protecting group and the nature of R¹ and R² (e.g. using NaSMe in dimethylformamide at 130°C wherein R^{P} is methyl and R¹ and R² are (C₁-C₄)alkyl).

The compounds of formula (9) may be prepared from the corresponding amino derivatives of formula (10) 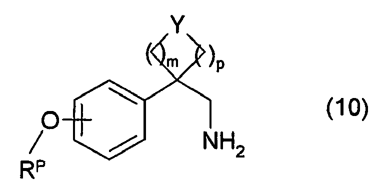 using analogous conditions to those described for the preparation of compounds of formula (1) from compounds of formula (2).

The compounds of formula (10) may be prepared from compounds of formula (5) wherein Z is O using analogous reduction conditions to those described for the preparation of compounds of formula (2) from compounds of formula (3).

A further example of performing the individual process steps described in this section in a different order to obtain compounds of formula (1) in an alternative or more convenient manner, is the preparation of compounds of formula (1) wherein Z is O by double alkylation of the compounds of formula (11) : 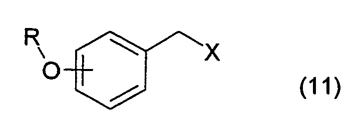 with the compounds of formula (7) as previously defined, using analogous conditions described for the preparation of compounds of formula (5) from compounds of formula (6).

The compounds of formula (11) may be prepared from the corresponding hydroxy derivatives of formula (12) : 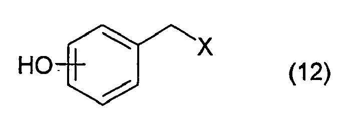 using analogous conditions described for the preparation of compounds of formula (3) from compounds of formula (4).

The compounds of formula (12) are either commercial or made using literature procedures well-known to the skilled person.

An example of performing the individual process steps described in this section in a different order to obtain compounds of formula (1) wherein Z is O and R is a group of formula : 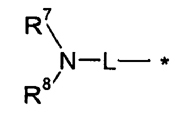 is the preparation of these compounds by reaction of the compounds of formula (13) : 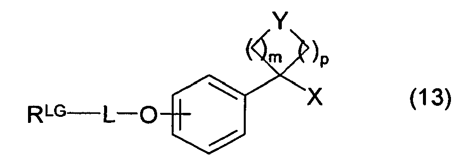 wherein R^{LG} is as previously defined, with the amino derivatives of formula NHR⁷R⁸ using analogous conditions to those previously described for the preparation of R-R^{LG}.

Alternatively, the same transformation can be achieved by heating the compounds of formula (13) with the amino derivatives of formula NHR⁷R⁸ and a base (e.g. diisopropylethylamine) in a suitable solvent (e.g. N-methylpyrrolidinone) at 150-200°C for 5-10 min using a microwave oven.

Alternatively, the compounds of formula (1) wherein Z is O, X is -CH₂NR¹R² and R¹ and R² are hydrogen or an optionally substituted (C₁-C₄)alkyl or together with the N atom to which they are attached form an optionally substituted 4-, 5- or 6-membered saturated heterocycle wherein a C atom may be replaced by N, O, S, SO or SO₂, may be prepared by reductive amination of the compounds compounds of formula (14) : 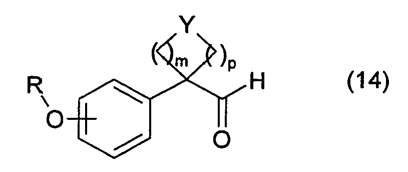 with the amino derivative of formula HNR¹R² and a reducing agent (e.g. NaHB(OAc)₃), optionally in the presence of a Lewis acid (e.g. Ti(OⁱPr)₄) in a suitable solvent (e.g. EtOH) at between 0°C and reflux.

The compounds of formula (14) may be prepared by reduction of the compounds of formula (3) wherein Z is O with a reducing agent (e.g. diisobutylaluminium hydride) in a suitable solvent (e.g. toluene) at between -78°C and room temperature (about 20°C).

Alternatively, the compounds of formula (14) may be prepared by oxidation of the compounds of formula (1) wherein Z is O and X is -CH₂OH, using an oxidant (e.g. pyridinium chlorochromate) in a suitable solvent (e.g. dichloromethane) at between 0°C and reflux.

The compounds of formula (1) wherein X is -CH₂OH, may be prepared by reduction of the compounds of formula (1) wherein X is -C(O)O(C₁-C₄)alkyl, with a reducing agent (e.g. LiAlH₄) in a suitable solvent (e.g. tetrahydrofuran) at between -78°C and reflux.

The compounds of formula (1) wherein X is -C(O)O(C₁-C₄)alkyl, may be prepared by esterification of the compounds of formula (1) wherein X is COOH, using the standard procedures well-known to the skilled person. For example, the esterification can be achieved by using a reagent capable of activating a carboxylic acid (e.g. thionyl chloride) and HO(C₁-C₄)alkyl, optionally in the presence of an additional solvent (e.g. dichloromethane) at between 0°C and reflux.

The compounds of formula (1) wherein X is C(O)OH, may be prepared by hydrolysis of the compounds of formula (3) as previously defined with a mineral acid (e.g. concentrated aqueous HCl), optionally in the presence of a suitable co-solvent (e.g. dioxane), at between 0°C and reflux.

The compounds of formula (1) wherein m and p are both equal to 2, Z is O and Y is CH(OH), may be prepared by reduction of the compounds of formula (1) wherein m and p are both equal to 2 and Y is C=O, with a reducing agent (e.g. LiAlH₄) in a suitable solvent (e.g. Et₂O) at between 0°C and reflux.

The compounds of formula (1) wherein m and p are both equal to 2, Z is O and Y is C=O, may be prepared by treatment of the compounds of formula (15) : 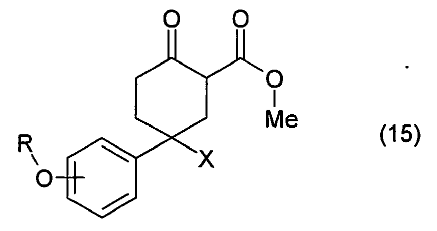 with sodium chloride in a suitable solvent (e.g. dimethylsulphoxide/water) at between 100°C and reflux.

The compounds of formula (15) may be prepared by treatment of the compounds of formula (16) : 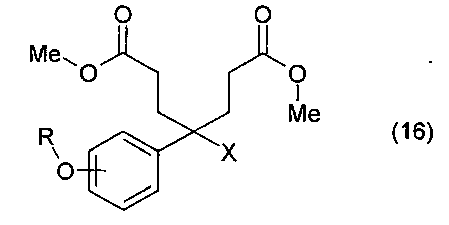 with a base (e.g. NaH) in a suitable solvent (e.g. 1,2-dimethoxyethane) at between room temperature and reflux.

The compounds of formula (16) may be prepared by alkylation of the compounds of formula (11) as previously defined with methyl acrylate and a base (e.g. benzyltrimethylammonium hydroxide) in a suitable solvent (e.g. methanol/acetonitrile) at between room temperature and reflux.

The compounds of formula (1) wherein X is -CONH₂, may be prepared by treatment of the compounds of formula (3) as previously defined with polyphosphoric acid or boron trifluoride-acetic acid complex at between room temperature and 100°C.

The compounds of formula (1) wherein X is -CONR³R⁴, may be prepared by reaction of the compounds of formula (1) wherein X is COOH, sequentially with a reagent capable of activating a carboxylic acid (e.g. thionyl chloride,) and then with the amino derivative of formula HNR³R⁴, in a suitable solvent (e.g. dichloromethane) at between 0°C and reflux. Alternatively the acid may be treated with the amine of formula HNR³R⁴, in the presence of a coupling agent (e.g. TBTU, O-benzotriazol-1-yl-N,N,N',N'-tetramethyluronium hexafluorophosphate, 1-(3-dimethylaminopropyl)-3-ethylcarbodiimide hydrochloride), optionally in the presence of a suitable additive (e.g. 1-hydroxybenzotriazole) and base (e.g. triethylamine) in a suitable solvent (e.g. dichloromethane, N,N-dimethylformamide) at room temperature.

Compounds of formula (1) wherein X is CH²NR¹R² may be prepared by reduction of compounds of formula (1) wherein X is -CONR³R⁴ and NR¹R² equals NR³R⁴, with a reducing agent (e.g. lithium aluminium hydride) in a suitable solvent (e.g. tetrahydrofuran, ether) at between room temperature and 50°C.

The compounds of formula (1) wherein X is -CH₂-O-(C₁-C₄)alkyl may be prepared by alkylation of the compounds of formula (1) wherein X is -CH₂-OH, with a derivative of formula R^{LG}(C₁-C₄)alkyl, wherein R^{LG} is as previously defined, and a base (e.g. NaH) in a suitable solvent (e.g. dimethylformamide) at between room temperature and reflux.

The compounds of formula (1) wherein X is -CH₂-O-het² may be prepared by alkylation of the compounds of formula (1) wherein X is -CH₂-OH, with a derivative of formula R^{LG}Het², by palladium catalysed cross-coupling with a haloheteroaromatic compound, using a suitable palladium source (e.g. tris(dibenzylideneacetone)palladium (0)), optionally in the presence of a chelating ligand (e.g. BINAP) and in the presence of a suitable base (e.g. sodium tert-butoxide) in a solvent (e.g. toluene), at between room temperature and reflux.

Compounds of formula (1) wherein X is het¹, may be prepared from compounds of formula (1) wherein X is COOH, or alternatively from compounds of formula (3) or (14) using methods known to those skilled in the art, such as those described in general heterocyclic texts such as Heterocyclic Chemistry, J.A. Joule and K. Mills, 4th Edition, Blackwell publishing, 2000 or Comprehensive Heterocyclic chemistry I and II, Pergamon Press.

For example, compounds of formula (1) where X is optionally substituted 2-thiazolyl and Z is O may be prepared by treating compounds of formula (17) : 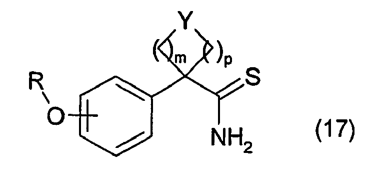 with a suitable reagent (e.g. bromoacetaldehyde dimethyl acetal, chloroacetone) and HCl in a suitable solvent (e.g. EtOH) at reflux.

Compounds of formula (17) may be prepared by reacting compounds of formula (3) wherein Z is O with diethyldithiophosphate and water at 60°C.

Compounds of formula (1) wherein X is -CH₂-het¹, and where said het¹ is N-linked, may be prepared from compounds of formula (1) wherein X is -CH₂-NH₂ using methods known to those skilled in the art, such as those described in general heterocyclic texts such as Heterocyclic Chemistry, J.A. Joule and K. Mills, 4th Edition, Blackwell publishing, 2000 or Comprehensive Heterocyclic chemistry I and II, Pergamon Press.

Compounds of formula (1) wherein X is -CH₂-het¹, and where said het' is C-linked, may be prepared from compounds of formula (1) wherein X is -COOH by a two step process of CH₂ homologation using the Arndt-Eistert synthesis (Advanced Organic Chemistry, J. March, 4th edition, Wiley-Interscience publication, 1992) followed by standard techniques for heterocycle construction from a carboxylic acid known to those skilled in the art.

The compounds of formula (1) and their precursors which contain a sulphide group can be oxidised to the corresponding sulphoxides or sulphones using standard techniques well-known to those skilled in the art, for example as described in Comprehensive Organic Transformations, R.C. Larock, 1st Edition, 1989 VCH Publishers Inc.

It will be appreciated by those skilled in the art that, certain compounds of formula (1) may be converted to alternative compounds of formula (1) using standard chemical transformations. Examples of these include, acylation and sulphonation of amine functions (e.g see examples 166, 229, 230), reductive amination reactions (e.g. see examples 167, 168), alkylation (e.g. see example 228) or hydrogenation of halo atoms (e.g. see examples 202, 203).

It will be appreciated by those skilled in the art that, in the course of carrying out the processes described above, the functional groups of intermediate compounds may need to be protected by protecting groups.

These functional groups include hydroxyl, amino and carboxylic acid. Suitable protecting groups for hydroxyl include trialkylsilyl and diarylalkylsilyl (e.g. tert-butyldimethylsilyl, tert-butyldiphenylsilyl or trimethylsilyl), alkyl (e.g. methyl or methoxyethyl) and tetrahydropyranyl. Suitable protecting groups for amino include tert-butyloxycarbonyl, 9-fluorenylmethoxycarbonyl or benzyloxycarbonyl. Suitable [protecting groups for carboxylic acid include (C₁-C₄)alkyl or benzyl esters.

The protection and deprotection of functional groups may take place before or after any of the reaction steps described hereinbefore.

The introduction and removal of protecting groups is fully described in Protective Groups in Organic Chemistry, edited by J.W.F. McOmie, Plenum Press (1973), Protective Groups in Organic Synthesis, 2nd edition, T.W. Greene & P.G.M. Wutz, Wiley-Interscience Publication, 1991) and Protecting Groups, P.J. Kocienski, Thieme, 1994.

Also, the compounds of formula (1) as well as intermediates for the preparation thereof, can be purified according to various well-known methods such as recrystallisation and chromatography.

The compounds of formula (1), their pharmaceutically acceptable salts and/or derived forms, are valuable pharmaceutically active compounds, which are suitable for the therapy and prophylaxis of numerous disorders in which the histamine H₃ receptor is involved or in which agonism or antagonism of this receptor may induce benefit.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

According to another aspect of the invention, there is provided a pharmaceutical composition including a compound of the formula (1) or a pharmaceutically acceptable salt and/or solvate thereof, as defined in any one of the preceding claims, together with a pharmaceutically acceptable excipient.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995).

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi- and nano-particulates, gels, solid solution, liposome, films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula (1), a film-forming polymer, a binder, a solvent, a humectant, a plasticiser, a stabiliser or emulsifier, a viscosity-modifying agent and a solvent. Some components of the formulation may perform more than one function.

The compound of formula (1) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula (1) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (1) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(*d*/-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (*e.g*. Powderject^{™}, Bioject^{™}, *etc*.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *I*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (1), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 1 µg to 4000 µg of the compound of formula (1). The overall daily dose will typically be in the range 1 µg to 20 mg which may be administered in a single dose or, more usually, as divided doses throughout the day.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

In as much as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (1) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the 0.001 mg to 2000 mg depending, of course, on the mode of administration. For example, oral administration may require a total daily dose of from 1 mg to 2000 mg, while an intravenous dose may only require from 0.01 mg to 100 mg. The total daily dose may be administered in single or divided doses and may, at the physician's discretion, fall outside of the typical range given herein.

These dosages are based on an average human subject having a weight of about 60kg to 70kg. The physician will readily be able to determine doses for subjects whose weight falls outside this range, such as infants and the elderly.

For the avoidance of doubt, references herein to "treatment" include references to curative, palliative and prophylactic treatment.

According to another embodiment of the present invention, the compounds of the formula (1), or pharmaceutically acceptable salts, derived forms or compositions thereof, can also be used as a combination with one or more additional therapeutic agents to be co-administered to a patient to obtain some particularly desired therapeutic end result. The second and more additional therapeutic agents may also be a compound of the formula (1), or a pharmaceutically acceptable salt, derived forms or compositions thereof, or one or more histamine H₃ receptor ligands known in the art. More typically, the second and more therapeutic agents will be selected from a different class of therapeutic agents.

As used herein, the terms "co-administration", "co-administered" and "in combination with", referring to the compounds of formula (1) and one or more other therapeutic agents, is intended to mean, and does refer to and include the following:
- simultaneous administration of such combination of compound(s) of formula (1) and therapeutic agent(s) to a patient in need of treatment, when such components are formulated together into a single dosage form which releases said components at substantially the same time to said patient,
- substantially simultaneous administration of such combination of compound(s) of formula (1) and therapeutic agent(s) to a patient in need of treatment, when such components are formulated apart from each other into separate dosage forms which are taken at substantially the same time by said patient, whereupon said components are released at substantially the same time to said patient,
- sequential administration of such combination compound(s) of formula (1) and therapeutic agent(s) to a patient in need of treatment, when such components are formulated apart from each other into separate dosage forms which are taken at consecutive times by said patient with a significant time interval between each administration, whereupon said components are released at substantially different times to said patient; and
- sequential administration of such combination of compound(s) of formula (1) and therapeutic agent(s) to a patient in need of treatment, when such components are formulated together into a single dosage form which releases said components in a controlled manner whereupon they are concurrently, consecutively, and/or overlapingly administered at the same and/or different times by said patient,
where each part may be administered by either the same or different route.

Suitable examples of other therapeutic agents which may be used in combination with the compound(s) of formula (1), or pharmaceutically acceptable salts, derived forms or compositions thereof, include, but are by no means limited to :
- Histamine H₁ receptor antagonists, for instance loratidine, desloratidine, fexofenadine and cetirizine
- Histamine H₄ receptor antagonists
- Histamine H₂ receptor antagonists
- Leukotriene antagonists, including antagonists of LTB₄, LTC₄, LTD₄, and LTE₄, in particular Montelukast
- Phosphodiesterase inhibitors such as PDE4 inhibitors or PDE5 inhibitors,
- neurotransmitter re-uptake inhibitors, for instance fluoxetine, sertraline, paroxetine, ziprasidone
- 5-Lipoxygenase (5-LO) inhibitors or 5-lipoxygenase activating protein (FLAP) antagonists,
- α₁- and α₂-adrenoceptor agonist vasoconstrictor sympathomimetic agents for decongestant use,
- Muscarinic M3 receptor antagonists or anticholinergic agents,
- β₂-adrenoceptor agonists,
- Theophylline,
- Sodium cromoglycate,
- COX-1 inhibitors (NSAIDs) and COX-2 selective inhibitors,
- Oral or inhaled Glucocorticosteroids,
- Monoclonal antibodies active against endogenous inflammatory entities,
- Anti-tumor necrosis factor (anti-TNF-α) agents,
- Adhesion molecule inhibitors including VLA-4 antagonists,
- Kinin-B₁ - and B₂ -receptor antagonists,
- Immunosuppressive agents,
- Inhibitors of matrix metalloproteases (MMPs),
- Tachykinin NK₁, NK₂ and NK₃ receptor antagonists,
- Elastase inhibitors,
- Adenosine A2a receptor agonists,
- Inhibitors of urokinase,
- Compounds that act on dopamine receptors, e.g. D2 agonists,
- Modulators of the NFκβ pathway, e.g. IKK inhibitors,
- Agents that can be classed as mucolytics or anti-tussive,
- antibiotics,
- modulators of cytokine signalling pathways such as p38 MAP kinase, syk kinase or JAK kinase inhibitors,
- HDAC (histone deacetylase) inhibitors and
- PI3 kinase inhibitors

According to the present invention, combination of the compounds of formula (1) with :
- Histamine H₁ receptor antagonists, for instance loratidine, desloratidine, fexofenadine and cetirizine
- Histamine H₄ receptor antagonists
- Histamine H₂ receptor antagonists
- Leukotriene antagonists, including antagonists of LTB₄, LTC₄, LTD₄, and LTE₄, in particular Montelukast
- Phosphodiesterase PDE4 inhibitors
- neurotransmitter re-uptake inhibitors, for instance fluoxetine, sertraline, paroxetine, ziprasidone are preferred.

The compounds of formula (1) have the ability to interact with the H₃ receptor and thereby have a wide range of therapeutic applications, as described further below, because of the essential role which the H₃ receptor plays in the physiology of all mammals. According to this invention H₃ ligands are meant to include H₃ receptor antagonists, agonists and inverse agonists. For the preferred indications to be treated according to the invention, H₃ antagonists are believed to be most suitable.

In another aspect of the invention there is provided a compound of the formula (1) as defined in herein, or a pharmaceutically acceptable salt and/or solvate thereof, for use as a medicament.

A further aspect of the present invention relates to the compounds of formula (1), or pharmaceutically acceptable salts, derived forms or compositions thereof, for use in the treatment of diseases, disorders, and conditions in which the H₃ receptor is involved. More specifically, the present invention also concerns the compounds of formula (1), or pharmaceutically acceptable salts, derived forms or compositions thereof, for use in the treatment of diseases, disorders, and conditions selected from the group consisting of :
- diseases of the central nervous system: sleep disorders, migraine, dyskinesia, stress-induced anxiety, psychotic disorders, epilepsy, Cognition deficiency diseases such as Alzheimer's disease or mild cognitive impairment, depression, mood disorders, schizophrenia, anxiety disorders, attention-deficit hyperactivity disorder (ADHD), psychotic disorders, obesity, dizziness, vertigo, epilepsy, motion sickness
- inflammatory diseases
- respiratory diseases (adult respiratory distress syndrome, acute respiratory distress syndrome, bronchitis, chronic bronchitis, chronic obstructive pulmonary disease, cystic fibrosis, asthma, emphysema, rhinitis, chronic sinusitis), allergy, allergy-induced airway responses, allergic rhinitis, viral rhinitis, non-allergic rhinitis, perennial and seasonal rhinitis, nasal congestion, allergic congestion
- Female sexual dysfunction including hypoactive sexual desire disorder, sexual arousal disorder, orgasmic disorder and sexual pain disorder
- Male sexual dysfunction including male desire disorders, male erectile dysfunction, male orgasmic disorders such as premature ejaculation
- cardiac dysfunctions such as myocardial ischaemia and arrythmia
- diseases of the gastrointestinal tract such as inflammatory bowel disease, Crohn's disease and colitis ulcerosa
- cancer
- hypotension
- pain and
- overactive bladder conditions

The compounds of formula (1) of the invention are particularly suitable for the treatment of allergy, allergy-induced airway responses, allergic rhinitis, viral rhinitis, non-allergic rhinitis, perennial and seasonal rhinitis, nasal congestion, allergic congestion.

A still further aspect of the present invention also relates to the use of the compounds of formula (1), or pharmaceutically acceptable salts, derived forms or compositions thereof, for the manufacture of a drug being a H₃ ligand. In particular, the present inventions concerns the use of the compounds of formula (1), or pharmaceutically acceptable salts, derived forms or compositions thereof, for the manufacture of a drug for the treatment of H3-mediated diseases and/or conditions, in particular the diseases and/or conditions listed above.

Another aspect of the invention relates to the use of a compound of formula (1), or a pharmaceutically acceptable salt, derived form or composition thereof, for the manufacture of a medicament for the treatment of female sexual dysfunction, including hypoactive sexual desire disorder, sexual arousal disorder, orgasmic disorder and sexual pain disorder, or for the treatment of male sexual dysfunction including male desire disorders, male erectile dysfunction or male orgasmic disorders such as premature ejaculation.

### EXAMPLES

The following examples illustrate the preparation of phenol and thiophenol derivatives of the formula (1) :

### glossary

| | |
|---|---|
| APCI | atmospheric pressure chemical ionisation |
| Arbocel® | filter agent |
| BINAP | 2,2'-Bis(diphenylphosphino)-1,1'-binaphthyl |
| BOC | *tert*-butoxycarbonyl |
| br | broad |
| CDI | carbonyldiimidazole |
| δ | chemical shift |
| d | doublet |
| Δ | heat |
| DCM | dichloromethane |
| DIAD | diisopropyl azodicarboxylate |
| DMF | *N,N*-dimethylformamide |
| DMSO | dimethylsulfoxide |
| EDCI | see WSCDI |
| ESI⁺ | electrospray ionisation positive scan |
| ESI⁻ | electrospray ionisation negative scan |
| h | hours |
| HBTU | O-(1*H*-benzotriazol-1-yl)-*N,N,N',N*-tetramethyluronium hexafluorophosphate |
| HOAT | 1-hydroxy-7-azabenzotriazole |
| HOBT | 1-hydroxybenzotriazole |
| HPLC | high pressure liquid chromatography |
| Hunig's base | Diisopropylethylamine |
| m/z | mass charge ratio |
| min | minutes |
| MS | mass spectrum |
| NH₃ | 0.88 ammonia aqueous solution |
| NMM | *N*-methyl morpholine |
| NMR | nuclear magnetic resonance |
| q | quartet |
| s | singlet |
| STAB | sodium triacetoxyborohydride |
| t | triplet |
| TBME | *tert*-butyl methyl ether |
| TBTU | 2-(1H-benzotriazot-1-yl)-1,1,3,3-tetramethyluronium tetrafluoroborate |
| Tf | trifluoromethanesulfonyl |
| TFA | trifluoroacetic acid |
| THF | tetrahydrofuran |
| TLC | thin layer chromatography |
| TosMIC | Tosylmethyl isocyanide |
| WSCDI | 1-(3-dimethylaminopropyl)-3-ethylcarbodiimide hydrochloride |

### Intermediate 1: 4-(4-methoxyphenyl)-tetrahydro-2H-pyran-4-carbonitrile

4-Methoxyphenylacetonitrile (10g, 67.9mmol) in DMF (50ml) was added slowly to a suspension of NaH (5.04g, 150mmol) in DMF (50ml) at 0°C under N₂. The reaction was allowed to warm up to room temperature and stirred for 30 min. The reaction was cooled to 0°C and bis(2-chloroethyl)ether (10.7g, 74.7mmol)) in DMF (100ml) was added dropwise over 80 min. The reaction was allowed to warm up to room temperature and stirred for 1 hour. The reaction was quenched with water (200ml) and extracted with ethyl acetate (3x300ml). The combined organic extracts were washed with water (2x200ml), brine (200ml), dried over MgSO₄, filtered, washed with ethyl acetate and concentrated *in vacuo* to give the title compound (17.2g, 100%).

### Intermediate 2: 4-(4-Hydroxy-phenyl)-tetrahydro-pyran-4-carbonitrile

Boron tribromide (1M in DCM, 262ml) was added to a solution of 4-(4-methoxyphenyl)-tetrahydro-2H-pyran-4-carbonitrile (14.7g, 67.9mmol) in DCM (294ml) at 0°C under N₂ keeping the temperature below 5°C. The reaction was allowed to warm to room temperature and stirred for 48hours. The mixture was cooled to -10-0°C with dry ice acetone and quenched with saturated aqueous sodium bicarbonate (294ml). The mixture was allowed to warm to room temperature and stirred for 1hour. The mixture was separated and the aqueous extracted with DCM (3x250ml). The combined organic extracts were dried over MgSO₄, filtered, washed with DCM and concentrated *in vacuo* to give the title compound (10.8g, 78%).

### Intermediate 3: 4-(4-Dimethylaminomethyl-tetrahydro-pyran-4-yl)-phenol

### Step 1:

To a stirred suspension of LiAlH₄ (79g, 2.08mol, 5eq) in THF (900ml) at 0 to 5°C under an atmosphere of nitrogen was added 4-(4-methoxyphenyl)- tetrahydro-2H-pyran-4-carbonitrile (90g, 0.414mol) in THF (900ml) over a 25 minutes maintaining the temperature at 5 to 10°C. The reaction mixture was allowed to warm to ambient temperature and stirred until complete. Sodium hydroxide (2N, 850ml) was added dropwise, the resulting solids filtered and washed with THF (2x800ml), the organics concentrated *in vacuo* at 40°C. The residue was dissolved in EtOAc (300ml) and dried over MgSO₄, filtered and concentrated *in vacuo* at 40°C to provide {[4-(4-methoxyphenyl)-tetrahydro-2H-pyran-4-yl]methyl}amine as a light yellow oil (92g, quantitative).

### Step 2:

To a solution of {[4-(4-methoxyphenyl)-tetrahydro-2H-pyran-4-yl]methyl}amine (60g, 0.271mol) in H₂O (444mL) and AcOH (213ml) was charged formaldehyde (37%ww solution in H₂O, 408 mL) and the mixture cooled to 0 to 5°C. Sodium triacetoxyborohydride (345g, 1.626mol, 6eq) was added portion wise while maintaining the temperature below 12°C. The mixture was stirred at ambient temperature until complete. Sodium hydroxide (2M, 1000ml) was added slowly and the mixture extracted with DCM (4x250ml). The organic extracts were dried over MgSO₄, filtered and concentrated *in vacuo* at 30°C to provide {[4-(4-methoxyphenyl)-tetrahydro-2H-pyran-4-yl]methyl}dimethylamine as a yellow oil (53.5g, 80%).

### Step 3:

To a suspension of Sodium thiomethoxide (49.2g, 0.702mol, 5eq) in DMF (140ml) was added {[4-(4methoxyphenyl)-tetrahydro-2H-pyran-4-yl]methyl}dimethylamine (35g, 0.140mol) and the resulting mixture was heated to 130°C. Once complete, allowed to cool to ambient temperature and saturated aqueous NH₄Cl (525ml) was added. The resultant was extracted with EtOAc (3x500ml), dried over MgSO₄, filtered and concentrated *in vacuo* at 35°C to provide 4-(4-dimethylaminomethyl-tetrahydro-pyran-4-yl)-phenol (intermediate 3) as a yellow solid (35.7g, 108%) containing residual DMF and EtOAc.

### Intermediate 4: 3-{4-[(dimethylamino)methyl]tetrahydro-pyran-4-yl}phenol

### Step 1:

3-Methoxyphenylacetonitrile (10g, 67.9mmol) in DMF (50ml) was added slowly to a suspension of NaH (5.04g, 150mmol) in DMF (50ml) at 0°C under N₂. The reaction was allowed to warm to room temperature and stirred for 30mns. The reaction was cooled to 0°C and bis(2-chloroethyl)ether (10.7g, 74.7mmol) in DMF (100ml) was added dropwise over 80 min. The reaction was allowed to warm to room temperature and stirred for 1 hour. The reaction was quenched with water (500ml) and extracted with ethyl acetate (3x100ml). The combined organic extracts were washed with brine (3x50ml), dried over MgSO₄, filtered, washed with ethyl acetate and concentrated *in vacuo*. The crude mixture was purified by column chromatography, eluting with heptane increasing the polarity to heptane:ethyl acetate (90 :10), to give 4-(3-methoxyphenyl)tetrahydro-pyran-4-carbonitrile (4.1 g, 28%).

### Step 2:

To a stirred suspension of LiAlH₄ (3.7 g, 96.7 mmol) in diethyl ether (100 mL) cooled at 0°C was added dropwise a solution of 4-(3-methoxyphenyl)-tetrahydro-pyran-4-carbonitrile (4.2 g, 19.3 mmol) in diethyl ether (100 mL). After being stirred at room temperature for 30 min, the reaction mixture was refluxed for another 15 min. The mixture was cooled to 10°C and water, sodium hydroxide (15% w/v in water, 0.48 mL) and water again were successively added dropwise. After being stirred for 15 min at room temperature, the mixture was filtered over diatomaceous earth and concentrated. The residue was purified with flash chromatography (DCM/MeOH: 95/5) to provide [4-(3-methoxyphenyl)tetrahydro-pyran-4-yl]methylamine (4 g, 94%).

### Step 3:

A mixture of [4-(3-methoxyphenyl)tetrahydro-pyran-4-yl]methylamine (0.51 g, 2.3 mmol), acetic acid (0.535 mL, 9.2 mmol), sodium triacetoxyborohydride (0.974 g, 4.6 mmol), 37% solution of formaldehyde in water (0.55 mL) and DCM (40 mL) was stirred 72 h at ambient temperature. The organic phase was washed twice with water. Concentrated NaOH was added to the aqueous phase (pH 12) and the mixture was extracted twice with (dichloromethane. The organic extracts were dried over Na₂SO₄, filtered and concentrated *in vacuo* to provide N-{[4-(3-methoxyphenyl)tetrahydro-pyran-4-yl]methyl}-N,N-dimethylamine as an oil (0.35 g, 61%).

### Step 4:

To a suspension of Sodium thiomethoxide (0.42 g, 6 mmol) in DMF (1.2 mL) was added N-{[4-(3-methoxyphenyl)tetrahydro-pyran-4-yl]methyl}-N,N-dimethylamine (0.3 g, 1.2 mmol) and the resulting mixture was heated to 65°C for 7 h, allowed to cool to ambient temperature and quenched with saturated aqueous NH₄Cl (6 mL). The mixture was extracted with DCM, dried over Na₂SO₄, filtered and concentrated *in vacuo* to provide 0.25 g of crude product. A mixture of the crude and HBr (3 mL) was heated to reflux for 1 h. After cooling, water was added to quench the reaction and the mixture was basified with NaHCO₃ and extracted with DCM. The organic extracts were dried over Na₂SO₄, filtered and concentrated. The residue was purified by flash chromatography (DCM / MeOH (10% NH₃)) to provide 3-{4-[(dimethylamino)methyl]tetrahydro-pyran-4-yl}phenol (0.100 g, 35.5%).

### General procedure A for the synthesis of chlorides (R-R^{LG} wherein R ^{LG} is chloride):

Amine (1eq.) was charged to a reaction flask followed by acetone (3vol or 20vol), 5M NaOH solution (1.2eq.) and 1-bromo-3-chloropropane (1.5 eq. or 3eq.). The reaction was stirred overnight at room temperature. The phases were separated and the acetone layer concentrated *in vacuo*. The aqueous was acidified with 2M HCl solution to pH 1 (∼10vol). The concentrate was diluted with TBME (30vol) and washed with water (15vol). The aqueous was extracted with TBME (2 x 15ml). The combined TBME was washed with 2M HCl solution (15ml) and combined with the first acidic phase. The combined acidic layers were washed with TBME (15vol) and basified to pH 14 with 4M NaOH solution (∼40ml). The aqueous was extracted with TBME (3 x 30ml). The combined organic layers were dried over MgSO₄, filtered, washed with TBME (5vol) and concentrated *in vacuo* to give the required chloride.

### General procedure B:

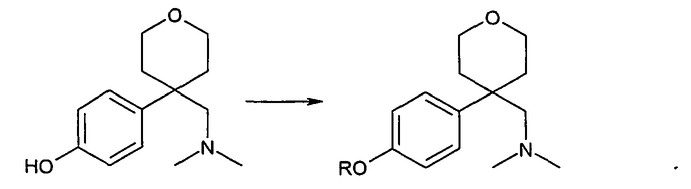

4-(4-Dimethylaminomethyl-tetrahydro-pyran-4-yl)-phenol (500mg, 2.13mmol), chloride (378mg, 2,13mmol), DMF (10ml), water (17ml) and K₂CO₃ (1.18g, 8.52mmol) was heated to 130°C. Cooled to ambient temperature, water (40ml) added, extracted with EtOAc (3x25ml) and the combined organic extracts washed with water (2x25ml). The organics were dried over MgSO₄, filtered and concentrated *in vacuo* at 35°C. Purification of the crude material by chromatography on silica, eluant (4% MeOH, 1% NH₃, in DCM) provided the title compounds.

### Intermediate 5: 1-(3-Chloro-propyl)-pyrrolidine

Pyrrolidine (10g, 0.14mol), acetone (28ml), 5M NaOH solution (21ml) and 1-bromo-3-chloropropane (24.4g, 0.15mol) were stirred together under N₂ for 8hours. The organic layer was separated and concentrated *in vacuo.* The crude product was purified by vacuum distillation (b.p. 90°C/30mbar) to give the title compound (11.7g, 57%) as a colourless oil.

### Example 1: Dimethyl-{4-[3-(3-pyrrolidin-1-ylpropoxy)phenyl]tetrahydro-pyran-4-ylmethyl}amine

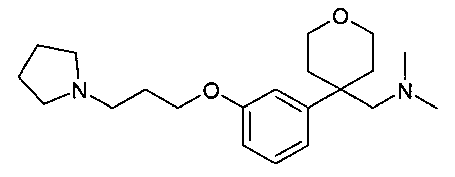

Alkylation of 3-[4-(dimethylamino)methyltetrahydro-2H-pyran-4-yl]phenol (0.1 g, 0.425 mmol) with 1-(3-chloropropyl)pyrrolidine (0.125 g, 0.85 mmol) according to general procedure B gave the desired compound after purification by flash chromatography (0.030 g, 20%).
¹H NMR (400MHz, CDCl₃) *δ* 7.27-7.18 (m, 1H), 6.92-6.84(m, 2H), 6.75 (dd, 1H), 4.05 (t, 2H), 3.8-3.7 (m, 2H), 3.55 (t, 2H), 2.65 (t, 2H), 2.6-2.5 (m, 4H), 2.4(s, 2H), 2.15-1.85 (m, 6H), 2.0 (s, 6H), 1.85-1.75 (m, 4H).

### Intermediate 6: 1-(3-Chloro-propyl)-2(R),5(R)-trans-dimethyl-pyrrolidine

2(R),5(R)-trans-Dimethyl-pyrrolidine (0.75g, 7.56mmol), acetone (15ml, 20vol), 5M NaOH solution (1.8ml, 1.2eq.) and 1-bromo-3-chloropropane (3.57g, 22.7mmol, 3eq.) were reacted together according to general procedure A to give the title compound (0.5g, 38%) as a yellow oil.

### Example 2: (4-{4-[3-(2,5-Dimethylpyrrolidin-1-yl)propoxy]pheny}tetra-hydro-pyran-4-ylmethyl)dimethylamine

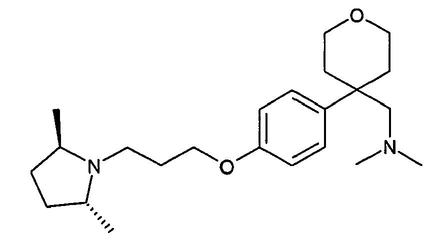

4-(4-Dimethylaminomethyl-tetrahydro-pyran-4-yl)-phenol (330mg, 1.43mmol), 1-(3-chloro-propyl)-2,5-trans-dimethyl-pyrrolidine (221mg, 1.26mmol), DMF (7.6ml) and K₂CO₃ (790mg, 5.72mmol) were reacted together according to general procedure B. The isolated material was subjected to chromatography on silica, eluant 95:4:1 (DCM, MeOH, NH₃) to give the title compound as a yellow oil (180mg, 34%).
¹H NMR (400MHz, CDCl₃) δ 7.20 (d, 2H), 6.87 (d, 2H), 4.08-3.95 (m, 2H), 3.79-3.70 (m, 2H), 3.59-3.49 (m, 2H), 3.11-3.00 (m, 2H), 2.77 (m, 1H), 2.54 (m, 1H), 2.40 (s, 2H), 2.14-1.81 (m, 8H), 1.96 (s, 6H), 1.44-1.31 (m, 2H), 0.97 (d, 6H).

### Intermediate 7: 1-(3-Chloro-propyl)-2-methyl-pyrrolidine

2-Methylpyrrolidine (0.9g, 10.6mmol), acetone (18ml, 20vol), 5M NaOH solution (2.50ml) and 1-bromo-3-chloropropane (5g, 31.8mmol, 3eq) were reacted together according to general procedure A to give the title compound (1g, 59%) as a pale yellow oil.

### Example 3: Dimethyl-(4-{4-[3-(2-methyl-pyrrolidin-1-yl)-propoxy]-phenyl}-tetrahydro-pyran-4-ylmethyl)-amine

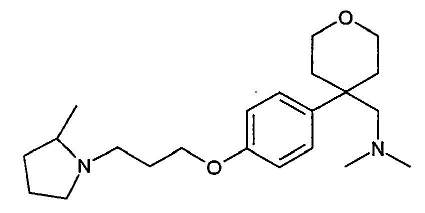

4-(4-Dimethylaminomethyl-tetrahydro-pyran-4-yl)-phenol (500mg, 2.13mmol), 1-(3-chloro-propyl)-2-methyl-pyrrolidine (344mg, 2,13mmol), DMF (10ml) and K₂CO₃ (1.18g, 8.52mmol) were reacted together according to general procedure B. The isolated material was subjected to chromatography on silica, eluant 95:4:1 (DCM, MeOH, NH₃) to give the title compound as a yellow oil (120mg, 16%).
¹H NMR (400MHz, CDCl₃) δ 7.20 (d, 2H), 6.87 (d, 2H), 4.08-3.96 (m, 2H), 3.75 (dt, 2H), 3.55 (td, 2H), 3.18 (td, 1H), 2.98 (m, 1H), 2.40 (s, 2H), 2.29 (m, 1H), 2.20 (m, 1H), 2.16-1.62 (m, 10H), 1.96 (s, 6H), 1.42 (m, 1H), 1.09 (d, 3H).

### Intermediate 8: 1-(3-Chloro-propyl)-2,6-cis-dimethyl-piperidine

### Step 1:

2,6-cis-Dimethyl-piperidine (1.0g, 8.83mmol), K₂CO₃ (1.52g, 1.25eq.) and 3-bromopropanol (6.14g, 44.2mmol, 4vol) were reacted together at 100°C for 2hours. The reaction was allowed to cool to room temperature, diluted with DCM (20ml) and quenched with 2M HCl solution (20ml). The aqueous was extracted with DCM (2 x 20ml) and basified to pH 14 with 2M NaOH solution (∼15ml). The aqueous was extracted with DCM (3 x 20ml). The combined DCM layers were dried over MgSO₄, filtered washed with DCM and concentrated *in vacuo* to give 1-(propan-1'-ol)-2,6-cis-dimethyl-piperidine (1.22g, 81 %) as a pale yellow oil.

### Step 2:

1-(Propan-1'-ol)-2,6-cis-dimethyl-piperidine (1.22g, 7.12mmol) was dissolved in DCM (24ml) and cooled to 0-5°C under N₂. Thionyl chloride (1.04ml, 14.25mmol) was added dropwise. The reaction was allowed to warm to room temperature and stirred for 30mins. The reaction was quenched with 2M HCl solution (25ml). The aqueous was extracted with DCM (25m1) and basified to pH 14 with 5M NaOH solution (∼25ml). The aqueous was extracted with TBME (3 x 25ml). The combined organic layers were dried over MgSO₄, filtered washed with TBME and concentrated *in vacuo* to give the title compound (1.26g, 93%) as a yellow oil.

### Example 4: (4-{4-[3-(2,6-Dimethylpiperidin-1-yl)propoxy]phenyl}tetra-hydro-pyran-4-ylmethyl)dimethyl-amine

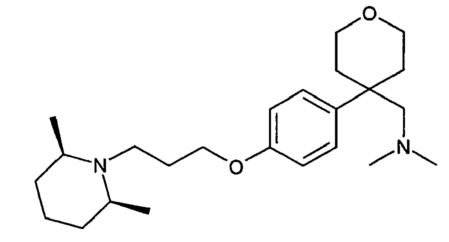

4-(4-Dimethylaminomethyl-tetrahydro-pyran-4-yl)-phenol (500mg, 2.13mmol), 1-(3-chloro-propyl)-2,6-cis-dimethyl-piperidine (410mg, 2,13mmol), DMF (10ml) and K₂CO₃ (1.18g, 8.52mmol) were reacted together according to general procedure B. The isolated material was subjected to chromatography on silica, eluant 95:4:1 (DCM, MeOH, NH₃) to give the title compound as a yellow oil (280mg, 33.9%).
¹H NMR (400MHz, CDCl₃) δ 7.20 (d, 2H), 6.85 (d, 2H), 3.93 (t, 2H), 3.75 (dt, 2H), 3.55 (td, 2H), 2.95 (t, 2H), 2.51-2.36 (m, 2H), 2.40 (s, 2H), 2.14-2.04 (m, 2H), 1.96 (s, 6H), 1.93-1.82 (m, 4H), 1.74-1.51 (m, 3H), 1.41-1.21 (m, 3H), 1.13 (d, 6H).

### Intermediate 9: 4-(3-Chloro-propyl)-thiomorpholine

Thiomorpholine (5g, 49mmol), acetone (15ml, 3vol), 5M NaOH solution (11.8ml) and 1-bromo-3-chloropropane (11.6g, 73.5mmol, 1.5eq.) were reacted together according to general procedure A to give the title compound (8.5g, 96%) as a pale yellow oil.

### Example 5: Dimethyl-{4-[4-(3-thiomorpholin-4-ylpropoxy)phenyl]tetra-hydro-pyran-4-ylmethyl}amine

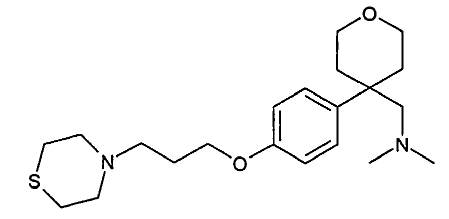

4-(4-Dimethylaminomethyl-tetrahydro-pyran-4-yl)-phenol (1000mg, 4.25mmol), 4-(3-chloro-propyl)-thiomorpholine (764mg, 2,13mmol), DMF (20ml), and K₂CO₃ (2,34g, 17mmol) were reacted together according to general procedure B. The isolated material was subjected to chromatography on silica, eluant DCM:MeOH:NH₃ (95:5:1) to give the title compound (320mg, 20%).
¹H NMR (400MHz, CDCl₃) δ 7.20 (d, 2H), 6.86 (d, 2H), 3.99 (t, 2H), 3.75 (dt, 2H), 3.55 (td, 2H), 2.79-2.64 (m, 8H), 2.55 (t, 2H), 2.40 (s, 2H), 2.16-2.04 (m, 2H), 2.01-1.82 (m, 4H), 1.97 (s, 6H).

### Example 6: Dimethyl-(4-{4-[3-(1-oxothiomorpholin-4-yl)propoxy]phenyl}-tetrahydropyran-4-ylmethyl)amine

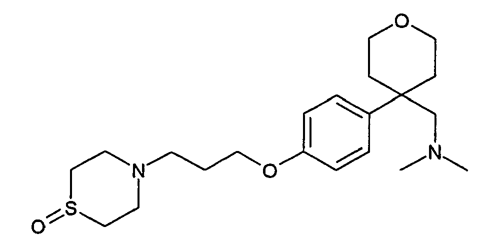

Dimethyl-{4-[4-(3-thiomorpholin-4-ylpropoxy)phenyl]tetrahydro-pyran-4-ylmethyl}amine (300mg, 0.792mmol) and TFA (0.99ml) were cooled to 0 to 5 °C and trifluoro-peracetic acid (4M, 0.77ml) [4M solution prepared by the addition of 27.5% H₂O₂ (0.94ml) to TFA (1.56ml)] was added and the reaction stirred for six hours at 0 to 5°C. The mixture was diluted with DCM (6ml), basified with NaOH (2M, 8ml) and extracted with DCM (2x20ml). The DCM extracts were dried over MgSO₄, filtered and concentrated *in vacuo* at 35°C. Purification by chromatography on silica, eluant DCM:MeOH:NH₃ (96:4:1) provided the title compound (200mg, 61%) as a white solid.
¹H NMR (400MHz, CDCl₃) δ 7.21 (d, 2H), 6.86 (d, 2H), 4.01 (t, 2H), 3.75 (dt, 2H), 3.55 (td, 2H), 3.15-3.02 (m, 2H), 2.93-2.79 (m, 4H), 2.72 (dt, 2H), 2.64 (t, 2H), 2.40 (s, 2H), 2.16-1.82 (m, 6H), 1.97 (s, 6H).

### Example 7: (4-14-[3-(1,1-Dioxo-thiomorpholin-4-yl)propoxylphenyl]tetra-hydro-pyran-4-ylmethyl)dimethyl-amine

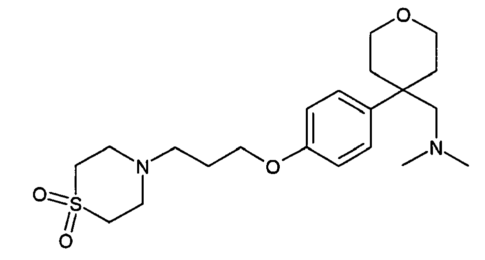

Dimethyl-{4-[4-(3-thiomorpholin-4-ylpropoxy)phenyl]tetrahydro-pyran-4-ylmethyl}amine (300mg, 0.792mmol) and TFA (0.99ml) were cooled to 0 to 5°C and trifluoro-peracetic acid (4M, 0.77ml) was added and the reaction allowed to warm to ambient temperature overnight. The mixture was diluted with DCM (6ml), basified with NaOH (2M, 8ml) and extracted with DCM (2x20ml). The DCM extracts were dried over MgSO₄, filtered and concentrated *in vacuo* at 35°C. Purification by chromatography on silica, eluant DCM:MeOH:NH₃ (96:4:1) provided the title compound (151mg, 46%) as a white solid.
¹H NMR (400MHz, CDCl₃) δ 7.22 (d, 2H), 6.85 (d, 2H), 4.01 (t, 2H), 3.75 (dt, 2H), 3.55 (td, 2H), 3.10-2.97 (m, 8H), 2.72 (t, 2H), 2.40 (s, 2H), 2.14-1.82 (m, 6H), 1.97 (s, 6H).

### Intermediate 10: 1-(3-Chloro-propyl)-piperidin-4-ol

4-Hydroxypiperidine (3g, 30mmol), acetone (60ml, 20vol), 5M NaOH solution (7.2ml) and 1-bromo-3-chloropropane (14.2g, 90mmol, 3eq.) were reacted together according to general procedure A to give the title compound (1.5g, 28%) as a pale yellow oil.

### Example 8: 1-{3-[4-(4-Dimethylaminomethyl-tetrahydro-pyran-4-yl)-phenoxy]-propyl}-piperidin-4-ol

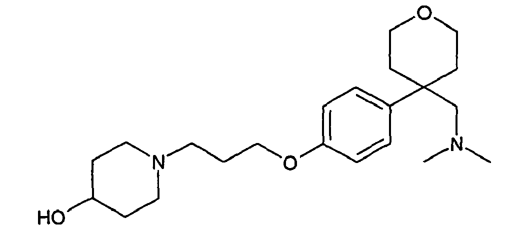

4-(4-Dimethylaminomethyl-tetrahydro-pyran-4-yl)-phenol (500mg, 2.13mmol), 1-(3-chloro-propyl)-piperidin-4-ol (378mg, 2,13mmol), DMF (10ml) and K₂CO₃ (1.18g, 8.52mmol) were reacted together according to general procedure B. The isolated material was dissolved in EtOAc (30ml), washed with NaOH (2M, 2x20ml) and water (25ml), dried over MgSO₄ filtered and concentrated *in vacuo* at 35°C to give the title compound (411 mg, 51%).
¹H NMR (400MHz, CDCl₃) δ 7.20 (d, 2H), 6.86 (d, 2H), 4.00 (t, 2H), 3.80-3.65 (m, 3H), 3.55 (td, 2H), 2.85-2.73 (m, 2H), 2.52 (t, 2H), 2.40 (s, 2H), 2.22-1.82 (m, 10H), 1.97 (s, 6H), 1.66-1.54 (m, 2H), 1.52 (br s, 1H).

### Intermediate 11: 1-(3-Chloro-propyl)-4-methoxy-piperidine

4-Methoxy-piperidine (1.5g, 13mmol), acetone (30ml, 20vol), 5M NaOH solution (3.13ml) and 1-bromo-3-chloropropane (6.14g, 39mmol, 3eq.) were reacted together according to general procedure A to give the title compound (1.5g, 60%) as a pale yellow oil.

### Example 9: (4-{4-[3-(4-Methoxy-piperidin-1-yl)-propoxy]-phenyl}-tetrahydro-pyran-4-ylmethyl)-dimethyl-amine

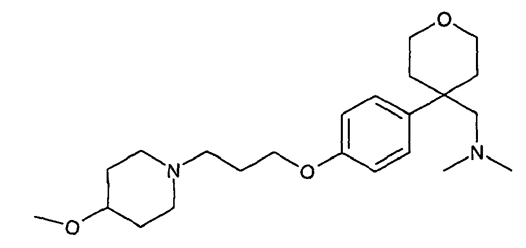

4-(4-Dimethylaminomethyl-tetrahydro-pyran-4-yl)-phenol (500mg, 2.13mmol), 1-(3-chloro-propyl)-4-methoxy-piperidine (410mg, 2,13mmol), DMF (10ml) and K₂CO₃ (1.18g, 8.62mmol) were reacted together according to general procedure B. The isolated material was subjected to chromatography on silica, eluant 95:4:1 (DCM: MeOH: NH₃) to give the title compound as a yellow oil (415mg, 50%).
¹H NMR (400MHz, CDCl₃) δ 7.20 (d, 2H), 6.86 (d, 2H), 4.00 (t, 2H), 3.75 (dt, 2H), 3.55 (td, 2H), 3.34 (s, 3H), 3.22 (m, 1H), 2.83-2.71 (m, 2H), 2.51 (t, 2H), 2.40 (s, 2H), 2.23-1.81 (m, 10H), 1.97 (s, 6H), 1.67-1.53 (m, 2H).

### Intermediate 12: 2-[1-(3-Chloro-propyl)-piperidin-4-yl]-ethanol

4-Piperidine ethanol (0.9g, 7.5mmol), acetone (18ml, 20vol), 5M NaOH solution (1.8ml) and 1-bromo-3-chloropropane (3.54g, 22.5mmol, 3eq.) were reacted together according to general procedure A to give the title compound (0.6g, 37%) as an orange oil.

### Example 10: 2-(1-{3-[4-(4-Dimethylaminomethyltetrahydropyran-4-yl)phenoxy]propyl}piperidin-4-yl)ethanol

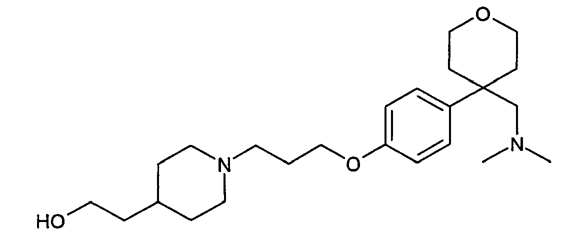

4-(4-Dimethylaminomethyl-tetrahydro-pyran-4-yl)-phenol (288mg, 1.22mmol), 2-[1-(3-chloro-propyl)-piperidin-4-yl]-ethanol (252mg, 1.22mmol), DMF (8ml) and K₂CO₃ (674mg, 4.88mmol) were reacted together according to general procedure B. Purification by chromatography on silica, eluant DCM:MeOH:NH₃ (96:4:1) provided the title compound (180mg, 36%) as an off white solid.
¹H NMR (400MHz, CDCl₃) *δ* 7.20 (d, 2H), 6.86 (d, 2H), 3.99 (t, 2H), 3.75 (dt, 2H), 3.70 (t, 2H), 3.55 (td, 2H), 2.98-2.88 (m, 2H), 2.49 (t, 2H), 2.40 (s, 2H), 2.14-1.82 (m, 6H), 1.97 (s, 6H), 1.75-1.15 (m, 10H).

### Intermediate 13:1-(3-Chloro-propyl)-4-(2-methoxy-ethyl)-piperidine

### Step 1:

N-Boc-4-(2-hydroxy-ethyl)-piperidine (5g, 21.8mmol) in THF (25ml) was added to a suspension of NaH (1.47g, 43.7mmol) in THF (75ml) at 0°C under N₂. The reaction was stirred for 1 hour at 0°C and Mel (2.72ml, 43.7mmol) added slowly. The reaction was allowed to warm to room temperature and stirred overnight. The reaction was quenched with water (100ml) and extracted with DCM (3 x 100ml). The combined DCM layers were dried over MgSO₄, filtered, washed with DCM and concentrated *in vacuo.* The crude reaction (7.5g) was treated with 3M HCl solution in EtOH (150ml) for 12hours. The reaction was concentrated *in vacuo* and azeotroped with toluene (2 x 150ml) to give 4-(2-methoxy-ethyl)-piperidine hydrochloride (3.5g, 89%) as a pale yellow solid.

### Step 2:

4-(2-Methoxy-ethyl)-piperidine hydrochloride (1.5g, 8.4mmol), acetone (30ml, 20vol), 5M NaOH solution (6.03ml, 3.6eq.) and 1-bromo-3-chloropropane (3.96g, 25.1mmol, 3eq.) were reacted together according to general procedure A to give the title compound (1.6g, 88%) as a pale yellow oil.

### Example 11: [4-(4-{3-[4-(2-Methoxy-ethyl)-piperidin-1-yl]-propoxy}-phenyl)-tetrahydro-pyran-4-ylmethyl]-dimethyl-amine

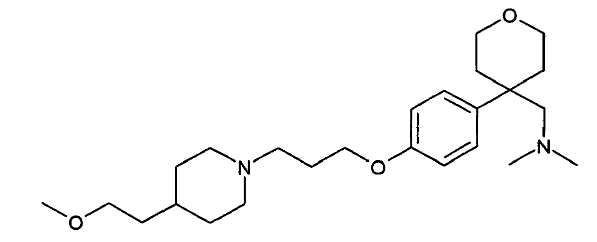

4-(4-Dimethylaminomethyl-tetrahydro-pyran-4-yl)-phenol (500mg, 2.13mmol), 1-(3-chloro-propyl)-4-(2-methoxy-ethyl)-piperidine (466mg, 2,13mmol), DMF (10ml) and K₂CO₃ (1.18g, 8.52mmol) were reacted together according to general procedure B. Purification by chromatography on silica, eluant DCM:MeOH:NH₃ (96:4:1) provided the title compound (409mg, 46%) as a yellow oil.
¹H NMR (400MHz, CDCl₃) δ 7.20 (d, 2H), 6.86 (d, 2H), 3.99 (t, 2H), 3.75 (dt, 2H), 3.55 (td, 2H), 3.41 (t, 2H), 3.33 (s, 3H), 2.97-2.86 (m, 2H), 2.49 (t, 2H), 2.40 (s, 2H), 2.15-1.78 (m, 8H), 1.97 (s, 6H), 1.74-1.17 (m, 7H).

### Intermediate 14:1-(3-Chloro-propyl)-piperidine-4-carboxylic acid amide

Piperidine-4-carboxylic acid amide (0.5g, 3.90mmol), acetone (1.5ml, 3vol), 5M NaOH solution (1.20ml, 1.2eq.) and 1-bromo-3-chloropropane (0.50ml, 5.04mmol, 1eq.) were reacted together according to general procedure A to give the title compound (0.16g, 20%) as a pale yellow oil.

### Example 12: 1-{3-[4-(4-Dimethylaminomethyl-tetrahydro-pyran-4-yl)-phenoxy]-propyl}-piperidine-4-carboxylic acid amide

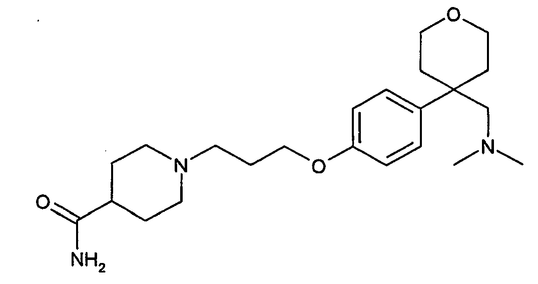

4-(4-Dimethylaminomethyl-tetrahydro-pyran-4-yl)-phenol (500mg, 2.13mmol), 1-(3-chloro-propyl)-piperidine-4-carboxylic acid amide (436mg, 2,13mmol), DMF (10ml) and K₂CO₃ (1.18g, 8.52mmol) were reacted together according to general procedure B. The isolated material was subjected to chromatography on silica, eluant 95:4:1 (DCM: MeOH: NH₃) to give the title compound as a white solid (175mg, 20%).
¹H NMR (400MHz, CDCl₃) δ 7.21 (d, 2H), 6.86 (d, 2H), 5.44 (br s, 1H), 5.25 (br s, 1H), 4.00 (t, 2H), 3.75 (dt, 2H), 3.55 (td, 2H), 3.03-2.93 (m, 2H), 2.51 (t, 2H), 2.40 (s, 2H), 2.22-1.66 (m, 13H), 1.97 (s, 6H).

### Intermediate 15: 4-(3-Chloro-propyl)-piperazine-1-carboxylic acid ethyl ester

Piperazine-1-carboxylic acid ethyl ester (3.0g, 18.9mmol), K₂CO₃ (2.8g, 1.1eq.), DMF (30ml, 10vol) and 1-bromo-3-chloropropane (1.8ml, 18.9mmol, 1eq.) were reacted together at room temperature overnight. The reaction was quenched with water (100ml) and extracted with DCM (3 x 50ml). The combined DCM extracts were dried over MgSO₄, filtered washed with DCM and concentrated *in vacuo* to give the title compound (3.1g, 70%) as a pale yellow oil.

### Example 13:{3-[4-(4-Dimethylaminomethyltetrahydropyran-4-yl)phenoxy]-propyl}piperazine-1-carboxylic acid ethyl ester

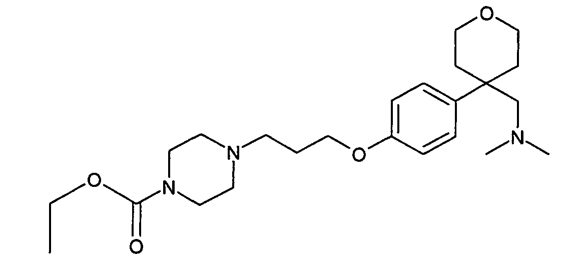

4-(4-Dimethylaminomethyl-tetrahydro-pyran-4-yl)-phenol (500mg, 2.13mmol), 4-(3-chloro-propyl)-piperazine-1-carboxylic acid ethyl ester (499mg, 2,13mmol), DMF (10ml) and K₂CO₃ (1.18g, 8.52mmol) were reacted together according to general procedure B. Purification by chromatography on silica, eluant (10%MeOH in DCM) the title compound (276mg, 26%).
¹H NMR (400MHz, CDCl₃) δ 7.21 (d, 2H), 6.86 (d, 2H), 4.14 (q, 2H), 4.01 (t, 2H), 3.75 (dt, 2H), 3.60-3.42 (m, 6H), 2.54 (t, 2H), 2.47-2.36 (m, 4H), 2.40 (s, 2H), 2.16-1.82 (m, 6H), 1.97 (s, 6H), 1.26 (t, 3H).

### Intermediate 16 : (3-Chloro-propyl)-diethyl-amine

Diethyl-amine (7.27ml, 70mmol), acetone (15ml, 3vol), 5M NaOH solution (16.8ml, 1.2eq.) and 1-bromo-3-chloropropane (16.5g, 105mmol, 1.5eq.) were reacted together according to general procedure A to give the title compound (6.2g, 59%) as a colourless oil.

### Example 14: {3-[4-(4-Dimethylaminomethyltetrahydropyran-4-yl)phenoxy]-propyl}diethyl-amine

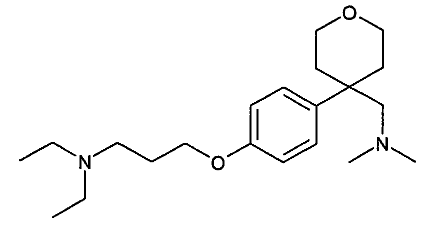

4-(4-Dimethylaminomethyl-tetrahydro-pyran-4-yl)-phenol (500mg, 2.13mmol), (3-chloro-propyl)-diethylamine (319mg, 2,13mmol), DMF (10ml) and K₂CO₃ (1.18g, 8.52mmol) were reacted together according to general procedure B. Purification by chromatography on silica, eluant (3%EtOH: 1%NH₃ in DCM) gave the title compound as a pale yellow clear oil (160mg, 22%). ¹H NMR (400MHz, CDCl₃) δ 7.20 (d, 2H), 6.86 (d, 2H), 4.00 (t, 2H), 3.75 (dt, 2H), 3.55 (td, 2H), 2.61 (t, 2H), 2.54 (q, 4H), 2.40 (s, 2H), 2.15-1.82 (m, 6H), 1.97 (s, 6H), 1.02 (t, 6H).

### Intermediate 17: 2-[(3-Chloro-propyl-ethyl-amino]-ethanol

2-Ethylamino ethanol (6.2g, 70mmol), acetone (18ml, 3vol), 5M NaOH solution (16.8ml, 1.2eq.) and 1-bromo-3-chloropropane (16.5g, 105mmol, 1.5eq.) were reacted together according to general procedure A to give the title compound (4.5g, 39%) as a colourless oil.

### Example 15: 2-({3-[4-(4-Dimethylaminomethyltetrahydropyran-4-yl)-phenoxy]propyl}ethylamino) ethanol

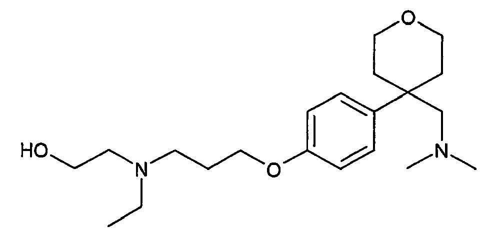

4-(4-Dimethylaminomethyl-tetrahydro-pyran-4-yl)-phenol (500mg, 2.13mmol), 2-[(3-chloro-propyl)-ethylamino]-ethanol (353mg, 2,13mmol), DMF (10ml) and K₂CO₃ (1.18g, 8.52mmol) were reacted together according to general procedure B. The isolated material was subjected to chromatography on silica, eluant 95:4:1 (DCM: MeOH: NH₃) to give the title compound as a white solid (150mg, 19%).
¹H NMR (400MHz, CDCl₃) δ 7.21 (d, 2H), 6.86 (d, 2H), 4.00 (t, 2H), 3.75 (dt, 2H), 3.60-3.49 (m, 4H), 2.68 (t, 2H), 2.62 (t, 2H), 2.59 (q, 2H), 2.40 (s, 2H), 2.14-1.82 (m, 6H), 1.97 (s, 6H), 1.62 (br s, 1H), 1.03 (t, 3H).

### Intermediate 18: (3-Chloro-propyl)-isopropyl-methyl-amine

Isopropylmethylamine (4g, 56mmol), acetone (12ml, 3vol), 5M NaOH solution (13.44ml, 1.2eq.) and 1-bromo-3-chloropropane (13.22g, 84mmol, 1.5eq.) were reacted together according to general procedure A to give the title compound (4.8g, 66%) as a colourless oil.

### Example 16: {3-[4-(4-Dimethylaminomethyltetrahydropyran-4-yl)phenoxyl-propyl} isopropylmethylamine

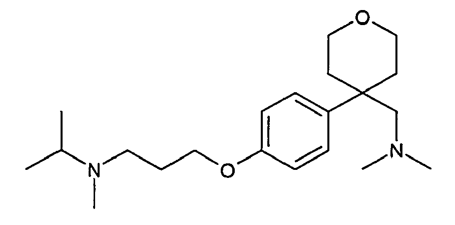

4-(4-Dimethylaminomethyl-tetrahydro-pyran-4-yl)-phenol (500mg, 2.13mmol), (3-chloro-propyl)-isopropyl-methyl-amine (318mg, 2,13mmol), DMF (10ml) and K₂CO₃ (1.18g, 8.52mmol) were reacted together according to general procedure B. Purification by chromatography on silica, eluant DCM:MeOH:NH₃ (96:3:1) provided the title compound (200mg, 43%) as a white solid.
¹H NMR (400MHz, CDCl₃) δ 7.20 (d, 2H), 6.87 (d, 2H), 4.00 (t, 2H), 3.75 (dt, 2H), 3.55 (td, 2H), 2.83 (sep, 1H), 2.55 (t, 2H), 2.40 (s, 2H), 2.22 (s, 3H), 2.14-2.05 (m, 2H), 1.96 (s, 6H), 2.00-1.82 (m, 4H), 1.00 (d, 6H).

### Intermediate 19: 1-(2-chloro-ethyl)-pyrrolidine

Pyrrolidine (10.4g, 0.15mol), acetone (200ml), 5M NaOH solution (35ml) and 1-bromo-2-chloroethane (62.9g, 0.44mol) were reacted together according to general procedure A. The crude mixture was purified by column chromatography eluting with ethyl acetate to give the title compound (2.0g, 10%) as a pale yellow oil.

### Example 17: Dimethyl-{4-[4-(2-pyrrolidin-1-ylethoxy)phenyl]tetrahydro-pyran-4-ylmethyl}amine

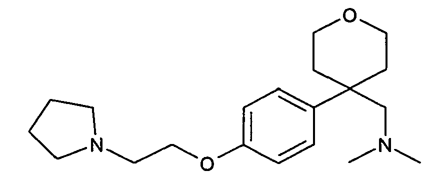

4-(4-Dimethylaminomethyl-tetrahydro-pyran-4-yl)-phenol (389mg, 1.65mmol), 1-(2-chloro-ethyl)-pyrrolidine (210mg, 1.57mmol), DMF (4.5ml) and K₂CO₃ (885g, 6.40mmol) was heated to 130°C for 30 minutes. The mixture was cooled to ambient temperature; water (9ml) was added and the mixture was extracted with EtOAc (3 x 4.5ml). The organic phase was washed with brine (10ml), NaOH solution (2M, 10ml) and water (10ml). The organic layer was dried over MgSO₄, filtered and concentrated *in vacuo* at 35°C and the residue subjected to chromatography on silica eluting with DCM:MeOH:NH3 (98:1:1) to give the title compound as a white solid (150mg, 27%).
¹H NMR (400MHz, CDCl₃) δ7.21 (d, 2H), 6.89 (d, 2H), 4.10 (t, 2H), 3.75 (dt, 2H), 3.55 (td, 2H), 2.89 (t, 2H), 2.67-2.58 (m, 4H), 2.40 (s, 2H), 2.13-2.05 (m, 2H), 1.97 (s, 6H), 1.88 (ddd, 2H), 1.84-1.77 (m, 4H).

### Intermediate 20: 1-(3-Chloro-2-methyl-propyl)-pyrrolidine

Pyrrolidine (3.0g, 42mmol), acetone (60ml), 5M NaOH solution (10ml) and 1-bromo-3-chloro-2-methylpropane (21.7g, 0.13mol) were reacted together according to general procedure A to give the title compound (3.9g, 58%) as a pale yellow oil.

### Example 18: Dimethyl-{4-[4-(2-methyl-3-pyrrolidin-1-yl-propoxy)-phenyl]-tetrahydro-pyran-4-ylmethyl}-amine

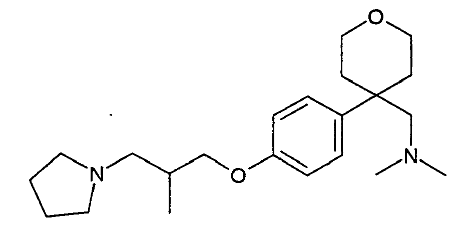

4-(4-Dimethylaminomethyl-tetrahydro-pyran-4-yl)-phenol (0.91g, 3.87mmol), 1-(3-chloro-2-methyl-propyl)-pyrrolidine (500mg, 3.10mmol), DMF (5ml) and K₂CO₃ (2.14g, 15.50mmol) were reacted together according to general procedure B. The organic phase was washed with 2M NaOH (3 x 20ml), water (2 x 20ml), dried over MgSO₄, fitered and concentrated *in vacuo* at 35°C. The crude material was subjected to chromatography on silica eluting with DCM :MeOH :NH₃ (97 :2 :1) to provide the title compound (464mg, 42%) as a pale yellow oil.
¹H NMR (400MHz, CDCl₃) δ 7.20 (d, 2H), 6.88 (d, 2H), 4.00 (dd, 1H), 3.80-3.70 (m, 3H), 3.55 (td, 2H), 2.59-2.43 (m, 5H), 2.40 (s, 2H), 2.33 (dd, 1H), 2.21-2.04 (m, 3H), 1.97 (s, 6H), 1.88 (ddd, 2H), 1.82-1.70 (m, 4H), 1.08 (d, 3H).

### Intermediate 21: Methanesulfonic acid 1-isopropyl-piperidin-4-yl ester

### Step 1:

4-Hydroxypiperidine (2.13g, 21.1mmol), K₂CO₃ (5.83g, 2eq.), 2-bromopropane (11.2g, 91mmol, 4.3eq.) and MeOH (21.3ml) were refluxed together overnight. The reaction was allowed to cool to room temperature and quenched with 2M HCl solution (40ml) and extracted with TBME (40ml). The aqueous phase was basified to pH 14 with 2M NaOH solution and extracted with DCM (9 x 50ml). The combined organic extracts were dried over MgSO₄, filtered, washed with DCM and concentrated *in vacuo* to give 1-isopropyl-4-hydroxypiperidine (2.41g, 80%) as a pale yellow oil.

### Step 2:

1-Isopropyl-4-hydroxypiperidine (1g, 6.98mmol), DCM (10ml) and triethylamine (1.08ml, 7.68mmol) were cooled to 0-5°C and mesyl chloride (0.54ml, 6.98mmol) was added dropwise under N₂. The reaction was allowed to warm to room temperature and stirred for 1hour. The reaction was quenched with sat. NaHC0₃ (20ml) and the aqueous phase extracted with DCM (2 x 5ml). The combined organic extracts were dried over MgSO₄, filtered, washed with DCM and concentrated *in vacuo* to give the title compound (1.55g, 100%) as a pale yellow oil.

### Example 19: {4-[4-(1-Isopropylpiperidin-4-yloxy)phenyl]tetrahydropyran-4-ylmethyl}dimethyl-amine

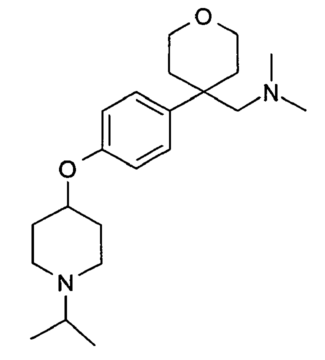

4-(4-Dimethylaminomethyl-tetrahydro-pyran-4-yl)-phenol (532mg, 2.26mmol) in DMF (2ml) was added to a solution of NaH (100mg, 2.5mmol) in DMF (2ml) at room temperature under N₂. The reaction was stirred for 1 hr and a solution of methanesulfonic acid 1-isopropyl-piperidin-4-yl ester (400mg, 1.81mmol) in DMF (1.3ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo.* The crude reaction was purified by column chromatography, eluting with DCM :MeOH : NH₃ (98 :1 :1) to give the title compound as a white crystalline solid (113mg, 17%).
¹H NMR (400MHz, CDCl₃) δ 7.20 (d, 2H), 6.87 (d, 2H), 4.27 (m, 1H), 3.75 (dt, 2H), 3.55 (td, 2H), 2.85-2.68 (m, 3H), 2.45-2.32 (m, 2H), 2.40 (s, 2H), 2.14-1.94 (m, 4H), 1.96 (s, 6H), 1.93-1.75 (m, 4H), 1.06 (d, 6H).

### Intermediate 22: Methanesulfonic acid 1-cyclopentyl-piperidin-4-yl ester

### Step 1:

4-Hydroxypiperidine (2.08g, 21.2mmol), K₂CO₃ (5.86g, 2eq.), cylopentylbromide (11.51g, 77.6mmol, 3.7eq.) and MeOH (20.8ml) were refluxed together overnight. The reaction was allowed to cool to room temperature and quenched with 2M HCl solution (40ml) and extracted with TBME (40ml). The aqueous phase was basified to pH 14 with a 2M NaOH solution and extracted with DCM (4 x 50ml). The combined organic extracts were dried over MgSO₄, filtered, washed with DCM and concentrated *in vacuo* to give 1-cyclopentyl-4-hydroxypiperidine (2.55g, 71 %) as a pale yellow oil.

### Step 2:

1-Cyclopentyl-4-hydroxypiperidine (1g, 5.91mmol), DCM (10ml) and triethylamine (0.91m, 6.5mmol) were cooled to 0-5°C and mesyl chloride (0.46ml, 5.91 mmol) was added dropwise under N₂. The reaction was allowed to warm to room temperature and stirred for 1 hour. The reaction was quenched with sat. NaHCO₃ (20ml) and the aqueous phase extracted with DCM (2 x 5ml). The combined organic extracts were dried over MgSO₄, filtered, washed with DCM and concentrated *in vacuo* to give the title compound (1.38g, 95%) as a pale yellow oil.

### Example 20: {4-[4-(1-Cyclopentylpiperidin-4-yloxy)phenyl]tetrahydropyran-4-ylmethyl}dimethylamine

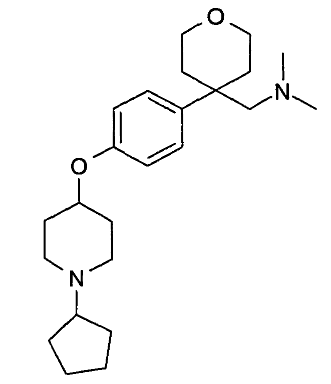

4-(4-Dimethylaminomethyl-tetrahydro-pyran-4-yl)-phenol (487mg, 2.07mmol) in DMF (2ml) was added to a solution of NaH (100mg, 2.5mmol) in DMF (2ml) at room temperature under N₂. The reaction was stirred for 1 hr and a solution of methanesulfonic acid 1-cyclopentyl-piperidin-4-yl ester (409mg, 1.65mmol) in DMF (1.5ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo.* The crude reaction was purified by column chromatography, eluting with DCM :MeOH : NH₃ (98:1 :1) to give the title compound as a white solid (78mg, 12%).
¹H NMR (400MHz, CDCl₃) δ 7.20 (d, 2H), 6.87 (d, 2H), 4.28 (m, 1H), 3.75 (dt, 2H), 3.55 (td, 2H), 2.89-2.76 (m, 2H), 2.52 (p, 1H), 2.40 (s, 2H), 2.37-2.25 (m, 2H), 2.14-1.94 (m, 4H), 1.96 (s, 6H), 1.93-1.77 (m, 6H), 1.76-1.34 (m, 6H).

### Example 21: {3-[4-(4-Dimethylaminomethyl-tetrahydro-pyran-4-yl)-phenoxy]-propyl}-isopropylamine

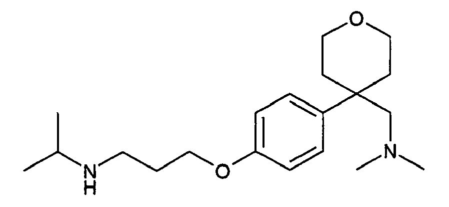

4-(4-Dimethylaminomethyl-tetrahydro-pyran-4-yl)-phenol (730mg, 3.10mmol), 3-chloro-1-bromopropane (980mg, 6.20mmol), DMF (10ml) and K₂CO₃ (1.72g, 12.40mmol) were reacted together according to general procedure B. Purification by chromatography on silica, eluant DCM:MeOH (20: 1) provided a mixture of bromo and chloro phenoxy ether (250mg) which was used in the next stage. The mixture (250mg) was refluxed with isopropyl amine (34°C) (10ml, 40vol) for 7 days. The reaction was concentrated *in vacuo* and partitioned between 2M NaOH (10ml) and DCM (10ml). The aqueous phase was extracted with DCM (3 x 10mL). The combined DCM layers were dried over MgSO₄, filtered, washed with DCM and concentrated *in vacuo.* Purification by chromatography on silica, eluant DCM :MeOH :NH₃ (95 :5 :0) increasing gradually to DCM :MeOH :NH₃ (90 :9 :1) gave the title compound as a pale yellow solid (180mg, 17% over the 2 steps).
¹H NMR (400MHz, CDCl₃) δ 7.20 (d, 2H), 6.88 (d, 2H), 4.03 (t, 2H), 3.75 (dt, 2H), 3.59-3.50 (m, 2H), 2.89-2.76 (m, 3H), 2.40 (s, 2H), 2.15-2.05 (m, 2H), 2.03-1.93 (m, 2H), 1.96 (s, 6H), 1.88 (ddd, 2H), 1.49 (br s, 1H), 1.07 (d, 6H).

### Example 22: Dimethyl-{4-[4-(4-pyrrolidin-1-ylbutoxy)phenyl]tetrahydro-pyran-4-ylmethyl}amine

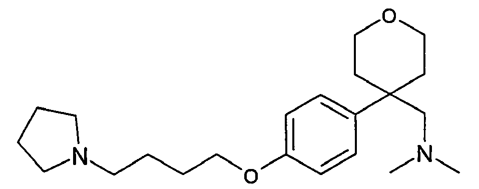

4-(4-Dimethylaminomethyl-tetrahydro-pyran-4-yl)-phenol (1.5g, 6.38mmol), 1-bromo-4-chlorobutane (1.5ml, 12.77mmol), DMF (20ml) and K₂CO₃ (3.5g, 25.53mmol) were reacted together according to general procedure B. Purification of a fraction of the crude (900mg) by chromatography on alumina, eluant ethyl acetate:heptanes (12: 88) provided a mixture of bromo and chloro phenoxy ether (220mg) which was used in the next stage. The purified mixture (220mg, 0.68mmol) and pyrrolidine (0.17ml, 2.03mmol) were refluxed in EtOH (3ml, 15vol) overnight. The reaction was concentrated *in vacuo* and partitioned between 2M NaOH (10ml) and ethyl acetate (10ml). The aqueous was extracted with ethyl acetate (3 x 5mL). The combined ethyl acetate layers were washed with brine (3 x 5ml), dired over MgSO₄, filtered, washed with ethyl acetate and concentrated *in vacuo.* Purification by chromatography on silica, eluant 95:3:2 (DCM: MeOH: NH₃) followed by a second column eluting with DCM :MeOH :NH₃ (96.5 :3 :0.5), gave the title compound as a colourless oil (134mg, 55%).
¹H NMR (400MHz, CDCl₃) δ 7.21 (d, 2H), 6.86 (d, 2H), 3.97 (t, 2H), 3.75 (dt, 2H), 3.59-3.50 (m, 2H), 2.57-2.45 (m, 6H), 2.40 (s, 2H), 2.14-2.05 (m, 2H), 1.96 (s, 6H), 1.92-1.65 (m, 10H).

### General procedure C:

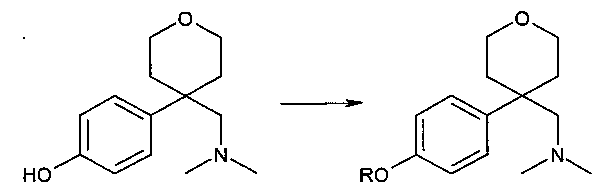

4-(4-Dimethylaminomethyl-tetrahydro-pyran-4-yl)-phenol (1eq.), alcohol (R-OH) (0.8eq.) and PPh₃ (1eq.) were mixed together in THF (10vol) and cooled to 0°C under N₂. DIAD (1eq.) in THF (10vol) was added slowly to the reaction and allowed to warm to room temperature overnight. The reaction was quenched with 2M HCl solution (10vol) and extracted with ethyl acetate (3 x 10vol). The aqueous phase was basified to pH 14 with NaOH (~30vol) and extracted with ethyl acetate (3 x 10vol). The organic phase was dried over MgSO₄, filtered and concentrated *in vacuo* at 35°C. Purification of the crude material by chromatography on silica, eluant (10%MeOH in DCM) provided the title compounds.

### Intermediate 23: 2,2-Dimethyl-3-pyrrolidin-1-yl-propan-1-ol

Pyrrolidine (1.82g, 25mmol), K₂CO₃ (3.0g, 1.04eq.), 3-bromo-2,2-dimethyl-propan-1-ol (8.45g, 50mmol, 2eq.) were heated together at 90°C overnight. The reaction was allowed to cool to room temperature and quenched with 2M HCl solution (50ml) and extracted with TBME (3 x 50ml). The aqueous phase was basified to pH 14 with 2M NaOH solution and extracted with DCM (3 x 50ml). The combined organic extracts were dried over MgSO₄, filtered, washed with DCM and concentrated *in vacuo* to give the title compound (1.03g, 26%) as a pale yellow oil.

### Example 23: {4-[4-(2,2-Dimethyl-3-pyrrolidin-1-yl-propoxy)-phenyl]-tetra-hydropyran-4-ylmethyl}-dimethyl-amine

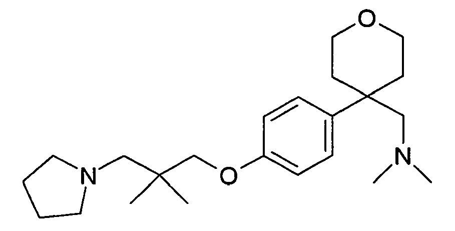

4-(4-Dimethylaminomethyl-tetrahydro-pyran-4-yl)-phenol (0.28g, 1.19mmol), 2,2-dimethyl-3-pyrrolidin-1-yl-propan-1-ol (0.15g, 0.96mmol), PPh₃ (0.31 g, 1.19mmol), THF (3ml) and DIAD (0.24ml, 1.19mmol) were reacted together according to general procedure C. The crude material was subjected to chromatography on silica eluting with DCM :MeOH :NH₃ (97 :2 :1) to provide the title compound (85mg, 24%) as an off-white solid.
¹H NMR (400MHz, CDCl₃) δ 7.23 (d, 2H), 6.91 (d, 2H), 3.84-3.69 (m, 4H), 3.69-3.54 (m, 2H), 2.67-2.56 (m, 4H), 2.50 (s, 2H), 2.44 (s, 2H), 2.18-2.08 (m, 2H), 2.01 (s, 6H), 1.92 (ddd, 2H), 1.79-1.67 (m, 4H), 1.04 (s, 6H).

### Intermediate 24: 4-Pyrrolidin-1-yl-butan-2-ol

Pyrrolidine (2ml, 24mmol), THF (20ml) and methyl vinylketone (2.5ml, 31mmol) were mixed together and stirred overnight. NaBH₄ (1.17g, 31mmol) was added to the reaction mixture and stirred for 3hours. The reaction was quenched with water (20ml) and extratcted with TBME (2 x 20ml). The combined organic extracts were dried over MgSO₄, filtered, washed with TBME and concentrated *in vacuo.* The crude product was purified by column chromatography, eluting with DCM :MeOH :NH₃ (94 :5 :1), to give the title compound as a pale yellow oil (1.3g, 38%).

### Example 24: Dimethyl-{4-[4-(1-methyl-3-pyrrolidin-1-ylpropoxy)phenyl]-tetrahydropyran-4-ylmethyl}amine

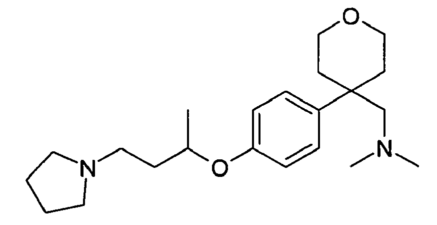

4-(4-Dimethylaminomethyl-tetrahydro-pyran-4-yl)-phenol (1.03g, 4.40mmol), 4-pyrrolidin-1-yl-butan-2-ol (0.5g, 3.50mmol), PPh₃ (1.15g, 4.40mmol), THF (10ml) and DIAD (0.86ml, 4.40mmol) were reacted together according to general procedure C. The crude product was purified by preparative HPLC, eluting with acetonitrile/water/0.1%TFA as a gradient, to give the title compound as a yellow oil (125mg, 10%).
¹H NMR (400MHz, CDCl₃) δ 7.19 (d, 2H), 6.86 (d, 2H), 4.43 (m, 1H), 3.75 (dt, 2H), 3.60-3.51 (m, 2H), 2.67-2.46 (m, 6H), 2.40 (s, 2H), 2.14-2.04 (m, 2H), 1.97 (s, 6H), 1.93-1.73 (m, 8H), 1.31 (d, 3H).

### Intermediate 25: 3-Pyrrolidin-1-yl-butan-1-ol

Pyrrolidine (28.15g, 0.4mol), toluene (200ml), catalytic p-TsOH (200mg) and ethyl acetoacetate (20g, 0.15mol) were refluxed together in a Dean-Stark apparatus under N₂ for 3hours. The reaction was cooled to room temperature and concentrated *in vacuo.* NaBH₄ (3.1g, 82mmol) was dissolved in MeOH (50ml) and cooled to 0-5°C. A portion of the previous crude reaction (5g, 27mmol) in MeOH (25ml) was added to the reaction mixture and stirred for 72hours. The reaction was quenched with an aqueous solution of NaOH (1%w/w, 50ml) and concentrated *in vacuo.* The aqueous phase was extracted with TBME (3 x 50ml). The combined organic extracts were dried over MgSO₄, filtered, washed with TBME and concentrated *in vacuo* to give a mixture of the title compound, 3-pyrrolidin-1-yl-butanoic acid ethyl ester and residual TBME (3.5g). The mixture (3.5g) in THF (20ml) was added to a solution of LiALH₄ (1 M in THF, 33.5ml, 33.5mmol) at 0-5°C under N₂. The reaction was allowed to warm to room temperature and stirred for 3hours. The reaction was quenched with an aqueous solution of NaOH (1%w/w, 50ml) at 0-5°C, filtered and washed with THF (3 x 20ml). The combined organic layers were dried over MgSO₄, filtered, washed with THF and concentrated *in vacuo* to give the title compound as a yellow oil (2g, 73%).

### Example 25: Dimethyl-{4-[4-(3-pyrrolidin-1-yl-butoxy)-phenyl]-tetrahydro-pyran-4-ylmethyl}-amine

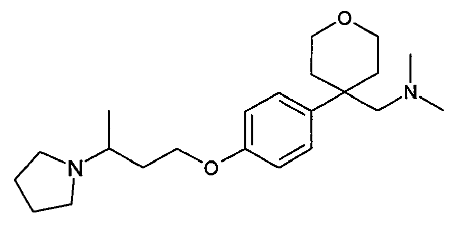

4-(4-Dimethylaminomethyl-tetrahydro-pyran-4-yl)-phenol (925mg, 3.93mmol), 3-pyrrolidin-1-yl-butan-1-ol (450mg, 3.14mmol), PPh₃ (1.03g, 3.93mmol), THF (9ml) and DIAD (0.77ml, 3.93mmol) were reacted together according to general procedure C. The crude material was subjected to chromatography on silica eluting with DCM :MeOH (99.5 :0.5) to provide the title compound (268mg, 24%) as a colourless oil. ¹H NMR (400MHz, CDCl₃) δ 7.21 (d, 2H), 6.88 (d, 2H), 4.11-3.97 (m, 2H), 3.75 (dt, 2H), 3.55 (td, 2H), 2.66-2.52 (m, 5H), 2.40 (s, 2H), 2.19-2.05 (m, 3H), 1.96 (s, 6H), 1.93-1.70 (m, 7H), 1.16 (d, 3H).

### Example 26: Dimethyl-(4-{4-[2-(1-methylpyrrolidin-2-yl)ethoxy]phenyl}-tetrahydropyran-4-ylmethyl)amine

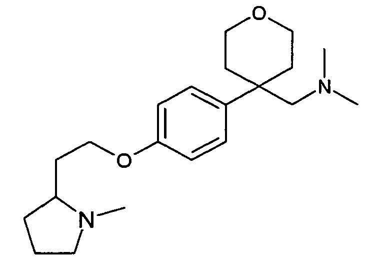

4-(4-Dimethylaminomethyl-tetrahydro-pyran-4-yl)-phenol (405mg, 1.72mmol), 2-(1-methyl-pyrrolidin-2-yl)-ethanol (178mg, 1.38mmol), PPh₃ (451mg, 1.72mmol), THF (8ml) and DIAD (348mg, 1.72mmol) were reacted together according to general procedure C. The crude product was subjected to chromatography on alumina eluting with ethyl acetate :heptane (50 :50) to provide the title compound (130mg, 27%) as a colourless oil.
¹H NMR (400MHz, CDCl₃) δ 7.21 (d, 2H), 6.87 (d, 2H), 4.09-3.95 (m, 2H), 3.75 (dt, 2H), 3.55 (td, 2H), 3.09 (m, 1H), 2.40 (s, 2H), 2.35 (s, 3H), 2.30-1.99 (m, 6H), 1.96 (s, 6H), 1.93-1.63 (m, 6H).

### Example 27: 4-[4-(3-Pprrolidin-1-yl-propoxy)-phenyl]-tetrahydro-pyran-4-carbonitrile

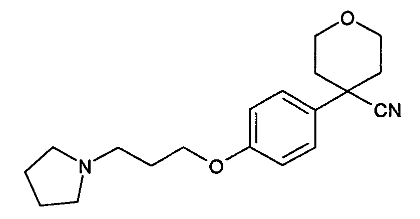

4-(4-Hydroxy-phenyl)-tetrahydro-pyran-4-carbonitrile (2g, 9.86mmol), 1-(3-chloro-propyl)-pyrrolidine (2.33g, 15.78mmol), DMF (40ml) and K₂CO₃ (5.45g, 39.44mmol) were reacted together according to general procedure E. The crude reaction product was diluted with 2M HCl solution (200ml) and washed with ethyl acetate (3 x 50ml). The aqueous phase was basified to pH 14 with 2M NaOH (200ml) and extracted with ethyl acetate (4 x 50ml). The combined organic extracts were dried over MgSO₄, filtered, washed with ethyl acetate and concentrated *in vacuo* to give a white solid. The solid was slurried in TBME (15ml) and heptane (15ml), filtered, washed with heptane (8ml) and dried on the filter for 2 hours to give the title compound as a white solid (1.6g, 53%).
¹H NMR (400MHz, DMSO-d₆) δ 7.43 (d, 2H), 6.99 (d, 2H), 4.08-3.94 (m, 4H), 3.64 (dt, 2H), 2.57-2.47 (m, 2H), 2.46-2.36 (m, 4H), 2.12-1.94 (m, 4H), 1.87 (p, 2H), 1.73-1.62 (m, 4H).

Alternatively, 4-[4-(3-Pyrrolidin-1-yl-propoxy)-phenyl]-tetrahydro-pyran-4-carbonitrile can be prepared by the following steps:

### Step 1:

4-Hydroxybenzylcyanide (20 g, 0.15 mol) was dissolved in DMF (500 mL) and then K₂CO₃ (41.4 g, 0.3 mol) and Nal (7.8 g, 0.052 mol) were added followed by dropwise addition of 3-(1-pyrrolidine)-propylchloride (26.5 g, 0.18 mol) at 60 °C. Heating was continued at 90 °C for 3 h and then the mixture was heated overnight at 60 °C. 3-(1-Pyrrolidine)-propylchloride (10 g, 0.067 mol) was again added as the reaction was not complete and heating was continued for a further 6 h. The reaction mixture was concentrated and taken up in EtOAc. The organic layer was washed with water, finally with brine. Crude [4-(3-pyrrolidin-1-ylpropoxy)phenyl]acetonitrile was purified by DCM/MeOH 9.5/0.5 over a silica gel column. Yield: 25.5g (70 %).
¹H-NMR (400 MHz, CDCl₃) δ 1.77 (m, 4H); 1.98 (m, 2H); 2.50 (m, 4H); 2.59 (t, 2H); 3.66 (s, 2H); 4.00 (t, 2H); 6.87(d, 2H); 7.19(d, 2H).

### Step 2:

NaH (14.6 g, 0.61 mol) was suspended in DMF (100 mL). [4-(3-Pyrrolidin-1-ylpropoxy)phenyl]acetonitrile (25.5 g, 0.10 mol) was dissolved in DMF (100 mL) and added dropwise to the ice-cooled suspension of NaH. The reaction mixture was stirred at room temperature for 30 min followed by dropwise addition of 2-bromoethylether (36.2 g, 0.16 mol) in DMF (200 mL) in ice-cold condition. The reaction mixture was poured into ice-cold water. The solid was filtered, washed with water, dried and the resulting solid was washed with 5 % EtOAc in hexane to give the title compound (22 g, 67 %).

### Example 28: {4-[4-(3-Pyrrolidin-1-ylpropoxy)phenyl]tetrahydropyran-4-yl} methylamine dihydrochloride

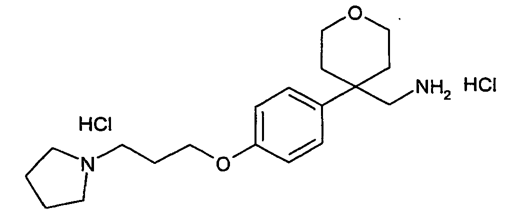

To a stirred suspension of LiAlH₄ (1.81g, 47.7mmol, 5eq) in Et₂O/DCM (1:1, 30mL) at 0 to 5°C under a nitrogen atmosphere was added 4-[4-(3-pyrrolidin-1-yl-propoxy)-phenyl]-tetrahydro-pyran-4-carbonitrile (3g, 9.55 mmol) in Et₂O/DCM (1:1, 30mL) over 15 minutes maintaining the temperature at 5 to 10°C. The reaction mixture was allowed to warm up to ambient temperature and stirred until complete. Sodium hydroxide (2N, 15mL) was added dropwise and the resulting solids were filtered, washed with Et₂O/DCM (1:1, 6 x 50mL) and concentrated *in vacuo* at 40°C. A portion of the crude product from the above reaction (0.5 g, 1.57 mmol) was dissolved in dioxane (5mL) and 4M HCl in dioxane (1.18mL) added under N₂. The reaction was stirred for 3 hours and filtered under a blanket of N₂ (hygroscopic), washed with TBME and dried in the oven at 40°C to give the title compound as a white solid (0.5g, 81 %).
¹H NMR (400MHz, DMSO-*d₆*) δ 11.24 (br s, 1H), 8.00 (br s, 3H), 7.33 (d, 2H), 6.98 (d, 2H), 4.08 (t, 2H), 3.76-3.63 (m, 2H), 3.60-3.46 (m, 2H), 3.45-3.18 (m, 4H), 3.07-2.87 (m, 4H), 2.25-1.77 (m, 10H).

### Example 29: Dimethyl-{4-[4-(3-pyrrolidin-1-ylpropoxy)phenyl]-tetrahydro-pyran-4-ylmethyl}amine

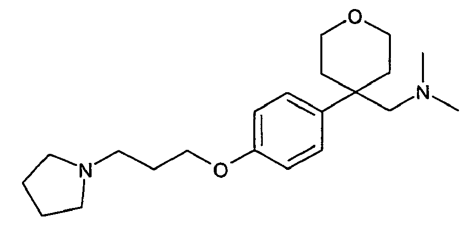

To a mixture of {4-[4-(3-pyrrolidin-1-ylpropoxy)phenyl]tetrahydropyran-4-yl} methylamine (2.5g, 7.86 mmol), acetic acid (6ml), and water (12.5ml) was added formaldehyde (11.5ml) and stirred for 15 minutes. STAB (10g, 4.72mmol) was added portionwise and stirred for one hour. Sodium hydroxide (2M, 100ml) was added slowly to attain a pH of 9. The resulting mixture was extracted with DCM (3x100ml) and the combined organic phases were dried over MgSO₄, filtered and concentrated *in vacuo* at 35°C. The crude mixture was purified by column chromatography on silica eluting with DCM:MeOH:NH₃ (95:4:1) to give the title compound as a white solid (1.09g, 40%).
¹H NMR (400MHz, CDCl₃) δ 7.21 (d, 2H), 6.87 (d, 2H), 4.02 (t, 2H), 3.75 (dt, 2H), 3.55 (td, 2H), 2.65 (t, 2H), 2.56 (br s, 4H), 2.40 (s, 2H), 2.15-1.74 (m, 10H), 1.97 (s, 6H).

### Example 30: 4-[4-(3-Piperidin-1-yl-propoxy)-phenyl]-tetrahydro-pyran-4-carbonitrile

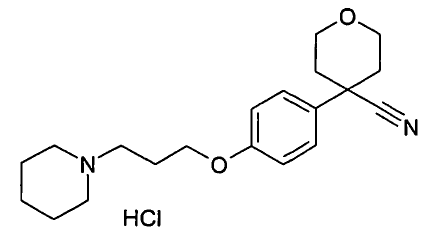

A mixture of 4-(4-hydroxyphenyl)tetrahydro-2H-pyran-4-carbonitrile ( 1.0 g, 5 mmol), 1-(3-chloropropyl)piperidine ( 1.2 g, 7.5 mmol) obtained following the procedure described in the *Patent* DD 60557, cesium carbonate ( 2.4g, 7.5 mmol), KI ( 1.2 g, 7.5 mmol) in DMF (50 mL) was heated to 70°C overnight. After DMF removal under reduced pressure, the residue was quenched with water and extracted with DCM. The organic phases were dried over Na₂SO₄, filtered and concentrated. The residue was purified by flash chromatography (50 g silica gel, DCM / MeOH) to provide the title compound (1.45 g, 88%).
The hydrochloride salt as analytical sample was prepared with ether/HCl 2M in dichloromethane and crystallization in ether.
¹H NMR (500MHz, CDCl₃) *δ* 12.14 (bs, 1H), 7.32 (d, 2H), 6.83(d,2H), 4.07 -3.97 (m, 4H), 3.85 -3.78 (m, 2H), 3.57-3.48 (m, 2H), 3.14-3.07 (m, 2H), 2.67 - 2.56 (m, 2H), 2.45 - 2.36 (m, 2H), 2.32 - 2.19 (m, 2H), 2.06 - 1.92 (m, 4H), 1.90-1.75 (m, 4H), 1.43-1.32 (m, 1H).

### Example 31: {4-[4-(3-Piperidin-1-ylpropoxy)phenyl]tetrahydropyran-4-yl} methylamine dihydrochloride

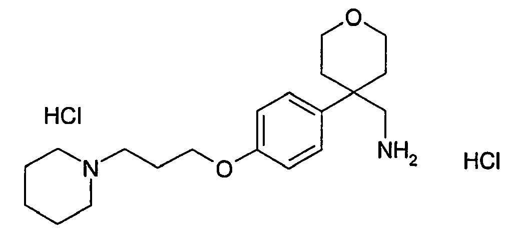

A mixture of 4-[4-(3-piperidin-1-yl-propoxy)-phenyl]-tetrahydro-pyran-4-carbonitrile (1.45 g, 4.4 mmol), isopropanol (12 mL), concentrated HCl (350µL), and PtO₂ (115 mg) was stirred and hydrogenated to 50°C overnight at atmospheric pressure. The mixture was filtrated over diatomaceous earth and concentrated. The residue was dissolved in DCM /MeOH mixture and washed with NaOH (1M). The organic layers were dried over Na₂SO₄, filtered and concentrated, purified by flash chromatography (10 g silica gel, DCM/ MeOH (10% NH₃)) to provide the title compound as free base (0.650 g, 44.5%). This was then converted to the dihydrochloride salt.
¹H NMR (400MHz, DMSO-D6) δ 10.66 (bs, 1H), 7.74 (bs, 3H), 7.32 (d, 2H), 6.97 (d, 2H), 4.07 (t, 2H), 3.73-3.65 (m, 2H), 3.47-3.28 (m, 4H), 3.18-3.10 (m, 2H), 2.98-2.81 (m, 4H), 2.25-2.16 (m, 2H), 2.14-2.06 (m, 2H), 1.90-1.67 (m, 7H), 1.44-1.31 (m, 1H).

### Example 32: Dimethyl-{4-[4-(3-piperidin-1-ylpropoxy)phenyl]tetrahydro-pyran-4-ylmethyl}amine dihydrochloride

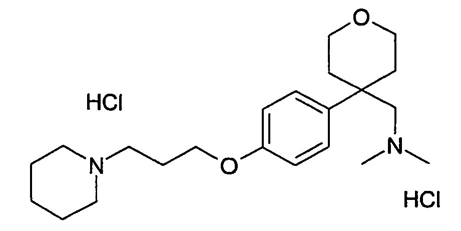

To (0.50 g, 1.5 mmol) of {4-[4-(3-Piperidin-1-ylpropoxy)phenyl]tetrahydropyran-4-yl} methylamine dihydrochloride was added 1.5 mL of formaldehyde (37% w/w solution in H₂O). 1.9 mL of formic acid was added and the mixture was heated to 100°C for 1.5 h. After cooling, water was added and the mixture extracted with ether. NaOH was added to the aqueous phase and the mixture extracted 3 times with ether. The organic extracts were washed with brine, dried over Na₂SO₄ filtered and concentrated *in vacuo.* The residue was purified by flash chromatography (25 g silica gel, DCM/ MeOH/ 10% NH₃). This was then converted to the dihydrochloride salt.
¹H NMR (400MHz, DMSO-D6) δ 10.63 (bs, 1H), 9.73 (bs, 1H), 7.43 (d, 2H), 6.99(d, 2H), 4.11-4.03 (t, 2H), 3.76-3.67 (m, 2H), 3.47-3.29 (m, 7H), 3.19-3.11 (m, 2H), 2.92-2.81 (m, 2H), 2.41-2.35 (m, 5H), 2.32-2.16 (m, 4H), 1.95-1.66 (m, 7H), 1.45-1.32 (m, 1H).

### Intermediate 26: 1-(4-hydroxyphenyl)cyclohexanecarbonitrile

To a solution of 1-(4-methoxyphenyl)cyclohexanecarbonitrile (2 g, 9.29 mmol) in DCM (40 mL) at 0°C under N₂ was added dropwise BBr₃ (2.63 mL, 27.87 mmol). The reaction was allowed to warm to room temperature and stirred for 48hours. The mixture was stirred for 3 hours at 0°C and quenched with water (30 mL). The mixture was separated and the organic layer was washed with a saturated aqueous solution of NaHCO₃ then water. The combined organic extracts were dried over Na₂SO₄, filtered, concentrated *in vacuo* and purified by column chromatography on silica gel eluting with DCM:acetone (97:3) to give the title compound (1.71 g, 91%).

### Example 33: 1-[4-(3-pyrrolidin-1-ylpropoxy)phenyl]cyclohexanecarbonitrile

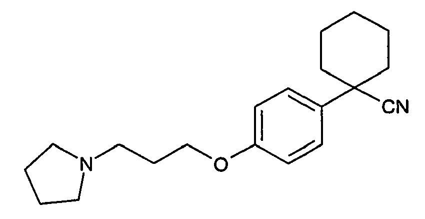

1-(4-Hydroxyphenyl)cyclohexanecarbonitrile (1.70 g, 8.45 mmol), 1-(3-chloro-propyl)-pyrrolidine (1.87 g, 12.67 mmol), acetone (50 mL), sodium iodide (0.444 g, 2.96 mmol) and K₂CO₃ (5.45g, 39.44 mmol) were heated to 50°C overnight. The solvent was evaporated and the crude product was taken into DCM, washed with 0.5N NaOH, extracted with 0.5N HCl solution and washed with DCM. The aqueous layer was basified with 0.5N NaOH and extracted with DCM. The combined organic extracts were dried over Na₂SO₄, filtered, washed with DCM and concentrated *in vacuo* to give a yellowish solid. The solid was slurried in ether, filtered, washed with ether to give the title compound as a white solid (1.34 g, 51 %).
¹H NMR (400MHz, CDCl₃) δ 7.30 (d, 2H), 6.83 (d, 2H), 3.95 (t, 2H), 2.54 (t, 2H), 2.44 (m, 4H), 2.06 (m, 2H), 1.92 (m, 2H), 1.84-1.58 (m, 12H).

### Example 34: N-({1-[4-(3-pyrrolidin-1-ylpropoxy)phenyl]cyclohexyl}methyl)-N,N-dimethylamine

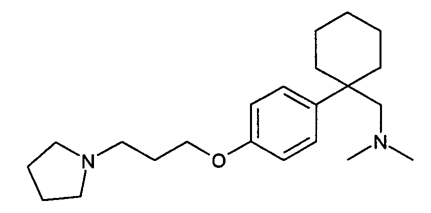

### Step 1:

To a stirred suspension of LiAlH₄ (0.607 g, 16 mmol) in diethyl ether (15 mL) cooled at 0°C was added dropwise a solution of 1-[4-(3-pyrrolidin-1-ylpropoxy)phenyl]cyclohexanecarbonitrile (1 g, 3.2 mmol) in diethyl ether (10 mL). After being stirred at room temperature for 30 min, the reaction mixture was refluxed for another 30 min. The mixture was cooled to 0°C and water (1.38 mL), a 5N aqueous solution of sodium hydroxide (1.38 mL) and water (5.52 mL) again were successively added dropwise. After being stirred for 15 min at room temperature, the mixture was filtered over diatomaceous earth and concentrated. The crude {1-[4-(3-pyrrolidin-1-ylpropoxy)phenyl]cyclohexyl}methylamine (0.961 g, 95%) was used in the next step without further purification.

### Step 2:

To {1-[4-(3-pyrrolidin-1-ylpropoxy)phenyl]cyclohexyl}methylamine (0.96 g, 3 mmol) was added formic acid (5 mL) followed by formaldehyde (37% w/w solution in H₂O, 0.915 mL, 33 mmol). The mixture was refluxed for 3 h and allowed to stir overnight at room temperature. It was then diluted with DCM, basified to pH 12 with an aqueous solution of NaOH and the mixture was extracted with DCM. The organic extracts were dried over Na₂SO₄, filtered, concentrated *in vacuo* and purified by column chromatography on silica gel eluting with a gradient of DCM:MeOH:NH₃ (from 99:0:1 to 90:10:1) to provide the title compound (0.447 g, 43%) as an oil.
¹H NMR (400MHz, CDCl₃) δ 7.18 (d, 2H), 6.78 (d, 2H), 3.94 (t, 2H), 2.55 (t, 2H), 2.45 (m, 4H), 2.22 (m, 2H), 2.06-1.86 (m, 10H), 1.72 (m, 4H), 1.58-1.19 (m, 8H).

### Intermediate 27: 4-[4-(3-pyrrolidin-1-ylpropoxy)phenyl]tetrahydro-2H-pyran-4-carbaldehyde

### Step 1:

A solution of 4-[4-(3-pyrrolidin-1-ylpropoxy)phenyl]tetrahydro-2*H-*pyran-4-carbonitrile (20g, 0.0636mol) in concentrated hydrochloric acid (200ml, 10vol) was heated and stirred at reflux for 16h after which time LC/LCMS analysis showed the reaction had stalled at 85-90% conversion. The solution was evaporated to dryness *in vacuo,* azeotroped with methanol (200ml, 20vol) and toluene (2x 200ml, 2x 20vol) to yield 4-[4-(3-pyrrolidin-1-ylpropoxy)phenyl]tetrahydro-2*H-*pyran-4-carboxylic acid as a beige solid which was used directly (no NMR data recorded).

### Step 2:

A suspension of 4-[4-(3-pyrrolidin-1-ylpropoxy)phenyl]tetrahydro-2*H-*pyran-4-carboxylic acid (23.5g, 0.0636mol) in methanol (235ml, 10vol) was stirred and cooled to 0 to -5°C under nitrogen. Thionyl chloride (9.3ml, 0.1272mol, 0.40vol) was charged dropwise over 20minutes, maintaining the temperature at -5 to +5°C. The resulting slurry was then heated and stirred at reflux under nitrogen for 16h after which time LC analysis showed 1.4% by area of acid remaining. The solution was evaporated to dryness *in vacuo* at 40°C to give a brown sludge. The sludge was dissolved in ethyl acetate (118min, 5vol) and water (235ml, 10vol) and the layers separated. The aqueous layer was washed with ethyl acetate (118ml, 5vol) and basified to pH 11 with saturated aqueous potassium carbonate (118ml, 5vol). The product was extracted into dichloromethane (3x 118ml, 3x 5vol) and the combined extracts dried over magnesium sulfate (23g) and concentrated *in vacuo* at 40°C to yield methyl 4-[4-(3-pyrrolidin-1-ylpropoxy)phenyl]tetrahydro-2*H-*pyran-4-carboxylate (21.7g, 98% from the nitrile) as a cream solid.

### Step 3:

Anhydrous tetrahydrofuran (141 ml, 10vol) was charged rapidly onto stirred and cooled (0-5°C) lithium aluminium hydride (6.2g, 0.1634mol, 0.44wt) under nitrogen. The resulting slurry was cooled to 0-5°C and then a solution of methyl 4-[4-(3-pyrrolidin-1-ylpropoxy)phenyl]tetrahydro-2*H-*pyran-4-carboxylate (14.1g, 0.04058mol) in tetrahydrofuran (99ml, 7vol) was added dropwise over 30 minutes maintaining 0-5°C. On complete addition the reaction was allowed to warm to room temperature over 30 minutes and stirred at 18-25°C for 30 minutes, LC analysis showed the reaction to be complete. The mixture was cooled to 0 to 5°C, a 1:1 mixture of tetrahydrofuran and water (18.6ml, 1.32vol) was added very slowly. The mixture was further quenched with 20% w/v sodium hydroxide solution (6.2ml, 0.44vol) and water (18.6m, 1.32vol) and the resulting white suspension stirred vigorously for 30 minutes at 18-25°C. The salts were removed by filtration and rinsed with tetrahydrofuran (3x 150ml). The combined filtrates were evaporated to dryness at 40°C to yield {4-[4-(3-pyrrolidin-1-ylpropoxy)phenyl]tetrahydro-2*H*-pyran-4-yl}methanol (12.1g, 94%) as a white solid.

### Step 4:

Pyridinium chlorochromate (20.3g, 0.09415mol), 1.69wt) was charged to a stirred suspension of celite (24g, 2wt) and {4-[4-(3-pyrrolidin-1-ylpropoxy)phenyl]tetrahydro-2*H-*pyran-4-yl}methanol (12.03g, 0.03766mol) in dichloromethane (120ml, 10vol) with stirring. The mixture was stirred at 18-25°C for 3.5 hours after which time LC analysis showed the reaction to be 70% complete. Magnesium sulphate (12g, 1wt) was added and the resulting slurry filtered through a pad of aluminium oxide (activated, basic, Brockmann I, standard grade, -150 mesh) (481g, 40wt) eluting with 1% methanol/dichloromethane. Clean fractions were combined and evaporated to dryness at 40°C. The resulting brown solid was stirred in *tert*butylmethylether (120ml, 10vol). Undissolved residue was removed by filtration and rinsed with *tert*butylmethylether (3x120ml, 3x 10vol). The combined filtrates were evaporated at 40°C to yield 4-[4-(3-pyrrolidin-1-ylpropoxy)phenyl]tetrahydro-2*H-*pyran-4-carbaldehyde (6.0g, 50.2%) as a beige waxy solid. Alternatively, 4-[4-(3-pyrrolidin-1-ylpropoxy)phenyl]tetrahydro-2*H-*pyran-4-carbaldehyde can be prepared as follows:
To a solution of 4-[4-(3-pyrrolidin-1-ylpropoxy)phenyl]tetrahydro-2H-pyran-4-carbonitrile (250 mg, 0.79 mmol) in anhydrous toluene (2 mL) at -78°C was added dropwise a 1.5M solution of diisobutyl aluminium hydride in toluene (1.59 mL, 2.38 mmol, 3 equiv). The reaction mixture was stirred for 2 h at -78°C. Ethyl acetate was then added followed by a saturated solution of sodium potassium tartrate and dichloromethane. After stirring at room temperature for 1h, the organic layer was separated. The aqueous layer was extracted three times with dichloromethane. The combined organic extracts were dried over sodium sulfate, filtered and concentrated under reduced pressure to give an 8:2 mixture of the desired aldehyde and starting nitrile as an orange oil.

### General procedure D:

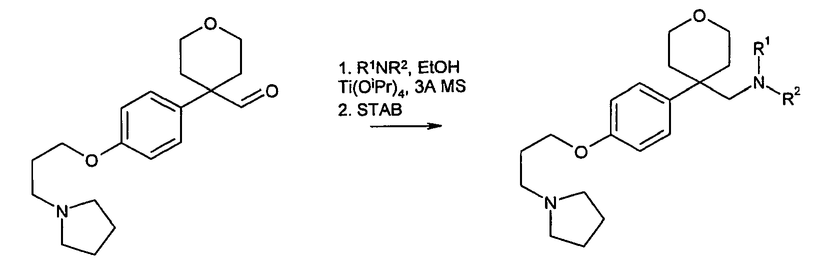

A solution of the 4-[4-(3-pyrrolidin-1-ylpropoxy)phenyl]tetrahydro-2*H-*pyran-4-carbaldehyde (700mg, 2.205mmol, 1wt) and secondary amine (NR¹R²) (4.21mmol) in absolute ethanol (28ml, 40vol) was stirred 18 to 25°C under nitrogen over activated 3A molecular sieves (700mg, 1wt). Titanium (IV) isopropoxide (3.25ml, 11.025mmol, 4.62vol) was added dropwise over 2 minutes. The resulting solution was stirred at 18 to 25°C under nitrogen for 20 to 24h, over which time precipitation was observed. Sodium triacetoxyborohydride (3.97g, 0.0187mol, 5.67wt) was then added in one portion (Note: not exothermic, but gentle gas evolution was observed) and the reaction was stirred for 1 to 8h until LC analysis showed the reaction to be either complete or stalled at 1-10% aldehyde by area). The reaction mixture was decanted from the sieves and evaporated to dryness *in vacuo* at 40°C. tert-Butylmethyl ether (28ml, 40vol), 50% w/v Rochelle's solution (28ml, 40vol) and sat. aqueous potassium carbonate solution (28ml, 40vol) were added and the mixture stirred vigorously for 1 to 2h until two layers were visible (the organic layer was clear, the aqueous cloudy). The layers were separated and the aqueous layer re-extracted with tert-butylmethyl ether (28ml, 40vol). The extracts were combined, washed with sat. aqueous sodium chloride solution (28ml, 40vol), dried over magnesium sulfate (700mg, 1wt) and evaporated *in vacuo* at 40°C to yield a viscous oil. The oil was purified by column chromatography on silica (20g, 28.6wt) eluting with DCM(95):MeOH(4):NH₃(1) to remove unreacted aldehyde, alcohol by-product (typically 1-10% by area) and the excess secondary amine to yield the pure product as a viscous oil contaminated by dichloromethane after evaporation. The oil was dissolved in ethanol (5ml, 7.1vol) and evaporated to dryness *in vacuo* at 40°C, then in a vacuum oven at 40°C for 12 to 24h to yield the required tertiary amine product as a viscous oil or waxy solid.

### Example 35: (1-{4-[4-(3-Pyrrolidin-1-ylpropoxy)phenyl]tetrahydropyran-4-ylmethyl}piperidin-4-yl)-methanol

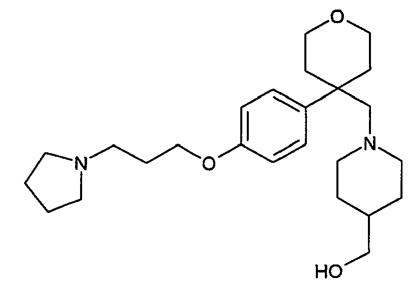

4-[4-(3-Pyrrolidin-1-ylpropoxy)phenyl]tetrahydro-2*H-*pyran-4-carbaldehyde (700mg, 2.205mmol, 1wt), 4-piperidinemethanol (485mg, 4.21mmol), absolute ethanol (28ml, 40vol), activated 3A molecular sieves (700mg), titanium (IV) isopropoxide (3.25ml, 11.03mmol) and STAB (3.97g, 18.7mmol) was reacted in accordance with the general procedure D. The isolated waxy solid was purified by column chromatography on silica eluting with DCM:MeOH:NH₃ 95:4:1 to give the title compound as a colorless oil (820mg, 72%).
¹H NMR (400MHz, CDCl₃) δ 57.20 (d, 2H), 6.85 (d, 2H), 4.02 (t, 2H), 3.75 (dt, 2H), 3.59-3.46 (m, 2H), 3.42 (d, 2H), 2.63 (t, 2H), 2.53 (br s, 4H), 2.39-2.26 (m, 4H), 2.15-1.03 (m, 18H).

### Example 36: 1-{4-[4-(3-Pyrrolidin-1-ylpropoxy)phenyl]tetrahydropyran-4-ylmethyl}piperidin-4-ol

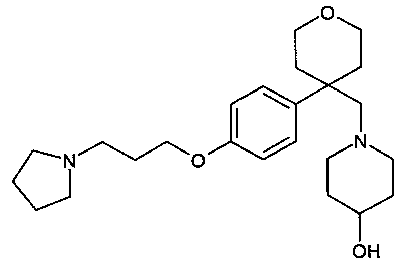

4-[4-(3-Pyrrolidin-1-ylpropoxy)phenyl]tetrahydro-2*H-*pyran-4-carbaldehyde (420mg, 1.30mmol, 1wt), 4-hydroxypiperidine (268mg, 2.60mmol), absolute ethanol (17ml, 40vol), activated 3A molecular sieves (420mg), titanium (IV) isopropoxide (1.94ml, 4.62vol) and STAB (2.34g, 11.1mmol) was reacted in accordance with the general procedure D. The isolated waxy solid was purified by preparative HPLC, eluting with acetonitrile/water/0.1%TFA as a gradient, to give the title compound as a white solid (380mg, 73%).
¹H NMR (400MHz, CDCl₃) δ 7.21 (d, 2H), 6.86 (d, 2H), 4.02 (t, 2H), 3.75 (dt, 2H), 3.59-3.47 (m, 2H), 2.63 (t, 2H), 2.53 (br s, 4H), 2.35 (s, 2H), 2.33-2.25 (m, 2H), 2.13-1.96 (m, 6H), 1.93-1.83 (m, 2H), 1.82-1.76 (m, 4H), 1.72-1.59 (m, 3H), 1.47-1.29 (m, 3H).

### Example 37: 1-{4-[4-(3-Pyrrolidin-1-yl-propoxy)-phenyl]-tetrahydro-pyran-4-ylmethyl}-piperidine-4-carboxylic acid amide

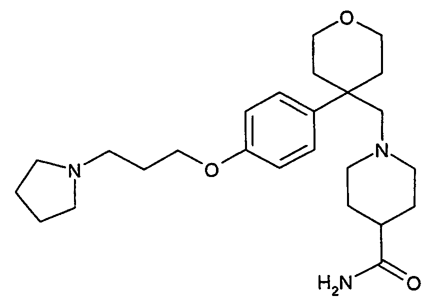

4-[4-(3-Pyrrolidin-1-ylpropoxy)phenyl]tetrahydro-2*H-*pyran-4-carbaldehyde (500mg, 1.58mmol), isonipecotamide (434mg, 3.03mmol), absolute ethanol (20ml), activated 3A molecular sieves (500mg), titanium (IV) isopropoxide (2.31ml, 7.88mmol) and STAB (2.84g, 13.6mmol) were reacted in accordance with the general procedure D. The isolated crude product was purified by column chromatography on silica eluting with DCM:MeOH:NH₃ (97:2:1) to give the title compound as a colorless solid (400mg, 59%).
¹H NMR (400MHz, CDCl₃) δ 7.20 (d, 2H), 6.85 (d, 2H), 5.45 (br s, 2H), 4.01 (t, 2H), 3.79-3.69 (m, 2H), 3.57-3.46 (m, 2H), 2.63 (t, 2H), 2.53 (br s, 4H), 2.35 (s, 2H), 2.31 (br s, 1H), 2.14-1.72 (m, 14H), 1.67-1.51 (m, 4H).

### Example 38: 1-Methyl-4-{4-[4-(3-pyrrolidin-1-yl-propoxy)-phenyl]-tetrahydro-pyran-4-ylmethyl}-piperazine

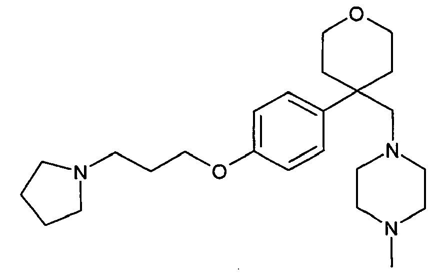

A solution of 4-[4-(3-pyrrolidin-1-ylpropoxy)phenyl]tetrahydro-2*H-*pyran-4-carbaldehyde (500mg, 1.58mmol, 1wt), 1-methylpiperazine (300mg, 2.99mmol), absolute ethanol (20ml, 40vol), activated 3A molecular sieves (500mg), titanium (IV) isopropoxide (2.32ml, 7.83mmol) and STAB (1.43g, 6.73mmol) were reacted in accordance with the general procedure D. The isolated oil was purified by column chromatography on silica (20g, 28.6wt) eluting with DCM(95):MeOH(4):NH₃(1) to give the title compound as a clear viscous oil (360mg, 56%).
¹H NMR (400MHz, CDCl₃) δ 7.21 (d, 2H), 6.86 (d, 2H), 4.02 (t, 2H), 3.75 (dt2H), 3.60-3.47 (m, 2H), 2.63 (t, 2H), 2.53 (br s, 4H), 2.38 (s, 2H), 2.34-2.14 (m, 11H), 2.13-1.73 (m, 10H).

### Example 39: 1-{4-[4-(3-Pyrrolidin-1-ylpropoxy)phenyl]tetrahydropyran-4-ylmethyl}piperazine

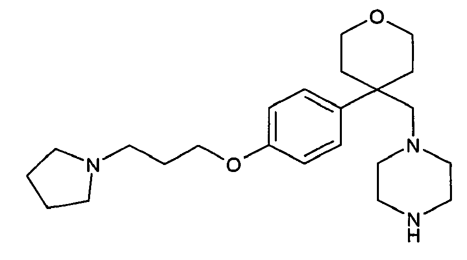

4-[4-(3-Pyrrolidin-1-ylpropoxy)phenyl]tetrahydro-2*H-*pyran-4-carbaldehyde (350mg, 1.103mmol), 4-piperazine-1-carboxylic acid tert-butyl ester (372mg, 1.98mmol), absolute ethanol (40ml), activated 3A molecular sieves (350mg), titanium (IV) isopropoxide (1.62ml, 5.48mmol) and STAB (2.0g, 9.74mmol) were reacted in accordance with the general procedure D. The isolated crude product was purified by column chromatography on silica eluting with DCM:MeOH:NH₃ (98:1:1) to give a white solid (295mg, 69%). This was re-dissolved in methanol (3.2ml) and 1,4-dioxane (8ml), HCl in dioxane (4M, 1.95ml) was added. The mixture was stirred overnight and concentrated *in vacuo* at 40°C. TBME (13ml) and NaOH (2M aqueous) were added to attain a pH of 12-14. The organic phase was separated and dried over MgSO₄ and concentrated *in vacuo* at 35°C. The crude product was subjected to chromatography on silica eluting with DCM:MeOH:NH₃ (94:4:2) to give the title compound as a yellow oil (100mg, 23%).
¹H NMR (400MHz, CDCl₃) δ 7.21 (d, 2H), 6.85 (d, 2H), 4.02 (t, 2H), 3.80-3.70 (m, 2H), 3.58-3.46 (m, 2H), 2.73-2.66 (m, 4H), 2.63 (t, 2H), 2.53 (br s, 4H), 2.34 (s, 2H), 2.17-1.48 (m, 15H).

### Example 40: 1-{4-[4-(3-Pyrrolidin-1-ylpropoxy)phenyl]tetrahydropyran-4-ylmethyl}pyrrolidin-3-ol

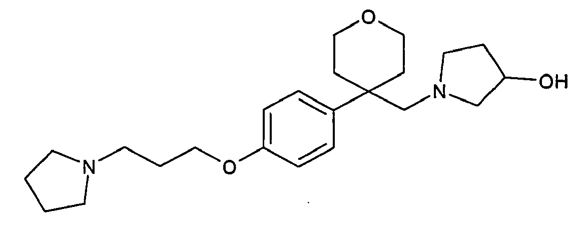

4-[4-(3-Pyrrolidin-1-ylpropoxy)phenyl]tetrahydro-2*H-*pyran-4-carbaldehyde (350mg, 1.103mmol), 3-pyrrolidinol (200mg, 2.02mmol), absolute ethanol (40ml), activated 3A molecular sieves (350mg), titanium (IV) isopropoxide (1.62ml, 5.48mmol) and STAB (2.0g, 9.74mmol) were reacted in accordance with the general procedure D. The isolated crude product was purified by column chromatography on silica eluting with DCM:MeOH:NH₃ (97:2:1) to give the title compound as a white solid (390mg, 91%).
¹H NMR (400MHz, CDCl₃) *δ* 7.20 (d, 2H), 6.87 (d, 2H), 4.10-4.01 (m, 3H), 3.81-3.68 (m, 3H), 3.55 (t, 2H), 2.67-2.48 (m, 9H), 2.28-1.61 (m, 15H).

### Example 41: (R)-2-Methoxymethyl-1-{4-[4-(3-pyrrolidin-1-yl-propoxy)-phenyl]-tetrahydro-pyran-4-ylmethyl}-pyrrolidine

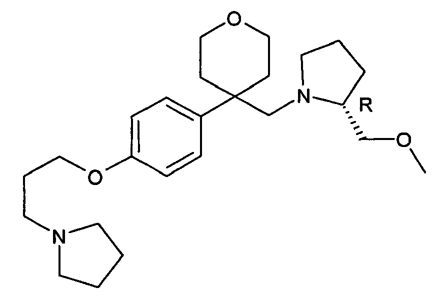

4-[4-(3-Pyrrolidin-1-ylpropoxy)phenyl]tetrahydro-2*H-*pyran-4-carbaldehyde (500mg, 1.575mmol), (R)-2-(methoxymethyl)pyrrolidine (347mg, 4.21mmol), absolute ethanol (20ml), activated 3A molecular sieves (500mg), titanium (IV) isopropoxide (2.33ml, 7.88mmol) and STAB (2.84g, 13.4mmol) were reacted in accordance with the general procedure D. The isolated waxy solid was purified by column chromatography on silica eluting with DCM:MeOH:NH₃ 95:14:1 to give the title compound as a viscous pale yellow oil (390mg, 59%).
¹H NMR (400MHz, CDCl₃) δ 7.18 (d, 2H), 6.86 (d, 2H), 4.02 (t, 2H), 3.85-3.69 (m, 2H), 3.59 (td, 1H), 3.45 (td, 1H), 3.27 (s, 3H), 3.10 (dd, 1H), 3.02 (dd, 1H), 2.94 (d, 1H), 2.63 (t, 2H), 2.58-2.45 (m, 6H), 2.39 (m,1H), 2.21 (m, 1H), 2.07-1.67 (m, 11H), 1.57-1.33 (m, 3H).

### Example 42: (S)-2-Methoxymethyl-1-{4-[4-(3-pyrrolidin-1-ylpropoxy)phenyl]tetrahydropyran-4-ylmethyl}pyrrolidine

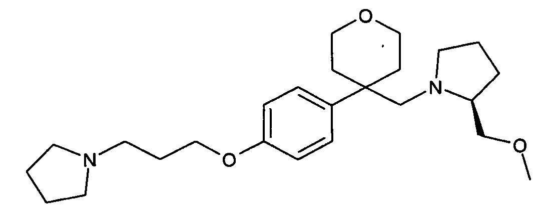

4-[4-(3-Pyrrolidin-1-ylpropoxy)phenyl]tetrahydro-2*H-*pyran-4-carbaldehyde (350mg, 1.103mmol), (s)-(+)-2-(methoxymethyl)pyrrolidine (230mg, 2.00mmol), absolute ethanol (40ml), activated 3A molecular sieves (350mg), titanium (IV) isopropoxide (1.62ml, 5.48mmol) and STAB (2.0g, 9.74mmol) were reacted in accordance with the general procedure D. The isolated crude product was purified by column chromatography on silica eluting with DCM:MeOH:NH₃ (98:1:1) to give the title compound as a white solid (330mg, 72%).
¹H NMR (400MHz, CDCl₃) δ 7.18 (d, 2H), 6.86 (d, 2H), 4.02 (t, 2H), 3.84-3.70 (m, 2H), 3.59 (td, 1H), 3.45 (td, 1H), 3.27 (s, 3H), 3.10 (dd, 1H), 3.02 (dd, 1H), 2.94 (d, 1H), 2.63 (t, 2H), 2.59-2.45 (m, 5H), 2.39 (m,1H), 2.21 (m, 1H), 2.06-1.34 (m, 15H).

### Example 43: 1-(1-{4-[4-(3-Pyrrolidin-1-ylpropoxy)phenyl]tetrahydropyran-4-ylmethyl}piperidin-4-yl)pyrrolidin-2-one

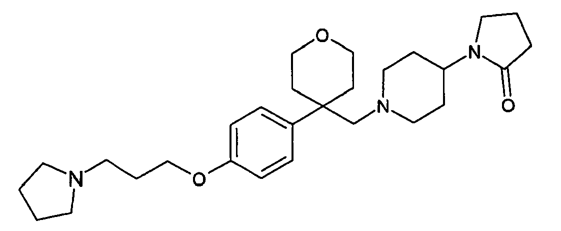

4-[4-(3-Pyrrolidin-1-ylpropoxy)phenyl]tetrahydro-2*H-*pyran-4-carbaldehyde (400mg, 1.260mmol), 4-(N-2-pyrrolidinone)piperidine hydrochloride (493mg, 4.407mmol), absolute ethanol (16ml), activated 3A molecular sieves (400mg), titanium (IV) isopropoxide (1.86ml, 6.30mmol) and STAB (2.27g, 10.71mmol) were reacted in accordance with the general procedure D. The isolated waxy solid was purified by column chromatography on silica eluting with DCM(95):MeOH(4):NH₃(1) to give the title compound as a pale yellow oil (133mg, 22%).
¹H NMR (400MHz, CDCl₃) δ 7.20 (d, 2H), 6.86 (d, 2H), 4.02 (t, 2H), 3.83-3.71 (m, 3H), 3.52 (dt, 2H), 3.32 (t, 2H), 2.63 (t, 2H), 2.53 (br s, 4H), 2.41-2.30 (m, 6H), 2.18 (dt, 2H), 2.12-1.36 (m, 16H).

### Example 44: 4-{4-[4-(3-Pyrrolidin-1-yl-propoxy)-phenyl]-tetrahydro-pyran-4-ylmethyl}-thiomorpholine

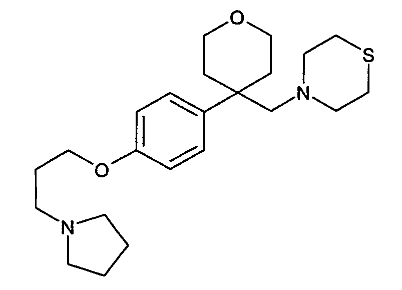

4-[4-(3-Pyrrolidin-1-ylpropoxy)phenyl]tetrahydro-2*H*-pyran-4-carbaldehyde (1.5g, 4.726mmol, 1wt), thiomorpholine (930mg, 9.027mmol), absolute ethanol (60ml), activated 3A molecular sieves (1.5g), titanium (IV) isopropoxide (6.97ml, 23.630mmol) and STAB (8.51g, 40.15mmol) were reacted in accordance with the general procedure D. The isolated waxy solid was purified by column chromatography on silica eluting with DCM(95):MeOH(4):NH₃(1) to give the title compound as a pale yellow oil (1.06g, 55%).
¹H NMR (400MHz, CDCl₃) δ 7.20 (d, 2H), 6.86 (d, 2H), 4.02 (t, 2H), 3.75 (dt, 2H), 3.57-3.45 (m, 2H), 2.63 (t, 2H), 2.59-2.39 (m, 12H), 2.36 (s, 2H), 2.14-1.95 (m, 4H), 1.92-1.73 (m, 6H).

### Example 45: 4-{4-[4-(3-Pyrrolidin-1-ylpropoxy)phenyl]tetrahydropyran-4-ylmethyl}thiomorpholine-1-oxide

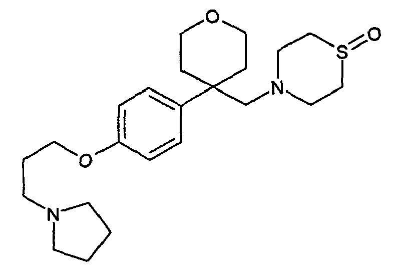

To a solution of 4-{4-[4-(3-pyrrolidin-1-yl-propoxy)-phenyl]-tetrahydro-pyran-4-ylmethyl}-thiomorpholine (200mg, 0.494mmol) in TFA (0.67ml) at 5 to 10°C was added dropwise a solution of trifluoro-peracetic acid [4M solution prepared by the addition of 27.5% H₂O₂ (0.94ml) to TFA (1.56ml)] and stirred at 5 to 10°C for 1 hour. The reaction mixture was diluted with DCM (5ml) and NaOH (5M solution, 4ml) was added to attain pH14. The mixture was extracted with DCM ((2x5ml), the combined DCM extracts were washed with saturated aqueous brine (10ml), dried over MgSO₄, filtered and concentrated *in vacuo.* The isolated yellow oil was purified by column chromatography on silica eluting with DCM(95):MeOH(4):NH₃(1) to give the title compound as a yellow oil (165mg, 79%).
¹H NMR (400MHz, CDCl₃) δ 7.20 (d, 2H), 6.87 (d, 2H), 4.02 (t, 2H), 3.81-3.69 (m, 2H), 3.59-3.43 (m, 2H), 2.99-2.82 (m, 2H), 2.76-2.2.59 (m, 6H), 2.53 (br s, 4H), 2.46 (s, 2H), 2.31-1.73 (m, 12H).

### Example 46: 4-{4-[4-(3-Pyrrolidin-1-yl-propoxy)-phenyl]-tetrahydro-pyran-4-ylmethyl}-thiomorpholine 1,1-dioxide

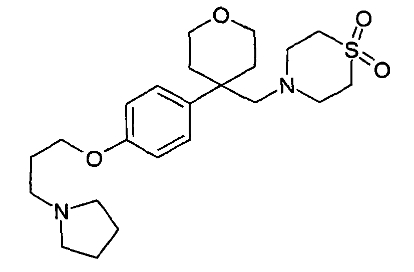

To a solution of 4-{4-[4-(3-Pyrrolidin-1-yl-propoxy)-phenyl]-tetrahydro-pyran-4-ylmethyl}-thiomorpholine (230mg, 0.56mmol) in TFA (0.77ml) at 0 to 5°C was added dropwise a solution of trifluoro-peracetic acid [4M solution prepared by the addition of 27.5% H₂O₂ (0.94ml) to TFA (1.56ml)]. The reaction was allowed to warm to room temperature and stirred overnight. The reaction mixture was cooled to 0 to 5°C, diluted with DCM (5ml) and quenched with NaOH (5M solution, 5ml) to attain pH14. The mixture was extracted with DCM (4x5ml), the combined DCM extracts were dried over MgSO₄, filtered and concentrated *in vacuo*. The isolated yellow oil was purified by column chromatography on silica eluting with DCM(95):MeOH(4):NH₃(1) to give the title compound as a colourless oil (96.6mg, 39%).
¹H NMR (400MHz, CDCl₃) δ 7.20 (d, 2H), 6.88 (d, 2H), 4.02 (t, 2H), 3.75 (dt, J = 11 and 4Hz, 2H), 3.57-3.45 (m, 2H), 2.89-2.79 (m, 4H), 2.73-2.65 (m, 4H), 2.63 (t, 2H), 2.55 (br s, 4H), 2.53 (s, 2H), 2.22-2.11 (m, 2H), 2.01 (m, 2H), 1.87-1.73 (m, 6H).

### Example 47: 4-{4-[4-(3-Pyrrolidin-1-yl-propoxy)phenyl]tetrahydropyran-4-ylmethyl}piperazine-1-carboxylic acid ethyl ester

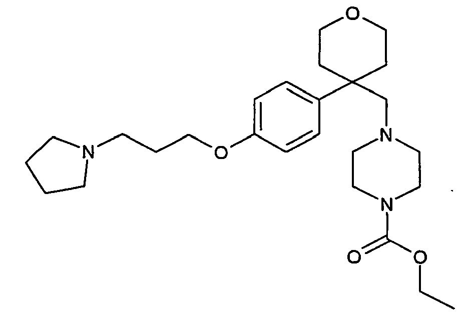

4-[4-(3-Pyrrolidin-1-ylpropoxy)phenyl]tetrahydro-2*H-*pyran-4-carbaldehyde (500mg, 1.58mmol), ethyl piperazine-1-carboxylate (471mg, 3.00mmol), absolute ethanol (20ml), activated 3A molecular sieves (500mg), titanium (IV) isopropoxide (2.31 ml, 7.88mmol) and STAB (2.84g, 13.6mmol) were reacted in accordance with the general procedure D. The isolated crude product was purified by column chromatography on silica eluting with DCM:MeOH:NH₃ (97:2:1) to give the title compound as a white solid containing residual 4-[4-(3-pyrrolidin-1-yfpropoxy)phenyl]tetrahydro-2*H-*pyran-4-carbaldehyde (350mg). The material was dissolved in DCM (14ml) and PS-AMPS (loading 2.04mmol⁻¹ g, 23mg) was added and shaken for 24 hours. The resin was filtered and the solution concentrated *in vacuo* at 30°C, the crude material was subjected to chromatography on silica eluting with DCM:MeOH:NH₃ (97:2:1) to give the title compound as a white solid (130mg, 18%).
¹H NMR (400MHz, CDCl₃) δ 7.20 (d, 2H), 6.86 (d, 2H), 4.08 (q, 2H), 4.02 (t, 2H), 3.79-3.70 (m, 2H), 3.57-3.47 (m, 2H), 3.27 (br s, 4H), 2.63 (t, 2H), 2.53 (br s, 4H), 2.38 (s, 2H), 2.21-1.73 (m, 14H), 1.21 (t, 3H).

### Intermediate 28: Piperazine-1-carboxylic acid amide hydrochloride

4-Piperazine-1-carboxylic acid tert-butyl ester (1.0g, 5.4mmol), acetic acid (3ml) and water (5ml) were mixed together. Potassium cyanate (2.25g, 27.7mmol) was added portionwise as a solution in water (5ml) and stirred for 4 hours, during which time a solid precipitated. The solid was filtered, re-dissolved in DCM (20ml), dried over MgSO₄, filtered, the filter cake washed with DCM (5vol) and concentrated *in vacuo* to give a white solid (0.38g). The white solid (0.38g) was dissolved in methanol (3.8ml) and 1,4-dioxane (0.7ml), 4M HCl in 1,4-dioxane (2.5ml, 10mmol) was added to the reaction and stirred overnight. The reaction was concentrated *in vacuo* to give the title compound (0.28g, 31% over 2 steps) as a white solid.

### Example 48: 4-{4-[4-(3-Pyrrolidin-1-ylpropoxy)phenyl]tetrahydropyran-4-ylmethyl}piperazine-1-carboxylic acid amide

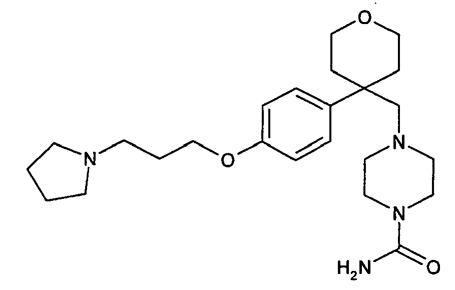

4-[4-(3-Pyrrolidin-1-ylpropoxy)phenyl]tetrahydro-2*H*-pyran-4-carbaldehyde (317mg, 1 mmol), piperazine-1-carboxylic acid amide hydrochloride (273mg, 1.65mmol), absolute ethanol (12.7ml), activated 3A molecular sieves (320mg), Et₃N (0.278ml), titanium (IV) isopropoxide (1.46ml, 5.20mmol) and STAB (1.8g, 8.5mmol) were reacted in accordance with the general procedure D. The isolated crude product was purified by column chromatography on silica eluting with DCM:MeOH:NH₃ (98:1:1) to give the title compound as a off white solid (70mg, 16%).
¹H NMR (400MHz, CDCl₃) δ 7.21 (d, 2H), 6.86 (d, 2H), 4.30 (br s, 2H), 4.02 (t, 2H), 3.80-3.71 (m, 2H), 3.58-3.48 (m, 2H), 3.23-3.15 (m, 4H), 2.63 (t, 2H), 2.53 (br s, 4H), 2.40 (s, 2H), 2.19-1.73 (m, 14H).

### Example 49: 1-{4-[4-(3-Pyrrolidin-1-ylpropoxy)phenyl]tetrahydropyran-4-ylmethyl}piperidine

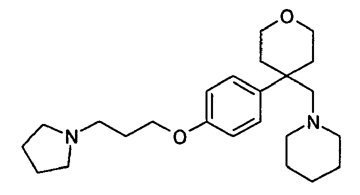

4-[4-(3-Pyrrolidin-1-ylpropoxy)phenyl]tetrahydro-2*H-*pyran-4-carbaldehyde (272 mg, 0.86 mmol), piperidine (0.161 mL, 1.63 mmol), absolute ethanol (11 mL), activated 4A molecular sieves, titanium (IV) isopropoxide (1.205 mL, 4.28 mmol) and STAB (1.54 g, 7.28 mmol) were reacted in accordance with the general procedure D. The isolated waxy solid was purified by column chromatography on silica eluting with a gradient of DCM:MeOH:NH₃ (from 99:0:1 to 90:9:1) to give the title compound (97.4 mg, 25%).
¹H NMR (400MHz, CDCl₃) δ 7.21(d, 2H), 6.85 (d, 2H), 4.02 (t, 2H), 3.77 -3.72 (m, 2H), 3.54 -3.48 (t, 2H), 2.63 (t, 2H), 2.53 (m, 4H), 2.29 (s, 2H), 2.13-1.87 (m, 10H), 1.79 (quint, 4H), 1.41-1.36 (m, 4H), 1.30-1.26 (m, 2H).

### Example 50: 4,4-difluoro-1-{4-[4-(3-Pyrrolidin-1-ylpropoxy)phenyl]tetra-hydropyran-4-ylmethyl}piperidine

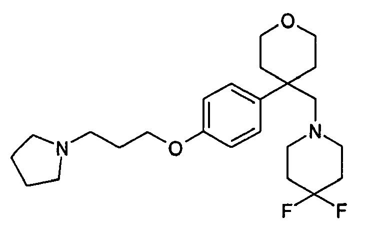

4-[4-(3-Pyrrolidin-1-ylpropoxy)phenyl]tetrahydro-2*H*-pyran-4-carbaldehyde (260 mg, 0.82 mmol), 4,4-difluoropiperidine hydrochloride (323 mg, 2.05 mmol), absolute ethanol (11 mL), activated 4A molecular sieves, titanium (IV) isopropoxide (1.209 mL, 4.09 mmol) and STAB (1.475 g, 6.96 mmol) were reacted in accordance with the general procedure D. The isolated waxy solid was purified by reverse phase preparative HPLC on a Xterra column eluting with a gradient of MeCN:H₂O:Et₃N (from 29.9:70:0.1 to 94.9:5:0.1 in 13 min) to give the title compound (26 mg, 6%).
¹H NMR (400MHz, CDCl₃) δ 7.21(d, 2H), 6.88 (d, 2H), 4.02 (t, 2H), 3.77 -3.73 (m, 2H), 3.53 -3.47 (t, 2H), 2.64 (t, 2H), 2.54 (m, 4H), 2.39 (s, 2H), 2.30-2.24 (m, 6H), 2.13 (d, 2H), 2.02 (m, 2H), 1.90-1.71 (m, 6H).

### Example 51: methyl-{4-[4-(3-pyrrolidin-1-ylpropoxy)phenyl]-tetrahydropyran-4-ylmethyl}amine

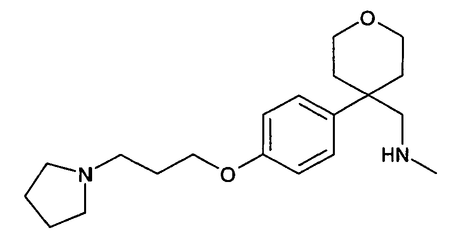

### Step 1:

To a stirred solution of {4-[4-(3-pyrrolidin-1-ylpropoxy)phenyl]tetrahydropyran-4-yl} methylamine (10.83 g, 34.05 mmol) and triethylamine (10.31 g, 102.16 mmol) in dry dichloromethane at 0 ° C was added dropwise a solution of di-*tert*-butyldicarbonate (9.65 g, 44.27 mmol) in dry dichloromethane. Stirring was continued for another hour at 0 °C and then overnight at room temperature. The reaction mixture was then diluted with dichloromethane, washed with water and brine, dried over Na₂SO₄ and concentrated under reduced pressure to provide a crude product. The crude product was purified by column chromatography on silica gel (EtOAc-MeOH-Et₃N) to give methyl-{4-[4-(3-pyrrolidin-1-ylpropoxy)phenyl]tetrahydro-pyran-4-ylmethyl}carbamic acid *tert*-butyl ester (9.80 g, 69 % yield).
¹H NMR (400 MHz, CDCl₃) δ 1.37 (s, 9H); 1.75-1.88 (m, 6H); 1.96-2.07 (m, 4H); 2.48-2.55 (m, 4H); 2.61 (t, 2H); 3.28 (d, 2H); 3.51-3.59 (m, 2H); 3.76-3.80 (m, 2H); 4.01 (t, 2H); 4.15 (brm, 1H); 6.89 (d, 2H); 7.16 (d, 2H).

### Step 2:

To a stirred solution of LiAlH₄ (2.59 g, 68.18 mmol) in dry THF at 0° C was added dropwise a solution of methyl-{4-[4-(3-pyrrolidin-1-ylpropoxy)phenyl]tetrahydro-pyran-4-ylmethyl}carbamic acid *tert*-butyl ester (9.50 g, 22.72 mmol). The reaction mixture was then brought to room temperature and finally heated to reflux for 3 h. The reaction mixture was quenched with aqueous NaOH solution and filtered over a short pad of celite, dried over Na₂SO₄ and concentrated under reduced pressure to provide the title compound (7.1 g, 94 % yield).
¹H NMR (400 MHz, CDCl₃) δ 1.71-1.78 (m, 4H); 1.80-1.90 (m, 2H); 1.88-2.02 (m, 2H); 2.06-2.14 (m, 2H); 2.23 (s, 3H); 2.46-2.53 (m, 4H); 2.51-2.63 (m, 4H); 3.47- 3.56 (m, 2H); 3.70-3.77 (m, 2H); 3.99 (t, 2H); 6.86 (d, 2H); 7.17 (d, 2H).

### Example 52: methyl-(3-methylsulfanyl-propyl)-{4-[4-(3-pyrrolidin-1-ylpropoxy)phenyl]tetrahydropyran-4-ylmethyl}amine

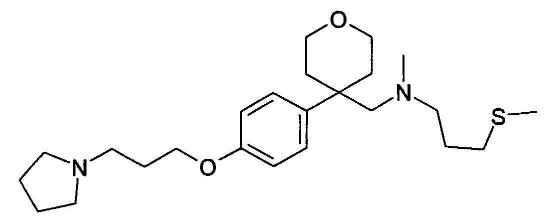

To a solution of methyl-{4-[4-(3-pyrrolidin-1-ylpropoxy)phenyl]tetrahydropyran-4-ylmethyl}amine (200 mg, 0.629 mmol) in dichloroethane (5 mL) under nitrogen atmosphere was added acetic acid (45 mg, 1.258 mmol), triethylamine (76 mg, 1.258 mmol) and 3-(methylthio)propanal (108 mg, 1.04 mmol). The mixture was stirred for 30 min and a solution of NaHB(OAc)₃ (236 mg, 1.15 mmol) in dichloroethane (5 mL) was added. After stirring at room temperature overnight, the reaction mixture was filtered over celite, concentrated *in vacuo* and purified by column chromatography on silica gel eluting with DCM:MeOH:NH₃ (94.9:5:0.1) to give the title compound (29 mg, 7%).
¹H NMR (400MHz, CDCl₃) δ 7.19 (d, 2H), 6.86 H), 4.02 (t, 2H), 3.78 - 3.73 (m, 2H), 3.54 - 3.49 (m, 2H), 2.67 (t, 2H), 2.59 (m, 4H), 2.42 (s, 2H), 2.35 (t, 2H), 2.22 (t, 2H), 2.11 - 2.00 (m, 7H), 1.91-1.86 (m, 5H), 1.85- 1.82 (m, 4H), 1.56 (q, 2H).

### Example 53: (3-methanesulfonyl-propyl)-methyl-{4-[4-(3-pyrrolidin-1-yl-propoxy)phenyl]tetrahydropyran-4-ylmethyl}amine

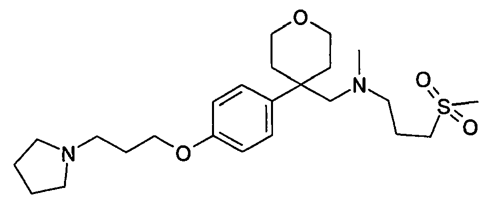

To a solution of methyl-(3-methylsulfanyl-propyl)-{4-[4-(3-pyrrolidin-1-ylpropoxy)phenyl]tetrahydropyran-4-ylmethyl}amine (170 mg, 0.404 mmol) in DCM (10 mL) under nitrogen atmosphere at 0°C was added TFA (0.240 mL, 3.2 mmol) followed by meta-chloroperbenzoic acid (70% pure, 199 mg, 0.808 mmol). The reaction mixture was stirred overnight allowing it to warm up to room temperature. It was quenched with an aqueous solution of Na₂S2O₃. The organic layer was separated and washed with an aqueous solution of NaHCO₃. The organic layer was then dried over sodium sulphate, filtered, concentrated and purified by column chromatography on silica gel eluting with DCM:MeOH:NH₃ (89.9:10:0.1) to give the title compound.
¹H NMR (400MHz, CDCl₃) δ 7.18 (d, 2H), 6.88 (d, 2H), 4.03 (t, 2H), 3.78 - 3.73 (m, 2H), 3.53 - 3.48 (m, 2H), 2.82 (s, 3H), 2.69-2.73 (m, 2H), 2.65 (m, 2H), 2.55 (m, 4H), 2.47 (s, 2H), 2.22 (t, 2H), 2.12 - 2.09 (m, 2H), 1.89-2.05 (m, 5H), 1.68-1.85 (m, 8H).

### Example 54: isopropyl-{4-[4-(3-pyrrolidin-1-ylpropoxy)phenyl]-tetrahydro-pyran-4-ylmethyl}amine

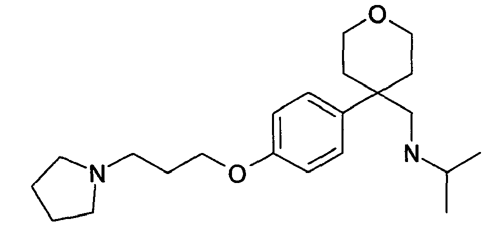

To a solution of {4-[4-(3-pyrrolidin-1-ylpropoxy)phenyl]tetrahydropyran-4-yl} methylamine (400 mg, 1.256 mmol) in dichloroethane (10 mL) under nitrogen atmosphere was added acetic acid (180.5 mg, 2.76 mmol), triethylamine (306.4 mg, 2.76 mmol) and acetone (80.2 mg, 1.38 mmol). The mixture was stirred for 1 h at ambient temperature and NaHB(OAc)₃ (601 mg, 2.76 mmol) was added. After stirring overnight, the reaction mixture was filtered over celite, concentrated *in vacuo* and purified by column chromatography on silica gel eluting with DCM:MeOH:NH₃ (89:10:1) to give the title compound as an oil (282 mg, 60%).
¹H NMR (400 MHz, CDCl3) δ 7.13 (d, 2H), 6.82 (d, 2H), 3.96 (t, 2H), 3.68 (m, 2H), 3.49 (m, 2H), 2.61 (s, 2H), 2.56 (t, 2H), 2.46 (m, 5H), 2.04 (m, 2H), 1.94 (m, 2H), 1.84 (m, 2H), 1.72 (m, 4H), 1.19 (s, 1H), 0.83 (d, 6H).

### Example 55: isopropyl-methyl-{4-[4-(3-pyrrolidin-1-ylpropoxy)phenyl]-tetrahydropyran-4-ylmethyl}amine

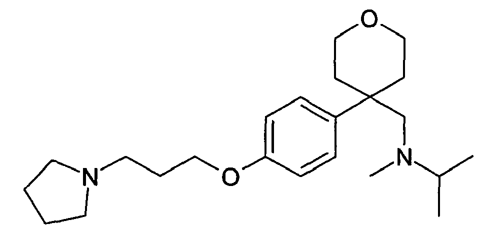

To isopropyl-{4-[4-(3-pyrrolidin-1-ylpropoxy)phenyl]tetrahydropyran-4-ylmethyl}-amine (258 mg, 0.71 mmol) was added formaldehyde (37% w/w solution in H₂O, 1 mL) and formic acid (1.5 mL).The resulting mixture was heated to 60°C overnight. After cooling, H₂O and ethyl acetate were added. The aqueous layer was separated, basified with 30% NaOH and extracted 3 times with ethyl acetate. The combined organic extracts were dried over Na₂SO₄, filtered and concentrated *in vacuo* to give the title compound as an oil (170 mg, 65%).
¹H NMR (400MHz, CDCl₃) *δ* 7.19 (d, 2H), 6.86 (d, 2H), 4.02 (t, 2H), 3.76 (m, 2H), 3.50 (m, 2H), 2.63 (t, 2H), 2.53 (m, 4H), 2.44 (m, 1H), 2.38 (s, 2H), 2.08 (m, 2H), 2.01 (m, 2H), 1.89 (m, 2H), 1.85 (s, 3H), 1.79 (m, 4H), 0.84 (d, 6H).

### Example 56: cyclopentyl-{4-[4-(3-pyrrolidin-1-ylpropoxy)phenyl]tetra-hydropyran-4-ylmethyl}amine

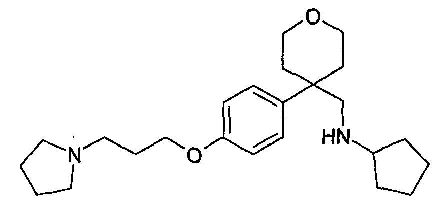

To a solution of {4-[4-(3-pyrrolidin-1-ylpropoxy)phenyl]tetrahydropyran-4-yl} methylamine (254 mg, 0.798 mmol) in dichloroethane (5 mL) under nitrogen atmosphere were added acetic acid (37 mg, 1.6 mmol), triethylamine (97 mg, 1.6 mmol) and cyclopentanone (10 mg, 1.19 mmol). The mixture was stirred for 30 min at ambient temperature and NaHB(OAc)₃ (204 mg, 1.6 mmol) was added at 0°C. After stirring overnight, the reaction mixture was filtered over celite, concentrated *in vacuo* and purified by column chromatography on silica gel eluting with DCM:MeOH:NH₃ (89:10:1) to give the title compound (226 mg, 58%).
¹H NMR (400MHz, CDCl₃) δ 7.20 (d, 2H), 6.88 H), 4.03 (t, 2H), 3.78-3.73 (m, 2H), 3.59-3.53 (m, 2H), 2.84 (q, 1H), 2.73 (t, 2H), 2.65-2.68 (m, 6H), 2.03-2.12 (m, 4H), 1.84-1.94 (m, 6H), 1.65-1.72 (m, 2H), 1.51-1.58 (m, 2H), 1.41-1.46 (m, 2H), 1.07-1.16 (m, 2H).

### Example 57:1-{4-[4-(3-Pyrrolidin-1-ylpropoxy)phenyl]tetrahydropyran-4-ylmethyl}pyrrolidine

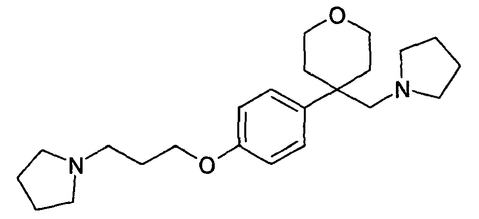

To a stirred solution of {4-[4-(3-pyrrolidin-1-ylpropoxy)phenyl]tetrahydropyran-4-yl} methylamine (150 mg, 0.47 mmol) and 1-bromo-4-chloroethane (54.2 µL, 0.47 mmol) in anhydrous acetonitrile (5 mL) was added potassium carbonate (65 mg, 0.47 mmol). The mixture was heated to 60°C overnight then concentrated under reduced pressure. The crude product was purified by column chromatography on silica gel eluting with DCM:MeOH:NH₃ (94:5:1) to give the title compound (40 mg, 23 % yield).
¹H NMR (400MHz, CDCl₃) δ 7.20 (d, 2H), 6.86 (d, 2H), 4.02 (t, 2H), 3.73 -3.78 (m, 2H), 3.57-3.53 (m, 2H), 2.65 -2.61 (m, 4H), 2.53 (m, 4H), 2.20 (m, 4H), 2.10-1.88 (m, 6H), 1.56 (m, 4H).

### Example 58: 4-{4-[4-(3-Pyrrolidin-1-ylpropoxy)phenyl]tetrahydropyran-4-ylmethyl}-morpholine

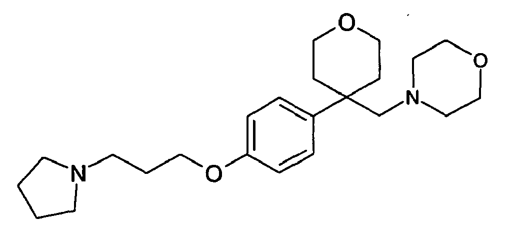

To a stirred solution of {4-[4-(3-pyrrolidin-1-ylpropoxy)phenyl]tetrahydropyran-4-yl} methylamine (207 mg, 0.65 mmol) and bis(2-bromoethyl)ether (82 µL, 1.3 mmol) in anhydrous acetonitrile (10 mL) was added potassium carbonate (180 mg, 1.3 mmol). The mixture was heated to 60°C overnight then concentrated under reduced pressure. The crude product was purified by column chromatography on silica gel eluting with DCM:MeOH (97:3) with 10% NH₃ to give the title compound (90 mg, 23 % yield).
¹H NMR (400MHZ, CDCL₃) δ 7.22 (d, 2H), 6.83 (d, 2H), 4.04 (m, 2H), 3.73 (m, 2H), 3.51 (m, 6H), 3 (m, 6H), 2.38 (m, 2H), 2.25 (m, 2H), 2.13 - 2.03 (m, 10H), 1.88 (m, 2H).

### Example 59 : methyl-phenethyl-{4-4-{3-pyrrolidin-1-ylpropoxy)phenyl]tetra-hydropyran-4-ylmethyl}amine

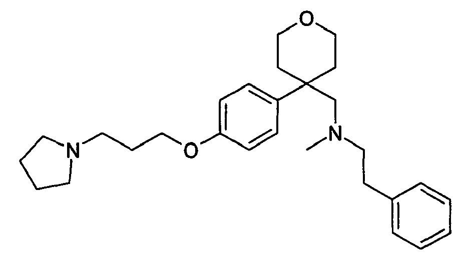

Methyl-{4-[4-(3-pyrrolidin-1-ylpropoxy)phenyl]tetrahydropyran-4-ylmethyl}amine (150 mg, 0.45 mmol) was treated with (2-bromoethyl)benzene (100 mg, 0.54 mmol) and potassium carbonate (187 mg, 1.35 mmol) in a sealed tube under microwave (Smith Personal Synthesiser) (55°C) for 80 min. The reaction mixture was taken in DCM (10 mL) and water (5 mL). The organic layer was separated and washed twice with water and brine. The combined organic extracts were dried over Na₂SO₄, filtered, concentrated *in vacuo* and purified by column chromatography on silica gel eluting with DCM:MeOH (90:10) with 10% NH₃ to give the title compound as a colorless oil (41.4 mg, 20%).
¹H NMR (400MHz,CDCl₃) δ 7.23 (m, 7H), 6.87 (d, 2H), 4.00 (t, 2H), 3.66 (m, 2H), 3.51 (m, 2H), 3.29 (t, 2H), 3.20 (t, 2H), 2.62 (t, 2H), 2.51 (m, 6H), 2.10 (m, 2H), 1.99 (m, 2H), 1.90 (m, 2H), 1.86 (s, 3H), 1.78 (m, 4H).

### Example 60 : methyl-benzyl-{4-[4-(3-pyrrolidin-1-ylpropoxy)phenyl]tetra-hydropyran-4-ylmethyl}amine

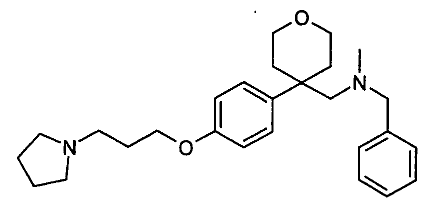

This example was prepared from methyl-{4-[4-(3-pyrrolidin-1-ylpropoxy)phenyl]tetrahydropyran-4-ylmethyl}amine similarly to the procedure used for example 59.

¹H NMR (400MHz, CDCl₃) δ 7.28-7.19 (m, 7H), 6.87 (d, 2H), 4.00 (t, 2H), 3.69-3.65 (m, 2H), 3.53-3.48 (t, 2H), 3.29 (s, 2H), 2.61 (t, 2H), 2.54-2.50 (m, 6H), 2.10-2.07 (m, 2H), 2.01-1.97 (m, 2H), 1.93-1.88 (m, 2H), 1.86 (s, 3H), 1.78-1.76 (m, 4H).

### Example 61 : (2-methoxyethyl)-methyl-{4-[4-(3-pyrrolidin-1-ylpropoxy)-phenyl]tetrahydropyran-4-ylmethyl}amine

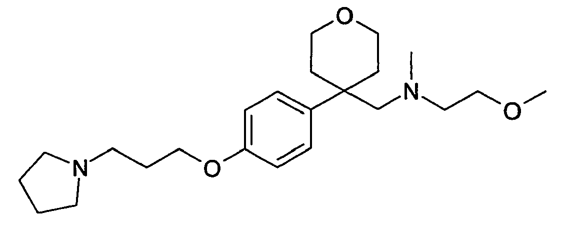

To a stirred solution of methyl-{4-[4-(3-pyrrolidin-1-ylpropoxy)phenyl]tetra-hydropyran-4-ylmethyl}amine (150 mg, 0.45 mmol) and 2-bromoethylmethylether (43 µL, 0.45 mmol) in anhydrous acetonitrile (5 mL) was added potassium carbonate (62.4 mg, 0.45 mmol). The mixture was heated to 60°C overnight then to 80°C for a day and concentrated under reduced pressure. The crude product was purified by column chromatography on silica gel eluting with DCM:MeOH (95:5) with 10% NH₃ to give the title compound as an oil (69 mg, 18% yield).
¹H NMR (400MHz, CDCl₃) δ 7.20 (d, 2H), 6.86 (d, 2H), 4.02 (t, 2H), 3.78 - 3.48 (m, 4H), 3.29 (t, 2H), 3.27 (s, 3H), 2.67 (t, 2H), 2.59 (m, 4H), 2.48 (s, 2H), 2.39 (t, 2H), 2.11-2.00 (m, 4H), 1.98 (s, 3H), 1.92 - 1.85 (m, 2H), 1.84-1.80 (m, 4H).

### Example 62: methyl-pyrymidin-2-yl-{4-[4-(3-pyrrolidin-1-ylpropoxy)-phenyl]-tetrahydropyran-4-ylmethyl}amine

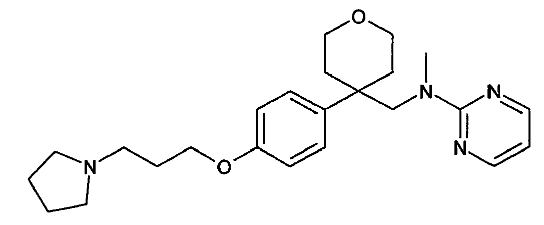

To a solution of methyl-{4-[4-(3-pyrrolidin-1-ylpropoxy)phenyl]tetrahydropyran-4-ylmethyl}amine (150 mg, 0.45 mmol) and 2-chloropyrymidine (52 mg, 0.45 mmol) in anhydrous acetonitrile (5 mL) was added potassium carbonate (62 mg, 0.45 mmol). The mixture was heated to 60°C overnight then refluxed for 2 days, filtered and concentrated under reduced pressure. The crude product was purified by column chromatography on silica gel eluting with DCM:MeOH (97:3) with 10% NH₃ to give the title compound (40 mg, 22% yield).
¹H NMR (400MHz, CDCl₃) δ 8.24 (m, 2H), 7.20 (d, 2H), 6.88 (d, 2H), 6.42 (m, 1H), 4.02 (m, 2H), 3.80 (m, 4H), 3.52 (t, 2H), 2.59 (m, 9H), 2.01 (m, 8H), 1.79 (s, 3H).

### Example 63: N-{4-4-(3-Pyrrolidin-1-yl-propoxy)-phenyl]-tetrahydro-pyran-4-ylmethyl}-methanesulfonamide

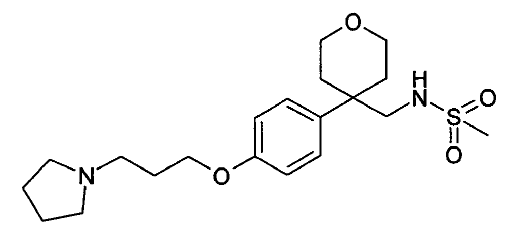

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of mesyl chloride (58 µL, 0.754 mmol) in anhydrous dichloromethane (2 mL). After stirring overnight, the reaction mixture was quenched with a saturated aqueous solution of NaHCO₃. The organic layer was separated and washed with a saturated aqueous solution of NaHCO₃ and water then dried over magnesium sulphate, filtered and concentrated under reduced pressure to give the title compound (140 mg, 56 %).
¹NMR (400MHz, CDCl₃) *δ* 7.19 (d, 2H), 6.94 (d, 2H), 4.04 (t, 2H), 3.79 (m, 2H), 3.57 (m, 2H), 3.24 (d, 2H), 2.75 (s, 3H), 2.63 (t, 2H), 2.54 (m, 4H), 2.12 (m, 2H), 2.01 (m, 2H), 1.88 (m, 2H), 1.80 (m, 4H).

### Example 64: C-Phenyl-N-{4-[4-(3-pyrrolidin-1-yl-propoxy)-phenyl]-tetra-hydropyran-4-ylmethyl}-methanesulfonamide

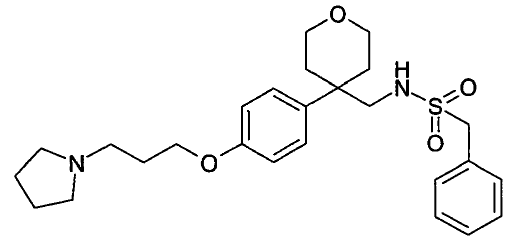

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of α-toluenesulfonylchloride (143 mg, 0.754 mmol) in anhydrous dichloromethane (2 mL). After stirring overnight, the reaction mixture was quenched with a saturated aqueous solution of NaHCO₃. The organic layer was separated and washed with water, dried over magnesium sulphate, filtered and concentrated under reduced pressure to give the title compound (207 mg, 69 %).
¹H NMR (400MHZ, CDCL₃) δ 7.27-7.22 (M, 3H), 7.16-7.14 (M, 2H), 7.07 (D, 2H), 6.83 (D, 2H), 4.05 (S, 2H), 3.95 (T, 2H), 3.68-3.63 (M, 2H), 3.55 (T, 1H), 3.49-3.44 (M, 2H), 3.01 (D,2H), 2.57 (T,2H), 2.47 (S,4H), 2.02-1.93 (M, 4H), 1.78-1.72 (M, 6H).

### Example 65: 3-Cyano-N-{4-[4-(3-pyrrolidin-1-yl-propoxy)-phenyl]-tetrahydro-pyran-4-ylmethyl}-benzenesulfonamide

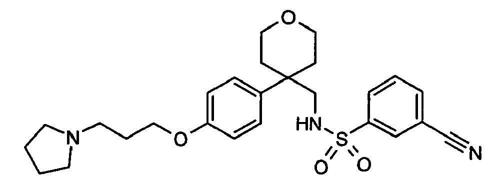

To a stirred solution of {4-[4-(3-pyrrolidin-1-ylpropoxy)phenyl]tetrahydropyran-4-yl} methylamine (100 mg, 0.314 mmol) and triethylamine (0.1 mL, 0.63 mmol) in anhydrous dichloromethane (3 mL) under nitrogen atmosphere at 0°C was added 3-cyanobenzenesulfonylchloride (100 mg, 0.47 mmol). The reaction mixture was stirred for 2 h allowing it to warm up to room temperature. It was then diluted with dichloromethane and quenched with water. The organic layer was separated and washed with water, dried over sodium sulfate, filtered and concentrated under reduced pressure. The crude product was purified by column chromatography on silica gel eluting with DCM:MeOH (95:5 to 90:10) to give the title compound.
¹H NMR (400MHz, CDCl₃) δ 7.97 (s, 1H), 7.93-7.91 (d, 1H), 7.83-7.81 (d, 1H), 7.62-7.58 (m, 1H), 7.11-7.09 (d, 2H), 6.89-6.86 (d, 2H ), 4.06-4.03 (m, 3H), 3.77-3.72 (m, 2H), 3.56-3.51 (m, 2H),3.08 (br s,2H), 2.70-2.67 (m, 2H), 3.08 (br s,4H), 2.08-2.01 (m, 4H), 1.85-1.82 (m, 6H).

### Example 66: 2-Fluoro-N-{4-[4-(3-pyrrolidin-1-yl-propoxy)-phenyl]-tetrahydro-pyran-4-ylmethyl}-benzenesulfonamide

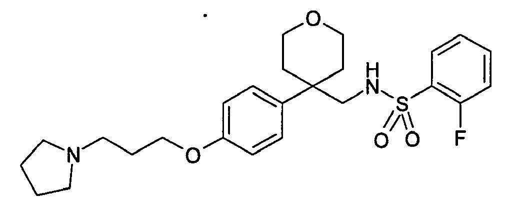

To a stirred solution of {4-[4-(3-pyrrolidin-1-ylpropoxy)phenyl]tetrahydropyran-4-yl} methylamine (100 mg, 0.314 mmol) and triethylamine (0.1 mL, 0.63 mmol) in anhydrous dichloromethane (3 mL) under nitrogen atmosphere at 0°C was added 2-fluorobenzenesulfonylchloride (91 mg, 0.47 mmol). The reaction mixture was stirred for 2 h allowing it to warm up to room temperature. It was then diluted with dichloromethane and quenched with a saturated aqueous solution of NaHCO₃. The organic layer was separated and washed with water, dried over sodium sulfate, filtered and concentrated under reduced pressure to give the title compound (120 mg, 80%).
¹H NMR (400MHz, CDCl₃) δ 7.86-7.82 (t, 1H), 7.58-7.52 (m, 1H), 7.28-7.24 (m, 1H), 7.13-7.12 (m, 3H), 6.90-6.88 (m, 2H), 4.19 (m, 1H), 4.05-4.02 (t, 2H), 3.73-3.70 (m, 2H), 3.56-3.51 (t, 2H), 3.10-3.08 (d, 2H), 2.66-2.62 (t, 2H), 2.54 (m, 4H), 2.06-1.99 (m, 4H), 1.85-1.8 (m, 6H).

### Example 67: N-methyl-N-{4-[4-(3-Pyrrolidin-1-yl-propoxy)-phenyl]-tetra-hydropyran-4-ylmethyl}-methanesulfonamide

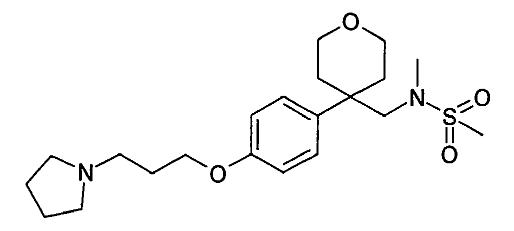

To a stirred solution of methyl-{4-[4-(3-pyrrolidin-1-ylpropoxy)phenyl]tetrahydropyran-4-ylmethyl}amine (200 mg, 0.60 mmol) and triethylamine (102 µL, 0.72 mmol) in anhydrous dichloromethane (8 mL) under nitrogen atmosphere was added dropwise a solution of mesyl chloride (56 µL, 0.72 mmol) in anhydrous dichloromethane (2 mL). After stirring overnight, the reaction mixture was quenched with a saturated aqueous solution of NaHCO₃. The organic layer was separated, washed with water then dried over magnesium sulfate, filtered and concentrated under reduced pressure to give the title compound (175 mg, 71 %).
¹NMR (400MHz, CDCl₃) δ 7.22 (d, 2H), 6.92-6.90 (d, 2H), 4.03 (t, 2H), 3.83-3.78 (m, 2H), 3.52 (t, 2H), 3.16 (s, 2H), 2.68 (s, 3H), 2.64 (t, 2H), 2.54 (m, 4H), 2.19-2.15 (m, 5H), 2.05-1.91 (m,4H), 1.81-1.78 (m, 4H).

### Example 68 : N'-{[4-(4-(3-Pyrrolidin-1-yl-propoxy)-phenyl)tetrahydro-2H-pyran-4-yl]methyl}-N,N-dimethylsulfamide

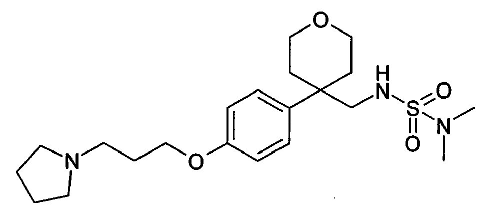

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of dimethylsulfamoyl chloride (81 µL, 0.754 mmol) in anhydrous dichloromethane (2 mL). After stirring overnight, the reaction mixture was quenched with a saturated aqueous solution of NaHCO₃. The organic layer was separated and washed with water then dried over magnesium sulfate, filtered and concentrated under reduced pressure to give the title compound (207 mg, 77 %).
¹H NMR (400MHZ, CDCL₃) δ 7.19 (D, 2H), 6.93-6.91 (D, 2H), 4.05 (T, 2H), 3.78-3.76 (M, 2H), 3.59-3.54 (M, 2H), 3.17-3.15 (D, 2H), 2.76-2.70 (M, 12H), 2.15-2.06 (M, 4H), 1.90-1.87 (M, 6H).

### Example 69: N-{4-[4-(3-Pyrrolidin-1-yl-propoxy)-phenyl]-tetrahydro-pyran-4-ylmethyl}-acetamide

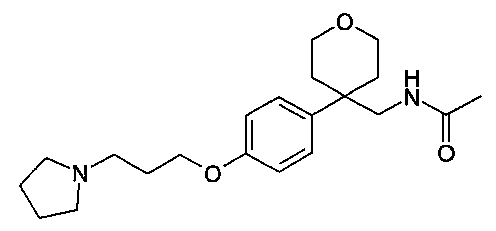

To a stirred solution of {4-[4-(3-pyrrolidin-1-ylpropoxy)phenyl]tetrahydropyran-4-yl} methylamine (200 mg, 0.628 mmol) and triethylamine (106 µL, 0.754 mmol) in anhydrous dichloromethane (8 mL) under nitrogen atmosphere was added dropwise a solution of acetyl chloride (54 µL, 0.754 mmol) in anhydrous dichloromethane (2 mL). After stirring overnight, the reaction mixture was quenched with a saturated aqueous solution of NaHCO₃. The organic layer was separated and washed with a saturated aqueous solution of NaHCO₃ and water then dried over magnesium sulfate, filtered and concentrated under reduced pressure to give the title compound (102 mg, 45 %).
¹H NMR (400MHz, CDCl₃) δ 7.18 (d, 2H), 6.94 (d, 2H), 4.97 (s, 1H), 4.05 (t, 2H), 3.80 (m, 2H), 3.59 (m, 2H), 3.46 (d, 2H), 2.66 (t, 2H), 2.57 (m, 4H), 2.03 (m, 4H), 1.80 (m,8H).

### Example 70: N-methyl-N-{4-[4-(3-Pyrrolidin-1-yl-propoxy)-phenyl]-tetrahydro-pyran-4-ylmethyl}-acetamide

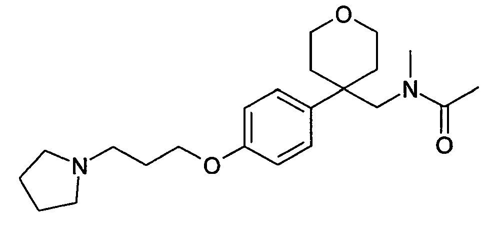

To a stirred solution of methyl-{4-[4-(3-pyrrolidin-1-ylpropoxy)phenyl]tetrahydropyran-4-ylmethyl}amine (200 mg, 0.60 mmol) and triethylamine (102 µL, 0.72 mmol) in anhydrous dichloromethane (8 mL) under nitrogen atmosphere was added dropwise a solution of acetyl chloride (52 µL, 0.72 mmol) in anhydrous dichloromethane (2 mL). After stirring overnight, the reaction mixture was quenched with a saturated aqueous solution of NaHCO₃. The organic layer was separated and washed with water then dried over magnesium sulfate, filtered, concentrated under reduced pressure and purified by column chromatography on silica gel eluting with DCM:MeOH (95:5 to 90:10) to give the title compound (86 mg, 36 %).
¹H NMR (400MHz, CDCl₃) δ Shows rotomers 7.19-7.13 (2d, 2H), 6.91-6.88 (d, 2H), 4.05-4.02 (m, 2H), 3.85-3.80 (m, 2H), 3.56-3.45 (m, 4H), 2.67-2.65 (m, 2H), 2.60-2.56 (m, 4H), 2.32-1.96 (several multiplets, 12H).

### Example 71: 2-Phenyl-N-{4-[4-(3-pyrrolidin-1-yl-propoxy)-phenyl]-tetrahydro-pyran-4-ylmethyl}-acetamide

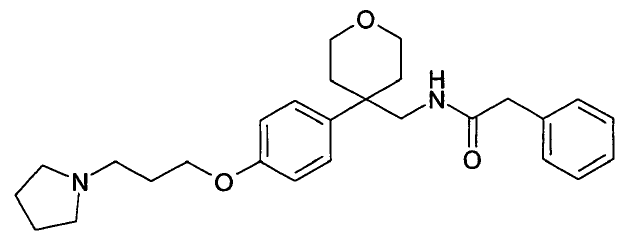

To a stirred solution of {4-[4-(3-pyrrolidin-1-ylpropoxy)phenyl]tetrahydropyran-4-yl} methylamine (200 mg, 0.63 mmol) and triethylamine (93 µL, 0.66 mmol) in anhydrous dichloromethane (8 mL) under nitrogen atmosphere at 0°C was added dropwise a solution of phenylacetyl chloride (93 µL, 0.66 mmol) in anhydrous dichloromethane (2 mL). After stirring for 3 h at 0°C, the reaction mixture was quenched with a saturated aqueous solution of NaHCO₃. The organic layer was separated and washed with water then dried over magnesium sulfate, filtered, concentrated under reduced pressure and purified by column chromatography on silica gel eluting with DCM:MeOH (98:2) to give the title compound (34 mg, 12 %).
¹H NMR (400MHz, CDCl₃) δ 7.32-7.31 (m, 3H), 7.12-7.10 (m, 2H), 6.89-6.87 (d, 2H), 6.76-6.74 (d, 2H), 4.91 (t, 1H), 4.02 (t, 2H), 3.79-3.74 (m, 2H), 3.53-3.51 (m, 2H), 3.47 (s, 2H), 3.37-3.35 (d, 2H), 2.62 (m, 6H), 2.08-2.06 (m, 2H), 1.93-1.85 (m, 6H), 1.76-1.74 (m, 2H).

### Example 72: 1,1-Dimethyl-3-{4-[4-(3-pyrrolidin-1-yl-propoxy)-phenyl]-tetrahydro-pyran-4-ylmethyl}-urea

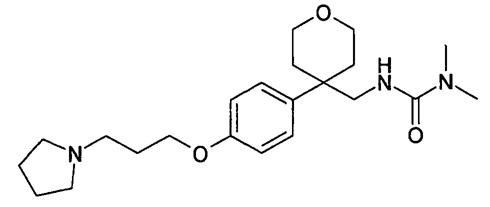

To a stirred solution of {4-[4-(3-pyrrolidin-1-ylpropoxy)phenyl]tetrahydropyran-4-yl} methylamine (200 mg, 0.63 mmol) and triethylamine (106 µL, 0.754 mmol) in anhydrous dichloromethane (9 mL) under nitrogen atmosphere was added dropwise a solution of dimethylcarbamoylchloride (69 µL, 0.754 mmol) in anhydrous dichloromethane (1 mL). After stirring for 3 h at 0°C, the reaction mixture was quenched with a saturated aqueous solution of NaHCO₃. The organic layer was separated and washed with water then dried over magnesium sulfate, filtered, concentrated under reduced pressure and purified by column chromatography on silica gel eluting with DCM:MeOH (95:5 to 90:10) to give the title compound (80 mg, 32 %).
¹H NMR (400MHz, CDCl₃) δ 7.13 (d, 2H), 6.86 (d, 2H), 3.98 (t, 2H), 3.84 (t, 1H), 3.74 (m, 2H), 3.52 (m, 2H), 3.36 (d, 2H), 2.69 (s, 6H), 2.65 (m, 2H), 2.57 (m, 4H), 2.02-1.95 (m,4H), 1.84-1.78 (m, 6H).

### Example 73: 1,1,3-Trimethyl-3-{4-[4-(3-pyrrolidin-1-yl-propoxyl-phenyl]-tetrahydro-pyran-4-ylmethyl}-urea

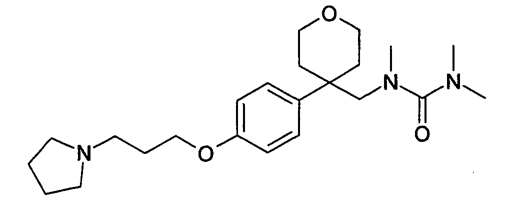

To a stirred solution of methyl-{4-[4-(3-pyrrolidin-1-ylpropoxy)phenyl]tetrahydropyran-4-ylmethyl}amine (200 mg, 0.60 mmol) and triethylamine (102 µL, 0.72 mmol) in anhydrous dichloromethane (8 mL) under nitrogen atmosphere was added dropwise a solution of dimethylcarbamoylchloride (67 µL, 0.72 mmol) in anhydrous dichloromethane (2 mL). After stirring overnight, the reaction mixture was quenched with a saturated aqueous solution of NaHCO₃. The organic layer was separated, washed with a saturated aqueous solution of NaHCO₃ and water then dried over magnesium sulfate, filtered, concentrated under reduced pressure and purified by column chromatography on silica gel eluting with DCM:MeOH (95:5) followed by DCM:MeOH:NH₃ (90:5:5) to give the title compound (84 mg, 39 %).
¹H NMR (400MHz, CDCl₃) δ 7.19 (d, 2H), 6.90-6.88 (d, 2H), 4.03 (t, 2H), 3.81-3.78 (m, 2H), 3.56-3.52 (m, 4H), 2.70 (s, 6H), 2.66 (t, 2H), 2.29 (s, 3H), 2.05-2.01 (m, 4H), 1.86-1.81 (m, 6H).

### Example 74: {4-[4-(3-Pyrrolidin-1-yl-propoxy)-phenyl]-tetrahydro-pyran-4-ylmethyl}-urea

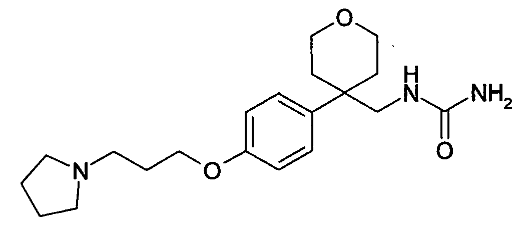

To a stirred solution of {4-[4-(3-pyrrolidin-1-ylpropoxy)phenyl]tetrahydropyran-4-yl} methylamine (200 mg, 0.63 mmol) and acetic acid (108 µL, 1.89 mmol) in water (12 mL) was added portionwise potassium cyanate (153 mg, 1.89 mmol). After stirring overnight, the reaction mixture was quenched with a 0.1N aqueous solution of NaOH. The organic layer was separated and the aqueous layer extracted twice with dichloromethane. The combined organic extracts were dried over magnesium sulfate, filtered, concentrated under reduced pressure and purified by column chromatography on silica gel eluting with DCM:MeOH (95:5) followed by DCM:MeOH (90:10) and DCM:MeOH:NH₃ (90:5:5) to give the title compound (25 mg, 11 %).
¹H NMR (400MHz, CDCl₃) δ 7.18 (d, 2H), 6.92-6.90 (d, 2H), 4.24 (s, 2H), 4.17 (t, 1H), 4.03 (t, 2H), 3.84-3.79 (m, 2H), 3.61-3.55 (m, 2H), 3.36-3.35 (d, 2H), 2.63 (t, 2H), 2.53 (m, 4H), 2.07-1.97 (m, 4H), 1.88-1.79 (m, 6H).

### Intermediate 29: 4-aminomethyl-4-[4-(3-pyrrolidin-1-yl-propoxy)phenyl]-cyclohexanol

### Step 1:

A three-neck reaction vessel was charged with [4-(3-pyrrolidin-1-ylpropoxy)phenyl]acetonitrile (7g, 28.6 mmol), benzyltrimethylammonium hydroxide (40% in methanol, 1.3 mL) in acetonitrile (185 mL). The solution was heated to reflux and methyl acrylate (25 mL, 286 mmol) was added dropwise. After refluxing for 5 hr, the mixture was concentrated to half, diethyl ether was added and the organics were washed with 1N HCl and brine, dried over Na₂SO₄, filtered and concentrated. The residue was purified by flash-chromatography to provide 4-cyano-4-[4-(3-pyrrolidin-1-ylpropoxy)-phenyl]-heptanedioic acid dimethyl ester (8.8 g, 74%).

### Step 2:

To a solution of 4-cyano-4-[4-(3-pyrrolidin-1-ylpropoxy)-phenyl]-heptanedioic acid dimethyl ester (8.8 g, 21.1 mmol) in 1,2-dimethoxyethane (176 mL) was charged portions of NaH (60% dispersion, 2.55 g, 63.4 mmol). The reaction mixture was heated to reflux. After 4.5 hr, the % of solvent were evaporated and the mixture cooled to 20°C with an ice bath and quenched with water (100 mL), HCl 1N (100 mL). The aqueous layer was extracted with ether (100 mL). After basification with NaOH the aqueous phases were extracted with dichloromethane. The combined organics were washed with water, dried over Na₂SO₄, filtrered and concentrated to provide 5-cyano-2-oxo-5-[4-(3-pyrrolidin-1-ylpropoxy)-phenyl]-cyclohexanecarboxylic acid methyl ester (6.6 g , 81%) as an oil.

### Step 3:

To a solution of 5-cyano-2-oxo-5-[4-(3-pyrrolidin-1-ylpropoxy)-phenyl]-cyclohexanecarboxylic acid methyl ester (6.6 g, 17 mmol) and dimethylsulfoxide (128 mL) was added water (8.0 mL) and sodium chloride (6.4 g, 109 mmol). The reaction mixture was heated to 142-146°C. After 5 hr, the mixture was concentrated and the residue was dissolved in DCM (120 mL), washed with water (100 mL), and brine, dried over Na₂SO₄, filtered and concentrated. The residue was purified by flash chromatography (70 g silica gel, eluant : gradient of DCM/MeOH 95/5 to 90/10) providing 4-oxo-1-[4-(3-pyrrolidin-1-ylpropoxy)-phenyl]-cyclohexanecarbonitrile as a crystallizing oil (2.2 g 45.5 %). After crystallization in ether, 0.2g of analytical sample were obtained.

### Step 4:

To a stirred suspension of LiAlH₄ (0.59 g, 15 mmol, 10 eq) in diethyl ether (8 mL) was added dropwise at ambient temperature a solution of 4-oxo-1-[4-(3-pyrrolidin-1-ylpropoxy)-phenyl]-cyclohexanecarbonitrile (0.5 g, 1.5 mmol) in diethyl ether (25 mL). The reaction mixture was heated to reflux for 2 h. The reaction mixture was cooled to 0°C and, successively, a solution of water, sodium hydroxide (15% w/v, 0.68 mL) and water again were carefully added dropwise. After being stirred for 15 min at room temperature, the mixture was filtered over diatomaceous earth and concentrated. The residue was purified by flash chromatography (10 g silica gel, DCM/ MeOH (10% NH₃ ) to provide the title compound (0.27 g, 54%) as an oil.

### Example 75: 4-dimethylaminomethyl-4-[4-(3-pyrrolidin-1-yl-propoxy)-phenyl]-cyclohexanol

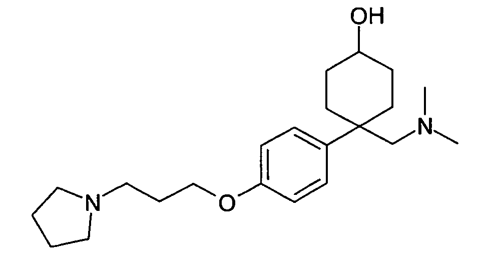

To 4-aminomethyl-4-[4-(3-pyrrolidin-1-yl-propoxy)-phenyl]-cyclohexanol (0.26 g, 0.78 mmol) was added 0.8 mL of formaldehyde (37% w/w solution in H₂O). 1 mL of formic acid was added and the mixture was heated to about 100°C for 20 min. After cooling, water was added and the mixture extracted with ether. NaOH was added at the aqueous phase and the mixture extracted 3 times with ether. The organic extracts were washed with brine, dried over Na₂SO₄, filtered and concentrated *in vacuo* to provide 0.23 g of oil. The residue was purified by flash chromatography (25 g silica gel, DCM/ MeOH (10% NH₃) to provide 0.18 g of product. After trituration with 10 mL hexanes (0.13 g, 46.4%) of title compound were obtained.
¹H NMR (400MHz, CDCl₃) δ 7.25 (d, 2H), 6.85(d, 2H), 4. (t, 2H), 3.8 (bs, 1H), 2.62 (t, 2H), 2.56-2.49 (m, 4H), 2.39 (s, 2H), 2.08-1.92 (m, 3H), 1.95 (s,6H), 1.85-1.74 (m, 6H), 1.73-1.64 (m, 4H), 1.62-1.52 (m, 2H).

### General procedure E:

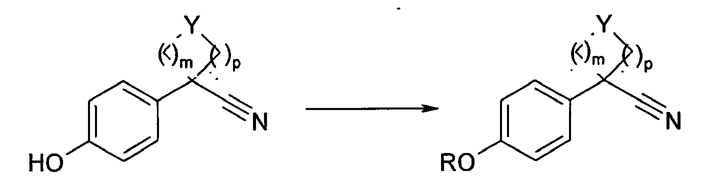

Starting phenol (3.69mmol), chloride (5.90mmol), DMF (15ml) and K₂CO₃ (14.83mmol) was heated to 130°C until the reaction is complete (1 to 2 hrs). Cooled to ambient temperature, water (30ml) was added, extracted with EtOAc (3x9ml) and the combined organic extracts were washed with NaOH (2M, 2x25ml). The organics were dried over MgSO₄, filtered and concentrated *in vacuo* at 35°C. Purification of the crude material by chromatography on silica, eluant (10%MeOH in DCM) provided the title compounds.

### Example 76: 4-[3-(3-Pyrrolidin-1-ylpropoxy)phenyl]tetrahydropyran-4-carbonitrile

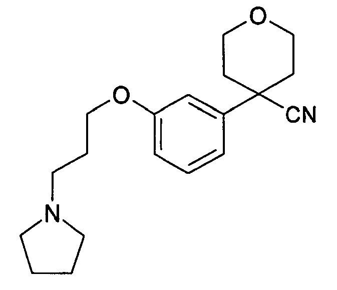

4-(3-Hydroxy-phenyl)-tetrahydro-2H-pyran-4-carbonitrile (1.03g, 5.10mmol), 1-(3-chloro-propyl)-pyrrolidine (600mg, 4.08mmol), DMF (6.8ml) and K₂CO₃ (2.82g, 20.4mmol) were reacted together according to general procedure E. The organic phase was washed with 2M NaOH (3 x 50ml), brine (3 x 50ml), dried over MgSO₄, fitered and concentrated *in vacuo* at 35°C. The crude mixture was slurried in TBME:heptane (1 :20, 10ml). The solid was filtered, washed with heptane (10ml) and dried on the filter for 2hours to give the title compound (0.4g, 31%).
¹H NMR (400MHz, CDCl₃) δ 7.32 (t, 1H), 7.07-6.99 (m, 2H), 6.87 (dd, 1H), 4.11-4.04 (m, 2H), 4.05 (t, 2H), 3.90 (td, 2H), 2.63 (t, 2H), 2.57-2.49 (m, 4H), 2.18-1.97 (m, 6H), 1.84-1.75 (m, 4H).

### Example 77: 4-{4-[3-(2,5-Dimethylpyrrolidin-1-yl)propoxy]phenyl}-tetra-hydropyran-4-carbonitrile

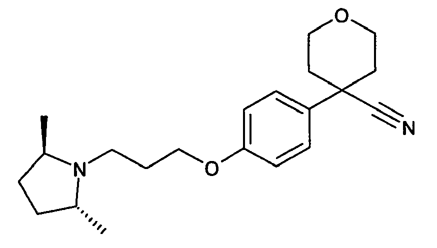

4-[4-(3-pyrrolidin-1-ylpropoxy)phenyl]tetrahydro-2H-pyran-4-carbonitrile (318mg, 1.56mmol), 1-(3-chloropropyl)-2,5-trans-dimethyl-pyrrolidine (250mg, 1.42mmol), DMF (5ml) and K₂CO₃ (785mg, 5.70mmol) were reacted together according to general procedure E. The crude was dissolved in ethyl acetate (20ml), washed with brine (2 x 10ml), dried over MgSO₄, filtered and concentrated *in vacuo* to give the title compound (200mg, 56%) as a light brown solid.
¹H NMR (400MHz, CDCl₃), δ 7.37 (d, 2H), 6.93 (d, 2H), 4.13-3.97 (m, 4H), 3.89 (td, 2H), 3.14-2.98 (m, 2H), 2.76 (m, 1H), 2.55 (m, 1H), 2.16-1.88 (m, 8H), 1.46-1.30 (m, 2H), 0.97 (d, 6H).

### Example 78: 4-{4-[3-(2-Methylpyrrolidin-1-yl)-propoxy]-phenyl}tetra-hydropyran-4-carbonitrile

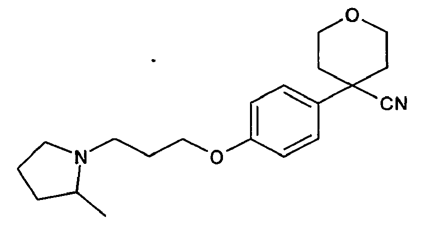

4-[4-(3-pyrrolidin-1-ylpropoxy)phenyl]tetrahydro-2H-pyran-4-carbonitrile (315mg, 1.55m mol), 1-(3-chloropropyl)-2-methyl-pyrrolidine (400mg, 2.48mmol), DMF (6ml) and K₂CO₃ (833mg, 6.03mmol) were reacted together according to general procedure E. Purification by chromatography on silica, eluant DCM:MeOH:NH₃ (92:6:2) provided the title compound (450mg, 88%) as an orange oil.
¹H NMR (400MHz, CDCl₃), δ 7.37 (d, 2H), 6.93 (d, 2H), 4.12-3.99 (m, 4H), 3.89 (td, 2H), 2.35-1.62 (m, 14H), 1.42 (m, 1H), 1.09 (d, 3H).

### Example 79: 4-[4-(3-Thiomorpholin-4-ylpropoxy)phenyl]tetrahydropyran-4-carbonitrite

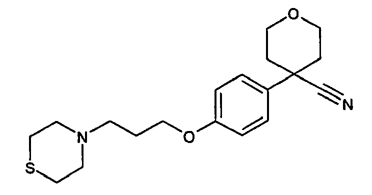

4-[4-(3-pyrrolidin-1-ylpropoxy)phenyl]tetrahydro-2H-pyran-4-carbonitrile (750mg, 3.69m mol), 4-(3-chloropropyl)-thiomorpholine (1.06g, 5.91mmol), DMF (15ml) and K₂CO₃ (2.05g, 14.83mmol) were reacted together according to general procedure E. Purification by chromatography on silica, eluant DCM:MeOH:NH₃ (96:3:1) provided the title compound (365mg, 29%) as an off white solid.
¹H NMR (400MHz, CDCl₃), δ 7.37 (d, 2H), 6.93 (d, 2H), 4.12-4.04 (m, 2H), 4.01 (t, 2H), 3.89 (td, 2H), 2.77-2.64 (m, 8H), 2.54 (t, 2H), 2.16-1.99 (m, 4H), 1.95 (p, 2H).

### Example 80: 4-{4-[3-(1-Oxo-thiomorpholin-4-yl)-propoxy]-phenyl}-tetra-hydropyran-4-carbonitrile

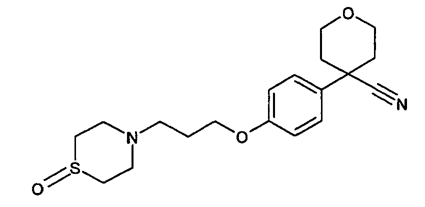

Sodium perborate tetrahydrate (221 mg, 1.43mmol) and glacial acetic acid (5ml) was heated to 50 to 60°C and 4-[4-(3-Thiomorpholin-4-ylpropoxy)phenyl]tetrahydropyran-4-carbonitrile (500mg, 1.43mmol) was added in one portion and the heating was maintained for 3 hours. The reaction mixture was cooled to ambient temperature and filtered. The filtrate was added to ice water (15ml) and extracted with EtOAc (3x5ml), which was discarded. The aqueous phase was basified to pH 8-9 with 2M sodium hydroxide and extracted with DCM (3x25ml), dried over MgSO₄, filtered and concentrated *in vacuo* at 35°C. The crude material was subjected to chromatography on silica eluting with DCM:MeOH:NH₃ (97:2:1 to 92:6:2) to provided the title compound (300mg, 58%) as a white solid.
¹H NMR (400MHz, CDCl₃), δ 7.38 (d, 2H), 6.92 (d, 2H), 4.11-4.05 (m, 2H), 4.03 (t, 2H), 3.89 (td, 2H), 3.15-3.03 (m, 2H), 2.94-2.67 (m, 6H), 2.63 (t, 2H), 2.16-1.92 (m, 6H).

### Example 81: 4-{4-[3-(1,1-Dioxo-1λ6-thiomorpholin-4-yl)propoxy]-phenyl}tetrahydro-pyran-4-carbonitrile

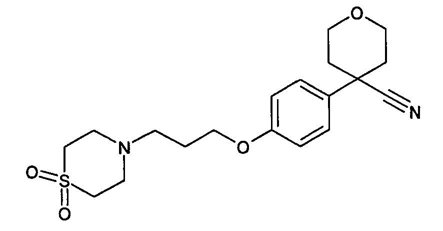

To a solution of 4-[4-(3-Thiomorpholin-4-ylpropoxy)phenyl]tetrahydropyran-4-carbonitrile (500mg, 1.44mmol) in TFA (1.65ml) at 0 to 5°C was added drop wise a solution of trifluoro-peracetic acid (4M in TFA, 0.72ml). The reaction was allowed to warm to room temperature and stirred for 3 hours. A further portion of trifluoro-peracetic acid (4M in TFA, 0.097ml) [4M solution prepared by the addition of 27.5% H₂O₂ (0.94ml) to TFA (1.56ml)] was added to the reaction and stirred overnight. The reaction mixture was cooled to 0 to 5°C, diluted with DCM (20ml) and quenched with NaOH (5M solution, 12ml) to attain pH10. The mixture was extracted with DCM (3 x 20ml), the combined DCM extracts were dried over MgSO₄, filtered and concentrated *in vacuo.* The isolated yellow oil was purified by column chromatography on basic alumina eluting with DCM(95):MeOH(4):NH₃(1) to give the title compound as an off-white solid (230mg, 42%).
¹H NMR (400MHz, CDCl₃), δ 7.39 (d, 2H), 6.92 (d, 2H), 4.12-4.05 (m, 2H), 4.03 (t, 2H), 3.89 (td, 2H), 3.11-2.96 (m, 8H), 2.71 (t, 2H), 2.15-1.99 (m, 4H), 1.96 (p, 2H).

### Example 82: 4-{4-[3-(4-Hydroxypiperidin-1-yl)propoxy]phenyl}tetrahydro-pyran-4-carbonitrile

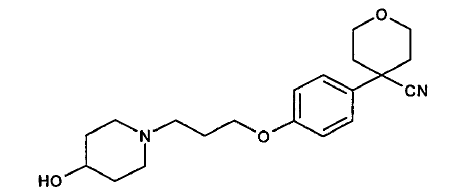

4-[4-(3-pyrrolidin-1-ylpropoxy)phenyl]tetrahydro-2*H*-pyran-4-carbonitrile (533mg, 2.62mmol), 1-(3-chloropropyl)-piperidin-4-ol (750mg, 4.22mmol), DMF (10ml) and K₂CO₃ (1.44g, 10.42mmol) were reacted together according to general procedure E. Purification by chromatography on silica, eluant DCM:MeOH:NH₃ (92:6:2) provided the title compound (550mg, 61%) as an off white solid.
¹H NMR (400MHz, CDCl₃), δ 7.38 (d, 2H), 6.93 (d, 2H), 4.13-4.04 (m, 2H), 4.02 (t, 2H), 3.89 (td, 2H), 3.70 (m, 1H), 2.87-2.71 (m, 2H), 2.50 (t, 2H), 2.23-1.84 (m, 10H), 1.68-1.49 (m, 3H).

### Example 83: 4-(4-{3-[4-(2-Hydroxyethyl)-piperidin-1-yl]propoxy}phenyl)-tetrahydro-pyran-4-carbonitrile

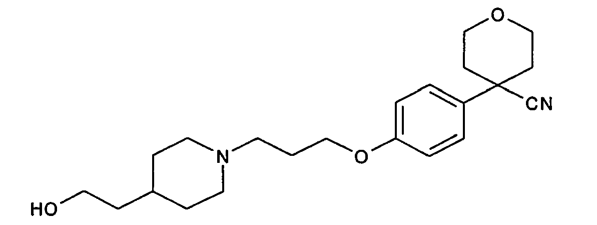

4-[4-(3-pyrrolidin-1-ylpropoxy)phenyl]tetrahydro-2*H*-pyran-4-carbonitrile (185mg, 0.91 mmol), 2-[1-(3-chloro-propyl)-piperidin-4-yl]-ethanol (300mg, 1.46mmol), DMF (4ml) and K₂CO₃ (497mg, 3.60mmol) were reacted together according to general procedure E. Purification by chromatography on silica, eluant DCM: MeOH:NH₃ (92:6:2) provided the title compound (220mg, 65%) as a pale pink solid.
¹H NMR (400MHz, CDCl₃), δ 7.37 (d, 2H), 6.93 (d, 2H), 4.11-4.04 (m, 2H), 4.01 (t, 2H), 3.89 (td, 2H), 3.70 (t, 2H), 2.97-2.87 (m, 2H), 2.48 (t, 2H), 2.15-1.88 (m, 8H), 1.77-1.57 (m, 3H), 1.57-1.38 (m, 3H), 1.35-1.21 (m, 2H).

### Example 84: 1-{3-[4-(4-Cyano-tetrahydro-pyran-4-yl)-phenoxy]-propyl}-piperidine-4-carboxylic acid amide

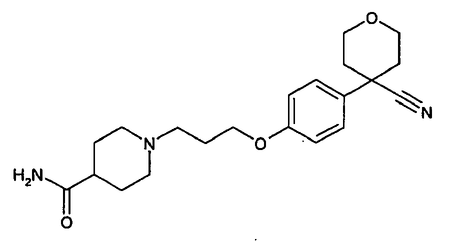

-[4-(3-pyrrolidin-1-ylpropoxy)phenyl]tetrahydro-2*H*-pyran-4-carbonitrile (200mg, 0.98mmol), 1-(3-chloropropyl)-piperidine-4-carboxylic acid amide (200mg, 0.98mmol), DMF (4ml) and K₂CO₃ (550mg, 3.98mmol) were reacted together according to general procedure E. The reaction mixture was poured onto water (30ml), the resulting precipitate was filtered and redissolved in DCM (20ml). The DCM solution was washed with NaOH solution (2M, 2x5ml) and water (5ml), dried over MgSO₄, filtered and concentrated *in vacuo* at 35°C, displacing residual DCM with EtOH to provide the title compound (210mg, 58%) as an off white solid.
¹H NMR (400MHz, CDCl₃), δ 7.38 (d, 2H), 6.93 (d, 2H), 5.45 (br s, 1H), 5.35 (br s, 1H), 4.12-4.05 (m, 2H), 4.02 (t, 2H), 3.89 (td, 2H), 3.03-2.92 (m, 2H), 2.50 (t, 2H), 2.22-1.84 (m, 11H), 1.81-1.67 (m, 2H).

### Example 85: 4-{3-[4-(4-Cyanotetrahydropyran-4-yl)-phenoxy]propyl}-piperazine-1-carboxylic acid ethyl ester

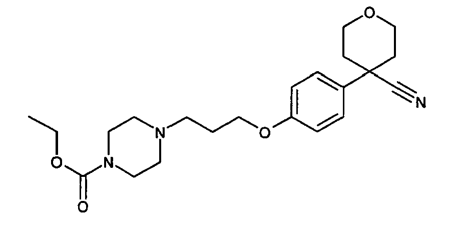

4-[4-(3-pyrrolidin-1-ylpropoxy)phenyl]tetrahydro-2*H*-pyran-4-carbonitrile (500mg, 2.46mmol), 4-(3-chloropropyl)-piperazine-1-carboxylic acid ethyl ester (570mg, 2.43mmol), DMF (10ml) and K₂CO₃ (1.36g, 9.84mmol) were reacted together according to general procedure E. The organic phase was washed with 2M NaOH (3.x 20ml), water (2x 20ml), dried over MgSO₄, fitered and concentrated *in vacuo* at 35°C. The crude product was subjected to chromatography on silica eluting with DCM:MeOH:NH₃ (97:2:1) to provide the title compound (280mg, 28%) as an off white solid.
¹H NMR (400MHz, CDCl₃), δ 7.38 (d, 2H), 6.93 (d, 2H), 4.14 (q, 2H), 4.11-4.05 (m, 2H), 4.03 (t, 2H), 3.89 (td, 2H), 3.55-3.42 (m, 4H), 2.53 (t, 2H), 2.47-2.36 (m, 4H), 2.15-1.92 (m, 6H), 1.26 (t, 3H).

### Intermediate 30: 4-(3-Chloro-propyl)-piperazine-1-carboxylic acid tert-butyl ester

4-Piperazine-1-carboxylic acid *tert*-butyl ester (4.0g, 21.5mmol), acetone (80ml, 20vol), 5M NaOH solution (5.16ml, 1.2eq.) and 1-bromo-3-chloropropane (10.15g, 64.5mmol, 3eq.) were reacted together according to general procedure A to give the title compound (2.78g, 66%) as a colourless oil.

### Example 86: 4-{3-[4-(4-Cyano-tetrahydro-pyran-4-yl)-phenoxy]-propyl}-piperazine-1-carboxylic acid tert-butyl ester

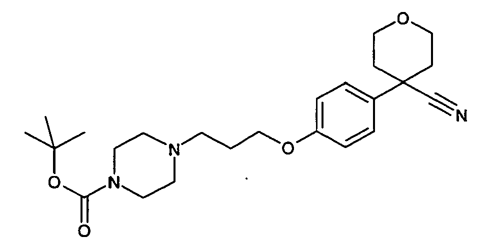

4-[4-(3-pyrrolidin-1-ylpropoxy)phenyl]tetrahydro-2*H*-pyran-4-carbonitrile (200mg, 0.99mmol), 4-(3-chloropropyl)-piperazine-1-carboxylic acid *tert*-butyl ester (203mg, 0.76mmol), DMF (4ml) and K₂CO₃ (553g, 4.00mmol) was heated to 70°C and work up according to general procedure E. The organic phase was washed with 2M NaOH (3.x 20ml), water (2x 20ml), dried over MgSO₄, fitered and concentrate in vacuo at 35°C to provided the title compound (272mg, 64%) as an off white solid.
¹H NMR (400MHz, CDCl₃), δ 7.38 (d, 2H), 6.93 (d, 2H), 4.11-4.04 (m, 2H), 4.03 (t, 2H), 3.89 (td, 2H), 3.50-3.37 (m, 4H), 2.53 (t, 2H), 2.45-2.34 (m, 4H), 2.15-2.00 (m, 4H), 1.97 (p, 2H), 1.46 (s, 9H).

### Example 87: 4-[4-(3-Piperazin-1-ylpropoxy)phenyl]tetrahydropyran-4-carbonitrile

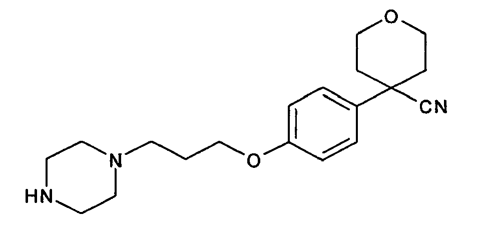

4-{3-[4-(4-Cyano-tetrahydro-pyran-4-yl)-phenoxy]-propyl}-piperazine-1-carboxylic acid *tert*-butyl ester (500mg, 1.16mmol), MeOH (5ml), 1,4-dioxane (1ml) and HCl in dioxane (4M, 1.8ml) was stirred at ambient temperature overnight. The reaction mixture was concentrated *in vacuo* at 40°C, and diluted with TBME (5ml). Aqueous NaOH (2M solution) was added to attain a pH of 14 and the mixture separated and extracted with TBME (2x5ml). The combined organic extracts were dried over MgSO₄, filtered and concentrated *in vacuo* to provide title compound as a yellow oil (275mg, 72%).
¹H NMR (400MHz, CDCl₃), δ 7.38 (d, 2H), 6.93 (d, 2H), 4.11-4.05 (m, 2H), 4.03 (t, 2H), 3.89 (td, 2H), 2.90 (t, 4H), 2.51 (t, 2H), 2.44 (br s, 4H), 2.15-2.00 (m, 4H), 1.97 (m, 2H), 1.74 (br s, 1H).

### Example 88: 4-(4-{3-[4-(2-Aminopropionyl)piperazin-1-yl]propoxy}phenyl)-tetrahydropyran-4-carbonitrile

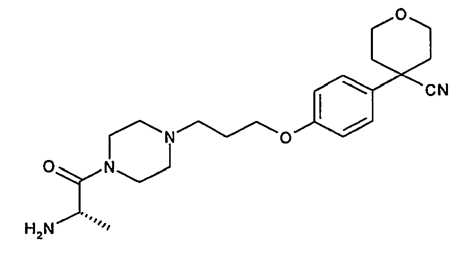

4-[4-(3-Piperazin-1-ylpropoxy)phenyl]tetrahydropyran-4-carbonitrile (265mg, 0.81 mmol), DCM (10.6ml), HOBT (120mg, 0.89mmmol), FMoc (L)-aniline (277mg, 0.89mmol) and EDCI.HCl (171mg, 0.89mmol) were stirred at ambient temperature overnight. Water (10ml) was added and stirred for one hour, the mixture was filtered and the organic phase separated and washed with water (10ml), the organic phase was dried over MgSO₄, filtered and concentrated *in vacuo* at 35°C. The residue was dissolved in DCM (9ml) and piperidine (86mg, 10mmol) was added, the mixture was stirred at ambient temperature for one hour. To the reaction mixture, water (5ml) was added and the organic phase separated, dried over MgSO₄, filtered and concentrated *in vacuo* at 35°C. The crude material was subjected to chromatography on silica eluting with 2% MeOH in DCM to provide title compound (136mg, 42%).
¹H NMR (400MHz, CDCl₃), δ 7.38 (d, 2H), 6.93 (d, 2H), 4.11-4.05 (m, 2H), 4.03 (t, 2H), 3.89 (td, 2H), 3.77 (q, 1H), 3.65 (br s, 2H), 3.49 (br s, 2H), 2.55 (t, 2H), 2.45 (br s, 4H), 2.14-2.00 (m, 4H), 1.97 (m, 2H), 1.58 (br s, 2H), 1.25 (d, 3H).

### Intermediate 31: 2-[(3-Chloro-propyl)-methyl-amino]-ethanol

2-(Methylamino)-ethanol (1.0g, 13.3mmol), acetone (20ml, 20vol), 5M NaOH solution (3.19ml, 1.2eq.) and 1-bromo-3-chloropropane (6.28g, 39.9mmol, 3eq.) were reacted together according to general procedure A to give the title compound (1.14g, 55%) as a colourless oil.

### Example 89: 4-(4-{3-[(2-Hydroxyethyl)methylamino]propoxy}-phenyl)tetra-hydropyran-4-carbonitrile

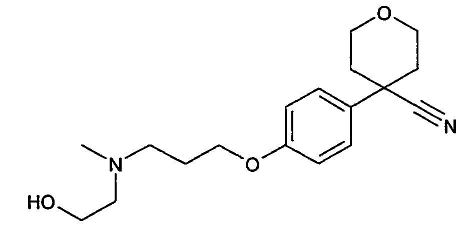

4-[4-(3-pyrrolidin-1-ylpropoxy)phenyl]tetrahydro-2H-pyran-4-carbonitrile (800mg, 3.94mmol), 2-[(3-chloropropyl)-methyl-amino]-ethanol (595mg, 3.94mmol), DMF (8ml) and K₂CO₃ (2.18g, 15.76mmol) were reacted together according to general procedure E. The organic phase was washed with 2M NaOH (3.x 20ml), water (2x 20ml), dried over MgSO₄, fitered and concentrated *in vacuo* at 35°C to provided the title compound (550mg, 44%) as a pale yellow oil.
¹H NMR (400MHz, CDCl₃), δ 7.37 (d, 2H), 6.92 (d, 2H), 4.11-4.04 (m, 2H), 3.88 (dt, 2H), 3.60 (t, 2H), 2.61 (t, 2H), 2.56 (t, 2H), 2.37 (br s, 1H), 2.29 (s, 3H), 2.15-1.91 (m, 6H)

### Example 90: 4-[4-(2-Pyrrolidin-1-ylethoxy)phenyl]tetrahydropyran-4-carbonitrile

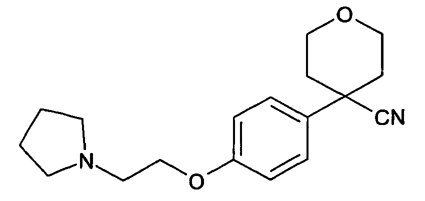

4-[4-(3-pyrrolidin-1-ylpropoxy)phenyl]tetrahydro-2*H*-pyran-4-carbonitrile (570mg, 2.82mmol), 1-(2-chloroethyl)-pyrrolidine (300mg, 2.25mmol), DMF (5.7ml) and K₂CO₃ (1.56g, 1.13mmol) were reacted together according to general procedure E. The organic phase was washed with 2M NaOH (3.x 20ml), water (2x 20ml), dried over MgSO₄, fitered and concentrated *in vacuo* at 35°C. The crude material was subjected to chromatography on silica eluting with 5% MeOH in DCM and gradient to 10% MeOH in DCM to provided the title compound (400mg, 47%) as an off white solid.
¹H NMR (400MHz, CDCL₃), δ 7.38 (d, 2H), 6.95 (d, 2H), 4.13 (t, 2H), 4.10-4.03 (m, 2H), 3.89 (td, 2H), 2.92 (t, 2H), 2.64 (br s, 4H), 2.15-1.99 (m, 4H), 1.88-1.76 (m, 4H).

### Example 91: 4-[4-(2-Methyl-3-pyrrolidin-1-ylpropoxy)phenyl]-tetrahydro-pyran-4-carbonitrile

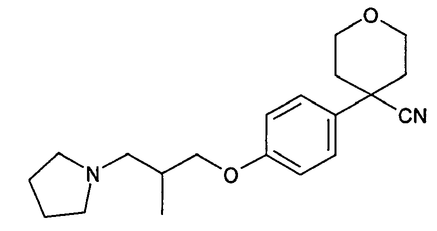

4-(4-Hydroxy-phenyl)-tetrahydro-pyran-4-carbonitrile (790mg, 3.87mmol), 1-(3-chloro-2-methyl-propyl)-pyrrolidine (500mg, 3.10mmol), DMF (5ml) and K₂CO₃ (2.14g, 15.50mmol) were reacted together according to general procedure E. The organic phase was washed with 2M NaOH (3 x 20ml), water (2 x 20ml), dried over MgSO₄, fitered and concentrated *in vacuo* at 35°C to provide the title compound (979mg, 96%) as a yellow solid.
¹H NMR (400MHz, CDCl₃), δ 7.37 (d, 2H), 6.95 (d, 2H), 4.11-4.03 (m, 2H), 4.02 (dd, 1H), 3.89 (td, 2H), 3.77 (dd, 1H), 2.60-2.42 (m, 5H), 2.32 (dd, 1H), 2.22-1.99 (m, 5H), 1.82-1.72 (m, 4H), 1.07 (d, 3H).

### Intermediate 32:1-(3-Chloro-1-methyl-propyl)-pyrrolidine hydrochloride

### Step 1:

Pyrrolidine (28.15g, 0.4mol), toluene (200ml), catalytic p-TsOH (200mg) and ethyl acetoacetate (20g, 0.15mol) were refluxed together with a Dean-Stark apparatus under N₂ for 3hours. The reaction was cooled to room temperature and concentrated *in vacuo*. NaBH₄ (3.1g, 82mmol) was dissolved in MeOH (50ml) and cooled to 0-5°C. A portion of the previous crude reaction (5g, 27mmol) in MeOH (25ml) was added to the reaction mixture and stirred for 72hours. The reaction was quenched with an aqueous solution of NaOH (1 %w/w, 50ml) and concentrated *in vacuo.* The aqueous was extratcted with TBME (3 x 50ml). The combined organic extracts were dried over MgSO₄, filtered, washed with TBME and concentrated *in vacuo* to give a mixture of the title compound, 3-pyrrolidin-1-yl-butanoic acid ethyl ester and residual TBME (3.5g). The mixture (3.5g) in THF (20ml) was added to a solution of LiALH₄ (1M in THF, 33.5ml, 33.5mmol) at 0-5°C under N₂. The reaction was allowed to warm to room temperature and stirred for 3hours. The reaction was quenched with an aqueous solution of NaOH (1%w/w, 50ml) at 0-5°C, filtered and washed with THF (3 x 20ml). The combined organics were dried over MgSO₄, filtered, washed with THF and concentrated *in vacuo* to give 3-pyrrolidin-1-yl-butan-1-ol as a yellow oil (2g, 73%).

### Step 2:

3-Pyrrolidin-1-yi-butan-1-ol (1.0g, 7mmol) was dissolved in DCM (20ml). The reaction was cooled to 0-5°C and thionyl chloride (1.65g, 14mmol) was added slowly. The reaction was allowed to warm to room temperature and stirred overnight. The reaction was concentrated *in vacuo* and azeotroped with DCM (20ml) to give the title compound (1.4g, 100%) as a brown oil.

### Example 92: 4-[4-(3-Pyrrolidin-1-ylbutoxy)phenyl]tetrahydropyran-4-carbonitrile

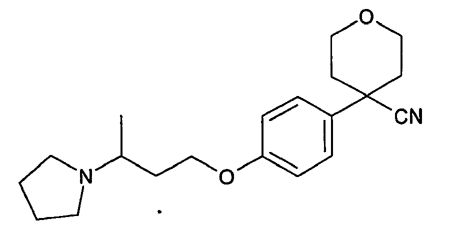

4-(4-Hydroxy-phenyl)-tetrahydro-pyran-4-carbonitrile (1.0g, 4.9mmol), 1-(3-chloro-1-methyl-propyl)-pyrrolidine (780mg, 3.90mmol), DMF (20ml) and K₂CO₃ (2.71g, 19.60mmol) were reacted together according to general procedure E. The organic phase was washed with 2M NaOH (3 x 20ml), water (2 x 20ml), dried over MgSO₄, fitered and concentrated *in vacuo* at 35°C and purified by preparative HPLC, eluting with acetonitrile/water/0.1%TFA as a gradient, to provide the title compound (120mg, 9%) as a yellow oil.
¹H NMR (400MHz, CDCl₃), δ 7.38 (d, 2H), 6.93 (d, 2H), 4.15-3.98 (m, 4H), 3.89 (td, 2H), 2.75-2.51 (m, 5H), 2.22-1.74 (m, 10H), 1.17 (d, 3H).

### Example 93: 4-{4-[2-(1-Methylpyrrolidin-2-yl)ethoxy]phenyl}tetrahydro-pyran-4-carbonitrile

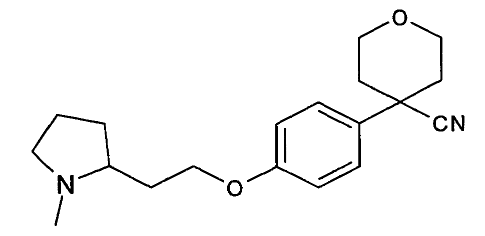

4-(4-Hydroxy-phenyl)-tetrahydro-pyran-4-carbonitrile (1.0g, 4.9mmol), 2-(2-chloro-ethyl)-1-methylpyrrolidine (722mg, 3.90mmol), DMF (20ml) and K₂CO₃ (2.71g, 19.60mmol) were reacted together according to general procedure E. The organic phase was washed with 2M NaOH (3 x 20ml), water (2 x 20ml), dried over MgSO₄, fitered and concentrated *in vacuo* at 35°C and purified by prep TLC, eluting with DCM :MeOH :NH₃ (95 :5 :3), to provide the title compound (180mg, 15%) as a yellow oil.
¹H NMR (400MHz, CDCl₃), δ 7.38 (d, 2H), 6.93 (d, 2H), 4.12-4.03 (m, 4H), 3.89 (td, 2H), 3.08 (m, 1H), 2.35 (s, 3H), 2.29-1.94 (m, 8H), 1.85-1.65 (m, 2H), 1.62-1.50 (m, 2H).

### Example 94: 4-[4-(1-Isopropylpiperidin-4-yloxy)phenyl]tetrahydropyran-4-carbonitrile

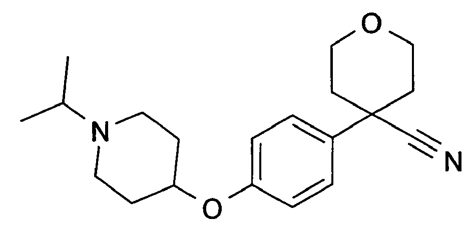

4-(4-Hydroxy-phenyl)-tetrahydro-pyran-4-carbonitrile (459mg, 2.26mmol) in DMF (2ml) was added to a solution of NaH (100mg, 2.5mmol) in DMF (2ml) at room temperature under N₂. The reaction was stirred for 1 hr and a solution of methanesulfonic acid 1-isopropyl-piperidin-4-yl ester (400mg, 1.81mmol) in DMF (1.3ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo*. The crude reaction was purified by successive column chromatography, eluting with DCM :MeOH : NH₃ (98 :1 :1) to give the title compound as a pale yellow solid (116mg, 19%).
¹H NMR (400MHz, CDCl₃) δ 7.37 (d, 2H), 6.93 (d, 2H), 4.30 (m, 1H), 4.12-4.02 (m, 2H), 3.89 (td, 2H), 2.84-2.68 (m, 3H), 2.45-2.34 (m, 2H), 2.15-1.96 (m, 6H), 1.88-1.75 (m, 2H), 1.06 (d, 6H).

### Example 95: 4-[4-(1-Cyclopentylpiperidin-4-yloxy)phenyl]tetrahydropyran-4-carbonitrile

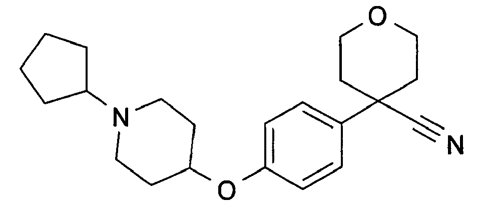

4-(4-Hydroxy-phenyl)-tetrahydro-pyran-4-carbonitrile (459mg, 2.26mmol) in DMF (2ml) was added to a solution of NaH (100mg, 2.5mmol) in DMF (2ml) at room temperature under N₂. The reaction was stirred for 1 hr and a solution of methanesulfonic acid 1-cyclopentyl-piperidin-4-yl ester (447mg, 1.81 mmol) in DMF (1.3ml) was slowly added. The reaction was heated to 75°C and stirred for 6hrs. The reaction was allowed to cool to room temperature and diluted with TBME (20ml), water (10ml) and 5M NaOH solution (10ml). The organic layer was washed with 2.5M NaOH (2 x 20ml), brine (2 x 20ml), dried over MgSO₄, filtered and concentrated *in vacuo*. The crude reaction was purified by successive column chromatography, eluting with DCM :MeOH : NH₃ (98 :1 :1) to give the title compound as a pale yellow solid (81mg, 13%).
¹H NMR (400MHz, CDCl₃) δ 7.37 (d, 2H), 6.93 (d, 2H), 4.36 (m, 1H), 4.11-4.04 (m, 2H), 3.89 (td, 2H), 2.91-2.77 (m, 2H), 2.67-2.25 (m, 2H), 2.15-1.99 (m, 7H), 1.95-1.81 (m, 4H), 1.78-1.64 (m, 2H), 1.62-1.39 (m, 4H).

### Intermediates 33 and 34: 4-[4-(3-chloro-propoxy)-phenyl]-tetrahydropyran-4-carbonitrile and 4-[4-(3-bromo-propoxy)-phenyl]-tetrahydropyran-4-carbonitrile

4-(4-hydroxy-phenyl)-tetrahydro-pyran-4-carbonitrile (3.4 g, 16.73 mmol) was taken in DMF (75 ml) and then Cs₂CO₃ (4.84 g, 25.095 mmol) was added followed by dropwise addition of 1-bomo-3-chloroproprane (2.45 ml) at 60 °C. The heating was continued at 60 °C overnight. First the reaction mixture was concentrated and quenched by adding water and extracted with ethyl acetate. The compound was purified by coloumn chromatography (0-14 %, ethyl acetate-hexane). Yield: 3.3 g (70 % of a 10:3 mixture of chloro and bromo compound).

### General procedure F:

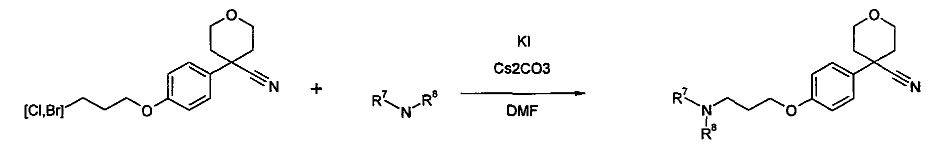

A mixture of halide (0.7 mmol), Cs₂CO₃ (0.350 g, 1.07 mmol), potassium iodide (0.180 g, 1.07 mmol) and amines (NR⁷R⁸) (1.07 mmol) in DMF (7 mL) was heated to 70°C overnight. The mixture was cooled to ambient temperature, water was added, and the resulting mixture was extracted with dichloromethane and the organic extracts were dried over Na₂SO₄, filtered and concentrated *in vacuo*. Purification of the crude material was carried out with flash chromatography (20 g silica gel, DCM/ MeOH gradient).

### Example 96: 4-[4-(3-morpholino-4-ylpropoxy)phenyl]tetrahydropyran-4-carbonitrile

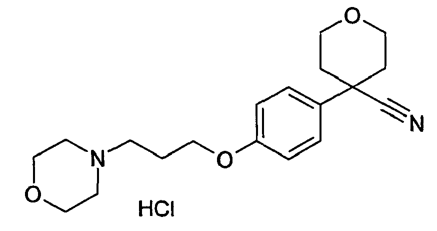

The compound was obtained according the general procedure F, using morpholine as amine. The hydrochloride salt as analytical sample was prepared with ether/HCl 2M in dichloromethane and crystallization in ether.
Yield = 34%
¹H NMR (400MHz, DMSO-D6) δ 1.95-2.12 (m, 4H); 2.16-2.25 (m, 2H); 3.01-3.12 (m, 2H); 3.19-3.27 (m, 2H); 3.40-3.48 (m, 2H); 3.60-3.69 (t, 2H); 3.80-3.89 (m, 2H); 3.91-4.03 (m, 4H); 4.08-4.13 (m, 2H); 7.20 (d, 2H); 7.46 (d, 2H); 11.33 (bs, 1H)

### Example 97: 4-{4-[3-(4-methyl-piperazin-1-yl)-propoxy]phenyl}tetrahydro-pyran-4-carbonitrile

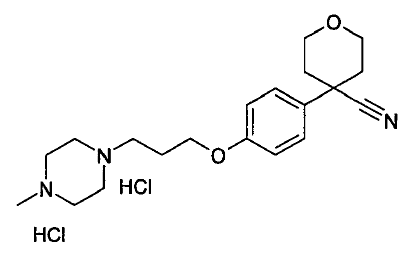

The compound was obtained according the general procedure F, using 1-methylpiperazine as amine. The hydrochloride salt as analytical sample was prepared with ether/HCl 1 M in dichloromethane and triturated with pentane.
Yield = 57%
¹H NMR (400MHz, DMSO-D₆) δ 11.85 (bs, 1H), 7.46 (d, 2H), 7.02 (d, 2H), 4.13 -4.07 (m, 2H), 4.03 -3.96 (m, 2H), 3.84-3.18 (m, 12H), 2.87-2.78 (m, 3H), 2.24-2.13 (m, 2H), 2.11-1.96 (m, 4H).

### Example 98: 4-{4-[3-(2-methyl-piperidin-1-yl)-propoxy]phenyl}-tetrahydro-pyran-4-carbonitrile

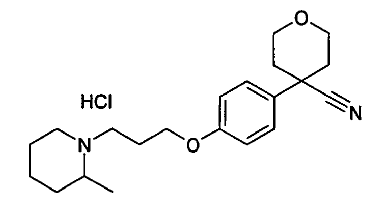

The compound was obtained according the general procedure F, using 2-methylpiperidine as amine. The hydrochloride salt as analytical sample was prepared with ether/HCl 1 M in dichloromethane and trituration with pentane.
Yield = 28%
¹H NMR (400MHz, DMSO-D6) δ 10.22 (bs, 1H), 7.46 (d, 2H), 7.01(d, 2H), 4.13-4.06 (m, 2H), 4.03-3.96 (dd, 2H), 3.69-3.60 (t, 2H), 3.48-3.38 (m, 1H), 3.25-3.06 (m, 3H), 3.01-2.90 (m, 1H), 2.21-1.95 (m, 7H), 1.88-1.57 (m, 5H), 1.56-1.39 (m, 1H), 1.32 (d, 2H), 1.24 (d, 1H).

### Example 99: 4-{4-[3-(3-methyl-piperidin-1-yl)-propoxy]phenyl}-tetrahydro-pyran-4-carbonitrile

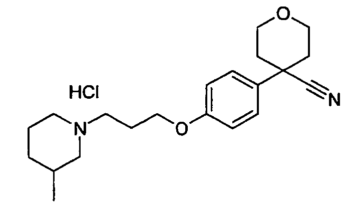

The compound was obtained according the general procedure F, using 3-methylpiperidine as amine. The hydrochloride salt as analytical sample was prepared with ether/HCl 1M in dichloromethane and trituration with pentane.
Yield = 52%
¹H NMR (400MHz, DMSO-D6) δ 10.32 (bs, 1H), 7.46 (d, 2H), 7.01 (d, 2H), 4.11-4.05 (t, 2H), 4.03-3.96 (dd, 2H), 3.69-3.60 (t, 2H), 3.48-3.35 (m, 2H), 3.19-3.11 (m, 2H), 2.82-2.72 (m, 1H), 2.58-2.45 (m, 1H), 2.24-2.16 (m, 2H), 2.12-1.90 (m, 5H), 1.85-1.70 (m, 3H), 1.12-1.02 (m, 1H), 0.89 (d, 3H).

### Example 100: 4-{4-[3-(4-methyl-piperidin-1-yl)-propoxy]phenyl}-tetrahydro-pyran-4-carbonitrile

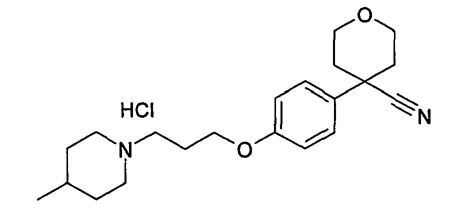

The compound was obtained according the general procedure F, using 4-methylpiperidine as amine. The hydrochloride salt as analytical sample was prepared with ether/HCl 1M in dichloromethane and trituration with pentane.
Yield = 60%
¹H NMR: (400MHz, DMSO-D6) δ 10.18 (bs, 1H), 7.46 (d, 2H), 7.01 (d, 2H), 4.11-4.05 (t, 2H), 4.03-3.96 (dd, 2H), 3.69-3.60 (t, 2H), 3.49-3.42 (m, 2H), 3.19-3.11 (m, 2H), 2.94-2.83 (m, 2H), 2.22-2.13 (m, 2H), 2.11-1.95 (m, 4H), 1.81-1.73 (m, 2H), 1.65-1.54 (m, 1H), 1.53-1.41 (m, 2H), 0.91 (d, 3H).

### Example 101: 4-[4-(3-azepan-1-ylpropoxy)phenyl]tetrahydropyran-4-carbonitrile

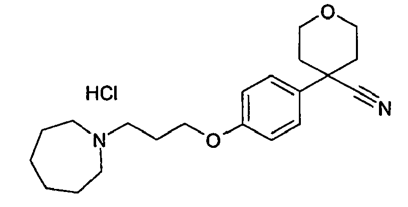

The compound was obtained according the general procedure F, using hexamethyleneimine as amine. The hydrochloride salt as analytical sample was prepared with ether/HCl 1M in dichloromethane and trituration with ether.
Yield = 9%
¹H NMR (400MHz, DMSO-D6) δ 10.54 (bs, 1H), 7.41 (d, 2H), 7.01(d, 2H), 4.11-4.05 (t, 2H), 4.03-3.96 (dd, 2H), 3.69-3.60 (dt, 2H), 3.41-3.30 (m, 2H), 3.24-3.17 (m, 2H), 3.16-3.06 (m, 2H), 2.24-2.16 (m, 2H), 2.12-1.95 (m, 4H), 1.92-1.76 (m, 4H), 1.72-1.52 (m, 4H).

### Example 102: 4-{4-[3-(4,4-difluoro-piperidin-1-yl)-propoxy]phenyl}-tetra-hydropyran-4-carbonitrile

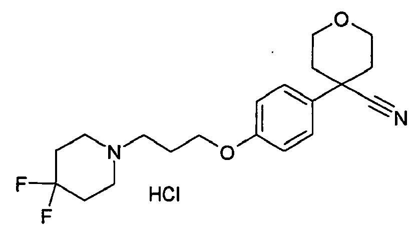

The compound was obtained according the general procedure F, using 4,4-difluoropiperidine hydrochloride as amine (add (C₂H₅)₃N to obtain the free base ). The hydrochloride salt as analytical sample was prepared with ether/HCl 1M in dichloromethane and trituration with ether.
Yield = 9%
¹H NMR : (400MHz, DMSO-D6) δ 10.28 (bs, 1H), 7.46 (d, 2H), 7.01(d, 2H), 4.12-4.07 (t, 2H), 4.03-3.96 (dd, 2H), 3.69-3.59 (dt, 4H), 3.33-3.25 (m, 2H), 3.21-3.11 (m, 2H), 2.39-2.27 (m, 2H), 2.27-2.17 (m, 2H), 2.12-1.95 (m, 4H).

### Example 103: 4-[4-(5-Pyrrolidin-1-ylpentyloxy)phenyl]tetrahydropyran-4-carbonitrile

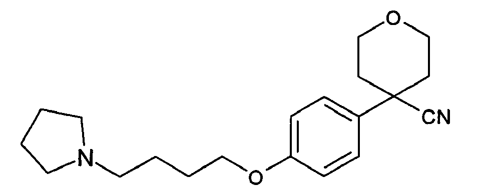

4-(4-Hydroxy-phenyl)-tetrahydro-pyran-4-carbonitrile (1.5g, 7.39mmol), 1-bromo-4-chlorobutane (1.7ml, 14.77mmol), DMF (20ml) and K₂CO₃ (4.1g, 29.56mmol) were reacted together according to general procedure E. Purification by chromatography on silica, eluant ethyl acetate:heptanes (33:66) provided a mixture of bromo and chloro phenoxy ether (1.3g, 60%) which was used in the next stage. A portion of the mixture (0.5g, 1.70mmol) and pyrrolidine (0.42ml, 5.11mmol) were refluxed in EtOH (5ml, 10vol) overnight. The reaction was concentrated *in vacuo* and partitioned between 2M NaOH (10ml) and ethyl acetate (10ml). The aqueous was extracted with ethyl acetate (3 x 10mL). The combined ethyl acetate layers were washed with brine (3 x 10ml), dried over MgSO₄, filtered, washed with ethyl acetate and concentrated *in vacuo.* Purification by chromatography on silica, eluant DCM :MeOH :NH₃ (95:4:1) increasing gradually to DCM :MeOH :NH₃ (93 :6 :1), gave the title compound as a pale yellow solid (350mg, 63%).
¹H NMR (400MHz, DMSO-d₆) δ 7.38 (d, 2H), 6.92 (d, 2H), 4.11-4.03 (m, 2H), 3.99 (t, 2H), 3.89 (td, 2H), 2.58-2.43 (m, 6H), 2.15-1.99 (m, 4H), 1.89-1.64 (m, 8H).

### Intermediate 35: 1-[4-(3-bromo-propoxy)-phenyl]-cyclohexanecarbonitrile

1-(4-Hydroxyphenyl)cyclohexanecarbonitrile (2.0 g, 10 mmol) was taken in acetone (150 mL) and then Cs₂CO₃ (8.1 g, 25 mmol) was added followed by dropwise addition of 1,3 dibromopropane (5.1 mL, 50 mmol). The mixture was heated for 3 h at 70°C. After cooling and filtration, the mixture was concentrated and the residue was purified by flash chromatography (50 g silica gel, DCM) to provide the title compound (2.8 g, 87%). Note: presence of allyl derivative as impurity.

### General procedure G:

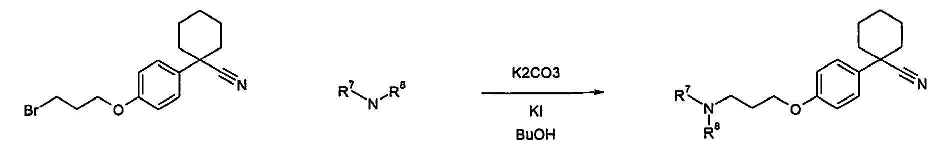

A mixture of 1-[4-(3-bromo-propoxy)-phenyl]-cyclohexanecarbonitrile (0.660 g, 2.0 mmol), amine (NR⁷R⁸) (0.260g, 3.0 mmol), sodium carbonate (0.320 g, 3 mmol), potassium iodide (20 mg) and 20 mL of butanol was heated for 4 h at 100°C. After cooling, water was added to quench the reaction and the mixture was extracted with DCM. The organics extracts were washed with a saturated aqueous solution of NaHCO₃, water and dried over Na₂SO₄, filtered and concentrated. The residue was purified by flash chromatography (DCM and DCM / MeOH (10% NH₃) to provide (0.440 g, 67%).

### Example 104: 1-[4-(3-piperidin-1-yl-propoxy)-phenyl]-cyclohexane-carbonitrile

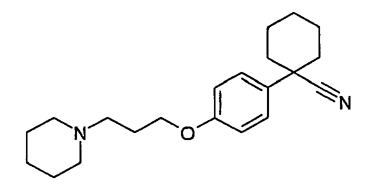

A mixture of 1-[4-(3-bromo-propoxy)-phenyl]-cyclohexanecarbonitrile (0.660 g, 2.0 mmol), piperidine (0.260g, 3.0 mmol), sodium carbonate (0.320 g, 3 mmol), potassium iodide (20 mg) and 20 mL of butanol was heated for 4 h at 100°C according to general procedure G. After cooling, water was added to quench the reaction and the mixture was extracted with DCM. The organics extracts were washed with a saturated aqueous solution of NaHCO₃, water and dried over Na₂SO₄, filtered and concentrated. The residue was purified by flash chromatography (DCM and DCM / MeOH (10% NH₃) to provide the title compound (0.440 g, 67%).
¹H NMR (400MHz, CDCl₃) δ 7.37 (d, 2H), 6.90(d, 2H), 4.00 (t, 2H), 2.49-2.36 (m, 6H), 2.17-2.09 (m, 2H), 1.97 (qt, 2H), 1.88-1.67 (m, 7H), 1.62-1.55 (m, 4H), 1.47-1.40 (m, 2H), 1.32-1.20 (m, 1H).

### General procedure H:

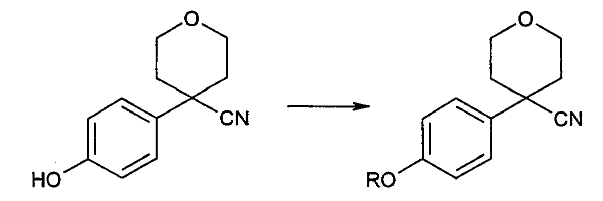

4-(4-Hydroxy-phenyl)-tetrahydro-pyran-4-carbonitrile (1eq.), alcohol (R-OH) (0.8eq.) and PPh₃ (1eq.) were mixed together in THF (10vol) and cooled to 0°C under N₂. DIAD (1eq.) in THF (10vol) was added slowly to the reaction and allowed to warm to room temperature overnight. The reaction was quenched with 2M HCl solution (10vol) and extracted with ethyl acetate (3 x 10vol). The aqueous was basified to pH 14 with NaOH (∼30vol) and extracted with ethyl acetate (3 x 10vol). The organics were dried over MgSO₄, filtered and concentrated *in vacuo* at 35°C. Purification of the crude material by chromatography on silica, eluant (10%MeOH in DCM) provided the title compounds.

### Example 105: 4-[4-(2,2-Dimethyl-3-pyrrolidin-1-ylpropoxy)phenyl]tetrahydro-pyran-4-carbonitrile

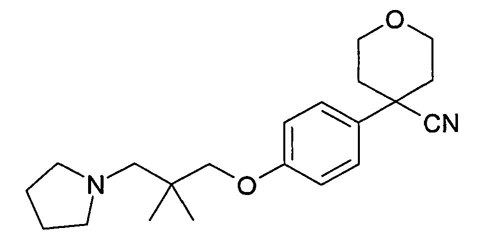

4-(4-Hydroxy-phenyl)-tetrahydro-pyran-4-carbonitrile (1.29g, 6.4mmol), 2,2-dimethyl-3-pyrrolidin-1-yl-propan-1-ol (0.8g, 5.1mmol), PPh₃ (1.67g, 6.4mmol), THF (16ml) and DIAD (1.25ml, 6.4mmol) were reacted together according to general procedure H. The crude material was subjected to chromatography on silica eluting with DCM :MeOH (98 :2). The resulting solid was slurried in TBME:heptanes (1:2, 1ml) to provide the title compound (120mg, 5%) as a white solid.
¹H NMR (400MHz, CDCl₃), δ 7.37 (d, 2H), 6.94 (d, 2H), 4.12-4.03 (m, 2H), 3.89 (td, 2H), 3.71 (s, 2H), 2.57 (br s, 4H), 2.47 (s, 2H), 2.15-1.99 (m, 4H), 1.70 (brs, 4H), 1.00 (s, 6H).

### Example 106: 4-[4-(3-Pyrrolidin-1-ylpropoxy)phenyl]tetrahydro-2H-pyran-4-carboxylic acid amide

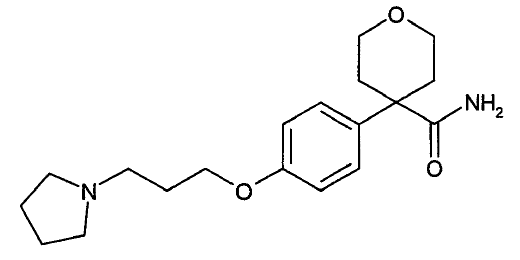

4-[4-(3-Pyrrolidin-1-yl-propoxy)-phenyl]-tetrahydro-pyran-4-carbonitrile (400 mg, 1.27 mmol) was treated with polyphosphoric acid at 80°C overnight. After allowing the mixture to cool to room temperature, ethyl acetate and water were added. The organic layer was separated. The aqueous layer was basified to pH 10 with concentrated NaOH and extracted with DCM. The combined organic extracts were dried over Na₂SO₄, filtered, concentrated *in vacuo* and purified by column chromatography on silica gel eluting to give the title compound (88 mg, 26%).
¹H NMR (400MHz, CDCl₃) *δ* 7.29 (d, 2H), 6.91(d, 2H), 5.64-5.30 (d, 2H), 4.02 (t, 2H), 3.76 (t, 4H), 2.62 (t, 2H), 2.52 (m, 4H), 2.37-2.30 (m, 2H), 2.08-1.96 (m, 4H), 1.81-1.75 (m, 4H).

### Example 107: 4-[4-(3-Pyrrolidin-1-ylpropoxy)phenyl]tetrahydro-2H-pyran-4-carboxylic acid

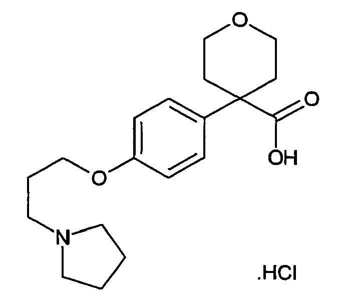

A solution of 4-[4-(3-pyrrolidin-1-ylpropoxy)phenyl]tetrahydro-2*H*-pyran-4-carbonitrile (20g, 0.0636mol) in concentrated hydrochloric acid (200ml, 10vol) was heated and stirred at reflux for 16h after which time LC/LCMS analysis showed the reaction had stalled at 85-90% conversion. The solution was evaporated to dryness *in vacuo,* azeotroped with methanol (200ml, 20vol) and toluene (2x 200ml, 2x 20vol) to yield title compound as a beige solid which was used directly.
¹H NMR (400 MHz, CDCl₃) δ 7.18 (d, 2H), 6.96 (d, 2H), 4.12 (t, 2H), 3.94-3.84 (m, 2H), 3.74-3.66 (m, 2H), 3.60 (dt, 2H), 3.42 (t, 2H), 2.50-2.44 (m, 2H), 2.26-2.00 (m, 8H), 2.96-2.84 (m, 2H);HRMS (ESI⁺) 334.2013 (M+H)⁺.

### Intermediate 36: Methyl 4-[4-(3-pyrrolidin-1-ylpropoxy)phenyl]tetrahydro-2H-pyran-4-carboxylate

A suspension of 4-[4-(3-pyrrolidin-1-ylpropoxy)phenyl]tetrahydro-2*H*-pyran-4-carboxylic acid (23.5g, 0.0636mol) in methanol (235ml, 10vol) was stirred and cooled to 0 to -5°C under nitrogen. Thionyl chloride (9.3ml, 0.1272mol, 0.40vol) was charged dropwise over 20minutes, maintaining the temperature - 5 to +5°C. The resulting slurry was then heated and stirred at reflux under nitrogen for 16h after which time LC analysis showed 1.4% by area of acid remaining. The solution was evaporated to dryness *in vacuo* at 40°C to give a brown sludge. The sludge was dissolved in ethyl acetate (118ml, 5vol) and water (235ml, 10vol) and the layers separated. The aqueous layer was washed with ethyl acetate (118ml, 5vol) and basified to pH 11 with saturated aqueous potassium carbonate (118ml, 5vol). The product was extracted into dichloromethane (3x 118ml, 3x 5vol) and the combined extracts dried over magnesium sulfate (23g, 1wt) and concentrated *in vacuo* at 40°C to yield title compound (21.7g, 98% from the nitrile) as a cream solid.

### Example 108: {4-[4-(3-Pyrrolidin-1-ylpropoxy)phenyl]tetrahydro-2H-pyran-4-yl}methanol

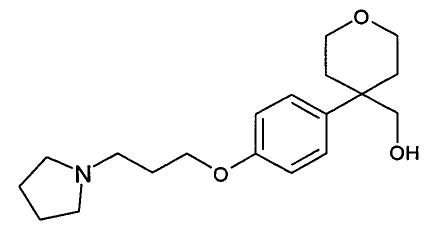

Anhydrous tetrahydrofuran (141ml, 10vol) was charged rapidly onto stirred and cooled (0-5°C) lithium aluminium hydride (6.2g, 0.1634mol, 0.44wt) under nitrogen (Note: very exothermic). The resulting slurry was cooled to 0-5°C and then a solution of methyl 4-[4-(3-pyrrolidin-1-ylpropoxy)phenyl]tetrahydro-2*H-*pyran-4-carboxylate (14.1g, 0.04058mol) in tetrahydrofuran (99ml, 7vol) was added dropwise over 30 minutes maintaining 0-5°C. On complete addition the reaction was allowed to warm to room temperature over 30 minutes and stirred at 18-25°C for 30 minutes, LC analysis showed the reaction to be complete. The mixture was cooled to 0 to 5°C, a 1:1 mixture of tetrahydrofuran and water (18.6ml, 1.32vol) was added very slowly (Note: very exothermic, gas evolution, temperature maintained below 16°C, toward the end of the quench the reaction mixture sets and then becomes freer with continued stirring.). The mixture was further quenched with 20% w/v sodium hydroxide solution (6.2ml, 0.44vol) and water (18.6ml, 1.32vol) and the resulting white suspension stirred vigorously for 30 minutes at 18-25°C. The slats were removed by filtration and rinsed with tetrahydrofuran (3x 150ml). The combined filtrates were evaporated to dryness at 40°C to yield title compound (12.1g, 94%) as a white solid.
¹H NMR (400MHz, CDCl₃) δ 7.25 (d, 2H), 6.90 (d, 2H), 4.05 (t, 2H), 3.80 (dt, 2H), 3.55 (td, 2H), 2.60 (t, 2H), 2.62-2.41 (m, 4H), 2.10 (dt, 2H), 2.00 (p, 2H), 2.00-1.85 (m, 2H), 1.95-1.72 (m, 4H).

### Example 109: 1-[4-(3-pyrrolidin-1-ylpropoxy)phenyl]cyclo-pentane-carbonitrile

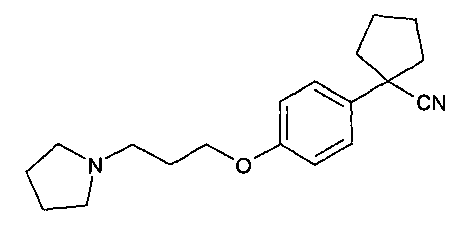

To a suspension of NaH (60%, 0.574 g, 14.35 mmol) in DMF (8 mL) at 0°C was added dropwise [4-(3-pyrrolidin-1-ylpropoxy)phenyl]acetonitrile (1 g, 4.10 mmol) in DMF (3 mL). The reaction mixture was stirred at 0°C for 5 min than at room temperature for 30 min. After cooling to 0°C, 1,4-dibromobutane (0.980 mL, 8.2 mmol) in DMF (2 mL) was added dropwise. The reaction mixture was allowed to warm up to room temperature then heated to 50°C overnight. It was poured into ice-cold water and extracted with DCM. The combined organic extracts were washed with brine, dried over Na₂SO₄, filtered and concentrated *in vacuo.* The crude mixture was purified by column chromatography, eluting with a gradient of DCM:MeOH:NH₃ (from 99:0:1 to 90:10:1) to give the title compound (0.443 g, 36%).
¹H NMR (400MHz, DMSO-*d₆*) *δ* 7.29 (d, 2H), 6.79 (d, 2H), 4.02 (m, 2H), 3.18 (m, 2H), 2.47-2.28 (m, 4H), 2.19-1.75 (m, 11H), 1.19 (m, 3H).

### Example 110: 4-oxo-1-[4-(3-pyrrolidin-1-ylpropoxy)-phenyl]-cyclohexane-carbonitrile

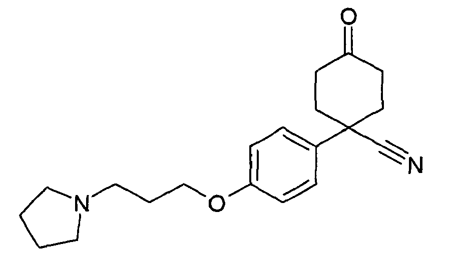

### Step 1:

A three neck reaction vessel was charged with [4-(3-pyrrolidin-1-ylpropoxy)phenyl]acetonitrile (7g, 28.6 mmol), benzyltrimethylammonium hydroxide (40% in methanol, 1.3 mL) in acetonitrile (185 mL). The solution was heated to reflux and methyl acrylate (25 mL, 286 mmol) was added dropwise. After refluxing for 5 hr, the mixture was concentrated to half, diethyl ether was added and the organics were washed with HCl 1N and brine, dried over Na₂SO₄, filtered and concentrated. The residue was purified by flash-chromatography to provide 4-cyano-4-[4-(3-pyrrolidin-1-ylpropoxy)-phenyl]-heptanedioic acid dimethyl ester (8.8 g, 74%).
¹H NMR (400MHz, CDCl₃) *δ* 7.28 (d, 2H); 6.95 (d, 2H); 4.08-4.0 (m, 2H); 3.6 (s, 6H); 2.68-2.60 (m, 2H); 2.58-2.45 (m, 6H); 2.40-2.20 (m, 4H); 2.20-2.12 (m, 2H); 2.08-2.0 (m, 2H); 1.80-1.75 (m, 4H)

### Step 2:

To a solution of 4-cyano-4-[4-(3-pyrrolidin-1-ylpropoxy)-phenyl]-heptanedioic acid dimethyl ester (8.8 g, 21.1 mmol) in 1,2-dimethoxyethane (176 mL) was charged portions of NaH (60% dispersion, 2.55 g, 63.4 mmol). The reaction mixture was heated to reflux. After 4.5 hr, the ¾ of solvent were evaporated and the mixture cooled to 20°C with an ice bath and quenched with water (100 mL), HCl 1N (100 mL). The aqueous layer was extracted with ether (100 mL). After basification with NaOH the aqueous phases were extracted with dichloromethane. The combined organics were washed with water, dried over Na₂SO₄, filtrered and concentrated to provide 5-cyano-2-oxo-5-[4-(3-pyrrolidin-1-ylpropoxy)-phenyl]-cyclohexanecarboxylic acid methyl ester (6.6 g, 81%) as an oil.
¹H NMR (400MHz, CDCl₃) δ 12.2 (bs, 1 H); 7.33 (d, 2H); 6.95 (d, 2H); 4.08-4.00 (m, 2H); 3.75 (s, 3H); 2.95 (d, 1H); 2.80-2.70 (m, 1H); 2.65-2.60 (m, 2H); 2.50-2.42 (m, 6H); 2.30-2.15 (m, 2H); 2.05-1.95 (m, 2H); 1.90-1.85 (m, 4H).

### Step 3:

To a solution of 5-cyano-2-oxo-5-[4-(3-pyrrolidin-1-ylpropoxy)-phenyl]-cyclohexanecarboxylic acid methyl ester (6.6 g, 17 mmol) and dimethylsulfoxide (128 mL) was added water (8.0 mL) and sodium chloride (6.4 g, 109 mmol). The reaction mixture was heated to 142-146°C. After 5 hr, the mixture was concentrated and the residue was dissolved in DCM (120 mL), washed with water (100 mL), and brine, dried over Na₂SO₄, filtered and concentrated. The residue was purified by flash chromatography (70 g silica gel, eluant: gradient of DCM/MeOH 95/5 to 90/10) providing 4-oxo-1-[4-(3-pyrrolidin-1-ylpropoxy)-phenyl]-cyclohexanecarbonitrile as a crystallizing oil (2.2 g, 45.5%). After crystallization in ether, 0.2g of analytical sample were obtained.
¹H NMR (400MHz, CDCl₃) *δ* 7.32 (d, 2H), 6.88 (d, 2H), 4.1 (t, 2H), 2.95-2.8 (m, 2H), 2.65-2.38 (m, 10H), 2.3-2.15 (m, 2H), 2.0-1.9 (m, 2H), 1.8-1.7 (m, 4H).

### Example 111: 4-hydroxy-1-[4-(3-pyrrolidin-1-ylpropoxy)-phenyl]cyclo-hexanecarbonitrile

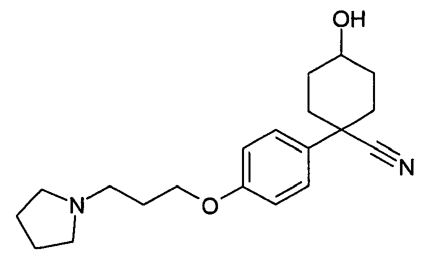

To a stirred suspension of LiAlH₄ (0.070 g, 1.85 mmol, 5 eq) in diethyl ether (1.9 mL) was added dropwise at 0°C a solution of 4-oxo-1-[4-(3-pyrrolidin-1-ylpfopoxy)-phenyl]-cyclohexanecarbonitrile (0.12 g, 0.37 mmol) in diethyl ether (5.5 mL). The reaction mixture was stirred at ambient temperature for 4 h .The reaction mixture was cooled to 0°C and, successively, a solution of water, sodium hydroxide (15% w/v, 0.08 mL) and water again were carefully dropped. After being stirred for 15 min at room temperature, the mixture was filtered over diatomaceous earth and concentrated. The residue was purified by flash chromatography (10 g silica gel, DCM/ MeOH (10% NH₃) to provide 0.050 g of title compound.
¹H NMR (400MHz,DMSO-D6) *δ* 10.6 (bs, 1H), 7.42 (d, 2H), 7(d, 2H), 4.1-3.95 (m, 2H), 3.6-3.2 (m, 5H), 3.03-2.87 (m, 2H), 2.15-1.8 (m, 10H), 1.65-1.5 (m, 2H).

### Intermediate 37: (R)-2-Methylpyrrolidine

Pure enantiomers of 2-methyl pyrrolidine can be obtained by resolution with +/- tartaric acid as described in Acta. Pharm. Suecica 15, 255-263; 1978.

### Intermediate 38: 3-[(2R)-2-Methylpyrrolidin-1-yl]propan-1-ol

3-Bromopropan-l-ol (5.28 mL, 58.4 mmol) was added to a solution of (R)-2-methylpyrrolidine (7.1 g, 58.4 mmol) in water (200 mL). KOH (7.53 g, 134 mmol) was then added and the mixture stirred at ambient temperature for 48 hours. The reaction mixture was then extracted with dichloromethane (5 x 80 mL) and ethyl acetate (5 x 80 mL). The combined organic extracts were dried using MgSO₄, filtered and concentrated *in vacuo.* The compound was purified by column chromatography on Biotage® silica gel, eluting with dichloromethane:methanol:ammonia, (90:10:1) to give the title compound as a pale brown oil (5.44, 65 %).
LCMS (APCl⁺) 144 (M+H)⁺.

### Intermediate 39: tert-Butyl (3S)-3-methoxypyrrolidine-1-carboxylate

Sodium hydride (80 % dispersion in mineral oil, 940 mg, 0.024 mmol) was added in two portions at 0 °C to a solution of *tert*-butyl (3*S*)-3-hydroxypyrrolidine-1-carboxylate (4.00 g, 0.02 mmol). The reaction mixture was allowed to warm to room temperature and stirred for 1 h before being cooled once more to 0 °C. Methyl iodide (2.00 mL, 0.032 mmol) was added slowly and the reaction mixture warmed to room temperature overnight. The mixture was poured slowly onto 200 mL of ice and stirred until the ice was fully thawed. The product was extracted using dichloromethane (2 x 250 mL), dried (Na₂SO4) and concentrated *in vacuo*. The compound was purified by column chromatography on Biotage® silica gel, eluting with dichloromethane to give the product as a colourless oil (2.88 g, 67 %).

### Intermediate 40: (3S)-3-Methoxypyrrolidine

A solution of trifluoroacetic acid (2.5 ml) in dichloromethane (5 mL) was added slowly at 0 °C to a solution of *tert*-Butyl (3*S*)-3-methoxypyrrolidine-1-carboxylate (2.88 g, mmol) and the reaction allowed to warm to room temperature and stirred for 2.5 h. The reaction mixture was quenched with saturated sodium carbonate solution (100mL) and extracted with dichloromethane (2 x 200mL). The organics were combined, dried over magnesium sulphate and concentrated *in vacuo*. The residue was taken up in dichloromethane (30 mL) and cooled to 0 °C in an ice bath. Hydrogen chloride gas was bubbled through the suspension for 1 hour and the reaction mixture allowed to stir at room temperature for 48 hours. The reaction mixture was basified with saturated sodium hydrogencarbonate solution (100 mL) and extracted with dichloromethane (2 x 200mL) and ethyl acetate (3 x 150mL). The aqueous was concentrated *in vacuo* and then extracted with warm methanol to yield the title product, 2.00g.

### Intermediate 41: (3R)-1-Benzyl-3-methoxypyrrolidine

(3R)-1-Benzyl-3-methoxypyrrolidine was prepared as described in the following patent WO 91/08206 and taken on to the next step as the crude mixture.

### Intermediate 42: (3R)-3-Methoxypyrrolidine

A solution of (3R)-1-Benzyl-3-methoxypyrrolidine (2.35 g, 12.3 mmol) in methanol (50 mL) containing concentrated HCl (1 mL) was hydrogenated at ambient temperature at 50 psi in the presence of a catalytic amount of 10 % Pd(OH)₂ on carbon (250 mg, 10% w/w). The reaction mixture was filtered over celite and rinsed with dichloromethane (60 mL) and methanol (60 mL) to remove the catalyst. The organic layer was basified with NaOH (2.0M, 10 mL) and extracted with diethylether (3 x 30 mL). The aqeous layer was further acidified to pH3 using concentrated HCl, concentrated *in vacuo* and azeotroped with toluene to give the product as a yellow solid which was further used without purification.

### Intermediate 43: 2,2-Dimethylpyrrolidine

Step 1: 1-Benzyl-2,2-dimethylpyrrolidine was prepared according to the procedure described in the following reference. S. M. Denton and A. Wood, Synlett, 1999, 55.

Step 2: A solution of 1-Benzyl-2,2-dimethylpyrrolidine (1.02g, 5.40 mmol) in ethanol (80 mL) and concentrated HCl (0.5 mL) was hydrogenated over 20 % Pd(OH)₂ (100 mg, 10 % w/w) at 60 psi at ambient temperature for 4 hours. The reaction mixture was filtered over arbocel to remove the catalyst and a further 2 mL of concentrated HCl was added to the crude product. The reaction mixture was then concentrated *in vacuo* and azeotropically dried using toluene to give the product as a brown solid, which appeared to be slightly hygroscopic (732 mg, 100 %).

### Intermediate 44: (2R,5S)-2,5-Dimethylpyrrolidine

The free base of cis-2,5-dimethylpyrrolidine was prepared as described by C. G. Overberger, L. C. Palmer, B. S. Marks and N. R. Byrd, J. Am. Chem. Soc., 1955, 77, 4100. This was then acidified using HCl (2.0 M solution in diethylether) which, upon filtration and subsequent recrystallisation from acetonitrile and i-propylalcohol gave the title compound as a white crystalline solid (5.29 g, 22 %) in a ratio of approximately 10:1 ratio of cis:trans.

### Intermediates 45 and 46: 1-[4-(3-bromo-propoxy)phenyl]cyclohexane-carbonitrile and 1-[4-(3-chloro-propoxyl-phenyl]-cyclohexane-carbonitrile

K₂CO₃ (3.02 g, 21.9 mmol) was added to a solution of 1-(4-hydroxyphenyl)cyclohexanecarbonitrile (4.0 g, 19.9 mmol) in DMF (50 mL). 1-bromo-3-chloroproprane (1.97 ml, 19.9 mmol) was then added dropwise and the reaction heated at 60°C overnight. The reaction mixture was concentrated, quenched by adding water (50 mL), extracted with dichloromethane (2 x 50 mL) and the organic extracts were dried over Na₂SO₄, filtered and concentrated *in vacuo.* The compound was purified by column chromatography on Biotage® silica gel, eluting with diethylether:dichloromethane, 50:50 to 0:100. Yield: 5.32 g (96 % of a 3:1 mixture of chloro and bromo compound).

### Example 112: 1-(4-{3-[(2R)-2-(methoxymethyl)pyrrolidin-1-yl]propoxy}-phenyl)cyclohexanecarbonitrile

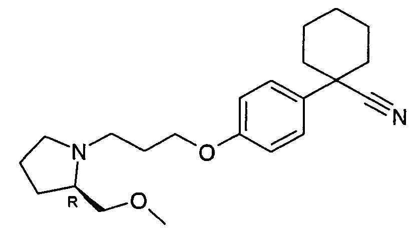

A (1:3) mixture of 1-[4-(3-bromo-propoxy)-phenyl]-cyclohexanecarbonitrile and 1-[4-(3-chloro-propoxy)-phenyl]-cyclohexanecarbonitrile (2.00g, 7.20 mmol), Cs₂CO₃ (2.58 g, 7.20 mmol), potassium iodide (0.20 g, 1.20 mmole) and (R)-(-)-2-(methoxymethyl)pyrrolidine (1.07 mL, 8.60 mmol) in NMP (20 mL) was heated to 75°C overnight. The reaction mixture was cooled to ambient temperature, concentrated *in vacuo,* and quenched by adding water (30 mL). The crude product was extracted with ethyl acetate (30 x mL) and the organic extracts were dried over Na₂SO₄, filtered and concentrated *in vacuo.* The compound was purified by column chromatography on Biotage® silica gel, eluting with dichloromethane:methanol:ammonia, 100:0:0 to 90:10:1 to give the title compound as a brown oil (1.39 g, 54%).
¹H NMR (400MHz, CDCl₃) *δ* 7.38 (d, 2H), 6.70 (d, 2H), 4.02 (t, 2H), 3.46-3.38 (m, 1H), 3.36-3.28 (m, 3H), 3.24-3.16 (m, 1H), 3.12-3.04 (m, 1H), 2.72-2.58 (m, 1H), 2.52-2.44 (m, 1H), 2.30-2.20 (m, 1H), 2.18-2.06 (m, 2H), 1.98 (pentet, 2H), 1.92-1.60 (m, 12H), 1.36-1.18 (m, 1H); HRMS (ESI⁺) 357.2537 (M+H)⁺.

### Example 113:1-(4-{3-[(3S)-3-methoxypyrrolidin-1-yl]propoxy}phenyl)cyclo-hexanecarbonitrile

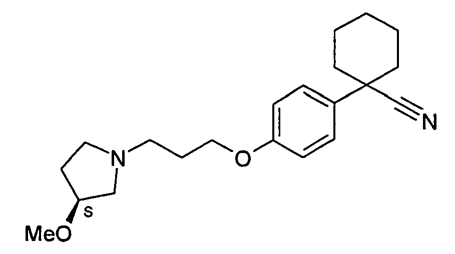

A (1:3) mixture of 1-[4-(3-bromo-propoxy)-phenyl]-cyclohexanecarbonitrile and 1-[4-(3-chloro-propoxy)-phenyl]-cyclohexanecarbonitrile (200 mg, 0.72 mmol), *N,N*-diisopropylethylamine (251 µL, 1.44 mmol) and (*S*)-3-methoxy-pyrrolidine (198 mg, 1.96 mmol) in NMP (0.5 mL) was heated at 160 °C in the microwave (Smith Personal Synthesiser) for 400 seconds. The reaction mixture was quenched with water (10 mL) and extracted with dichloromethane (2 x 10 mL), dried over Na₂SO₄, filtered and concentrated *in vacuo.* The compound was purified by column chromatography on Biotage® silica gel, eluting with dichloromethane:methanol:ammonia, 97:3:0.3 to 95:5:0.5. to give the product as a clear oil (102 mg, 41%).
¹H NMR (400MHz, CDCl₃) *δ* 7.36 (d, 2H), 6.90 (d, 2H), 4.02 (t, 2H), 3.96-3.88 (m, 1H), 3.28 (s, 3H), 2.86-2.52 (m, 6H), 2.18-1.96 (m, 5H), 1.88-1.66 (m, 8H), 1.38-1.16 (m, 1H); LCMS (APCI⁺) 343 (M+H)⁺.

### Example 114:1-(4-{3-[(3R)-3-methoxypyrrolidin-1-yl]propoxy}phenyl)-cyclohexanecarbonitrile

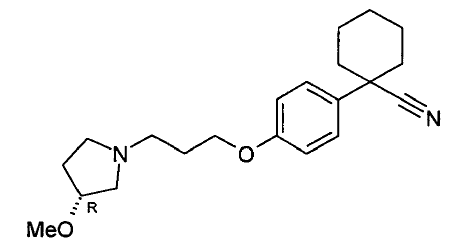

A (1:3) mixture of 1-[4-(3-bromo-propoxy)-phenyl]-cyclohexanecarbonitrile and 1-[4-(3-chloro-propoxy)-phenyl]-cyclohexanecarbonitrile (200 mg, 0.72 mmol), *N,N*-diisopropylethylamine (251 µL, 1.44 mmol) and (R)-3-methoxy-pyrrolidine (198 mg, 1.44 mmol) in NMP (0.5 mL) was heated at 160 °C in the microwave (Smith Personal Synthesiser) for 600 seconds. The reaction mixture was quenched with water (30 mL) and extracted with ethylacetate (2 x 30 mL), dried over Na₂SO₄, filtered and concentrated *in vacuo.* The compound was purified by column chromatography on Biotage® silica gel, eluting with dichloromethane:methanol:ammonia, 98:2:0.2 to 95:5:0.5 to give a brown oil (37 mg, 11 %).
¹H NMR (400MHz, CDCl₃) *δ* 7.38 (d, 2H), 6.90 (d, 2H), 4.02 (t, 2H), 3.94-3.88 (m, 1H), 3.28 (s, 3H), 2.74-2.66 (m, 2H), 2.64-2.56 (m, 2H), 2.46 (quintet, 2H), 2.17-1.94 (m, 5H) 1.88-1.66 (m, 8H) 1.32-1.18 (m, 1H); HRMS (ESI⁺) 343.2380 (M+H)⁺.

### Example 115: 4-(4-{3-[(2R,5S)-2,5-dimethylpyrrolidin-1-yl]propoxy}-phenyl)-tetrahydro-2H-pyran-4-carbonitrile

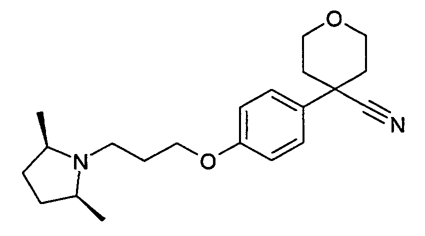

A (1:3) mixture of 1-[4-(3-bromo-propoxy)-phenyl]-cyclohexanecarbonitrile and 1-[4-(3-chloro-propoxy)-phenyl]-cyclohexanecarbonitrile (150 mg, 0.54 mmol), *N,N*-diisopropylethylamine ( 187 µL, 1.07 mmol) and (2R,5S)-2,5-dimethylpyrrolidine (145 mg, 1.07 mmol) in NMP (0.5 mL) was heated at 160°C in the microwave (Smith Personal Synthesiser) for 400 seconds then 170 °C for 600 seconds. The reaction mixture was quenched with NaHCO₃ (15 mL) and extracted with ethyl acetate (2 x 15 mL), dried over Na₂SO₄, filtered and concentrated *in vacuo.* The compound was purified by column chromatography on Biotage® silica gel, eluting with dichloromethane:methanol:ammonia, 97:3:0.3 to 90:10:1. Yield = 28 %
¹H NMR (400MHz, CDCl₃) *δ* 7.36 (d, 2H), 6.92 (d, 2H), 4.10-4.04 (m, 2H), 4.00 (t, 2H), 3.88 (dt, 2H), 2.72 (t, 2H), 2.64-2.52 (m, 2H), 2.14-1.98 (m, 4H), 1.94 (pentet, 2H), 1.86-1.78 (m, 2H), 1.42-1.28 (m, 2H), 1.10 (d, 6H); HRMS (ESI⁺) 343.2380 (M+H)⁺.

### Example 116: 1-{4-[3-(2,2-dimethylpyrrolidin-1-yl)propoxy]phenyl}cyclo-hexanecarbonitrile

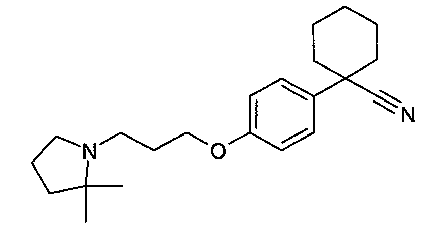

A (1:3) mixture of 1-[4-(3-bromo-propoxy)-phenyl]-cyclohexanecarbonitrile and 1-[4-(3-chloro-propoxy)-phenyl]-cyclohexanecarbonitrile (150 mg, 0.54 mmol), *N,N*-diisopropylethylamine (188 µL, 1.08 mmol) and 2,2-dimethyl-pyrrolidine (131 mg, 1.08 mmol) in NMP (0.5 mL) was heated at 180°C in the microwave (Smith Personal Synthesiser) for 600 seconds. The reaction mixture was quenched with water (15 mL) and extracted with ethyl acetate (2 x 15 mL), dried over Na₂SO₄. filtered and concentrated *in vacuo.* The compound was purified by column chromatography on Biotage® silica gel, eluting with dichloromethane:methanol:ammonia, 98:2:0.2 to 95:5:0.5 to give the title compound as a brown oil (31 mg, 17 %).
¹H NMR (400MHz, CDCl₃) *δ* 7.38 (d, 2H), 6.90 (d, 2H), 4.04 (t, 2H), 2.78 (t, 2H), 2.54 (t, 2H), 2.16-2.12 (m, 2H), 1.94 (pentet, 2H), 1.90-1.64 (m, 11H), 1.20-1.34 (m, 1H), 0.98 (s, 6H); HRMS (ESI⁺) 341.2588 (M+H)⁺.

### Example 117: -1-[1-(4-{3-[(2R)-2-(methoxymethyl)pyrrolidin-1-yl]propoxy} phenyl)cyclohexyl]methanamine

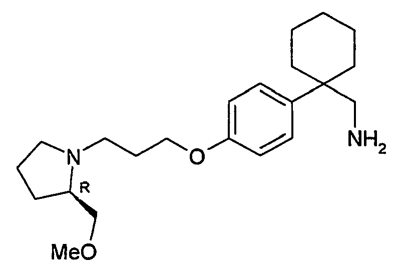

To a stirred suspension of LiAlH₄ (1.0M in diethyl ether, 13.7 ml, 13.7 mmol) at 0°C under an atmosphere of nitrogen was added 1-(4-{3-[(2R)-2-(methoxymethyl)pyrrolidin-1-yl]propoxy}phenyl)cyclohexanecarbonitrile (0.980 g, 2.7 mmol) in Et₂O (15 mL) over 15 minutes maintaining the temperature at 5 to 10°C. The reaction mixture was allowed to warm up to ambient temperature for 20 minutes then refluxed for 30 minutes until complete. The reaction mixture was cooled to 0°C, water (0.5 mL) was added followed by NaOH (2.0M, 1.5 mL) and water (0.5 mL). Ethyl acetate (5 mL) was added and the mixture filtered through a short pad of celite, eluting with ethyl acetate (2 x 15 mL). The organic washings were dried over Na₂SO₄ and concentrated *in vacuo.* The compound was purified by column chromatography on Biotage® silica gel, eluting with dichloromethane:methanol:ammonia, 98:2:0.2 to 95:5:0.5 to give the title compound as a clear oil (309 mg, 32 %).
¹H NMR (400MHz, CDCl₃) *δ* 7.22 (d, 2H), 6.88 (d, 2H), 4.04-3.96 (m, 2H), 3.32 (m, 3H), 3.26-3.20 (m, 1H), 3.14-3.04 (m, 1H), 2.74-2.62 (m, 1H), 2.60 (s, 2H), 2.56-2.46 (m, 1H), 2.28 (quartet, 1H), 2.12-1.98 (m, 4H), 1.96-1.90 (m, 1H), 1.82-1.70 (m, 2H), 1.68-1.46 (m, 8H), 1.36-1.30 (m, 3H); HRMS (ESI⁺) 361.2850 (M+H)⁺.

### Example 118: {[1-(4-{3-[(3S)-3-methoxypyrrolidin-1-ylpropoxy}-phenyl)-cyclohexyl]methyl}amine

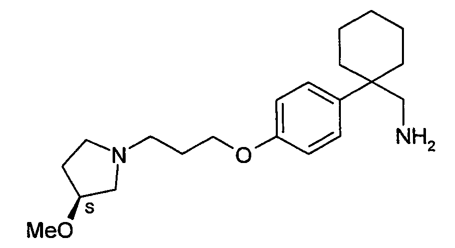

To a stirred suspension of LiAlH₄ (1.0M in diethyl ether, 1.46 ml, 1.46 mmol) at 0°C under an atmosphere of nitrogen was added a solution of 1-(4-{3-[(3*S*)-3-methoxypyrrolidin-1-yl]propoxy}phenyl)cyclohexanecarbonitrile (0.10 g, 0.29 mmol) in Et₂O (2 mL) over 15 minutes maintaining the temperature at 5 to 10°C. The reaction mixture was allowed to warm up to ambient temperature for 20 minutes then refluxed for 30 minutes until complete. The reaction mixture was cooled to 0°C, water (0.5 ml) was added dropwise followed by sodium hydroxide (2.0M, 1.5 ml) and water (0.5 ml). Ethyl acetate (5 mL) was added and the mixture filtered through a short pad of celite, eluting with ethyl acetate (2 x 15 mL). The organic washings were dried over Na₂SO₄ and concentrated *in vacuo*. The compound was purified by column chromatography on Biotage® silica gel, eluting with dichloromethane:methanol:ammonia, 97:3:0.3 to 90:10:1 to give the title compound as a clear oil (75 mg, 75%).
¹H NMR (400MHz, CDCl₃) *δ* 7.20 (d, 2H), 6.86 (d, 2H), 4.02 (t, 2H), 3.94-3.88 (m, 1H), 3.28 (s, 3H), 2.76-2.68 (m, 2H), 2.58-2.66 (m, 3H), 2.45-2.52 (m, 1H), 2.12-2.04 (m, 3H), 1.98 (pentet, 2H), 1.84-1.74 (m, 1H), 1.56-1.44 (m, 5H), 1.38-1.30 (m, 3H), 1.16-1.25 (m, 2H); HRMS (ESI⁺) 347.2693 (M+H)⁺.

### Example 119: N-methyl-1-{1-[4-(3-pyrrolidin-1-ylpropoxy)phenyl]cyclo-hexyl}methanamine

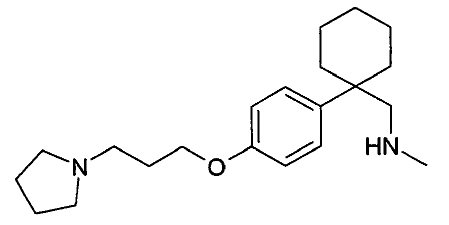

To a stirred solution of ({1-[4-(3-pyrrolidin-1-ylpropoxy)phenyl]cyclo-hexyl}methyl)amine (0.60 g, 1.90 mmol) and *N,N*-diisopropylethylamine (330µL, 1.90 mmol) in dry dichloromethane (6 mL) at 0 ° C was added dropwise a solution of di-*tert*-butyldicarbonate (455 mg, 2.09 mmol) in dry dichloromethane (4 mL). The reaction was allowed to warm to ambient temperature and stirred for a further 2 hours. The reaction mixture was concentrated *in vacuo*, partitioned between NaHCO₃ (20 mL) and dichloromethane (20 mL) and extracted with a further 20 mL of dichloromethane. The organic layers were combined, dried over Na₂SO₄ and concentrated under reduced pressure to provide the crude product. The compound was purified by column chromatography on silica gel, eluting with DCM/MeOH/NH₃ (95:5:0.5) to give *tert*-butyl ({1-[4-(3-pyrrolidin-1-ylpropoxy)phenyl]cyclohexyl}methyl)-carbamate as a clear oil (642 mg, 81 %).
¹H NMR complicated by the presence of rotamers.
¹H NMR (400 MHz, CDCl₃) δ 7.24 (d, 2H), 6.90 (d, 2H), 4.12 (brs, 1H), 4.02 (t, 2H), 3.24-3.06 (m, 2H), 2.66 (t, 2H) 2.56-2.46 (m, 4H), 2.08-1.94 (m, 4H), 1.84-1.72 (m, 4H), 1.62-1.54 (m, 4H), 1.48-1.28 (m, 13H) LCMS (ESI⁺) 417 (M+H)⁺ 439 (M+Na)⁺.

### Step 2:

A solution of *tert*-butyl ({1-[4-(3-pyrrolidin-1-ylpropoxy)phenyl]cyclohexyl}methyl)-carbamate (600 mg, 1.44 mmol) in Et₂O (5 mL) was added dropwise to a stirred solution of LiAlH₄ (1.0M solution in Et₂O, 4.32 mL, 4.32 mmol) at 0°C. THF (5 mL) was added and the reaction mixture heated at reflux overnight. Reaction mixture was cooled to 0°C quenched with water (0.5 mL), aqueous NaOH (2.0M, 0.5 mL) and water (0.5 mL). Dichloromethane and ethyl acetate were added and the mixture filtered over a short pad of celite, and concentrated *in vacuo*. The compound was purified by column chromatography on Biotage® silica gel, eluting with dichloromethane:methanol:ammonia, 96:4:0.4 to 90:10:1 to give the title compound as a clear oil (230 mg, 48 %).
¹H NMR (400 MHz, CDCl₃) δ 7.16 (d, 2H), 6.88 (d, 2H), 4.02 (t, 2H), 2.64 (t, 2H) 2.54 (s, 2H), 2.50-2.58 (m, 4H), 2.26 (s, 3H), 2.14-2.08 (m, 2H), 1.98 (pentet, 2H), 1.84-1.76 (m, 4H), 1.68-1.56 (m, 2H), 1.52-1.32 (m, 6H); HRMS (ESI⁺) 331.2744 (M+H)⁺.

### Example 120: [(1-{4-[3-(2,2-dimethylpyrrolidin-1-yl)propoxy]phenyl}-cyclo-hexyl)methyl]amine

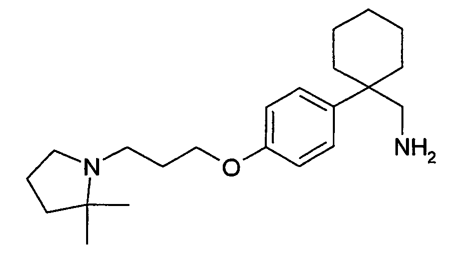

To a stirred suspension of LiAlH₄ (1.0M in diethyl ether, 0.4 ml, 0.40 mmol) at 0°C under an atmosphere of nitrogen was added 1-{4-[3-(2,2-dimethylpyrrolidin-1-yl)propoxy]phenyl}cyclohexanecarbonitrile (27 mg, 0.079 mmol) in Et₂O (15 mL) over 15 minutes maintaining the temperature at 5 to 10°C. The reaction mixture was allowed to warm up to ambient temperature for 20 minutes then refluxed for 30 minutes until complete. The reaction mixture was cooled to 0°C, water (0.5 mL) was added followed by NaOH (2.0M, 1.5 mL) and water (0.5 mL). Ethyl acetate (5 mL) was added and the mixture filtered through a short pad of celite, eluting with ethyl acetate (2 x 15 mL) and concentrated *in vacuo.* The compound was purified by column chromatography on Biotage® silica gel, eluting with dichloromethane:methanol:ammonia, 97:3:0.3 to give the title compound as a clear oil (10 mg, 37 %).
¹H NMR (400 MHz, CDCl₃) δ 7.22 (d, 2H), 6.84 (d, 2H), 4.02 (t, 2H), 2.86-2.76 (m, 2H), 2.66 (s, 2H), 2.58-2.50 (m, 2H), 2.16-2.06 (m, 2H), 2.00-2.90 (m, 2H), 1.84-1.72 (m, 2H), 1.70-1.64 (m, 2H), 1.56-1.46 (m, 4H), 1.40-1.32 (m, 4H), 1.02 (s, 6H). HRMS (ESI⁺) 345.2901 (M+H)⁺.

### Example 121: N-ethyl-N-({4-[4-(3-pyrrolidin-1-ylpropoxy)phenyl]tetrahydro-2H-pyran-4-yl}methyl)ethanamine

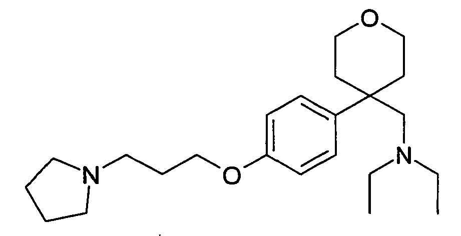

A solution of *N*-ethyl-*N*-({4-[4-(3-pyrrolidin-1-ylpropoxy)phenyl]tetrahydro-2*H*-pyran-4-yl}methyl)acetamide (119mg, 0.307 mmol) in tetrahydrofuran (1 mL) was added dropwise at 0°C to a solution of LiAlH₄ (1.0M in diethylether, 0.614 mL, 0.614 mmol) under an atmosphere of nitrogen. The reaction mixture was allowed to warm to ambient temperature overnight. The reaction mixture was cooled to 0°C, water (0.5 mL) was then added dropwise followed by sodium hydroxide (2.0M, 0.5 mL) and water (0.5 mL) again. The resulting solid was filtered over a small pad of celite, washed with DCM (100 mL) and concentrated *in vacuo.* The compound was purified by column chromatography on Biotage® silica gel, eluting with dichloromethane:methanol:ammonia, 94.5:5.5:0.55 to 91:9:0.9 to give the title compound as a pale yellow oil (61 mg, 53%).
¹H NMR (400 MHz, CDCl₃) δ 7.18 (d, 2H), 6.86 (d, 2H), 4.02 (t, 2H), 3.70-3.78 (m, 2H), 3.48 (dt, 2H), 2.64 (t, 2H), 2.58-2.46 (m, 4H), 2.38 (s, 2H), 2.20 (quartet, 4H), 2.13-1.96 (m, 4H), 1.92-1.86 (m, 2H), 1.82-1.78 (m, 4H), 0.82 (t, 6H); HRMS (ESI⁺) 375.3006 (M+H)⁺.

### Example 122: N-ethyl-N-({4-[4-(3-pyrrolidin-1-ylpropoxy)phenyl]tetrahydro-2H-pyran-4-yl}methyl)acetamide

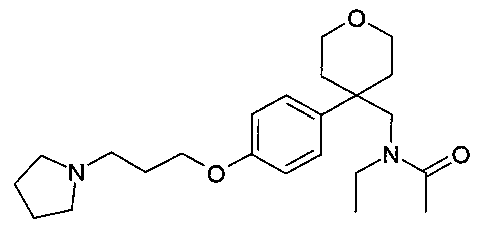

Acetyl chloride (14 µL, 0.20 mmol) was added dropwise to a solution of *N*-({4-[4-(3-pyrrolidin-1-ylpropoxy)phenyl]tetrahydro-2*H*-pyran-4-yl}methyl)ethanamine (70 mg, 0.20 mmol) and triethylamine (28 µL, 0.20 mmol) in THF (0.50 mL) at 0°C and the reaction stirred for 2 hours. The reaction was quenched using NaHCO₃ (10 mL), extracted with dichloromethane (3x10 mL) and the organic extracts dried over Na₂SO₄, filtered and concentrated *in vacuo.* The compound was purified by column chromatography on silica, eluting with DCM/MeOH/NH₃ (95:5:0.5) to provide the title compound as a clear oil (33 mg, 42 %).
¹H NMR complicated by the presence of rotamers.
¹H NMR (400 MHz, CDCl₃) δ 7.20-7.12 (m, 2H), 6.92-6.86 (m, 2H), 4.06-4.00 (m, 2H), 3.88-3.76 (m, 2H), 2.56-2.48 (m, 2H), 3.42 (s, 2H), 2.66 (t, 2H), 2.58-2.52 (m, 4H), 2.48 (quartet, 2H), 2.08 (s, 3H), 2.06-1.92 (m, 6H), 1.84-1.76 (m, 4H), 0.98 (t, 0.6 H) 0.86 (t, 2.4H). HRMS (ESI⁺) 389.2799 (M+H)⁺.

### Example 123: N-methyl-N-({4-[4-(3-pyrrolidin-1-ylpropoxy)phenyl]-tetrahydro-2H-pyran-4-yl}methyl)ethanesulfonamide

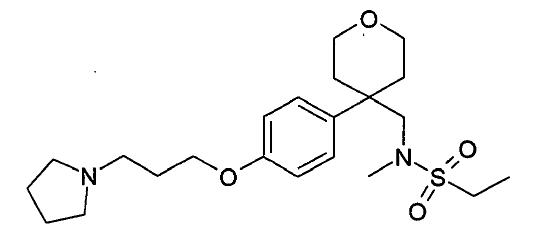

Ethanesulfonyl chloride (43 µL, 0.456 mmol) was added dropwise to a solution of triethylamine (58 µL, 0.418 mmol) and *N-*methyl-1-{1-[4-(3-pyrrolidin-1-ylpropoxy)phenyl]cyclohexyl}methanamine (125 mg, 0.38 mmol) in dichloromethane (1 mL) at 0°C and the reaction stirred for 30 minutes. The reaction was quenched with NaHCO₃ (20 mL), extracted using dichloromethane (3x10 mL), dried using Na₂SO₄ and concentrated *in vacuo.* The compound was purified by column chromatography on Biotage® silica gel, eluting with dichloromethane:methanol:ammonia, 98:2:0.2 to 96:4:0.4 to give the title compound as a pale brown oil (152 mg, 94%).
¹H NMR (400 MHz, CDCl₃) δ 7.24 (d, 2H), 6.90 (d, 2H), 4.04 (t, 2H), 3.84-3.76 (m, 2H), 2.56-2.50 (m, 2H), 3.26 (s, 2H), 2.88 (quartet, 2H), 2.68 (t, 2H), 2.60-2.52 (m, 4H), 2.22 (s, 3H), 2.30-2.12 (m, 2H), 2.02 (pentet, 2H), 1.98-1.90 (m, 2H), 1.86-1.78 (m, 4H) 1.34 (t, 3H); HRMS (ESI⁺) 425.2469 (M+H)⁺.

### Example 124: N-methyl-N-({4-[4-(3-pyrrolidin-1-yl)propoxy)phenyl]-tetrahydro-2H-pyran-4-yl}methyl)propane-1-sulfonamide

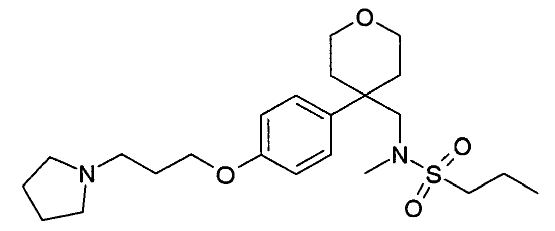

n-Propylsulfonyl chloride (33 µL, 0.289 mmol) was added dropwise to a solution of triethylamine (37 µL, 0.265 mmol) and *N-*methyl-1-{1-[4-(3-pyrrolidin-1-ylpropoxy)phenyl]cyclohexyl}methanamine (80 mg, 0.24 mmol) in dichloromethane (1 mL) at 0°C and the reaction stirred for 1 hour. The reaction was quenched with NaHCO₃ (20 mL), extracted using dichloromethane (3 x 15 mL), dried using Na₂SO₄ and concentrated *in vacuo*. The compound was purified by column chromatography on Biotage® silica gel, eluting with dichloromethane:methanol:ammonia, 98:2:0.2 to 92:8:0.8 to give the title compound as a pale brown oil (62 mg, 59%).
¹H NMR (400 MHz, CDCl₃) δ 7.22 (d, 2H), 6.90 (d, 2H), 4.02 (t, 2H), 3.82-3.74 (m, 2H), 3.50 (dt, 2H), 3.20 (s, 2H), 2.74 (dt, 2H), 2.64 (t, 2H), 2.56-2.48 (m, 4H), 2.20 (s, 3H), 2.18-2.12 (m, 2H), 2.02 (pentet, 2H), 1.98-1.84 (m, 2H), 1.82-1.68 (m, 6H) 1.00 (t, 3H); HRMS (ESI⁺) 439.2625 (M+H)⁺.

### Example 125: N-ethyl-N-({4-[4-(3-pyrrolidin-1-ylpropoxy)phenyl]tetrahydro-2H-pyran-4-yl}methyl)methanesulfonamide

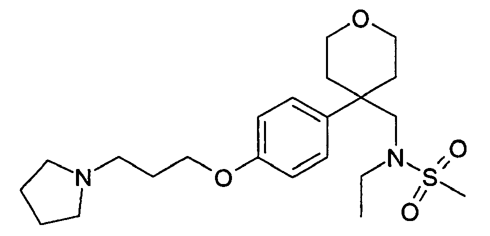

Methanesulfonyl chloride (19 µL, 0.24 mmol) was added dropwise to a solution of triethylamine (31 µL, 0.22 mmol) and *N*-({4-[4-(3-pyrrolidin-1-ylpropoxy)phenyl]tetrahydro-2*H*-pyran-4-yl}methyl)ethanamine (70 mg, 0.20 mmol) in dichloromethane (0.5 mL) at 0°C then was allowed to warm to ambient temperature and stirred overnight. The reaction was quenched with NaHCO₃ (10 mL), extracted using dichloromethane (3 x 15 mL), dried using Na₂SO₄ and concentrated *in vacuo*. The compound was purified by column chromatography on Biotage® silica gel, eluting with dichloromethane:methanol:ammonia, 96.5:3.5:0.35 to 95:5:0.5 to give the title compound as a pale brown oil (60 mg, 71%).
¹H NMR (400 MHz, CDCl₃) δ 7.24 (d, 2H), 6.90 (d, 2H), 4.02 (t, 2H), 3.84-3.72 (m, 2H), 3.50 (dt, 2H), 3.28 (s, 2H), 2.72-2.60 (m, 7H), 2.58-2.52 (m, 4H), 2.16 (m, 2H), 2.04 (pentet, 2H), 1.96-1.90 (m, 2H), 1.84-1.76 (m, 4H), 0.90 (t, 3H); HRMS (ESI⁺) 425.2469 (M+H)⁺.

### Example 126: N-ethyl-N-({4-[4-(3-pyrrolidin-1-ylpropoxy)phenyl]tetrahydro-2H-pyran-4-yl}methyl)ethanesulfonamide

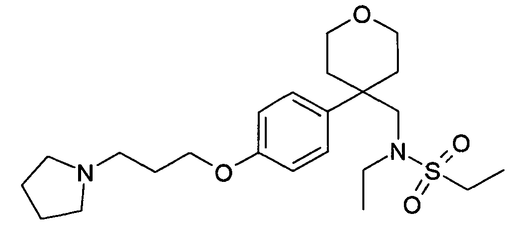

Ethanesulfonyl chloride (26 µL, 0.28 mmol) was added dropwise to a solution of triethylamine (35 µL, 0.25 mmol) and *N-*({4-[4-(3-pyrrolidin-1-ylpropoxy)phenyl]tetrahydro-2H-pyran-4-yl}methyl)ethanamine (78 mg, 0.23 mmol) in dichloromethane (0.5 mL) at 0°C allowed to warm to ambient temperature and stirred overnight. The reaction was quenched with NaHCO₃ (10 mL), extracted using dichloromethane (3 x 10 mL), dried using Na₂SO₄ and concentrated *in vacuo.* The compound was purified by column chromatography on Biotage® silica gel, eluting with dichloromethane:methanol:ammonia, 96.5:3.5:0.35 to 95:5:0.5 to give the title compound as a pale brown oil (55 mg, 54%).
¹H NMR (400 MHz, CDCl₃) δ 7.24 (d, 2H), 6.90 (d, 2H), 4.02 (t, 2H), 3.84-3.72 (m, 2H), 3.48 (t, 2H), 3.32 (s, 2H), 2.76 (quartet, 2H), 2.72-2.60 (m, 4H), 2.60-2.48 (m, 4H), 2.20-2.08 (m, 2H), 2.04 (pentet, 2H), 1.98-1.90 (m, 2H), 1.86-1.76 (m, 4H), 1.30 (t, 3H), 0.88 (t, 3H). HRMS (ESI⁺) 439.2625 (M+H)⁺.

### Example 127: N-({4-[4-(3-pyrrolidin-1-ylpropoxy)phenyl]tetrahydro-2H-pyran-4-yl}methyl)ethanamine

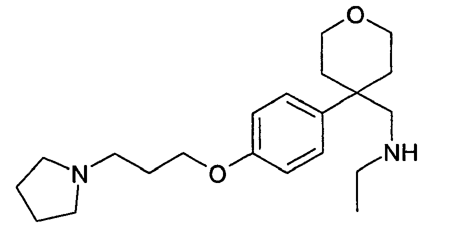

LiAlH₄ (1.0M in diethylether, 4.72 mL, 4.72 mmol) was added dropwise at 0°C to a solution of (*N*-{4-[4-(3-pyrrolidin-1-yl-propoxy)-phenyl]-tetrahydro-pyran-4-ylmethyl}-acetamide (848 mg, 2.36 mmol) in tetrahydrofuran (2 mL) under an atmosphere of nitrogen. The reaction mixture was allowed to warm to ambient temperature for 2 hours then heated to 50°C for 10 hours. The reaction mixture was cooled to 0°C, water (3 mL) was added dropwise followed by sodium hydroxide (2.0M, 6 mL) and water (3 mL). The resulting solid was filtered over a small pad of celite, washed with Et₂O/DCM (1:1 6 x 50 mL) and concentrated *in vacuo* to give the title compound as a pale yellow solid (635 mg, 78 %).
¹H NMR (400 MHz, CDCl₃) δ 7.20 (d, 2H), 6.88 (d, 2H), 4.02 (t, 2H), 3.80-3.68 (m, 2H), 3.55 (dt, 2H), 2.68 (s, 2H), 2.60 (t, 2H), 2.58-2.42 (m, 6H), 2.18-2.08 (m, 2H), 2.00 (pentet, 2H) 1.94-1.86 (m, 2H), 1.84-1.76 (m, 4H), 1.92 (t, 3H). HRMS (ESI⁺) 347.2693 (M+H)⁺.

### Example 128: N-methyl-N-({4-[4-(3-pyrrolidin-1-ylpropoxy)phenyl]tetra-hydro-2H-pyran-4-yl}methyl)ethanamine

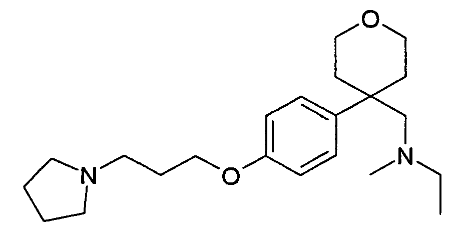

A solution of LiAlH₄ (1.0M in diethylether, 1.20 mL, 0.60 mmol) was added dropwise at 0°C to a solution of *N*-methyl-*N*-{4-[4-(3-pyrrolidin-1-yl-propoxy)-phenyl]-tetrahydro-pyran-4-ylmethyl}-acetamide (220 mg, 0.60 mmol) in tetrahydrofuran (1 mL) under an atmosphere of nitrogen. The reaction mixture was allowed to warm to ambient temperature overnight. The reaction mixture was cooled to 0°C, water (1 mL) was added dropwise followed by sodium hydroxide (2.0M, 3 mL) and water (1 mL). The resulting solid was filtered over a small pad of celite, washed with DCM (150 mL) and concentrated *in vacuo.* The compound was purified by column chromatography on Biotage® silica gel, eluting with dichloromethane:methanol:ammonia, 97.5:2.5:0.25 to 95.5:4.5:0.45 to give the title compound as a pale yellow oil (114 mg, 88%).
¹H NMR (400 MHz, CDCl₃) δ 7.18 (d, 2H), 6.86 (d, 2H), 4.04 (t, 2H), 3.78-3.70 (m, 2H), 3.52 (dt, 2H), 2.66 (t, 2H), 2.52-2.66 (m, 4H), 2.38 (s, 2H), 2.18 (quartet, 2H), 2.00-2.12 (m, 4H), 1.92-1.86 (m, 5H), 1.82-1.76 (m, 4H), 0.86 (t, 3H); HRMS (ESI⁺) 361.2850 (M+H)⁺.

### Intermediate 47: 4-(4-{3-[(2S)-2-methylpyrrolidinyl]propoxy}phenyl)-tetrahydro-2H-pyran-4-carbonitrile

A solution of 4-(4-hydroxy-phenyl)-tetrahydro-pyran-4-carbonitrile (203 mg, 1.0 mmol) and tri-n-butylphosphine (299 µL, 1.20 mmol) in toluene (6 mL) were added to a solution of 3-[(2R)-2-methylpyrrolidin-1-yl]propan-1-ol (172 mg, 1.20 mmol) in toluene (6 mL) and tetrahydrofuran (2 mL) at ambient temperature. 1'1-azobis(*N,N*-dimethylformamide) (206 mg, 1.20 mmol) was added and the reaction heated to 85°C overnight. The reaction mixture was concentrated *in vacuo* followed by addition on dichloromethane (30 mL). The organic solution was then washed with NaOH (2.0M, 2 x 20 mL), dried with Na₂SO₄ and concentrated *in vacuo.* The compound was purified by column chromatography on Biotage® silica gel, eluting with dichloromethane:methanol:ammonia, 98:2:0.2 to 96:4:0.4 to give the title compound as a pale yellow oil (70mg, 21%). HRMS (ESI⁺) 329.2224 (M+H)⁺.

### Example 129: 4-(4-{3-[(2R)-2-methylpyrrolidin-1-yl]propoxy}phenyl-tetra-hydro-2H-pyran-4-carboxylic acid

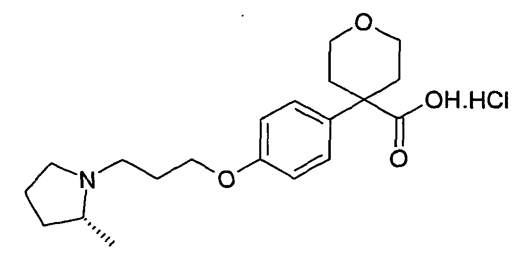

A solution of 4-(4-{3-[(2*S*)-2-methylpyrrolidinyl]propoxy}phenyl)tetrahydro-2*H*-pyran-4-carbonitrile (70 mg, 0.21 mmol) in concentrated hydrochloric acid (1 mL) was heated at reflux for 3 days. A further 2mL of concentrated hydrochloric acid was added and the reaction heated at reflux for a further 2 days. The solution was evaporated to dryness *in vacuo,* triturated using acetone and azeotroped with toluene to give a pale brown solid (51 mg, 73 %).
¹H NMR (400 MHz, CDCl₃) δ 7.36 (d, 2H), 6.94 (d, 2H), 4.12 (t, 2H), 3.92-3.84 (m, 2H), 3.76-3.46 (m, 6H), 2.54-2.44 (m, 2H), 2.20-2.02 (m, 1H), (m, 8H), (m, 3H); HRMS (ESI⁺) 348.2170 (M+H)⁺.

### Intermediate 48: tert-butyl 4-[4-(4-cyanotetrahydro-2H-pyran-4-yl)phenoxy]-1-piperidinecarboxylate

*tert*-Butyl 4-[(methylsulfonyl)oxy]-1-piperidinecarboxylate (1.35g, 4.93mmol), 4-(4-hydroxyphenyl)tetrahydro-2H-pyran-4-carbonitrile (1.00g, 4.93mmol) and potassium carbonate (1.36g, 9.85mmol) were stirred in 10ml DMF at 56°C for 28 hours. The reaction mixture was partitioned between ethyl acetate (50ml) and water (50ml). The ethyl acetate was dried over Na₂SO₄ and concentrated under reduced pressure. The crude product was then purified by column chromatography on silica gel eluting with 2% diethylamine in ethylacetate. This only gave a poor separation, so the impure material was triturated with diisopropylether (20ml) to give a white powder (1.5g). This solid was dissolved in diethylether (100ml) and washed with 2M NaOH (50ml) then with water (50ml). The diethylether was dried over Na₂SO₄ and evaporated to give *tert*-butyl 4-[4-(4-cyanotetrahydro-2*H*-pyran-4-yl)phenoxy]-1-piperidinecarboxylate as white crystals (740mg, 39%).

### Intermediate 49: 4-[4-(4-piperidinyloxy)phenyl]tetrahydro-2H-pyran-4-carbonitrile

*tert*-Butyl 4-[4-(4-cyanotetrahydro-2H-pyran-4-yl)phenoxy]-1-piperidinecarboxylate (720mg) was dissolved in dichloromethane (50ml) at 0°C. TFA (10ml) was added and the mixture stirred whilst warming to room temperature over 2 hours. The reaction mixture was then concentrated *in vacuo,* and partitioned between dichloromethane (80ml) and 0.5M NaOH (50ml). The dichlorormethane solution was dried over Na₂SO₄ and evaporated to give 4-[4-(4-piperidinyloxy)phenyl]tetrahydro-2*H*-pyran-4-carbonitrile as a colurless oil which crystallized on standing (500mg, 94%).

### Example 130: 4-{4-[1-cyclobutyl-4-piperidinyl)oxy]phenyl}tetrahydro-2H-pyran-4-carbonitrile

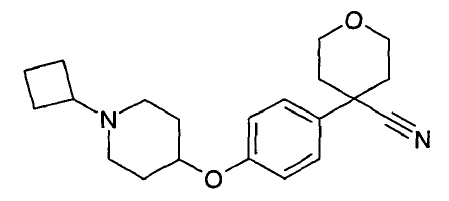

4-[4-(4-Piperidinyloxy)phenyl]tetrahydro-2*H*-pyran-4-carbonitrile (130mg, 0.45mmol) and cyclobutanone (140µl, 1.9mmol) were stirred in THF (2ml) and acetic acid (26µl, 0.45mmol) at ambient temperature for 15 mins. Sodium triacetoxyborohydride (340mg, 1.6mmol) was added and the mixture stirred for 18 hrs. The reaction was quenched by adding 10% aqueous Na₂CO₃ (5ml) and then partitioned between ethyl acetate (50ml) and water (30ml). The organics were dried over Na₂SO₄ and evaporated to give a solid which was purified by column chromatography on silica gel eluting with dichloromethane:methanol:ammonia, 98:2:0.2 to 94:6:0.6 to give the product which was then crystallized from diisopropylether (5ml) to give 4-{4-[(1-cyclobutyl-4-piperidinyl)oxy]phenyl}tetrahydro-2*H*-pyran-4-carbonitrile as a white powder (50mg, 33%).
¹H NMR (400MHz, CDCl₃) *δ* 7.38 (d, 2H), 6.93 (d, 2H), 4.33 (m, 1H), 4.08 (m, 2H), 3.89 (m, 2H), 2.75 (pentet, 1H), 2.62 (m, 2H), 2.20-1.58 (m, 16H)

### Example 131: 4-{4-[(1-isopropylpiperidin-4-yl)oxy]phenyl}tetrahydro-2H-pyran-4-carboxamide

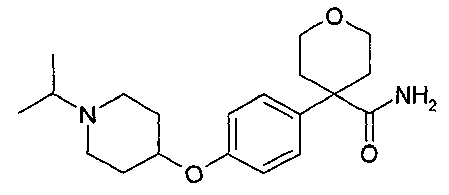

4-[4-(1-Isopropylpiperidin-4-yloxy)phenyl]tetrahydropyran-4-carbonitrile (50 mg, 0.152 mmol) was combined with boron trifluoride-acetic acid complex (500 µL, 3.6 mmol) and stirred at ambient temperature for 18 hours, followed by 65°C for 3 hours. The reaction mixture was allowed to cool and then basified with saturated NaHCO₃ solution (15 mL). The product was extracted with dichloromethane (2 x 35 mL). The combined organic extracts were dried using Na₂SO₄ filtered and concentrated *in vacuo.* The compound was purified by column chromatography on silica gel (15 g), eluting with dichloromethane:methanol:ammonia, (96:4:0.4) to dichloromethane:methanol:ammonia, (90:10:1) to give the title compound as a white solid (22 mg, 42%).
¹H NMR (400 MHz, CD₃OD) δ 7.3 (d, 2H), 6.9 (d, 2H), 4.4 (m, 1H), 3.8 (m, 2H), 3.65 (m, 2H), 2.78-2.85 (m, 3H), 2.5 (m, 2H), 2.4 (d, 2H), 1.95-2.08 (m, 4H), 1.8 (m, 2H), 1.1 (d, 6H)

### Example 132: 4-{4-[(1-isopropylpiperidin-4-yl)oxy]phenyl}tetrahydro-2H-pyran-4-carboxylic acid

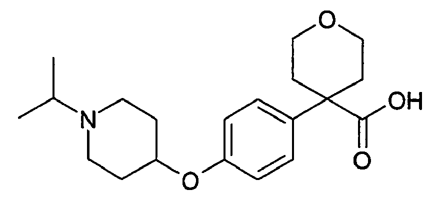

4-[4-(1-Isopropylpiperidin-4-yloxy)phenyl]tetrahydropyran-4-carbonitrile (1g, 3 mmol) and concentrated hydrochloric acid (10 mL) were combined and heated at reflux for 44 hours. Reaction mixture was concentrated *in vacuo* the resulting grey solid was triturated with acetone (20 mL) to give a cream coloured solid. The compound was purified using an SCX isolelute column flushed with methanol (60 mL) and then flushed with 2M NH₃ in methanol (150 mL). The title compound was isolated as a cream coloured solid (550mg, 52%).
¹H NMR (400 MHz, CD₃OD) *δ* 7.4 (d, 2H), 6.9 (d, 2H), 4.4 (m, 1H), 3.85 (m, 2H), 3.65 (m, 2H), 3.4 (m, 1H), 3.2 (m, 2h), 3.03 (m, 2H), 2.5 (m, 2H), 2.0-1.8 (m, 6H), 1.3 (d, 6H)

### Example 133: 4-{4-[(1-isopropylpiperidin-4-yl)oxy]pheny]-N,N-dimethyltetrahydro-2H-pyran-4-carboxamide

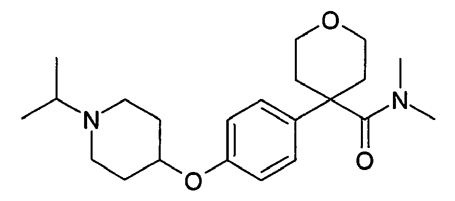

4-(4-[(1-Isopropylpiperidin-4-yl)oxy]phenyl)tetrahydro-2*H*-pyran-4-carboxylic acid (150 mg, 3.9 mmol), O-(1H-benzotriazol-1-yl)-N,N,N',N'-tetramethyluronium hexafluorophosphate (222 mg, 5.8 mmol), dimethylamine hydrochloride (96mg, 1.1 mmol), pyridine (126uL, 1.56 mmol) were combined and dissolved in dimethylformamide (2 mL). The reaction mixture was stirred at ambient temperature for 18 hours. Further O-(1H-benzotriazol-1-yl)-N,N,N',N'-tetramethyluronium hexafluorophosphate (222 mg, 5.8 mmol) and dimethylamine in ethanol (33%) (500 µL) was added and the reaction mixture was stirred at ambient temperature for 18 hours. The reaction mixture was diluted with water (20 mL) and was then extracted with ethyl acetate (2 x 40 mL). The combined organic extracts were dried using NaSO₄, filtered and concentrated *in vacuo.* The compound was purified by column chromatography on silica gel, eluting with dichloromethane:methanol:ammonia (97:3:0.3) to dichloromethane:methanol:ammonia (90:10:1) to give a compound as a clear colourless oil. This was dissolved in ethyl acetate (70 mL) and washed with saturated NaHCO₃ solution (2 x 10 mL). The organic extract was dried using Na₂SO₄, filtered and concentrated *in vacuo* to give the title compound as a clear crystaline solid (72mg, 50%).
¹H NMR (400 MHz, CD₃OD) *δ* 7.2 (d, 2H), 6.95 (d, 2H), 4.4 (m, 1H), 3.85 (m, 2H), 3.7 (m, 2H), 2.9-2.5 (m, 11H), 2.28 (m, 2H), 2.0 (m, 4H), 1.8 (m, 2H), 1.1 (d, 6H)

### Example 134: 4-{4-[(1-isopropylpiperidin-4-yl)oxy]phenyl]-N,N-diethyltetra-hydro-2H-pyran-4-carboxamide

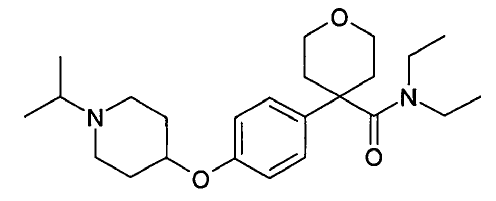

4-{4-[(1-Isopropylpiperidin-4-yl)oxy]phenyl}tetrahydro-2*H*-pyran-4-carboxylic acid (86mg, 0.2 mol), diethylamine (27 µL, 0.26 mmol), O-(1H-benzotriazol-1-yl)-N,N,N',N'-tetramethyluronium hexafluorophosphate (108mg, 0.29 mmol), pyridine (53 uL, 0.66 mmol) were combined and dissolved in dimethylformamide (1.5 mL). The reaction mixture was stirred at 60°C for 5 hours. Further diethylamine was added (500 µL). The reaction mixture was stirred at ambient temperature for 60 hours. The reaction mixture was diluted with water (25 mL), and extracted with ethyl acetate (2 x 35 mL). The combined organics were dried using Na₂SO₄, filtered and concentrated *in vacuo.* The compound was purified by column chromatography using 15g of silica gel, eluting with dichloromethane:methanol:ammonia (96:4:0.4) to dichloromethane:methanol:ammonia (90:10:1) to give the title compound as a clear yellow oil (10mg, 11%).
¹H NMR (400 MHz, CD₃OD) *δ* 7.2 (d, 2H), 6.95 (d, 2H), 4.4 (m, 1H), 3.85-3.7 (m, 4H), 3.3 (m, 2H), 3.05-2.9 (m, 5H), 2.6 (m, 2H), 2.25 (d, 2H), 2.0 (m, 4H), 1.8 (m, 2H), 1.1 (m, 9H), 0.63(m, 3H)

### Example 135: 4-{4-[(1-isopropylpiperidin-4-yl)oxy]phenyl)tetrahydro-2H-pyran-4-yl]carbonyl}pyrrolidine

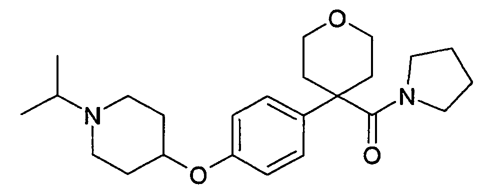

4-{4-[(1-Isopropylpiperidin-4-yl)oxy]phenyl}tetrahydro-2*H*-pyran-4-carboxylic acid (93mg, 0.27 mmol) was supended in dimethylformamide (3mL), O-(1H-benzotriazol-1-yl)-N,N,N',N'-tetramethyluronium hexafluorophosphate (203mg, 0.53 mmol), and triethylamine (112µL, 0.8 mmol) were added. The reaction mixture was stirred at ambient temperature for 1 hour. Pyrrolidine (300µL) was added and the reaction mixture was stirred at ambient temperature for 2.5 hours. The reaction mixture was concentrated *in vacuo,* then partitioned between saturated NaHCO₃ solution (50 mL) and dichloromethane (2 x 75 mL). The combined organics were dried using Na₂SO₄, filtered and concentrated *in vacuo.* The compound was purified by column chromatography using 20g of silica gel, eluting with dichloromethane:methanol:ammonia (96:4:0.4) to dichloromethane:methanol:ammonia (94:6:0.6) to give the title compound as a clear oil (34mg, 32%).
¹H NMR (400 MHz, CD₃OD) *δ* 7.2 (d, 2H), 6.9 (d, 2H), 4.4 (m, 1H), 3.85-3.7 (m, 4H), 3.45 (m, 2H), 2.95 (m, 2H), 2.8-2.7 (m, 3H), 2.5 (m, 2H), 2.3 (m, 2H), 2.0 (m, 4H), 1.8-1.7 (m, 4H), 1.6 (m, 2H), 1.1 (d, 6H).

### Intermediate 50: 4-[4-(3-pyrrolidin-1-ylpropoxy)phenyl]tetrahydro-2H-pyran-4-carbothioamide

4-[4-(3-Pyrrolidin-1-yl-propoxy)-phenyl]-tetrahydro-pyran-4-carbonitrile (2g, 6.4 mmol), diethyl dithiophosphate (15 mL, 8.9 mmol), and water (1.5 mL) were combined and stirred at ambient temperature for 5 hours, 55 °C for 2 hours then 70°C for 2 hours. The reaction mixture was diluted with dichloromethane (100mL) and basified to pH 8 with a saturated solution of NaHCO₃ in water, and solid NaHCO₃. The layers were separated, and further dichloromethane (100 mL) was used to extract the product. The combined organics were dried using Na₂SO₄, filtered and concentrated *in vacuo* to leave a yellow oil. The compound was purified with column chromatography on 50g of silica, eluting with dichloromethane:methanol:ammonia (96:4:0.4) to dichloromethane:methanol:ammonia (90:10:1) to give a clear oil. The clear oil was dissolved in ethyl acetate (150 mL) and washed with 10% Na₂CO₃ solution. The organic layer was dried using Na₂SO₄, filtered and concentrated *in vacuo* to give the title compound as a white solid (0.61g, 28%).

### Example 136: 4-metyl-2-[4-(4-(3-pyrrolidin-1-ylpropoxy)phenyl)tetrahydro-2H-pyran-4-yl]-1,3-thiazole

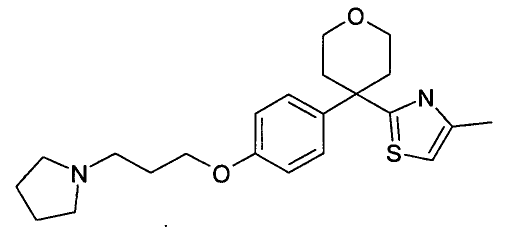

4-[4-(3-Pyrrolidin-1-ylpropoxy)phenyl]tetrahydro-2*H*-pyran-4-carbothioamide (100mg, 0.29 mmol), chloroacetone (46 µL, 0.57 mmol) and ethanol (5 mL) were combined and heated at reflux for 18 hours. The reaction mixture was concentrated *in vacuo* to give a cream coloured solid. This was dissolved in dichloromethane (100 mL) and washed with saturated NaHCO₃ solution (50 mL). The organic extract was dried using Na₂SO₄, filtered and concentrated *in vacuo.* The compound was purified by column chromatography using 15g of silica gel, eluting with dichloromethane:methanol:ammonia (97:3:0.3) to dichloromethane:methanol:ammonia (94:6:0.6) to give the title compound as a white solid (68mg, 61%).
¹H NMR (400 MHz, CDCl₃) *δ* 7.3 (d, 2H), 6.9 (d, 2H), 6.75 (s, 1H), 4.0 (t, 2H), 3.83 (m, 2H), 3.7 (m, 2H), 2.75-2.58 (m, 8H), 2.4 (s, 3H), 2.35 (m, 2H), 2.03 (m, 2H), 1.8 (m, 4H)

### Example 137: 2-[4-(4-(3-pyrrolidin-1-ylpropoxy)phenyl)tetrahydro-2H-pyran-4-yl]-1,3-thiazole

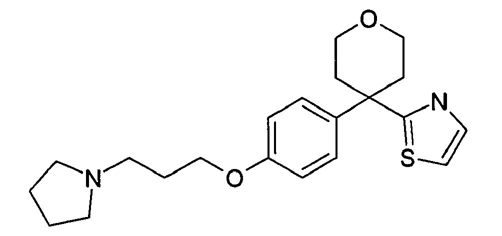

4-[4-(3-Pyrrolidin-1-ylpropoxy)phenyl]tetrahydro-2H-pyran-4-carbothioamide (150 mg, 0.43 mmol), bromoacetaldehyde diethylacetal (67 µL, 0.43 mmol), concentrated hydrochloric acid (5 drops) and ethanol were combined and heated at reflux for 5 hours. The reaction mixture was concentrated *in vacuo* and partitioned between 10% Na₂CO₃ (20mL) and dichloromethane (2 x 35 mL). The combined organics were dried using Na₂SO₄, filtered and concentrated *in vacuo.* The compound was purified using column chromatography using 15g of silica gel, eluting with dichloromethane:methanol:ammonia (95:5:0.5) to give a cream coloured solid. This was triturated with 3mL of diethyl ether to give the title compound as a cream coloured solid (25mg, 16%).
¹H NMR (400 MHz, CD₃OD) *δ* 7.7 (s, 1H), 7.45 (s, 1H), 7.3 (d, 2H), 6.9 (d, 2H), 4.0 (t, 2H), 3.8 (m, 2H), 3.7 (m, 2H), 2.85-2.75 (m, 6H), 2.6 (m, 2H), 2.4 (m, 2H), 2.03 (m, 2H), 1.9 (m, 4H)

### Intermediate 51: (4-{4-[(1-isopropylpiperidin-4-yl)oxy]phenyl}tetrahydro-2H-pyran-4-yl)methylamine

To a stirred solution 4-[4-(1-isopropylpiperidin-4-yloxy)phenyl]tetrahydropyran-4-carbonitrile (2.5 g, 7.62 mmol) in dry diethyl ether (15 ml) at 0°C was added dropwise a solution of LiAlH₄ (1.0M solution in Et₂O, 22.87 ml, 22.9 mmol). Reaction stirred at 0°C for 30 mins, then warmed up to ambient temperature overnight under a nitrogen atmosphere until complete. The reaction was cooled to 0°C, water (0.9 ml) was added dropwise followed by sodium hydroxide (2.0M, 0.9 ml) and water (2.7 ml). Dichloromethane (25 ml) and methanol (1 ml) were added and the mixture filtered through a short pad of arbocel, eluting with 2% methanol in dichloromethane (200 ml). The organic washings were dried over Na₂SO₄ and concentrated *in vacuo* to give the title compound as an orange oil (2.59 g, 100%).

### Intermediate 52: tert-butyl [4-(4-[(1-isopropylpiperidin-4-yloxylphenyl)tetrahydro-2H-pyran-4-yl]methylcarbamate

To a stirred solution of (4-{4-[(1-isopropylpiperidin-4-yl)oxy]phenyl}tetrahydro-2*H*-pyran-4-yl)methylamine (600 mg, 1.81 mmol) and triethylamine (578 µl. 5.42 mmol) in dichloromethane (2.5 ml) at 0°C, was added dropwise a solution of di-*tert*-butyldicarbonate (540 µl, 2.35 mmol) in dichloromethane (2.5 ml). Solution stirred at 0°C for 2 hours then allowed to warm up to ambient temperature and stirred until reaction complete. Reaction mixture diluted with dichloromethane (10 ml), washed with water (3x10 ml) then brine (2x10 ml). Organic washings dried over Na₂SO₄ and concentrated *in vacuo*. The compound was purified by column chromatography on silica gel, eluting with dichloromethane:methanol:ammonia, 100:0:0 to 96:4:0:0.4 to give the title compound as a foam (532 mg, 68%).

### Example 138: N-[(4-{4-[(1-isopropylpiperidin-4-yl)oxy]phenyl}tetrahydro-2H-pyran-4-yl)methyl]-N-methylamine

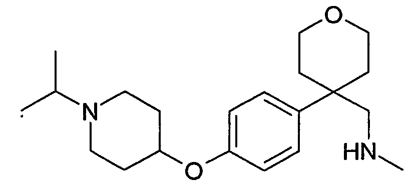

To a stirred solution of *tert*-butyl [4-(4-[(1-isopropylpiperidin-4-yl)oxy]phenyl)tetrahydro-2H-pyran-4-yl]methylcarbamate (532 mg, 1.23 mmol) in THF (3.5 ml) at 0°C was added dropwise a solution of LiAlH₄ (1.0M solution in Et₂O, 3.69 ml, 3.69 mmol). Reaction stirred for 60 hours at ambient temperature before a further dropwise addition of LiAlH₄ (1.0M solution in Et₂O, 0.6 ml, 0.6 mmol). Heated at 40°C until reaction complete. The reaction was then cooled to 0°C, water (0.2 ml) was added dropwise followed by sodium hydroxide (2.0M, 0.2 ml) and water (0.6 ml). 1% methanol in dichloromethane (10ml) was added and the mixture filtered through a short pad of arbocel, eluting with 1% methanol in dichloromethane (25 ml). The organic washings were dried over Na₂SO₄ and concentrated *in vacuo* to give the title compound as a solid (399 mg, 94%).
¹H NMR (400MHz, CDCl₃) δ 7.20 (d, 2H), 7.88 (d, 2H), 4.22-4.34 (m, 1H), 3.70-3.80 (m, 2H), 3.60-4.01 (d, 2H), 2.70-2.88 (m, 3H), 2.64 (s, 2H), 2.32-2.44 (m, 2H) 2.24 (s, 3H), 2.10-2.18 (M, 2H), 1.99-2.08 (m, 2H), 1.79-1.96 (m, 4H), 1.03 (d, 6H).

### Example 139: N-{[4-(4-[(1-isopropylpiperidin-4-yl)oxy]phenyl)tetrahydro-2H-pyran-4-yl]methyl}-N-methylacetamide

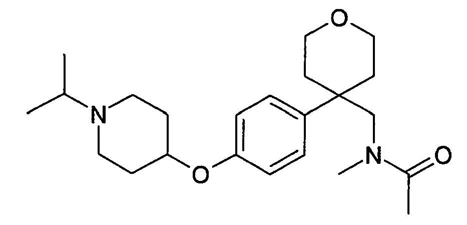

To a stirred, solution of *N-*[(4-{4-[(1-isopropylpiperidin-4-yl)oxy]phenyl}tetrahydro-2H-pyran-4-yl)methyl]-*N* methylamine (120 mg, 0.347 mmol) and triethylamine (121 µl, 0.867 mmol) in dichloromethane (1ml) at 0°C was added dropwise acetic anhydride (36 µl, 0.382 mmol). The reaction mixture was warmed to ambient temperature until reaction complete. Water (1 ml) and dichloromethane (15 ml) added to the reaction, the extracted with saturated NaHCO₃ (2x10 ml). Organic washings were dried over Na₂SO₄ and concentrated *in vacuo* to give the title compound as an oil (113 mg, 83%).
¹H NMR (400MHz, CDCl₃) δ 7.10-7.22 (2 doublets due to rotamers, 2H), 6.80 (d, 2H), 4.24-4.38 (m, 1H), 3.79-3.80 (m, 2H), 3.42-3.60 (m, 2H), 2.70-2.90 (m, 3H), 2.39-2.48 (m, 2H), 3.20-3.35 (singlet and doublet due to rotamers, 3H), 1.75-2.10 (m, 11H), 1.10 (d, 6H).

### Example 140: N-{[4-(4-[(1-isopropylpiperidin-4-yl)oxy]phenyl)tetrahydro-2H-pyran-4-yl]methyl}-N-methylpropanamide

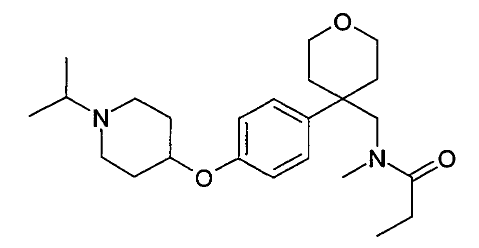

To a stirred solution of *N-*[(4-{4-[(1-isopropylpiperidin-4-yl)oxy]phenyl}tetrahydro-2H-pyran-4-yl)methyl]-*N-*methylamine (120 mg, 0.347 mmol) and triethylamine (121 µl, 0.867 mmol) in dichloromethane (1 ml) at 0°C was added dropwise propionic anhydride (44 µl, 0.382 mmol). Reaction allowed to warm up to ambient temperature until reaction complete. Water (0.5 ml) and dichloromethane (15 ml) added to the reaction. The organic phase was separated and extracted with saturated NaHCO₃ (3x10 ml). Organic washings were dried over Na₂SO₄ and concentrated *in vacuo* to give the title compound as an oil (153 mg, 82%).
¹H NMR (400MHz, CD₃OD) δ 7.20-7.32 (2 doublets due to rotamers, 2H), 7.95 (d, 2H), 3.95-4.05 (m, 1H), 3.76-3.85 (m, 2H), 3.40-3.60 (multiple peaks due to rotamers, 4H), 2.70-2.88 (multiple peaks due to rotamers, 4H), 2.42-2.58 (m, 2H), 2.40 (s, 2H), 2.20-2.38 (m, 2H), 1.52-2.58 (several multiplets, 8H), 1.10 (d, 6H), 0.70-1.08 (two triplets due to rotamers, 3H).

### Example 141: N-{[4-(4-[(1-isopropylpiperidin-4-yl)oxy]phenyl)tetrahydro-2H-pyran-4-yl]methyl}-acetamide

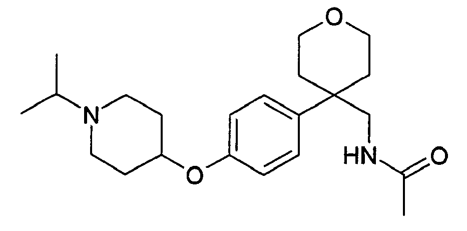

To a stirred solution of (4-{4-[(1-isopropylpiperidin-4-yl)oxy]phenyl}tetrahydro-2*H*-pyran-4-yl)methylamine (250 mg, 0.753 mmol) and triethylamine (200 µl. 1.88mmol) in dichloromethane (2ml) was added dropwise acetic anhydride (78 µl, 0.828 mmol). Reaction left at ambient temperature until reaction complete. Dichloromethane (10 ml) added to the reaction, the organic phase extracted with saturated NaHCO₃ (3x10 ml). Organic washings were dried over Na₂SO₄ and concentrated *in vacuo* to give the title compound as an oil (203 mg, 72%).
¹H NMR (400MHz, CDCl₃) δ 7.19 (d, 2H), 6.94 (d, 2H), 4.95-5.02 (broad, 1H), 4.24-4.38 (m, 1H), 3.79-3.88 (m, 2H), 3.52-3.64 (m, 2H), 3.45 (d, 2H), 2.70-2.88 (m, 3H), 2.36-2.45 (m, 2H), 1.98-2.10 (m, 4H), 1.78-1.90 (m, 7H), 1.05 (d, 6H).

### Example 142: N-[(4-{4-[(1-isopropylpiperidin-4-yl)oxy]phenyl}tetrahydro-2H-pyran-4-yl)methyl]-N-ethylamine

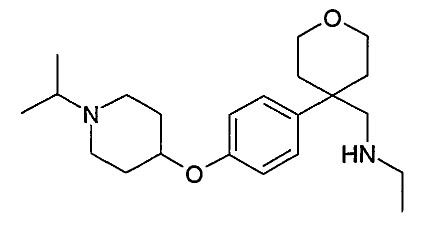

To a stirred solution of *N-*{[4-(4-[(1-isopropylpiperidin-4-yl)oxy]phenyl)tetrahydro-2*H*-pyran-4-yl]methyl}-acetamide (192 mg, 0.513 mmol) in THF (1.5 ml) at 0°C was added dropwise a solution of LiAlH₄ (1.0M solution in Et₂O, 1.54 ml, 1.54 mmol). Reaction heated at 40°C for 24 hours, then increased to 50°C. A further dropwise addition of LiAlH₄ (1.0M solution in Et₂O, 2 ml, 2 mmol) and further heating at 50°C for 24 hours was required for reaction to reach completion. The reaction was then cooled to 0°C, water (0.15 ml) was added dropwise followed by sodium hydroxide (2.0M, 0.15 ml) and water (0.45 ml). 1% methanol in dichloromethane (15 ml) was added and the mixture filtered through a short pad of Arbocel®, eluting with 1% methanol in dichloromethane (25 ml). The organic washings were dried over Na₂SO₄ and concentrated *in vacuo* (185 mg crude, 82%). The compound was purified by column chromatography on silica gel, eluting with dichloromethane:methanol:ammonia, 98:2:0.2, to give the title compound as a white solid (9.9 mg, 4.4%).
¹H NMR (400MHz, CDCl₃) δ 7.20 (d, 2H), 7.88 (d, 2H), 4.24-4..56 (m, 1H), 3.71-3.82 (m, 2H), 3.52-3.63 (m, 2H), 2.72-2.88 (m, 3H), 2.70 (s, 2H), 2.46-2.52 (quartet, 2H), 2.34-2.42 (m, 2H), 1.78-2.18 (several multiplets, 8H), 1.08 (d, 6H), 0.96 (t, 3H).

### Example 143: N-{[4-(4-[(1-isopropylpiperidin-4-yl)oxy]phenyl)tetrahydro-2H-pyran4-ylmethyl}-N-ethylacetamide

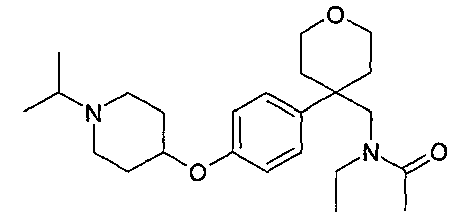

To a stirred solution of *N*-[(4-{4-[(1-isopropylpiperidin-4-yl)oxy]phenyl}tetrahydro-2*H*-pyran-4-yl)methyl]*-N-*ethylamine (102 mg, 0.283 mmol) and triethylamine (99 µl. 0.708 mmol) in dichloromethane (1 ml) was added dropwise acetic anhydride (29 µl, 0.312 mmol). Reaction left at ambient temperature for 4 hours until reaction complete. Water (1 ml) and dichloromethane (10 ml) added to the reaction, the extracted with saturated NaHCO₃ (2 x 10 ml). Organic washings were dried over Na₂SO₄ and concentrated *in vacuo* to give the title compound as an oil (107mg, 94%).
¹H NMR (400MHz, CD₃OD) δ 7.20-7.31 (multiplet due to rotamers, 2H), 6.92-7.00 (multiplet due to rotamers, 2H), 4.36-4.46 (m, 1H), 3.70-3.88 (m, 2H), 3.20-3.55 (several peaks due to rotamers, 4H), 2.78-2.90 (m, 3H), 2.60, (q, 2H), 2.25-2.52 (multiple peaks due to rotamers, 2H), 1.70-2.10 (multiple peaks, 11H), 1.10 (d, 6H), 0.80-1.00 (two triplets due to rotamers, 3H).

### Example 144: 1-{[4-(4-[(1-isopropylpiperidin-4-yl)oxy]phenyl)tetrahydro-2H-pyran-4-yl]methyl}pyrrolidin-2-one

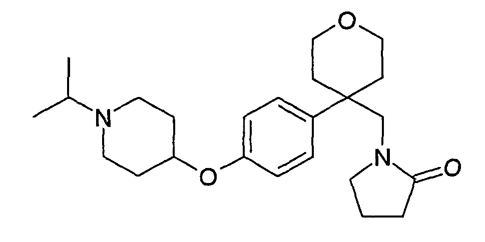

To a solution of (4-{4-[(1-isopropylpiperidin-4-yl)oxy]phenyl}tetrahydro-2H-pyran-4-yl)methylamine (100 mg, 0.301 mmol) in toluene (2 ml) was added ethyl-4-bromobutyrate (43 µl, 0.301 mmol), *N,N-*diisopropylethylamine (157 µl, 0.904 mmol) and potassium iodide (5 mg, 0.030 mmol). Reaction mixture heated at 80°C for 48 hours, then increased to reflux. Potassium carbonate (83 mg, 0.602 mmol) was added and reaction left at reflux for a further 24 hours until reaction complete. Reaction mixture was partitioned between ethyl acetate (20 ml) and saturated NaHCO₃ (10 ml). Organic washings were dried over Na₂SO₄ and concentrated *in vacuo* to give a brown oil (101 mg crude, 84%). The compound was purified by column chromatography on silica gel, eluting with dichloromethane:methanol:ammonia, 100:0:0 to 98:2:0.2, to give the title compound as an oil (7 mg, 6%).
¹H NMR (400MHz, CDCl₃) δ 7.20 (d, 2H), 7.90 (d, 2H), 4.22-4.36 (m, 1H), 4.80-4.90 (m, 2H), 3.52-3.67 (m, 2H), 3.40 (s, 2H), 2.72-3.88 (several multiplets, 3H), 2.50 (t, 2H), 2.34-2.44 (m, 2H), 2.21-2.30 (t, 2H), 1.60-2.10 (several multiplets, 10H), 1.08 (d, 6H).

### Example 145: N-{[4-(4-[(1-isopropylpiperidin-4-yl)oxy]phenyl)tetrahydro-2H-pyran-4-yl]methyl}pyrimidin-2-amine

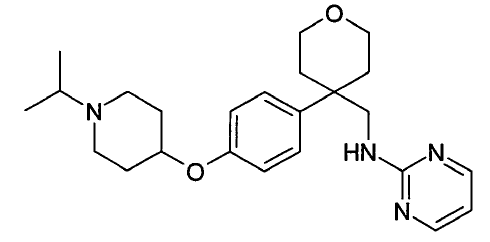

A mixture of (4-{4-[(1-isopropylpiperidin-4-yl)oxy]phenyl}tetrahydro-2*H*-pyran-4-yl)methylamine (150 mg, 0.422 mmol), 2-chloropyrimidine (52 mg, 0.422 mmol) and *N,N*-diisopropylethylamine (161 µl, 0.904 mmol) in N-methyl pyrrolidinone (0.5 ml) was heated at 150°C in the microwave (Smith Personal Synthesiser) for 900 seconds. The reaction mixture was partitioned between ethyl acetate (10 ml) and saturated NaHCO₃ (10 ml). The organic washings were dried over Na₂SO₄ and concentrated *in vacuo* to give an orange oil. The compound was purified by column chromatography on silica gel, eluting with dichloromethane:methanol:ammonia, 100:0:0 to 96:4:0.4, to give the title compound as a solid (87 mg, 47%).
¹H NMR (400MHz, CDCl₃) δ 8.20 (d, 2H), 7.21 (d, 2H), 6.90 (d, 2H), 6.48 (t, 2H), 4.71 (m, 1H), 4.20-4.35 (broad, 1H), 3.80-3.90 (m, 2H), 3.55-3.70 (multiple peaks, 4H), 2.70-2.90 (m, 3H), 2.32-2.49 (m, 2H), 1.78-2.19 (several multiplets, 8H), 1.10 (d, 6H).

### Example 146: N-{4-(4-(3-Pyrrolidin-1-ylpropoxy)phenyl)tetrahydro-2H-pyran-4-ylmethyl}pyrimidin-2-amine

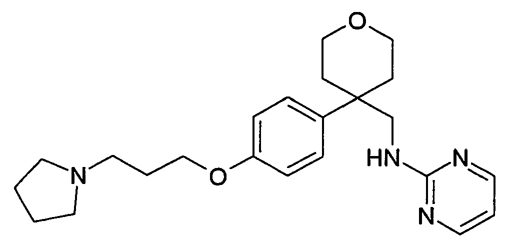

A mixture of {4-[4-(3-pyrrolidin-1-ylpropoxy)phenyl]tetrahydropyran-4-yl} methylamine (100 mg, 0.314 mmol), 2-chloropyrimidine (50 mg, 0.346 mmol) and *N,N-*diisopropylethylamine (112 µl, 0.629 mmol) in N-methyl pyrrolidinone (0.5 ml) was heated at 180°C in the microwave (Smith Personal Synthesiser) for 600 seconds. The reaction mixture was partitioned between ethyl acetate (10 ml) and saturated NaHCO₃ (10 ml). The organic washings were dried over Na₂SO₄ and concentrated *in vacuo* to give an oil. The compound was purified by column chromatography on silica gel, eluting with dichloromethane:methanol:ammonia, 100:0:0 to 88:12:1.2. Compound still impure so purified further on the Fraction Lynx automated LC system to give the title compound as a solid (22 mg, 17%)
¹H NMR (400MHz, CDCl₃) δ 8.30 (brs, 2H), 7.34 (d, 2H), 6.92 (d, 2H), 6.70 (t, 1H), 4.10 (t, 2H), 3.78-3.84 (m, 2H), 3.62-3.72 (multiple peaks, 4H), 3.44-3.55 (m, 2H), 3.40 (m, 2H), 3.04-3.18 (m, 2H), 1.85-2.25 (multiple peaks, 10H).

### Example 147: 2-{[4-(4-[(1-isopropylpiperidin-4-yl)oxy]phenyl)tetrahydro-2H-pyran-4-yl]methyl}isothiazolidine 1,1-dioxide

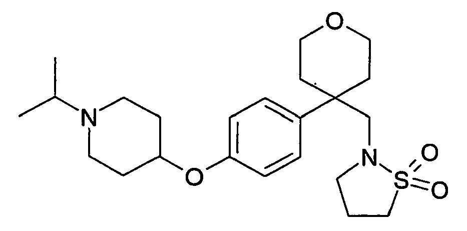

A solution of (4-{4-[(1-isopropylpiperidin-4-yl)oxy]phenyl}tetrahydro-2*H*-pyran-4-yl)methylamine (100mg, 0.30 mmol), 3-chloropropanesulphonyl chloride (51µl, 0.40 mmol) and *N,N*-diisopropylethylamine (52µl, 0.30 mmol) in dichloromethane (2ml) was stirred at room temperature for 18hrs. The reaction mixture was concentrated *in vacuo* and then partitioned between dichloromethane (10ml) and sodium bicarbonate solution (10ml). The aqueous layer was extracted with a further 10ml dichloromethane and the combined organics dried over sodium sulphate and concentrated *in vacuo* to give an oil. This was dissolved in tetrahydrofuran (2ml). Potassium *tert*-butoxide (65mg, 0.60 mmol) was added to the solution and the reaction mixture stirred at 40°C for 18hrs. This was then concentrated *in vacuo* and partitioned between diethyl ether (10ml) and water (10ml). The aqueous layer was extracted with a further 10ml diethyl ether and the combined organics dried over sodium sulphate and concentrated *in vacuo* to give a pale yellow oil. The compound was purified by column chromatography on Biotage® silica gel, eluting with dichloromethane:methanol:ammonia, 96:4:0.4 to give the title compound as a pale yellow oil (19mg, 15%).
¹H NMR (400 MHz, CDCl₃) δ 7.20 (d, 2H), 6.90 (d, 2H), 4.26 (m, 2H), 3.80 (m, 2H), 3.58 (m, 2H), 3.15 (s, 2H), 2.99 (t, 2H), 2.80 (m, 2H), 2.75(m, 2H), 2.40 (t, 2H), 2.38 (m, 2H), 2.16 (m, 2H), 2.02 (m, 4H), 1.90 (m, 2H) 1.81 (m, 2H), 1.03 (d, 6H); HRMS (ESI⁺) 437.2457 (M+H)⁺.

### Intermediate 53: 4-{4-[(1-isopropylpiperidin-4-yl)oxy]phenyl}tetrahydro-2H-pyran-4-carbothioamide

The title compound (320mg, 74%) was prepared from 4-[4-(1-isopropylpiperidin-4-yloxy)phenyl]tetrahydropyran-4-carbonitrile and diethyl dithiophosphate similarly to the procedure used for intermediate 50.
LRMS APCI⁺ m/z 363 [MH]⁺.

### Example 148: 1-isopropyl-4-{4-[4-(1,3-thiazol-2-yl)tetrahydro-2H-pyran-4-yl]phenoxy}piperidine

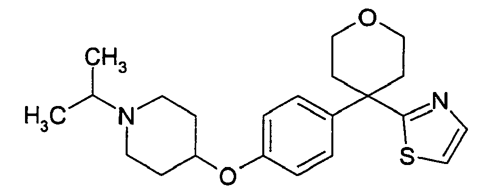

The title compound (320mg, 74%) was prepared from 4-{4-[(1-isopropylpiperidin-4-yl)oxy]phenyl}tetrahydro-2*H*-pyran-4-carbothioamide and bromoacetaldehyde diethylacetal similarly to the procedure used for example 137.
¹H NMR (400 MHz, CD₃OD) δ 1.10 (d, 6H), 1.75-1.80 (m, 2H), 1.98-2.02 (m, 2H), 2.37-2.44 (m, 2H), 2.47-2.51 (m, 2H), 2.61-2.65 (m, 2H), 2.75-2.84 (m, 3H), 3.66-3.70 (m, 2H), 3.78-3.83 (m, 2H), 4.37 (m, 1H), 6.89 (d, 2H), 7.28 (d, 2H), 7.46 (d, 1H), 7.70 (d, 1H).
HRMS ESI⁺ m/z 387.2090 [MH]⁺.

Microanalysis: Found: C, 67.42; H, 7.83; N, 7.08%. C₂₂H₃₀N₂O₂S. 0.2H₂O requires C, 67.73; H, 7.85; N, 7.18%.

### Example 149: 4-{4-[4-(azetidin-1-ylcarbonyl)tetrahydro-2H-pyran-4-yl]phenoxy}-1-isopropylpiperidine

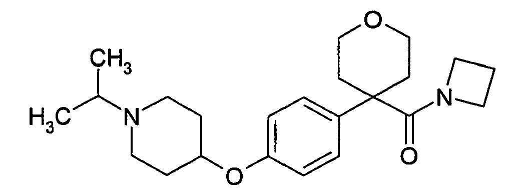

4-{4-[(1-Isopropylpiperidin-4-yl)oxy]phenyl}tetrahydro-2*H*-pyran-4-carboxylic acid (200mg, 0.58mmol), azetidine hydrochloride (81mg, 0.87mmol), 1-(3-dimethylaminopropyl)-3-ethylcarbodiimide hydrochloride (121mg, 0.63mmol), 1-hydroxybenzotriazole hydrate (97mg, 0.63mmol) and triethylamine (210µL, 1.51mmol) were stirred in dichloromethane (8mL) at room temperature for 18 hours. The reaction was diluted with dichloromethane (60mL) and washed with aqueous sodium bicarbonate. The aqueous were extracted again with dichloromethane. The organics were combined, dried over sodium sulphate, filtered and concentrated under reduced pressure. The crude product was purified by flash chromatography on silica gel eluting with dichloromethane:methanol:ammonia (96:4:0.5, by volume) to provide the title compound (97mg, 43%) as a white solid.
¹H NMR (400 MHz, CD₃OD) δ 1.11 (d, 6H), 1.77-1.82 (m, 2H), 1.88-1.95 (m, 2H), 2.01-2.08 (m, 4H), 2.26-2.29 (m, 2H), 2.47-2.52 (m, 2H), 2.76-2.86 (m, 3H), 3.66-3.74 (m, 4H), 3.79-3.82 (m, 2H), 3.92-3.96 (m, 2H), 4.40 (m, 1H), 6.95 (d, 2H), 7.22 (d, 2H).
HRMS ESI⁺ m/z 387.2634 [MH]⁺.

### Example 150: N-ethyl-4-{4-[(1-isopropylpiperidin-4-yl)oxy]phenyl}-N-methyltetrahydro-2H-pyran-4-carboxamide

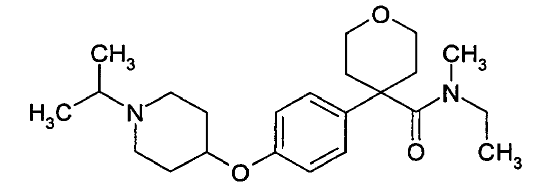

O-(1*H*-Benzotriazol-1-yl)-*N,N,N',N'*-tetramethyluronium hexafluorophosphate (286mg, 0.75mmol) was added to a solution of 4-{4-[(1-isopropylpipe/din-4-yl)oxy]phenyl}tetrahydro-2*H*-pyran-4-carboxylic acid (200mg, 0.58mmol) in *N,N*-dimethylformamide (6mL). Then triethylamine (243µL, 1.74mmol) was added. The mixture was stirred at room temperature for 15 minutes. Ethylmethylamine (500µL, 5.8mmol) was added and the reaction mixture stirred at room temperature for 18 hours. The reaction was concentrated *in vacuo.* The residue was partitioned between aqueous sodium bicarbonate solution (30mL) and ethyl acetate (2x75mL). The organics were combined, dried over sodium sulphate, filtered and concentrated under reduced pressure. The crude product was purified by flash chromatography on silica gel eluting with dichloromethane:methanol:ammonia (96:4:0.5, by volume) to provide the title compound (80mg, 35%).
¹H NMR (400 MHz, CD₃OD) δ 1.06-1.25 (m, 9H), 1.90-2.02 (m, 4H), 2.08-2.12 (m, 2H), 2.25-2.28 (m, 2H), 2.58 (m, 2H), 2.87 (m, 3H), 2.95 (m, 2H), 3.09-3.12 (m, 3H), 3.71-3.76 (m, 2H), 3.82-3.86 (m, 2H), 4.53 (m, 1H), 6.98 (d, 2H), 7.20 (d, 2H).
HRMS ESI⁺ m/z 389.2791 [MH]⁺.

### Example 151: 1-[(4-{4-[(1-isopropylpiperidin-4-yl)oxy]phenyl}tetrahydro-2H-pyran-4-yl)carbonyl]-4-methylpiperazine

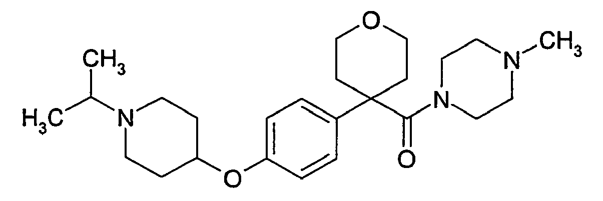

The title compound (60mg, 48%) was prepared from 4-{4-[(1-isopropylpiperidin-4-yl)oxy]phenyl}tetrahydro-2*H*-pyran-4-carboxylic acid and *N-*methyl piperazine similarly to the procedure used for example 150.
¹H NMR (400 MHz, CDCl₃) δ 1.05 (d, 6H), 1.79-1.81 (m, 2H), 2.00-2.03 (m, 7H), 2.15 (s, 3H), 2.18-2.22 (m, 2H), 2.37-2.42 (m, 2H), 2.77-2.79 (m, 3H), 2.99 (m, 1H), 3.27-3.56 (m, 4H), 3.78 (t, 2H), 3.87-3.89 (m, 2H), 4.28 (m, 1H), 6.88 (d, 2H), 7.14 (d, 2H).
HRMS ESI⁺ m/z 430.3062 [MH]⁺.

### Example 152: N-[(4-{4-[(1-isopropylpiperidin-4-yl)oxy]phenyl}tetrahydro-2H-pyran-4-yl)methyl]pyridin-2-amine

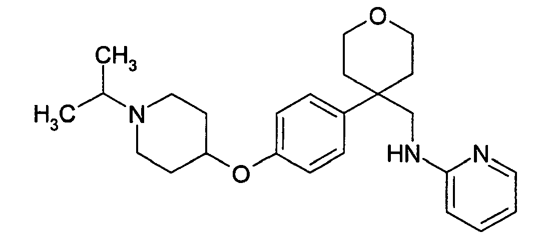

(4-{4-[(1-Isopropylpiperidin-4-yl)oxy]phenyl}tetrahydro-2*H*-pyran-4-yl)methylamine (300mg, 0.9mmol), 2-bromopyridine (50µL, 0.75mmol), tris(dibenzylideneacetone)dipalladium(0) (15mg, 0.018mmol), 2,2'-bis(diphenylphosphino)-1,1'-binaphthyl (19mg, 0.036mmol) and sodium *tert*-butoxide (100mg, 1mmol) were heated at 70°C in toluene (6ml) for 24 hours under nitrogen. The reaction was partitioned between aqueous sodium bicarbonate (302ml) and dichloromethane (2x75ml). The organics were combined, dried over sodium sulphate, filtered and concentrated under reduced pressure. The crude product was purified by flash chromatography on silica gel eluting with dichloromethane:methanol:ammonia (96:4:0.4, to 94:6:0.6 by volume) to provide the title compound (81mg, 22%) as a white solid after trituration in diethyl ether.
¹H NMR (400 MHz, CDCl₃) δ 1.11 (d, 6H), 1.86-1.95 (m, 4H), 2.07-2.16 (m, 4H), 2.46-2.51 (m, 2H), 2.82-2.86 (m, 3H), 3.49 (d, 2H), 3.56-3.61 (m, 2H), 3.80-3.85 (m, 2H), 4.03 (m, 1H), 4.33 (m, 1H), 6.22 (d, 1H), 6.51 (t, 1H), 6.91 (d, 2H), 7.24 (d, 2H), 7.32 (t, 1H), 8.01 (d, 1H).
HRMS ESI⁺ m/z 410.2793 [MH]⁺.

### Example 153: N-[(4-{4-[(1-isopropylpiperidin-4-yl)oxy]phenyl}tetrahydro-2H-pyran-4-yl)methyl]pyridin-3-amine

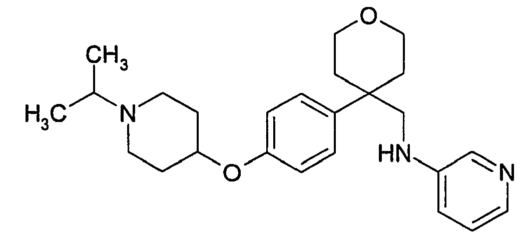

The title compound (20mg, 5%) was prepared from (4-{4-[(1-isopropylpiperidin-4-yl)oxy]phenyl}tetrahydro-2H-pyran-4-yl)methylamine and 3-iodopyridine similarly to the procedure used for example 152.
¹H NMR (400 MHz, CDCl₃) δ 1.12 (d, 6H), 1.88-1.95 (m, 4H), 2.10-2.21 (m, 4H), 2.51 (m, 2H), 2.83-2.88 (m, 3H), 3.22-3.27 (m, 3H), 3.55-3.59 (m, 2H), 3.77-3.82 (m, 2H), 4.35 (m, 1H), 6.73 (d, 1H), 6.93 (d, 2H), 7.00 (t, 1H), 7.24 (d, 2H), 7.87-7.90 (m, 2H).
HRMS ESI⁺ m/z 410.2791 [MH]⁺.

### Intermediate 54: 1-cyclobutylpiperidin-4-ol

4-Hydroxypiperidine (2.41g, 23.8mmol), cyclobutanone (5g, 71.4mmol) and acetic acid (1.36ml, 23.8mmol) were stirred in tetrahydrofuran (35ml) at 0°C for 1.5 hours. Sodium triacetoxyborohydride (10.1g, 47.7mmol) was then added at 0°C and the reaction mixture stirred at 0°C for 1 hour. The mixture was warmed to room temperature and stirred for 1 hour, then heated to 40°C for 18 hours. The reaction was concentrated *in vacuo*. The residue was taken up in water (30ml). The aqueous layer was basified to pH 9 with ammonia and extracted with diethyl ether (3x50ml). The organics were combined, dried over magnesium sulphate, filtered and concentrated *in vacuo.* The crude product was purified by flash chromatography on silica gel eluting with dichloromethane:methanol:ammonia (100:0:0 to 93:7:0.7 by volume) to provide the title compound (2.47g, 67%) as an oil.
LRMS APCI⁺ m/z 156 [MH]⁺.

### Example 154: 1-(4-{4-[(1-cyclobutylpiperidin-4-yl)oxy]phenyl}tetrahydro-2H-pyran-4-yl)-N,N-dimethylmethanamine

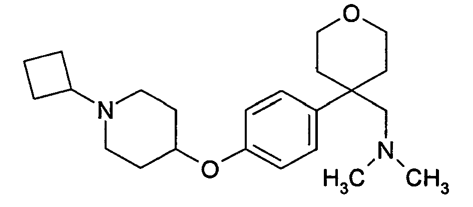

4-(4-Dimethylaminomethyl-tetrahydro-pyran-4-yl)-phenol (0.75g, 3.19mmol), 1-cyclobutylpiperidin-4-ol (0.45g, 2.90mmol), PPh₃ (0.84g, 3.19mmol), THF (10ml) and DIAD (0.66ml, 3.19mmol) were reacted together similarly to general procedure C. The crude material was subjected to chromatography on silica gel eluting with dichloromethane:methanol:ammonia (98:2:0.2) to provide the title compound (114mg, 11 %) as an off-white solid.
¹H NMR (400MHz, CD₃OD) δ 1.71-1.77 (m, 4H), 1.85-1.90 (m, 4H), 1.94 (s, 6H), 1.99-2.06 (m, 4H), 2.12-2.16 (m, 2H), 2.20-2.28 (m, 2H), 2.45 (s, 2H), 2.58-2.70 (m, 2H), 2.79 (m, 1H), 3.46-3.53 (m, 2H), 3.72-3.75 (m, 2H), 4.39 (m, 1H), 6.91 (d, 2H), 7.26 (d, 2H).
HRMS ESI⁺ m/z 373.2846 [MH]⁺.

### Intermediate 55: ethyl {4-[(1-cyclobutylpiperidin-4-yl)oxy]phenyl}acetate

DIAD (9.8ml, 47.3mmol) was added dropwise over 10 minutes to a solution of ethyl-4-hydroxy phenylacetate (7.7g, 42.7mmol), 1-cyclobutylpiperidin-4-ol (6.58g, 42.5mmol) and PPh₃ (12.3g, 47mmol) in THF (150ml) at 0°C. The reaction mixture was then stirred at room temperature for 18 hours. The reaction was concentrated *in vacuo.* The residue was taken up in ethyl acetate (150ml). The organic layer was washed with water (150ml) and 2N aqueous hydrochloric acid (50ml) twice. The acid layers were basified with sodium carbonate and extracted with dichloromethane (2x80ml). These organics were combined, dried over sodium sulphate, filtered and concentrated *in vacuo*. The crude product was purified by flash chromatography on silica gel eluting with ethyl acetate:pentane:diethylamine (70:30:2 by volume) to provide the title compound (2.0g, 15%) as a colourless oil. LRMS APCI⁺ m/z 318 [MH]⁺.

### Intermediate 56: ethyl 4-{4-[(1-cyclobutylpiperidin-4-yl)oxy]phenyl}tetrahydro-2H-pyran-4-carboxylate

NaH (60% dispersion in oil, 800mg, 20mmol) was added at 0°C to a solution of ethyl {4-[(1-cyclobutylpiperidin-4-yl)oxy]phenyl}acetate (2g, 6.3mmol) in THF (25ml) and *N-*methylpyrrolidinone (2ml) under nitrogen. The reaction was stirred at 0°C for 15 minutes. Bis(2-bromoethyl)ether (1.2ml, 9.54mmol) and potassium iodide (500mg, 3mmol) were added and the mixture stirred at 0°C for 45 minutes. The reaction was then warmed to room temperature and stirred for 18 hours. The reaction was cooled to 0°C, quenched with ice and solid carbon dioxide. The mixture was diluted with water and extracted with ethyl acetate (100ml and 20ml). The combined organic extracts were dried over sodium sulphate, filtered and concentrated *in vacuo*. The crude product was purified by flash chromatography on silica gel eluting with ethyl acetate:diethylamine (99:1 by volume) to provide the title compound (870mg, 36%) as a pale yellow oil.
LRMS APCI⁺ m/z 388 [MH]⁺.

### Intermediate 57: 4-{4-[(1-cyclobutylpiperidin-4-yl)oxy]phenyl}tetrahydro-2H-pyran-4-carboxylic acid

Sodium hydroxide (200mg, 5mmol) was added to a solution of ethyl 4-{4-[(1-cyclobutylpiperidin-4-yl)oxy]phenyl}tetrahydro-2*H*-pyran-4-carboxylate (870mg, 2.25mmol) in dioxane (10ml) and water (2ml). The reaction mixture was heated to reflux for 16 hours under nitrogen. The reaction was not complete. More sodium hydroxide (190mg, 4.75mmol) in water (4ml) was added and the reaction heated at reflux for 18 hours. 2N Hydrochloric acid (5ml, 9.75mmol) was added and the mixture concentrated *in vacuo* to a white powder (2.0g) containing the title compound and sodium chloride.
LRMS APCI⁺ m/z 360 [MH]⁺.

### Examples 155-160

The compounds of the following tabulated Examples of the general formula 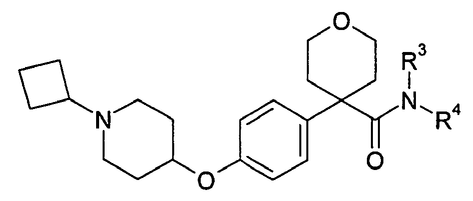 were prepared using 4-{4-[(1-cyclobutylpiperidin-4-yl)oxy]phenyl}tetrahydro-2*H*-pyran-4-carboxylic acid and the appropriate amines similarly to the procedure used for example 149.

| Ex No | NR³R⁴ | Analytical Data |
|---|---|---|
| **155** | NMe₂ | 4-{4-[(1-cyclobutylpiperidin-4-yl)oxy]phenyl}-*N*,*N-*dimethyltetrahydro-2H-pyran-4-carboxamide |
| | | ¹H NMR (400MHz, CD₃OD) δ 1.70-1.79 (m, 4H), 1.89-2.02 (m, 6H), 2.07-2.10 (m, 2H), 2.25 (d, 4H), 2.63-2.87 (m, 9H), 3.73 (t, 2H), 3.82-3.85 (m, 2H), 4.41 (m, 1H), 6.94 (d, 2H), 7.16 (d, 2H). |
| | | HRMS ESI⁺ m/z 387.2637 [MH]⁺. |
| | | 25% yield |
| **156** | NHMe | 4-{4-[(1-cyclobutylpiperidin-4-yl)oxy]phenyl}-N-methyltetrahydro-2H-pyran-4-carboxamide |
| | | ¹H NMR (400MHz, CD₃OD) δ 1.70-1.76 (m, 4H), 1.88-2.08 (m, 8H), 2.22-2.25 (m, 2H), 2.38-2.42 (m, 2H), 2.59-2.63 (m, 2H), 2.66 (s, 3H), 2.80 (m, 1H), 3.61 (t, 2H), 3.75-3.80 (m, 2H), 4.39 (m, 1H), 6.90 (d, 2H), 7.27 (d, 2H). |
| | | HRMS ESI⁺ m/z 373.2480 [MH]⁺. |
| | | 10% yield |
| **157** | 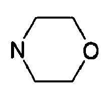 | 4-[(4-{4-[(1-cyclobutylpiperidin-4-yl)oxy]phenyl}tetrahydro-2H-pyran-4-yl)carbonyl]morpholine |
| | | ¹H NMR (400MHz, CD₃OD) δ 1.70-1.76 (m, 4H), 1.85-2.09 (m, 8H), 2.20-2.24 (m, 4H), 2.63-2.67 (m, 2H), 2.81 (m, 1H), 3.25-3.31 (m, 6H), 3.33-3.39 (m, 2H), 3.73 (t, 2H), 3.82-3.87 (m, 2H), 4.41 (m, 1H), 6.96 (d, 2H), 7.20 (d, 2H). |
| | | HRMS ESI⁺ m/z 429.2740 [MH]⁺. |
| | | 20% yield |
| **158** | NH₂ | 4-{4-[(1-cyclobutylpiperidin-4-yl)oxy]phenyl}tetrahydro-2H-pyran-4-carboxamide |
| | | ¹H NMR (400MHz, CD₃OD) δ 1.76-1.82 (m, 2H), 1.94-2.00 (m, 4H), 2.06-2.10 (m, 4H), 2.20-2.22 (m, 2H), 2.42 (d, 2H), 2.65-2.79 (m, 2H), 2.89-2.99 (m, 2H), 3.26 (m, 1H), 3.66 (t, 2H), 3.78-3.82 (m, 2H), 4.55 (m, 1H), 6.95 (d, 2H), 7.34 (d, 2H). |
| | | HRMS ESI⁺ m/z 359.2322 [MH]⁺. |
| | | 13% yield |
| **159** | NHiPr | 4-{4-[(1-cyclobutylpiperidin-4-yl)oxy]phenyl}-N-isopropyltetrahydro-2H-pyran-4-carboxamide |
| | | ¹H NMR (400MHz, CD₃OD) δ 1.04 (d, 6H), 1.70-1.77 (m, 4H), 1.88-2.00 (m, 6H), 2.05-2.08 (m, 2H), 2.20-2.26 (m, 2H), 2.42 (d, 2H), 2.60-2.68 (m, 2H), 2.81 (m, 1H), 3.62 (t, 2H), 3.77-3.82 (m, 2H), 3.99 (m, 1H), 4.39 (m, 1H), 6.90 (d, 2H), 7.28 (d, 2H). |
| | | HRMS ESI⁺ m/z 401.2789 [MH]⁺. |
| | | 13% yield |
| **160** | NHEt | 4-{4-[(1-cyclobutylpiperidin-4-yl)oxy]phenyl}-N-ethyltetrahydro-2H-pyran-4-carboxamide |
| | | ¹H NMR (400MHz, CD₃OD) δ 1.01 (t, 3H), 1.69-1.75 (m, 4H), 1.88-2.01 (m, 6H), 2.04-2.07 (m, 2H), 2.16-2.28 (m, 2H), 2.41 (d, 2H), 2.56-2.68 (m, 2H), 2.79 (m, 1H), 3.15 (q, 2H), 3.62 (t, 2H), 3.76-3.80 (m, 2H), 4.38 (m, 1H), 6.89 (d, 2H), 7.28 (d, 2H). |
| | | HRMS ESI⁺ m/z 387.2636 [MH]⁺. |
| | | 22% yield |

### Intermediate 58: 4-{4-[(1-cyclobutylpiperidin-4-yl)oxy]phenyl}tetrahydro-2H-pyran-4-carbothioamide

The title compound (223mg, 50%) was prepared from 4-{4-[(1-cyclobutyl-4-piperidinyl)oxy]phenyl}tetrahydro-2*H*-pyran-4-carbonitrile and diethyl dithiophosphate similarly to the procedure used for intermediate 50.
LRMS APCI⁺ m/z 375 [MH]⁺.

### Example 161: 1-cyclobutyl-4-{4-[4-(1,3-thiazol-2-yl)tetrahydro-2H-pyran-4-yl]phenoxy}piperidine

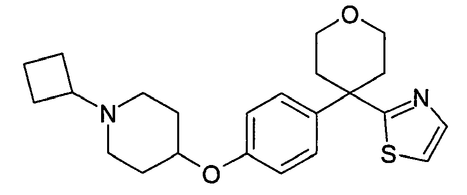

The title compound (92mg, 39%) was prepared from 4-{4-[(1-cyclobutylpiperidin-4-yl)oxy]phenyl}tetrahydro-2*H*-pyran-4-carbothioamide and bromoacetaldehyde diethylacetal similarly to the procedure used for example 137.
¹H NMR (400 MHz, CDCl₃) δ 1.66-1.72 (m, 2H), 1.77-1.89 (m, 5H), 1.95-2.04 (m, 4H), 2.11 (m, 1H), 2.36-2.43 (m, 2H), 2.58-2.72 (m, 5H), 3.71 (t, 2H), 3.84-3.88 (m, 2H), 4.27 (m, 1H), 6.84 (d, 2H), 7.23-7.26 (m, 3H), 7.70 (s, 1H).
LRMS APCI⁺ m/z 399 [MH]⁺.

### Example 162: 1-cyclobutyl-4-{4-[4-(4-methyl-1,3-thiazol-2-yl)tetrahydro-2H-pyran-4-yl]phenoxy}piperidine

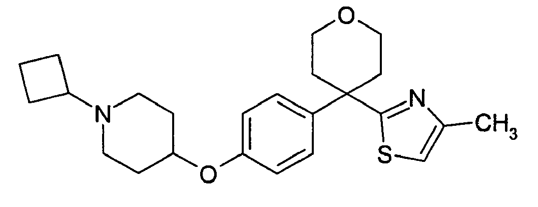

The title compound (155mg, 83%) was prepared from 4-{4-[(1-cyclobutylpiperidin-4-yl)oxy]phenyl}tetrahydro-2*H*-pyran-4-carbothioamide and chloroacetone similarly to the procedure used for example 136.
¹H NMR (400 MHz, CDCl₃) δ 1.64-1.71 (m, 2H), 1.78-1.81 (m, 2H), 1.88-2.04 (m, 6H), 2.10-2.20 (m, 2H), 2.31-2.38 (m, 2H), 2.42 (s, 3H), 2.61-2.64 (m, 4H), 2.74 (m, 1H), 3.71 (t, 2H), 3.81-3.85 (m, 2H), 4.28 (m, 1H), 6.75 (s, 1H), 6.83 (d, 2H), 7.23 (d, 2H).
HRMS ESI⁺ m/z 413.2257 [MH]⁺.

### Intermediate 59: 1-(4-{4-[(1-cyclobutylpiperidin-4-yl)oxy]phenyl}tetrahydro-2H-pyran-4-yl)methanamine

The title compound (1.05g, 100%) was prepared from 4-{4-[(1-cyclobutyl-4-piperidinyl)oxy]phenyl}tetrahydro-2*H*-pyran-4-carbonitrile and lithium aluminium hydride similarly to the procedure used for intermediate 51.
LRMS APCI⁺ m/z 345 [MH]⁺.

### Example 163: N-[(4-{4-[(1-cyclobutylpiperidin-4-yl)oxy]phenyl}tetrahydro-2H-pyran-4 yl)methyl]pyrimidin-2-amine

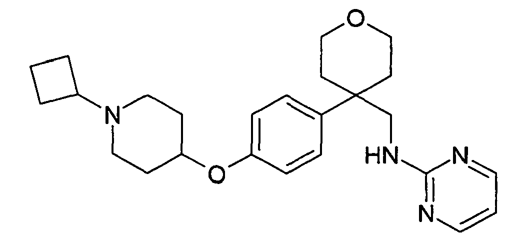

The title compound (31mg, 25%) was prepared from 1-(4-{4-[(1-cyclobutylpiperidin-4-yl)oxy]phenyl}tetrahydro-2*H*-pyran-4-yl)methanamine and 2-chloropyrimidine similarly to the procedure used for example 145.
¹H NMR (400 MHz, CDCl₃) δ 1.66-1.73 (m, 2H), 1.83-1.95 (m, 6H), 1.98-2.15 (m, 8H), 2.60-2.66 (m, 2H), 2.75 (m, 1H), 3.57-3.62 (m, 2H), 3.66 (d, 2H), 3.81-3.85 (m, 2H), 4.31 (m, 1H), 4.68 (t, 1H), 6.47 (t, 1H), 6.90 (d, 2H), 7.23 (d, 2H), 8.20 (d, 2H).
LRMS APCI⁺ m/z 423 [MH]⁺.

### Example 164: N-[(4-{4-[(1-cyclobutylpiperidin-4-yl)oxy]phenyl}tetrahydro-2H-pyran-4-yl)methyl]pyridin-2-amine

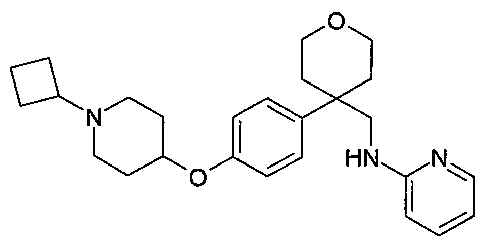

The title compound (25mg, 31%) was prepared from 1-(4-{4-[(1-cyclobutylpiperidin-4-yl)oxy]phenyl}tetrahydro-2*H*-pyran-4-yl)methanamine and 2-bromopyridine similarly to the procedure used for example 152.
¹H NMR (400 MHz, CDCl₃) δ 1.68-1.72 (m, 2H), 1.84-1.94 (m, 6H), 2.00-2.16 (m, 8H), 2.63-2.67 (m, 2H), 2.74 (m, 1H), 3.49 (d, 2H), 3.56-3.62 (m, 2H), 3.80-3.85 (m, 2H), 4.02 (m, 1H), 4.31 (m, 1H), 6.22 (d, 1H), 6.51 (t, 1H), 6.91 (d, 2H), 7.24 (d, 2H), 7.32 (t, 1H), 8.01 (d, 1H).
HRMS ESI⁺ m/z 422.2792 [MH]⁺.

### Example 165: N-[(4-{4-[(1-cyclobutylpiperidin-4-yl)oxy]phenyl}tetrahydro-2H-pyran-4-yl)methyl]pyridin-3-amine

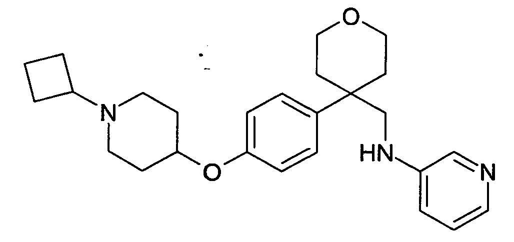

The title compound (28mg, 18%) was prepared from 1-(4-{4-[(1-cyclobutylpiperidin-4-yl)oxy]phenyl}tetrahydro-2*H*-pyran-4-yl)methanamine and 3-iodopyridine similarly to the procedure used for example 153.
¹H NMR (400 MHz, CDCl₃) δ 1.66-1.94 (m, 8H), 2.01-2.06 (m, 4H), 2.17-2.20 (m, 4H), 2.60-2.68 (m, 2H), 2.76 (m, 1H), 3.22-3.27 (m, 3H), 3.57 (t, 2H), 3.77-3.81 (m, 2H), 4.32 (m, 1H), 6.73 (d, 1H), 6.92 (d, 2H), 6.99 (m, 1H), 7.23 (d, 2H), 7.88 (m, 2H).
HRMS ESI⁺ m/z 422.2789 [MH]⁺.

### Example 166: 1-acetyl-4-({4-[4-(3-pyrrolidin-1-ylpropoxy)phenyl]tetrahydro-2H-pyran-4-yl}methyl)piperazine

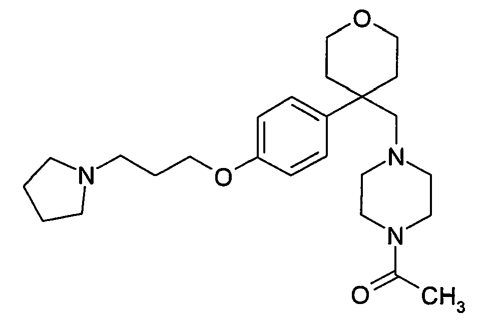

To a stirred solution of 1-{4-[4-(3-pyrrolidin-1-ylpropoxy)phenyl]tetrahydropyran-4-ylmethyl}piperazine (160mg, 0.41mmol) and *N,N*-diisopropylethylamine (180µl, 1.03mmol) in dichloromethane (2ml) at 0°C was added dropwise acetic anhydride (47µl, 0.49mmol). The reaction was allowed to warm up to room temperature and stirred for 18 hours. The reaction mixture was concentrated under reduced pressure and the residue partitioned between dichloromethane (20ml) and aqueous sodium carbonate solution (20ml). The layers were separated and the aqueous layer was extracted again with dichloromethane (20ml). The organics were combined, dried over sodium sulphate, filtered and concentrated *in vacuo.* The crude product was purified by flash chromatography on silica gel eluting with dichloromethane:methanol:ammonia (96:4:0.4 to 90:10:1 by volume) to provide the title compound (34mg, 19%) as a clear oil.
¹H NMR (400MHz, CDCl₃) δ 1.78-1.81 (m, 4H), 1.83-1.89 (m, 2H), 1.99 (s, 3H), 2.01-2.03 (m, 2H), 2.06-2.11 (m, 3H), 2.15-2.18 (m, 3H), 2.40 (s, 2H), 2.51-2.53 (m, 4H), 2.63 (t, 2H), 3.22 (t, 2H), 3.42-3.44 (m, 2H), 3.52 (t, 2H), 3.73-3.76 (m, 2H), 4.02 (t, 2H), 6.86 (d, 2H), 7.21 (d, 2H).
HRMS ESI⁺ m/z 430.3052 [MH]⁺.

### Example 167: 1-isopropyl-4-({4-[4-(3-pyrrolidin-1-ylpropoxy)phenyl]tetrahydro-2H-pyran-4-yl}methyl)piperazine

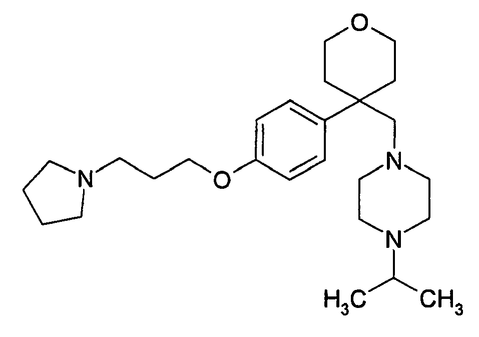

The title compound (78mg, 44%) was prepared from 1-{4-[4-(3-pyrrolidin-1-ylpropoxy)phenyl]tetrahydropyran-4-ylmethyl}piperazine and acetone similarly to the procedure used for example 54.
¹H NMR (400MHz, CDCl₃) δ 0.98 (d, 6H), 1.77-1.80 (m, 4H), 1.86-1.93 (m, 2H), 1.98-2.02 (m, 2H), 2.06-2.09 (m, 2H), 2.20-2.22 (m, 4H), 2.34-2.36 (m, 6H), 2.50-2.54 (m, 5H), 2.62 (t, 2H), 3.52 (t, 2H), 3.71-3.75 (m, 2H), 4.02 (t, 2H), 6.85 (d, 2H), 7.21 (d, 2H).
HRMS ESI⁺ m/z 430.3417 [MH]⁺.

### Example 168: 1-(cyclopropylmethyl)-4-({4-[4-(3-pyrrolidin-1-yl-propoxy)-phenyl]-tetrahydro-2H-pyran-4-yl}methyl)-piperazine

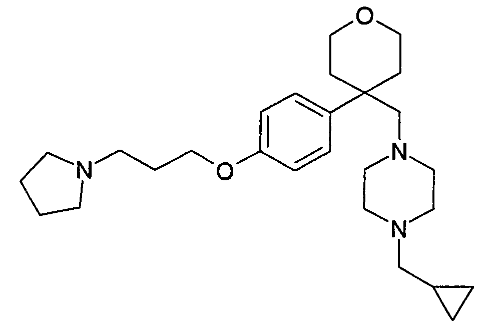

The title compound (26mg, 14%) was prepared from 1-{4-[4-(3-pyrrolidin-1-ylpropoxy)phenyl]tetrahydropyran-4-ylmethyl}piperazine and cyclopropane carboxaldehyde similarly to the procedure used for example 54.
¹H NMR (400MHz, CDCl₃) δ 0.02 (q, 2H), 0.43 (q, 2H), 0.76 (m, 1H), 1.74-1.76 (m, 4H), 1.83-1.89 (m, 2H), 1.95-1.99 (m, 2H), 2.03-2.06 (m, 3H), 2.13 (d, 2H), 2.19-2.21 (m, 4H), 2.26-2.40 (m, 5H), 2.47-2.51 (m, 4H), 2.59 (t, 2H), 3.49 (t, 2H), 3.68-3.72 (m, 2H), 3.98 (t, 2H), 6.82 (d, 2H), 7.17 (d, 2H).
HRMS ESI⁺ m/z 442.3412 [MH]⁺.

### Example 169: 1-cyclopropyl-N-(cyclopropylmethyl)-N-({4-[4-(3-pyrrolidin-1-ylpropoxy)phenyl]-tetrahydro-2H-pyran-4-yl}methyl)methanamine

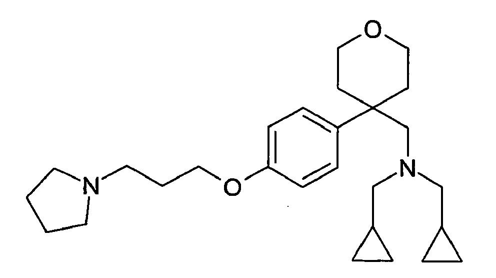

The title compound (169mg, 51%) was prepared from {4-[4-(3-pyrrolidin-1-ylpropoxy)phenyl]tetrahydropyran-4-yl} methylamine free base and cyclopropane carboxaldehyde similarly to the procedure used for example 54. 3 equivalents of cyclopropane carboxaldehyde were required for the synthesis of this Example.
¹H NMR (400MHz, CDCl₃) δ -0.04 (q, 4H), 0.39 (q, 4H), 0.71-0.76 (m, 2H), 1.80-1.86 (m, 4H), 1.94-2.12 (m, 6H), 2.16 (d, 4H), 2.55-2.72 (m, 8H), 3.51 (t, 2H), 3.75-3.79 (m, 2H), 4.04 (t, 2H), 6.88 (d, 2H), 7.21 (d, 2H).
HRMS ESI⁺ m/z 427.3306 [MH]⁺.

### Examples 170-178

The compounds of the following tabulated Examples of the general formula 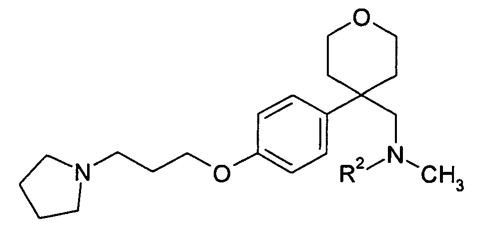 were prepared using methyl-{4-[4-(3-pyrrolidin-1-ylpropoxy)phenyl]-tetrahydropyran-4-ylmethyl}amine and the appropriate aldehydes or ketones similarly to the procedure used for example 54.

| Ex No | R² | Analytical Data |
|---|---|---|
| **170** | 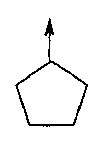 | N-methyl-N-({4-[4-(3-pyrrolidin-1-ylpropoxy)phenyl]tetrahydro-2H-pyran-4-yl}methyl)cyclopentanamine |
| | | ¹H NMR (400MHz, CDCl₃) δ 1.21-1.28 (m, 2H), 1.39-1.43 (m, 2H), 1.53-1.60 (m, 4H), 1.83-1.94 (m, 9H), 2.07-2.12 (m, 4H), 2.41 (s, 2H), 2.61-2.72 (m, 7H), 3.49 (t, 2H), 3.73-3.79 (m, 2H), 4.02 (t, 2H), 6.89 (d, 2H), 7.20 (d, 2H). |
| | | HRMS ESI⁺ m/z 401.3162 [MH]⁺. |
| | | 53% yield |
| **171** | 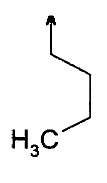 | N-methyl-N-({4-[4-(3-pyrrolidin-1-ylpropoxy)phenyl]tetrahydro-2H-pyran-4-yl}methyl)butan-1-amine |
| | | ¹H NMR (400MHz, CDCl₃) δ 0.83 (t, 3H), 1.15-1.20 (m, 2H), 1.23-1.29 (m, 2H), 1.85-1.92 (m, 9H), 2.07-2.15 (m, 6H), 2.40 (s, 2H), 2.69-2.77 (m, 6H), 3.51 (t, 2H), 3.73-3.78 (m, 2H), 4.03 (t, 2H), 6.86 (d, 2H), 7.20 (d, 2H). |
| | | HRMS ESI⁺ m/z 389.3153 [MH]⁺. |
| | | 57% yield |
| **172** | 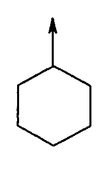 | N-methyl-N-({4-[4-(3-pyrrolidin-1-ylpropoxy)phenyl]tetrahydro-2H-pyran-4-yl}methyl)cyclohexanamine |
| | | ¹H NMR (400MHz, CD₃OD) δ 1.02-1.14 (m, 6H), 1.53 (m, 1H), 1.60 (d, 2H), 1.69 (d, 2H), 1.81-1.87 (m, 6H), 1.91 (s, 3H), 1.99-2.03 (m, 2H), 2.14 (d, 2H), 2.49 (s, 2H), 2.61 (t, 4H), 2.69 (t, 2H), 3.48 (t, 2H), 3.72-3.76 (m, 2H), 4.03 (t, 2H), 6.89 (d, 2H), 7.24 (d, 2H). |
| | | HRMS ESI⁺ m/z 415.3314 [MH]⁺. |
| | | 61% yield |
| **173** | 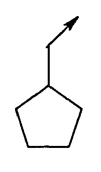 | 1-cyclopentyl-N-methyl-N-({4-[4-(3-pyrrolidin-1-ylpropoxy)phenyl]tetrahydro-2H-pyran-4-yl}methyl)methanamine |
| | | ¹H NMR (400MHz, CD₃OD) δ 1.07-1.12 (m, 2H), 1.46-1.50 (m, 4H), 1.60-1.64 (m, 2H), 1.82-1.85 (m, 5H), 1.87-1.91 (m, 5H), 1.99-2.01 (m, 2H), 2.04 (d, 2H), 2.14 (d, 2H), 2.43 (s, 2H), 2.59-2.62 (m, 4H), 2.69 (t, 2H), 3.49 (t, 2H), 3.71-3.76 (m, 2H), 4.02 (t, 2H), 6.88 (d, 2H), 7.24 (d, 2H). |
| | | HRMS ESI⁺ m/z 415.3313 [MH]⁺. |
| | | 44% yield |
| **174** | 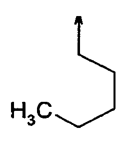 | N-methyl-N-({4-[4-(3-pyrrolidin-1-ylpropoxy)phenyl]tetrahydro-2H-pyran-4-yl}methyl)pentan-1-amine |
| | | ¹H NMR (400MHz, CD₃OD) δ 0.86 (t, 3H), 1.08-1.13 (m, 2H), 1.20-1.28 (m, 4H), 1.82-1.88 (m, 6H), 1.92 (s, 3H), 1.97-2.01 (m, 2H), 2.08 (t, 2H), 2.14 (d, 2H), 2.44 (s, 2H), 2.60-2.64 (m, 4H), 2.70 (t, 2H), 3.49 (t, 2H), 3.71-3.76 (m, 2H), 4.02 (t, 2H), 6.89 (d, 2H), 7.25 (d, 2H). |
| | | HRMS ESI⁺ m/z 403.3313 [MH]⁺. |
| | | 30% yield |
| **175** | 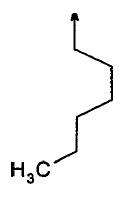 | N-methyl-N-({4-[4-(3-pyrrolidin-1-ylpropoxy)phenyl]tetrahydro-2H-pyran-4-yl}methyl)hexan-1-amine |
| | | ¹H NMR (400MHz, CDCl₃) δ 0.87 (t, 3H), 1.13-1.20 (m, 4H), 1.23-1.29 (m, 4H), 1.83-1.91 (m, 9H), 2.06-2.13 (m, 6H), 2.40 (s, 2H), 2.58-2.72 (m, 6H), 3.51 (t, 2H), 3.73-3.77 (m, 2H), 4.02 (t, 2H), 6.85 (d, 2H), 7.19 (d, 2H). |
| | | HRMS ESI⁺ m/z 417.3469 [MH]⁺. |
| | | 74% yield |
| **176** | 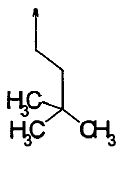 | N,3,3-trimethyl-N-({4-[4-(3-pyrrolidin-1-ylpropoxy)phenyl]tetrahydro-2H-pyran-4-yl}methyl)butan-1-amine |
| | | ¹H NMR (400MHz, CDCl₃) δ 0.77 (s, 9H), 1.17 (t, 2H), 1.84-1.90 (m, 6H), 1.92 (s, 3H), 2.08-2.12 (m, 6H), 2.41 (s, 2H), 2.57-2.70 (m, 6H), 3.52 (t, 2H), 3.73-3.78 (m, 2H), 4.01 (t, 2H), 6.86 (d, 2H), 7.20 (d, 2H). |
| | | HRMS ESI⁺ m/z 417.3457 [MH]⁺. |
| | | 59% yield |
| **177** | 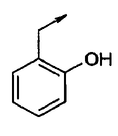 | 2-{[methyl({4-[4-(3-pyrrolidin-1-ylpropoxy)phenyl]tetrahydro-2H-pyran-4-yl}methyl)amino]methyl}phenol |
| | | ¹H NMR (400MHz, CDCl₃) δ 1.78 (s, 3H), 1.79-1.85 (m, 6H), 2.06-2.09 (m, 2H), 2.21 (d, 2H), 2.67-2.75 (m, 8H), 3.48-3.54 (m, 4H), 3.70-3.75 (m, 2H), 4.03 (t, 2H), 6.73 (t, 1H), 6.77 (d, 1H), 6.86 (d, 1H), 6.90 (d, 2H), 7.13 (t, 1H), 7.25 (d, 2H). |
| | | HRMS ESI⁺ m/z 439.2943 [MH]⁺. |
| | | 30% yield |
| **178** | 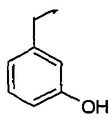 | 3-{[methyl({4-[4-(3-pyrrolidin-1-ylpropoxy)phenyl]tetrahydro-2H-pyran-4-yl}methyl)amino]methyl}phenol |
| | | ¹H NMR (400MHz, CD₃OD) δ 1.83 (brs, 7H), 1.88-1.91 (m, 2H), 1.95-2.01 (m, 2H), 2.11-2.15 (d, 2H), 2.55 (s, 2H), 2.58-2.62 (m, 4H), 2.69 (t, 2H), 3.17 (s, 2H), 3.49 (t, 2H), 3.63-3.67 (m, 2H), 4.02 (t, 2H), 6.62 (d, 1H), 6.67 (d, 1H), 6.72 (s, 1H), 6.89 (d, 2H), 7.05 (t, 1H), 7.29 (d, 2H). |
| | | HRMS ESI⁺ m/z 439.2941 [MH]⁺. |
| | | 68% yield |

### Examples 179-191

The compounds of the following tabulated Examples of the general formula 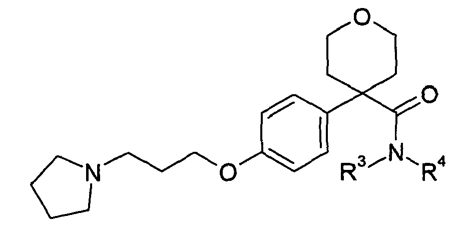 were prepared using 4-[4-(3-pyrrolidin-1-ylpropoxy)phenyl]tetrahydro-2H-pyran-4-carboxylic acid, the appropriate amines and O-(1*H*-benzotriazol-1-yl)-*N,N,N',N'*-tetramethyluronium hexafluorophosphate (HBTU), O-(1*H*-benzotriazol-1-yl)-*N,N,N',N'*-tetramethyluronium tetrafluoroborate (TBTU) or 1-[3-(Dimethylamino)propyl]-3-ethylcarbodiimide hydrochloride (WSCDI) similarly to the procedure used for example 150.

| Ex No | NR³R⁴ | Analytical Data |
|---|---|---|
| **179** | NEt₂ | N,N-diethyl-4-[4-(3-pyrrolidin-1-ylpropoxy)phenyl]tetrahydro-2H-pyran-4-carboxamide |
| | | ¹H NMR (400MHz, CDCl₃) δ 0.64-0.74 (m, 3H), 1.04-1.12 (m, 3H), 1.79-1.82 (m, 4H), 1.91-2.03 (m, 4H), 2.23 (d, 2H), 2.54-2.58 (m, 4H), 2.65 (t, 2H), 2.86-2.94 (m, 2H), 3.27-3.35 (m, 2H), 3.77-3.89 (m, 4H), 4.01 (t, 2H), 6.86 (d, 2H), 7.15 (d, 2H). |
| | | HRMS ESI⁺ m/z 389.2789 [MH]⁺. |
| | | Microanalysis: Found: C, 69.16; H, 9.18; N, 7.05%. C₂₃H₃₆N₂O₃. 0.5H₂O requires C, 69.49; H, 9.38; N, 7.05%. |
| | | 68% yield |
| **180** | 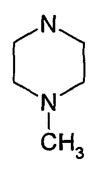 | 1-methyl-4-({4-[4-(3-pyrrolidin-1-ylpropoxy)phenyl]tetrahydro-2H-pyran-4-yl}carbonyl)piperazine |
| | | ¹H NMR (400MHz, CDCl₃) δ 1.76-1.81 (m, 4H), 1.97-2.03 (m, 8H), 2.15 (s, 3H), 2.19-2.22 (m, 2H), 2.50-2.54 (m, 4H), 2.62 (t, 2H), 3.26-3.44 (m, 4H), 3.78 (t, 2H), 3.87-3.90 (m, 2H), 4.02 (t, 2H), 6.88 (d, 2H), 7.15 (d, 2H). |
| | | HRMS ESI⁺ m/z 416.2908 [MH]⁺. |
| | | 9% yield |
| **181** | 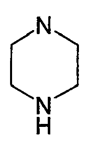 | 1-({4-[4-(3-pyrrolidin-1-ylpropoxy)phenyl]tetrahydro-2H-pyran-4- yl}carbonyl)piperazine |
| | | ¹H NMR (400MHz, CDCl₃) δ 1.77-1.80 (m, 6H), 1.97-2.02 (m, 4H), 2.20 (d, 2H), 2.50-2.54 (m, 6H), 2.61 (t, 2H), 3.24-3.38 (m, 4H), 3.78 (t, 2H), 3.86-3.90 (m, 2H), 4.00 (t, 2H), 6.87 (d, 2H), 7.15 (d, 2H). |
| | | HRMS ESI⁺ m/z 402.2746 [MH]⁺. |
| | | 43% yield |
| **182** | 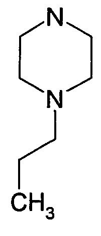 | 1-propyl-4-({4-[4-(3-pyrrolidin-1-ylpropoxy)phenyl]tetrahydro-2H-pyran-4-yl}carbonyl)piperazine |
| | | ¹H NMR (400MHz, CDCl₃) δ 0.84 (t, 3H), 1.37-1.43 (m, 2H), 1.1.69-1.77 (m, 2H), 1.84-1.88 (m, 4H), 1.98-2.07 (m, 6H), 2.14-2.19 (m, 4H), 2.65-2.69 (m, 4H), 2.74 (t, 2H), 3.26-3.40 (m, 4H), 3.78 (t, 2H), 3.87-3.89 (m, 2H), 4.02 (t, 2H), 6.86 (d, 2H), 7.15 (d, 2H). |
| | | HRMS ESI⁺ m/z 444.3196 [MH]⁺. |
| | | 71% yield |
| **183** | 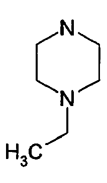 | 1-ethyl-4-({4-[4-(3-pyrrolidin-1-ylpropoxy)phenyl]tetrahydro-2H-pyran-4-yl}carbonyl)piperazine |
| | | ¹H NMR (400MHz, CDCl₃) δ 0.97 (t, 3H), 1.76-1.83 (m, 4H), 1.98-2.01 (m, 8H), 2.18 (d, 2H), 2.25 (d, 2H), 2.51-2.55 (m, 4H), 2.62 (t, 2H), 3.20-3.40 (m, 4H), 3.77 (t, 2H), 3.85-3.88 (m, 2H), 3.98-4.03 (m, 2H), 6.85 (d, 2H), 7.13 (d, 2H). |
| | | HRMS ESI⁺ m/z 430.3047 [MH]⁺. |
| | | 81 % yield |
| **184** | 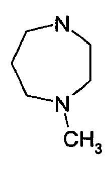 | 1-methyl-4-({4-[4-(3-pyrrolidin-1-ylpropoxy)phenyl]tetrahydro-2H-pyran-4-yl}carbonyl)-1,4-diazepane |
| | | ¹H NMR (400MHz, CDCl₃) δ 1.75-1.81 (m, 8H), 1.98-2.03 (m, 4H), 2.14-2.29 (m, 7H), 2.51-2.54 (m, 4H), 2.62 (t, 2H), 3.12-3.24 (m, 2H), 3.54-3.65 (m, 2H), 3.77-3.88 (m, 4H), 4.01 (t, 2H), 6.88 (d, 2H), 7.16 (d, 2H). |
| | | HRMS ESI⁺ m/z 430.3057 [MH]⁺. |
| | | 68% yield |
| **185** | 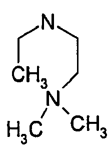 | N-[2-(dimethylamino)ethyl]-N-ethyl-4-[4-(3-pyrrolidin-1-ylpropoxy)phenyl]tetrahydro-2H-pyran-4-carboxamide |
| | | ¹H NMR (400MHz, CDCl₃) δ 0.66-0.72 (m, 3H), 1.76-1.81 (m, 4H), 1.98-2.02 (m, 6H), 2.23-2.26 (m, 6H), 2.40-2.45 (m, 2H), 2.50-2.55 (m, 4H), 2.61 (t, 2H), 2.93-3.00 (m, 2H), 3.30-3.39 (m, 2H), 3.80 (t, 2H), 3.86-3.89 (m, 2H), 4.01 (t, 2H), 6.88 (d, 2H), 7.16 (d, 2H). |
| | | HRMS ESI⁺ m/z 432.3215 [MH]⁺. |
| | | 62% yield |
| **186** | 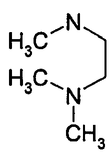 | N-[2-(dimethylamino)ethyl]-N-methyl-4-[4-(3-pyrrolidin-1-ylpropoxy)phenyl]tetrahydro-2H-pyran-4-carboxamide |
| | | ¹H NMR (400MHz, CDCl₃) δ 1.77-1.80 (m, 4H), 1.96-2.01 (m, 6H), 2.18-2.27 (m, 6H), 2.35-2.42 (m, 3H), 2.50-2.53 (m, 4H), 2.62 (t, 2H), 3.03 (s, 2H), 3.35-3.45 (m, 2H), 3.79 (t, 2H), 3.86-3.88 (m, 2H), 4.01 (t, 2H), 6.87 (d, 2H), 7.15 (d, 2H). |
| | | HRMS ESI⁺ m/z 418.3052 [MH]⁺. |
| | | 61 % yield |
| **187** | 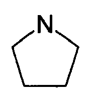 | 1-(3-{4-[4-(pyrrolidin-1-ylcarbonyl)tetrahydro-2H-pyran-4-yl]phenoxy}propyl)pyrrolidine |
| | | ¹H NMR (400MHz, CDCl₃) δ 1.40-1.52 (m, 3H), 1.52-1.80 (m, 3H), 1.82-2.10 (m, 6H), 2.10-2.30 (m, 2H), 2.30-2.38 (m, 2H), 2.60-3.05 (m, 6H), 3.40-3.60 (m, 2H), 3.75-3.90 (m, 4H), 4.05 (t, 2H), 6.84 (d, 2H), 7.18 (d, 2H). |
| | | LRMS APCI⁺m/z 387 [MH]⁺. |
| | | HRMS ESI⁺ m/z 387.2634[MH]⁺. |
| **188** | 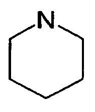 | 1-({4-[4-(3-pyrrolidin-1-ylpropoxy)phenyl]tetrahydro-2H-pyran-4-yl}carbonyl)piperidine |
| | | ¹H NMR (400MHz, CDCl₃) δ 1.18-1.38 (m, 2H), 1.40-1.50 (m, 2H), 1.50-1.80 (m, 6H), 1.84-2.05 (m, 2H), 2.10-2.30 (m, 8H), 2.30-2.48 (m, 2H), 3.10-3.40 (m, 4H), 3.75-3.90 (m, 4H), 4.10 (t, 2H), 6.88 (d, 2H), 7.20 (d, 2H). |
| | | LRMS APCI⁺ m/z 401 [MH]⁺. |
| | | HRMS ESI⁺ m/z 401.2670 [MH]⁺. |
| **189** | 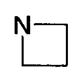 | 1-(3-{4-[4-(azetidin-1-ylcarbonyl)tetrahydro-2H-pyran-4-yl]phenoxy}propyl)pyrrolidine |
| | | ¹H NMR (400MHz, CDCl₃) δ 1.80-2.00 (m, 6H), 2.00-2.20 (m, 4H), 2.20-2.30 (m, 2H), 2.60-2.82 (m, 6H), 3.50-3.65(m, 2H), 3.72-3.90(m, 4H), 3.90-4.05(m, 2H), 4.08 (t, 2H), 6.90 (d, 2H), 7.20 (d, 2H). |
| | | LRMS APCI⁺ m/z 373 [MH]⁺. |
| | | HRMS ESI⁺ m/z 373.2477[MH]⁺. |
| **190** | 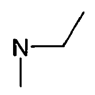 | N-ethyl-N-methyl-4-[4-(3-pyrrolidin-1-ylpropoxy)phenyl]tetrahydro-2H-pyran-4-carboxamide |
| | | ¹H NMR (400MHz, CDCl₃) δ 1.00-1.20(m, 3H), 1.78-1.86(m, 4H), 1.92-2.08(m, 4H), 2.18-2.30(m, 2H), 2.45-2.60(m, 6H), 2.60-2.70(t, 3H), 3.22-3.50(m, 2H), 3.70-3.94 (m, 4H), 4.00 (t, 2H), 6.88 (d, 2H), 7.15 (d, 2H). |
| | | LRMS APCI⁺ m/z 375 [MH]⁺. |
| | | HRMS ESI⁺ m/z 375.2634[MH]⁺. |
| **191** | 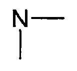 | N,N-dimethyl-4-[4-(3-pyrrolidin-1-ylpropoxy)phenyl]tetrahydro-2H-pyran-4-carboxamide |
| | | ¹H NMR (400MHz, CDCl₃) δ 1.70-1.90(m, 4H), 1.92-2.10(m, 4H), 2.20-2.30(m, 2H), 2.50-2.90(m, 12H), 3.70-3.82(m, 2H), 2.82-2.92(m, 2H), 4.00 (t, 2H), 6.88 (d, 2H), 7.15 (d, 2H). |
| | | LRMS APCI⁺m/z 361 [MH]⁺. |
| | | HRMS ESI⁺ m/z 361.2478[MH]⁺. |

### Intermediate 60: 1-(methoxyacetyl)-4-({4-[4-(3-pyrrolidin-1-ylpropoxy)phenyl]tetrahydro-2H-pyran-4-yl}carbonyl)piperazine

Methoxyacetyl chloride (55µl, 0.6mmol) was added dropwise at 0°C to a solution of 1-({4-[4-(3-pyrrolidin-1-ylpropoxy)phenyl]tetrahydro-2H-pyran-4-yl}carbonyl)piperazine of example 181 (200mg, 0.5mmol) and *N,N*-diisopropylethylamine (100µl, 0.6mmol) in dichloromethane (3ml). The reaction mixture was stirred at room temperature for 1 hour. The reaction mixture was diluted with dichloromethane (20ml) and washed with 2N sodium hydroxide (20ml). The layers were separated and the aqueous layer was further extracted with dichloromethane (20ml). The organics were combined, dried over sodium sulphate, filtered and concentrated *in vacuo.* The crude product was purified by flash chromatography on silica gel eluting with dichloromethane:methanol:ammonia (97:3:0.3 to 90:10:1 by volume) to provide the title compound (160mg, 67%).
HRMS ESI⁺ m/z 474.2952 [MH]⁺.

### Examples 192-195

The compounds of the following tabulated Examples of the general formula 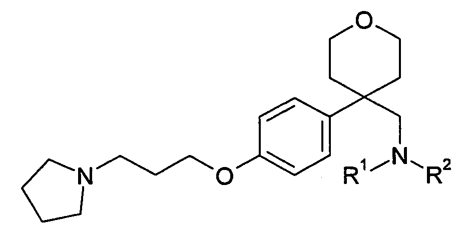 were prepared using lithium aluminium hydride, the amides of examples 182, 183, 184 and intermediate 60 similarly to the procedure used for example 142.

| Ex No | NR¹R² | Analytical Data |
|---|---|---|
| **192** (from Ex 182) | 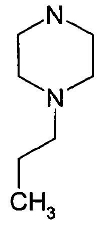 | 1-propyl-4-({4-[4-(3-pyrrolidin-1-ylpropoxy)phenyl]tetrahydro-2H-pyran-4-yl}methyl)piperazine |
| | | ¹H NMR (400MHz, CDCl₃) δ 0.85 (t, 3H), 1.40-1.46 (m, 2H), 1.77-1.80 (m, 4H), 1.84-1.91 (m, 2H), 1.98-2.03 (m, 2H), 2.08 (d, 2H), 2.18-2.22 (m, 6H), 2.24-2.28 (m, 4H), 2.36 (s, 2H), 2.51-2.54 (m, 4H), 2.63 (t, 2H), 3.51 (t, 2H), 3.72-3.75 (m, 2H), 4.01 (t, 2H), 6.84 (d, 2H), 7.20 (d, 2H). |
| | | HRMS ESI⁺ m/z 430.3410 [MH]⁺. |
| | | 96% yield |
| **193** (from Ex 183) | 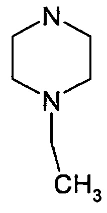 | 1-ethyl-4-({4-[4-(3-pyrrolidin-1-ylpropoxy)phenyl]tetrahydro-2H-pyran-4-yl}methyl)piperazine |
| | | ¹H NMR (400MHz, CDCl₃) δ 1.02 (t, 3H), 1.77-1.81 (m, 4H), 1.86-1.91 (m, 2H), 1.99-2.02 (m, 2H), 2.08 (d, 2H), 2.19-2.23 (m, 4H), 2.27-2.35 (m, 6H), 2.38 (s, 2H), 2.51-2.54 (m, 4H), 2.63 (t, 2H), 3.52 (t, 2H), 3.73-3.76 (m, 2H), 4.02 (t, 2H), 6.85 (d, 2H), 7.21 (d, 2H). |
| | | HRMS ESI⁺ m/z 416.3267 [MH]⁺. |
| | | 54% yield |
| **194** (from Ex 184) | 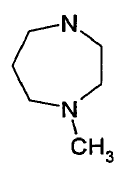 | 1-methyl-4-({4-[4-(3-pyrrolidin-1-ylpropoxy)phenyl]tetrahydro-2H-pyran-4-yl}methyl)-1,4-diazepane |
| | | ¹H NMR (400MHz, CDCl₃) δ 1.60 (p, 2H), 1.77-1.80 (m, 4H), 1.86-1.92 (m, 4H), 1.98-2.02 (m, 2H), 2.08 (d, 2H), 2.28 (s, 3H), 2.38-2.40 (m, 2H), 2.47-2.53 (m, 8H), 2.57 (s, 2H), 2.62 (t, 2H), 3.50 (t, 2H), 3.73-3.77 (m, 2H), 4.01 (t, 2H), 6.85 (d, 2H), 7.19 (d, 2H). |
| | | HRMS ESI⁺ m/z 416.3265 [MH]⁺. |
| | | 13% yield |
| **195** (from Int 60) | 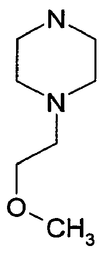 | 1-(2-methoxyethyl)-4-({4-[4-(3-pyrrolidin-1-ylpropoxy)phenyl]tetrahydro-2H-pyran-4-yl}methyl)piperazine |
| | | ¹H NMR (400MHz, CDCl₃) δ 1.76-1.79 (m, 4H), 1.84-1.89 (m, 2H), 1.97-2.01 (m, 2H), 2.06 (d, 2H), 2.19-2.21 (m, 4H), 2.28-2.33 (m, 4H), 2.35 (s, 2H), 2.47 (t, 2H), 2.50-2.53 (m, 4H), 2.61 (t, 2H), 3.29 (s, 3H), 3.43 (t, 2H), 3.50 (t, 2H), 3.71-3.74 (m, 2H), 4.00 (t, 2H), 6.83 (d, 2H), 7.19 (d, 2H). 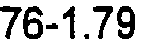 |
| | | HRMS ESI⁺ m/z 446.3374 [MH]⁺. |
| | | 31% yield |

### Intermediate 61: 2-nitro-N-({4-[4-(3-pyrrolidin-1-ylpropoxy)phenyl]tetrahydro-2H-pyran-4-yl}methyl)aniline

{4-[4-(3-Pyrrolidin-1-ylpropoxy)phenyl]tetrahydropyran-4-yl} methylamine (300mg, 0.94mmol), 2-fluoronitrobenzene (100µl, 0.95mmol) and potassium carbonate (150mg, 1.08mmol) were stirred together at room temperature in *N,N*-dimethylformamide (1ml) for 2 days. The reaction mixture was concentrated *in vacuo* and the residue partitioned between water (30ml) and ethyl acetate (100ml). The layers were separated and the organic phase washed with water (2x20ml), dried over sodium sulphate, filtered and concentrated *in vacuo* to give the title compound as a yellow gum (570mg, 100%).
HRMS ESI⁺ m/z 440.2537 [MH]⁺.

### Intermediate 62: N-({4-[4-(3-pyrrolidin-1-ylpropoxy)phenyl]tetrahydro-2H-pyran-4-yl}methyl)benzene-1,2-diamine

A solution of 2-nitro-*N-*({4-[4-(3-pyrrolidin-1-ylpropoxy)phenyl]tetrahydro-2*H*-pyran-4-yl}methyl)aniline (570mg, 1.3 mmol) in ethanol (40mL) was hydrogenated for 5 hours at room temperature at 50 psi in the presence of 10% Pd/C (60mg, 10%w/w). The reaction mixture was filtered over Arbocel® and rinsed with ethanol. The filtrate was concentrated *in vacuo* to give the title compound as a gum, used without further purification (500mg, 94%).
HRMS ESI⁺ m/z 410.2791 [MH]⁺.

### Example 196: 2-{4-[4-(3-pyrrolidin-1-ylpropoxy)phenyl]tetrahydro-2H-pyran-4-yl}-1H-benzimidazole

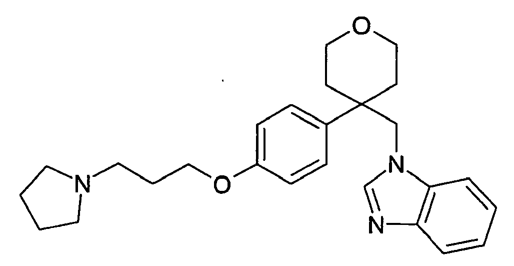

*N-*({4-[4-(3-Pyrrolidin-1-ylpropoxy)phenyl]tetrahydro-2*H*-pyran-4-yl}methyl)benzene-1,2-diamine (500mg, 1.22mmol), trimethylorthoformate (5ml) and formic acid (0.5ml) were stirred at room temperature for 18 hours. The reaction mixture was concentrated *in vacuo* and the residue partitioned between aqueous sodium carbonate (50ml) and ethyl acetate (100ml). The layers were separated and the organic phase washed with water (50ml), dried over sodium sulphate, filtered and concentrated *in vacuo.* The crude product was purified by flash chromatography on silica gel eluting with dichloromethane:methanol:ammonia (98:2:0.2 to 92:8:1 by volume). The relevant fractions were evaporated and the residue taken up in diethyl ether (20ml). The unsoluble material was filtered off and the filtrate concentrated *in vacuo* to provide the title compound (150mg, 29%).
¹H NMR (400MHz, CD₃OD) δ 1.83-1.85 (m, 4H), 1.98-2.06 (m, 4H), 2.23 (d, 2H), 2.62 (s, 4H), 2.69 (t, 2H), 3.42 (t, 2H), 3.82 (d, 2H), 4.00 (t, 2H), 4.33 (s, 2H), 6.84 (d, 2H), 7.06 (d, 2H), 7.14-7.18 (m, 3H), 7.26 (s, 1H), 7.55 (d, 1H).
HRMS ESI⁺ m/z 420.2637 [MH]⁺.

### Examples 197-200

The compounds of the following tabulated Examples of the general formula 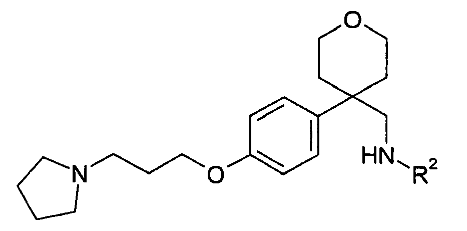 were prepared using {4-[4-(3-pyrrolidin-1-ylpropoxy)phenyl]tetrahydropyran-4-yl} methylamine and the appropriate bromo or iodopyridines similarly to the palladium coupling procedure used for example 152.

| Ex No | R² | Analytical Data |
|---|---|---|
| **197** | 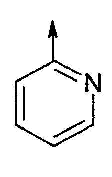 | N-({4-[4-(3-pyrrolidin-1-ylpropoxy)phenyl]tetrahydro-2H-pyran-4-yl}methyl)pyridin-2-amine |
| | | ¹H NMR (400MHz, CDCl₃) δ 1.81-1.86 (m, 4H), 1.90-1.96 (m, 2H), 2.05-2.17 (m, 4H), 2.58-2.75 (m, 6H), 3.50 (d, 2H), 3.59 (t, 2H), 3.81-3.85 (m, 2H), 4.00 (t, 1H), 4.05 (t, 2H), 6.23 (d, 1H), 6.51 (t, 1H), 6.92 (d, 2H), 7.25 (d, 2H), 7.32 (t, 1H), 8.02 (d, 1H). |
| | | HRMS ESI⁺ m/z 396.2633 [MH]⁺. |
| | | 14% yield |
| **198** | 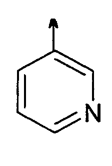 | N-({4-[4-(3-pyrrolidin-1-ylpropoxy)phenyl]tetrahydro-2H-pyran-4-yl}methyl)pyridin-3-amine |
| | | ¹H NMR (400MHz, CDCl₃) δ 1.80-1.84 (m, 4H), 1.88-1.94 (m, 2H), 2.03-2.07 (m, 2H), 2.19 (d, 2H), 2.56-2.63 (m, 4H), 2.66-2.70 (m, 2H), 3.24 (s, 3H), 3.57 (t, 2H), 3.77-3.82 (m, 2H), 4.05 (t, 2H), 6.74 (d, 1H), 6.93 (d, 2H), 7.01 (t, 1H), 7.24 (d, 2H), 7.88-7.91 (m, 2H). |
| | | HRMS ESI⁺ m/z 396.2640 [MH]⁺. |
| | | 13% yield |
| **199** | 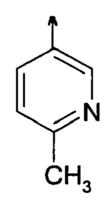 | 6-methyl-N-({4-[4-(3-pyrrolidin-1-ylpropoxy)phenyl]tetrahydro-2H-pyran-4-yl}methyl)pyridin-3-amine |
| | | ¹H NMR (400MHz, CDCl₃) δ 1.83 (d, 7H), 1.94-2.01 (m, 2H), 2.04 (t, 2H), 2.24 (d, 2H), 2.2.58-2.62 (m, 4H), 2.68 (t, 2H), 2.98 (t, 1H), 3.29 (d, 2H), 3.59 (t, 2H), 3.79-3.83 (m, 2H), 4.04 (t, 2H), 6.85 (d, 1H), 6.94 (d, 2H), 7.27 (d, 2H), 7.86 (d, 1H), 7.92 (s, 1H). |
| | | HRMS ESI⁺ m/z 410.2793 [MH]⁺. |
| | | 11 % yield |
| **200** | 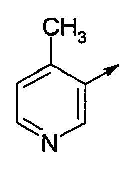 | 4-methyl-N-({4-[4-(3-pyrrolidin-1-ylpropoxy)phenyl]tetrahydro-2H-pyran-4-yl}methyl)pyridin-3-amine |
| | | ¹H NMR (400MHz, CDCl₃) δ 1.81-1.83 (m, 4H), 1.87-1.94 (m, 2H), 2.05 (t, 2H), 2.18 (d, 2H), 2.38 (s, 3H), 2.58-2.61 (m, 4H), 2.69 (t, 2H), 3.10 (t, 1H), 3.20 (d, 2H), 3.56 (t, 2H), 3.77-3.82 (m, 2H), 4.05 (t, 2H), 6.69 (dd, 1H), 6.87 (d, 1H), 6.93 (d, 2H), 7.24 (d, 2H), 7.78 (s, 1H). |
| | | HRMS ESI⁺ m/z 410.2793 [MH]⁺. |
| | | 13% yield |

### Example 201: 1-methyl-N-({4-[4-(3-pyrrolidin-1-ylpropoxy)phenyl]tetrahydro-2H-pyran-4-yl}methyl)-1H-benzimidazol-2-amine

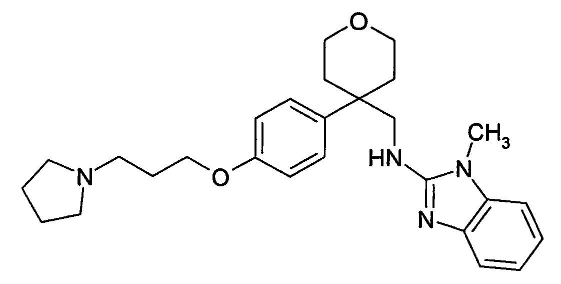

A mixture of {4-[4-(3-pyrrolidin-1-ylpropoxy)phenyl]tetrahydropyran-4-yl} methylamine (150 mg, 0.47mmol), 1-methylbenzimidazole-2-sulfonic acid (83.4mg, 0.39mmol) and *N,N*-diisopropylethylamine (175µl), 0.98mmol) in acetonitrile (1.5ml) was heated at 155°C in the Smith Personal Synthesiser for 2400 seconds and then a further 900 seconds. The reaction mixture was partitioned between dichloromethane (10 ml) and saturated aqueous sodium bicarbonate solution (10 ml). The organic layer was dried over sodium sulphate, filtered and concentrated *in vacuo.* The crude product was purified by flash chromatography on silica gel eluting with dichloromethane:methanol:ammonia (100:0:0 to 95:5:0.5 by volume) to provide the title compound (29mg, 14%).
¹H NMR (400MHz, CD₃OD) δ 1.83-1.86 (m, 4H), 1.96-2.02 (m, 4H), 2.19 (d, 2H), 2.63-2.66 (m, 4H), 2.70 (t, 2H), 3.41 (s, 3H), 3.50 (t, 2H), 3.57 (s, 2H), 3.79-3.82 (m, 2H), 3.97 (t, 2H), 6.91 (d, 2H), 6.97-7.02 (m, 2H), 7.08 (d, 1H), 7.22 (d, 1H), 7.36 (d, 2H).
LRMS APCI⁺ m/z 449 [MH]⁺.

### Intermediate 63: 6-chloro-N-({4-[4-(3-pyrrolidin-1-ylpropoxy)phenyl]tetrahydro-2H-pyran-4-yl}methyl)pyrimidin-4-amine

A mixture of {4-[4-(3-pyrrolidin-1-ylpropoxy)phenyl]tetrahydropyran-4-yl} methylamine (100 mg, 0.31mmol), 4,6-dichloropryrimidine (46.8mg, 0.31mmol) and *N,N*-diisopropylethylamine (137µl, 0.78mmol) in isopropanol (1ml) was stirred at room temperature for 18 hours. The reaction mixture was partitioned between ethyl acetate (3x10 ml) and saturated aqueous sodium bicarbonate (10 ml). The combined organic layers were dried over sodium sulphate, filtered and concentrated *in vacuo*. The crude product was purified by flash chromatography on silica gel eluting with dichloromethane:methanol:ammonia (100:0:0 to 92:8:0.8 by volume) to provide the title compound (50mg, 37%).
LRMS APCI⁺ m/z 431 [MH]⁺.

### Example 202: N-({4-[4-(3-pyrrolidin-1-ylpropoxy)phenyl]tetrahydro-2H-pyran-4-yl}methyl)pyrimidin-4-amine

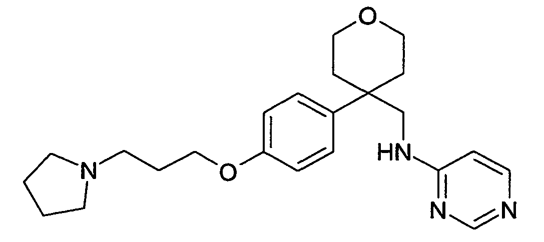

A solution of 6-chloro-*N-*({4-[4-(3-pyrrolidin-1-ylpropoxy)phenyl]tetrahydro-2*H*-pyran-4-yl}methyl)pyrimidin-4-amine (50mg, 0.12 mmol) and triethylamine (48.6µl, 0.35mmol) in ethanol (2mL) was hydrogenated for 16 hours at room temperature at 50 psi in the presence of 10% Pd/C (5mg, 10% w/w). The reaction mixture was filtered through Arbocel® and rinsed with ethanol and water. The filtrate was concentrated in *vacuo.* The residue was partitioned between ethyl acetate (20 ml) and saturated aqueous sodium carbonate (20 ml). The organic layer was dried over sodium sulphate, filtered and concentrated *in vacuo*. The crude product was purified by flash chromatography on silica gel eluting with dichloromethane:methanol:ammonia (100:0:0 to 95:5:0.5 by volume) to provide the title compound (9mg, 20%).
¹H NMR (400MHz, CDCl₃) δ 1.78-1.81 (m, 4H), 1.85-1.90 (m, 2H), 2.00-2.05 (m, 2H), 2.09-2.16 (m, 2H), 2.53-2.60 (m, 4H), 2.66 (t, 2H), 3.54-3.58 (m, 4H), 3.79-3.83 (m, 2H), 4.02 (t, 2H), 4.44 (brs, 1H), 6.12 (d, 1H), 6.92 (d, 2H), 7.20 (d, 2H), 8.05 (d, 1H), 8.47 (s, 1H).
HRMS ESI⁺ m/z 397.2596 [MH]⁺.

### Intermediate 64: 4-chloro-N-({4-[4-(3-pyrrolidin-1-ylpropoxy)phenyl]tetrahydro-2H-pyran-4-yl}methyl)phthalazin-1-amine

A mixture of {4-[4-(3-pyrrolidin-1-ylpropoxy)phenyl]tetrahydropyran-4-yl} methylamine (300 mg, 0.94mmol), 1,4-dichlorophthalazine (188mg, 0.94mmol) and *N,N*-diisopropylethylamine (410µl, 2.36mmol) in *N,N-*dimethylformamide (1ml) was stirred at room temperature for 18 hours. More 1,4-dichlorophthalazine (188mg, 0.94mmol) was added and the reaction stirred at room temperature for 36 hours, then heated at 55°C for 18 hours. The reaction mixture was partitioned between ethyl acetate (20 ml) and saturated aqueous sodium bicarbonate solution (20 ml). The organic layer was washed with more aqueous sodium bicarbonate, brine, dried over sodium sulphate, filtered and concentrated *in vacuo.* The crude product was purified by flash chromatography on silica gel eluting with dichloromethane:methanol:ammonia (100:0:0 to 92:8:0.8 by volume) to provide the title compound (183mg, 61%).
LRMS APCI⁺ m/z 481 [M]⁺.

### Example 203: N-({4-[4-(3-pyrrolidin-1-ylpropoxy)phenyl]tetrahydro-2H-pyran-4-yl}methyl)phthalazine-1-amine

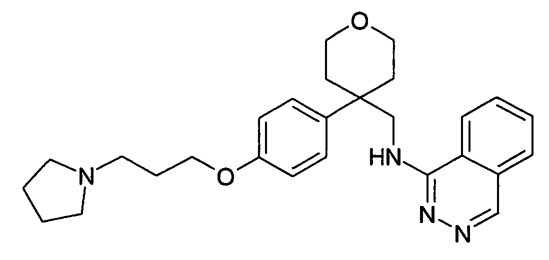

The title compound (86mg, 51%) was prepared from 4-chloro-*N-*({4-[4-(3-pyrrolidin-1-ylpropoxy)phenyl]tetrahydro-2*H*-pyran-4-yl}methyl)phthalazin-1-amine by hydrogenation similarly to the procedure used for example 202.
¹H NMR (400MHz, CDCl₃) δ 1.80-1.84 (m, 4H), 2.01-2.08 (m, 4H), 2.15-2.20 (m, 2H), 2.55-2.60 (m, 4H), 2.68 (t, 2H), 3.61-3.66 (m, 2H), 3.86-3.92 (m, 2H), 3.98 (d, 2H), 4.07 (t, 2H), 4.62 (t, 1H), 7.00 (d, 2H), 7.32-7.36 (m, 3H), 7.68-7.77 (m, 3H), 8.89 (s, 1H).
HRMS ESI⁺ m/z 447.2746 [MH]⁺.

### Example 204: 2-{4-[4-(3-pyrrolidin-1-ylpropoxy)phenyl]tetrahydro-2H-pyran-4-yl}-1H-benzimidazole

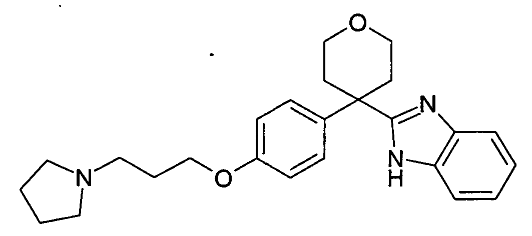

A mixture of 4-[4-(3-pyrrolidin-1-ylpropoxy)phenyl]tetrahydro-2*H*-pyran-4-carboxylic acid (400mg, 1.20mmol), 1,2-diaminobenzene (129mg, 1.20mmol) and polyphosphoric acid (2g) was heated at 130°C for 2 days. The cooled reaction mixture was partitioned between dichloromethane (20 ml) and aqueous ammonia (20 ml). The aqueous layer was further extracted with dichloromethane (2x50ml). The organic layers were combined, dried over sodium sulphate, filtered and concentrated *in vacuo.* The crude product was purified by preparative HPLC on Fraction-Lynx® eluting with water:acetonitrile:TFA (95:5:0.1) to provide the title compound (3.5mg, 1%).
¹H NMR (400MHz, CDCl₃) δ 1.92-1.96 (m, 2H), 2.06-2.21 (m, 4H), 2.40-2.46 (m, 2H), 2.96-3.00 (m, 4H), 3.05-3.09 (m, 2H), 3.16-3.20 (m, 2H), 3.57-3.65 (m, 4H), 3.93 (d, 2H), 5.45 (d, 2H), 7.06 (d, 2H), 7.45 (d, 2H), 7.71-7.74 (m, 2H).
LCMS ESI⁺ m/z 406 [MH]⁺.

### Example 205: 2-{4-[4-(3-pyrrolidin-1-ylpropoxy)phenyl]tetrahydro-2H-pyran-4-yl}-pyridine

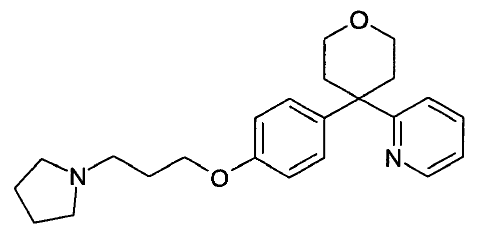

Trimethylsilylacetylene (0.9ml, 6mmol) was added at room temperature to a solution of 4-[3-(3-pyrrolidin-1-ylpropoxy)phenyl]tetrahydropyran-4-carbonitrile (200mg, 0.6mmol) and dicarbonylcyclopentadienyl cobalt (12mg, 0.07mmol) in toluene (3ml). Tetrabutylammonium fluoride (1.54g, 6mmol) was added portionwise over 10 minutes. The reaction mixture was then stirred at room temperature under visible light under nitrogen for 12 days. The reaction mixture was concentrated *in vacuo.* The residue was partitioned between dichloromethane (50ml) and aqueous sodium hydroxide solution (50ml). The aqueous layer was extracted again with dichloromethane (50ml). The organic layers were combined, dried over sodium sulphate, filtered and concentrated *in vacuo*. The crude product was purified by preparative HPLC on Fraction-Lynx® eluting with water:acetonitrile:TFA (95:5:0.1) to provide the title compound (4mg, 2%).
¹H NMR (400MHz, CDCl₃) δ 1.78-1.81 (m, 4H), 1.95-2.02 (m, 2H), 2.32-2.39 (m, 2H), 2.51-2.56 (m, 4H), 2.61-2.70 (m, 4H), 3.67 (t, 2H), 3.78-3.84 (m, 2H), 3.98 (d, 2H), 6.81 (d, 2H), 7.06 (t, 1H), 7.13 (d, 1H), 7.21 (d, 2H), 7.55 (t, 1H), 8.58 (d, 1H).
HRMS ESI⁺ m/z 367.2375 [MH]⁺.

### Example 206: 5-{4-[4-(3-pyrrolidin-1-ylpropoxy)phenyl]tetrahydro-2H-pyran-4-yl}-1,3-oxazole

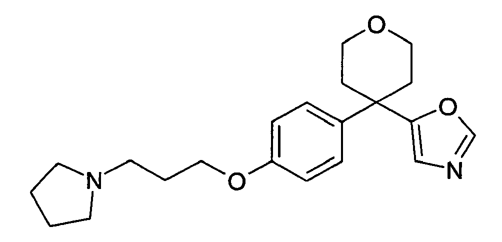

Tosylmethyl isocyanide (370mg, 1.89mmol) was added to a solution of 4-[4-(3-pyrrolidin-1-ylpropoxy)phenyl]tetrahydro-2H-pyran-4-carbaldehyde (500mg, 1.58mmol) and potassium carbonate (652mg, 4.73mmol) in methanol (7ml). The reaction mixture was heated at reflux for 3 hours. The reaction mixture was partitioned between ethyl acetate (2x75ml) and water (20ml). The organic layers were combined, dried over sodium sulphate, filtered and concentrated *in vacuo.* The crude product was taken up in 2N hydrochloric acid (3ml) and stirred at room temperature for 1.5 hours. The mixture was then partitioned between aqueous sodium carbonate and dichloromethane (2x75ml). The combined organic layers were dried over sodium sulphate, filtered and concentrated *in vacuo.* The crude product was purified by flash chromatography on silica gel eluting with dichloromethane:methanol:ammonia (95:5:0.5 by volume) to provide the title compound (12mg, 2%).
¹H NMR (400MHz, CD₃OD) δ 1.81-1.85 (m, 4H), 1.97-2.02 (m, 2H), 2.25-2.31 (m, 2H), 2.38-2.43 (m, 2H), 2.60-2.63 (m, 4H), 2.69 (t, 2H), 3.61 (t, 2H), 3.78-3.83 (m, 2H), 4.01 (t, 2H), 6.87 (d, 2H), 6.98 (s, 1H), 7.22 (d, 2H), 8.09 (s, 1H).
LRMS APCI⁺ m/z 357 [MH]⁺.

### Example 207: 4-({4-[4-(3-pyrrolidin-1-ylpropoxy)phenyl]tetrahydro-2H-pyran-4-yl}methoxy)pyridine

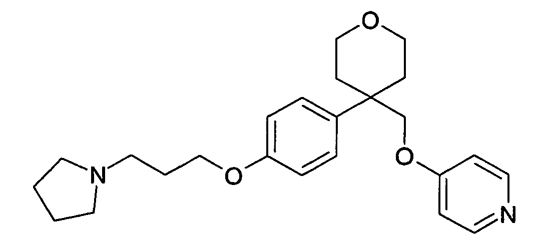

The title compound (75mg, 60%) was prepared from {4-[4-(3-pyrrolidin-1-ylpropoxy)phenyl]tetrahydro-2H-pyran-4-yl}methanol and 4-bromopyridine similarly to the procedure used for example 152.
¹H NMR (400MHz, CDCl₃) δ 1.76-1.82 (m, 4H), 1.98-2.04 (m, 2H), 2.12-2.17 (m, 4H), 2.49-2.58 (m, 4H), 2.61-2.66 (m, 2H), 3.54-3.60 (m, 2H), 3.81-3.85 (m, 2H), 3.87 (s, 2H), 4.03 (t, 2H), 6.71 (d, 2H), 6.91 (d, 2H), 7.29 (d, 2H), 8.36 (d, 2H).
HRMS ESI⁺ m/z 397.2479 [MH]⁺.

### Intermediate 65: 1-{4-[4-(3-chloropropoxy)phenyl]tetrahydro-2H-pyran-4-yl}-N,N-dimethylmethanamine

1-Bromo-3-chloropropane (0.42ml, 4.25mmol) was added to a solution of 4-(4-dimethylaminomethyltetrahydro-pyran-4-yl)-phenol (1.0g, 4.25mmol) and potassium carbonate (1.5g, 10.8mmol) in *N,N-*dimethylformamide (10ml). The reaction mixture was stirred at 45°C for 18 hours. The reaction mixture was concentrated *in vacuo*. The residue was partitioned between ethyl acetate (100ml) and water (50ml). The organic layer was separated, dried over sodium sulphate, filtered and concentrated *in vacuo* to provide the title compound (800mg, 60%).
LRMS APCI⁺ m/z 412 [MH]⁺.

### Example 208: N,N-dimethyl-1-(4-{4-[3-(4-methylpiperazin-1-yl)propoxy]phenyl}tetrahydro-2H-pyran-4-yl)methanamine

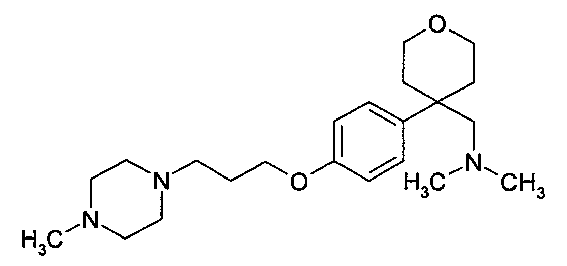

A mixture of 1-{4-[4-(3-chloropropoxy)phenyl]tetrahydro-2*H*-pyran-4-yl}-*N,N*-dimethylmethanamine (200mg, 0.7mmol), *N-*methyl piperazine (87µl, 0.79mmol), solid sodium bicarbonate (66mg, 0.79mmol) and a catalytic amount of potassium iodide was heated at 50°C in *N,N*-dimethylformamide (2ml) for 18 hours. The reaction mixture was concentrated *in vacuo.* The residue was partitioned between ethyl acetate (2x75ml) and water (20ml). The organic layers were combined, dried over sodium sulphate, filtered and concentrated *in vacuo.* The crude product was purified by flash chromatography on silica gel eluting with dichloromethane:methanol:ammonia (96:4:0.4 by volume) to provide the title compound (102mg, 39%).
¹H NMR (400MHz, CDCl₃) δ 1.84-1.91 (m, 2H), 1.95-1.99 (m, 8H), 2.07-2.11 (m, 2H), 2.30 (s, 3H), 2.40 (s, 2H), 2.42-2.59 (m, 10H), 3.54 (t, 2H), 3.72-3.78 (m, 2H), 4.00 (t, 2H), 6.86 (d, 2H), 7.20 (d, 2H). HRMS ESI⁺ m/z 376.2951 [MH]⁺.

### Intermediate 66: N,N-dimethylazetidin-3-amine ditrifluoroacetate

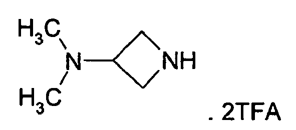

This amine was described in patent WO 2004096810 (prep 170, p240).

### Examples 209-211

The compounds of the following tabulated examples of the general formula 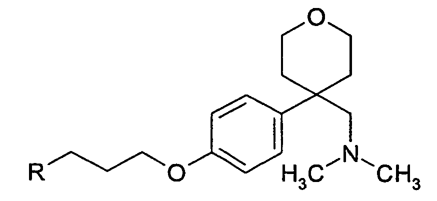 were prepared using 1-{4-[4-(3-chloropropoxy)phenyl]tetrahydro-2*H*-pyran-4-yl}-*N,N-*dimethylmethanamine and the appropriate amines similarly to the procedure used for example 208.

| Ex No | R | Analytical Data |
|---|---|---|
| **209** | 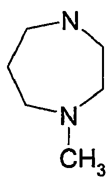 | N, N-dimethyl-1-(4-{4-[3-(4-methyl-1,4-diazepan-1-yl)propoxy]phenyl}tetrahydro-2H-pyran-4-yl)methanamine |
| | | ¹H NMR (400MHz, CDCl₃) δ 1.77-1.91 (m, 6H), 1.94 (s, 6H), 2.04-2.08 (m, 2H), 2.33 (s, 3H), 2.38 (s, 2H), 2.57-2.65 (m, 6H), 2.70-2.73 (m, 4H), 3.52 (t, 2H), 3.70-3.75 (m, 2H), 3.98 (t, 2H), 6.84 (d, 2H), 7.18 (d, 2H). |
| | | LRMS APCI⁺ m/z 390 [MH]⁺. |
| | | 30% yield |
| **210** | 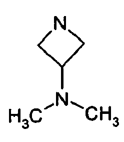 | 1-[3-(4-{4-[(dimethylamino)methyl]tetrahydro-2H-pyran-4-yl}phenoxy)propyl]-N,N-dimethylazetidin-3-amine |
| | | ¹H NMR (400MHz, CDCl₃) δ 1.62-1.90 (m, 6H), 1.96 (s, 6H), 2.11 (s, 6H), 2.40 (s, 2H), 2.63 (t, 3H), 2.80-2.86 (m, 3H), 3.52-3.56 (m, 4H), 3.72-3.76 (m, 2H), 3.96-3.99 (m, 2H), 6.85 (d, 2H), 7.20 (d, 2H). |
| | | HRMS ESI⁺ m/z 376.2959 [MH]⁺. |
| | | 53% yield |
| **211** | 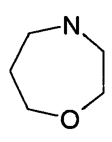 | Dimethyl-{4-[4-(3-[1,4]oxazepan-4-yl-propoxy)-phenyl]-tetrahydro-pyran-4-ylmethyl}-amine |
| | | ¹H NMR (400MHz, CDCl₃) δ 1.85-2.04 (m, 12H), 2.11 (M, 2H), 2.42 (s, 2H), 2.7-2.8 (m, 6H), 3.55 (t, 2H), 3.72-3.82 (m, 6H), 4.02 (t, 2H), 6.87 (d, 2H), 7.22 (d, 2H) |
| | | HRMS ESI⁺ m/z 377.2792 [MH]⁺ |
| | | 35% yield |

### Intermediate 67: 1-benzhydryl-3-chloromethyl-azetidine hydrochloride

(1-Benzhydryl-azetidin-3-yl)-methanol (3.07g, 12.1 mmol) was dissolved in dichloromethane (60ml). The reaction was cooled to 0-5°C and thionyl chloride (1.07ml, 14.6mmol) was added slowly. The reaction was allowed to warm to room temperature and stirred for 18 hours. The reaction was concentrated *in vacuo* and azeotroped with toluene (3x20ml) to give the title compound (3.8g, 100%) as a pale brown solid.

### Example 212: 1-(4-{4-[(1-cyclopentylazetidin-3-yl)methoxy]phenyl}tetrahydro-2H-pyran-4-yl)-N,N-dimethylmethanamine

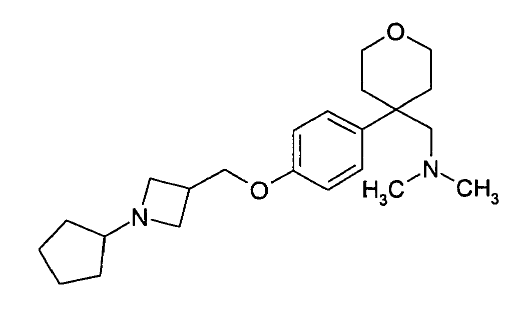

### Step 1:

4-(4-Dimethylaminomethyl-tetrahydro-pyran-4-yl)-phenol (1.15g, 5.65mmol), 1-benzhydryl-3-chloromethylazetidine hydrochloride (1.58g, 5.13mmol), *N,N*-dimethylformamide (32ml) and potassium carbonate (2.84g, 20.52mmol) were reacted together according to general procedure B. The reaction mixture was diluted with ethyl acetate. The organic layer was washed with 2M sodium hydroxide (3x50ml), brine (3x50ml), dried over magnesium sulphate, filtered and concentrated *in vacuo* to provide 1-[4-(4-{[1-benzhydrylazetidin-3-yl]methoxy}phenyl)tetrahydro-2*H*-pyran-4-yl]-*N,N*-dimethylmethanamine (1.82g, 81%) as an off-white solid.

### Step 2:

This intermediate was hydrogenated in ethanol (20ml) for 4 hours at 60°C at atmospheric pressure in the presence of 10% Pd/C (300mg). The reaction mixture was filtered through Arbocel® and rinsed with ethanol and water. The filtrate was concentrated *in vacuo*. The residue was taken up in 2N hydrochloric acid (50ml) and extracted with *tert*-butylmethyl ether (3x50ml). The aqueous layer was then basified to pH14 with 2M sodium hydroxide and extracted with dichloromethane (3x50ml). These organic layers were combined, dried over magnesium sulphate, filtered and concentrated *in vacuo* to provide 1-{4-[4-(azetidin-3-ylmethoxy)phenyl]tetrahydro-2*H*-pyran-4-yl]-*N,N*-dimethylmethanamine (850mg, 85%) as an off-white solid.

### Step 3:

Cyclopentyl bromide (0.17ml, 1.54mmol) and 5M aqueous sodium hydroxide (0.34ml, 1.68mmol) were added to a solution of 1-{4-[4-(azetidin-3-ylmethoxy)phenyl]tetrahydro-2*H*-pyran-4-yl]-*N,N-*dimethylmethanamine (425mg, 1.4mmol) in acetone (9ml). The reaction mixture was stirred at room temperature for 18 hours. The reaction mixture was concentrated *in vacuo.* The residue was taken up in 2N hydrochloric acid to reach pH 1 and extracted with *tert*-butylmethyl ether (2x15ml). The aqueous layer was then basified to pH14 with 4M sodium hydroxide (ca 40ml) and extracted with dichloromethane (3x30ml). These organic layers were combined, dried over magnesium sulphate, filtered and concentrated *in vacuo.* The crude product was purified by flash chromatography on silica gel eluting with dichloromethane:methanol:ammonia (95:4:1 by volume) to provide the title compound (55mg, 11%) as a yellow solid.
¹H NMR (400MHz, CDCl₃) δ 1.28-1.73 (m, 8H), 1.83-1.93 (m, 2H), 1.96 (s, 6H), 2.05-2.13 (m, 2H), 2.40 (s, 2H), 2.71 (m, 1H), 2.90 (m, 1H), 3.00 (t, 2H), 3.39-3.58 (m, 4H), 3.72-3.78 (m, 2H), 4.05 (d, 2H), 6.87 (d, 2H), 7.21 (d, 2H).
LRMS ESI⁺ m/z 373 [MH]⁺.

### Example 213: N,N-dimethyl-1-{4-[4-(3-morpholin-4-ylpropoxy)phenyl]tetrahydro-2H-pyran-4-yl}methanamine

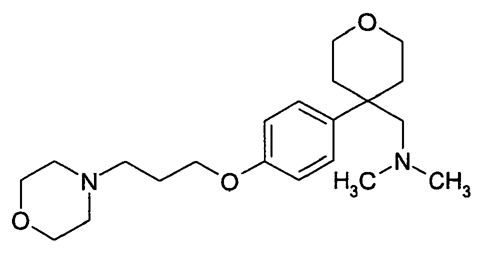

A mixture of 1-{4-[4-(3-chloropropoxy)phenyl]tetrahydro-2*H*-pyran-4-yl}-*N,N*-dimethylmethanamine (100mg, 0.32mmol), morpholine (100mg, 1.1mmol) and potassium carbonate (100mg, 0.72mmol) was heated at reflux in acetonitrile (5ml) for 8 hours. Further morpholine (100mg, 1.1mmol) and potassium carbonate (100mg, 0.72mmol) were added and the reaction mixture heated at reflux for 6 hours. The reaction mixture was concentrated *in vacuo.* The residue was purified by flash chromatography on silica gel eluting with dichloromethane:methanol:ammonia (90:10:1 by volume) to provide the title compound (61mg, 52%).
¹H NMR (400MHz, CD₃OD) δ 0.81-0.87 (m, 2H), 1.04-2.01 (m, 8H), 2.13-2.18 (m, 2H), 2.48-2.50 (m, 6H), 2.56 (t, 2H), 3.50 (t, 2H), 3.68-3.77 (m, 6H), 4.03 (t, 2H), 6.90 (d, 2H), 7.27 (d, 2H).
HRMS ESI⁺ m/z 363.2635 [MH]⁺.

### Example 214: 4-(4-{3-[(2S)-2-(hydroxymethyl)pyrrolidin-1-yl]propoxy}phenyl)tetrahydro-2H-pyran-4-carbonitrile

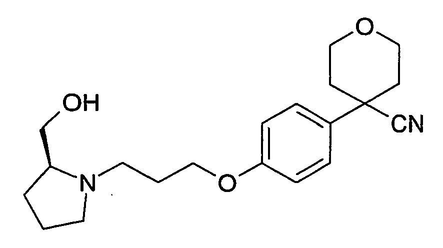

The title compound (30mg, 12%) was prepared using 4-[4-(3-chloro-propoxy)-phenyl]-tetrahydropyran-4-carbonitrile and (S)-(+)-2-pyrrolidine methanol similarly to the procedure used for example 208.
¹H NMR (400MHz, CDCl₃) δ 1.79-1.85 (m, 3H), 1.94 (m, 1H), 2.01-2.12 (m, 7H), 2.41 (m, 1H), 2.58 (m, 1H), 2.78 (m, 1H), 3.08 (m, 1H), 3.33 (m, 1H), 3.49 (m, 1H), 3.71 (d, 1H), 3.88 (t, 2H), 4.04-4.09 (m, 4H), 6.92 (d, 2H), 7.38 (d, 2H).
HRMS ESI⁺ m/z 345.2167 [MH]⁺.

### Intermediate 68: 1-{4-[4-(3-thiomorpholin-4-ylpropoxy)phenyl]tetrahydro-2H-pyran-4-yl}methanamine

The title compound (1.58g, 92%) was prepared using 4-[4-(3-thiomorpholin-4-ylpropoxy)phenyl]tetrahydro-2H-pyran-4-carbonitrile and lithium aluminium hydride similarly to the procedure used for intermediate 51. LRMS APCI⁺ m/z 351 [MH]⁺.

### Example 215: N-({4-[4-(3-thiomorpholin-4-ylpropoxy)phenyl]tetrahydro-2H-pyran-4-yl}methyl)acetamide

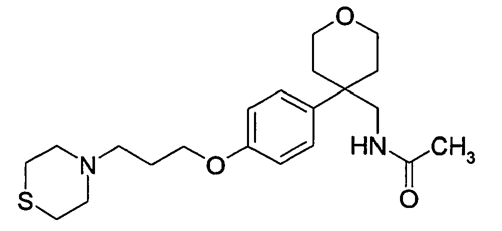

The title compound (1.16g, 100%) was prepared using 1-{4-[4-(3-thiomorpholin-4-ylpropoxy)phenyl]tetrahydro-2H-pyran-4-yl}methanamine and acetic anhydride similarly to the procedure used for example 140.
¹H NMR (400MHz, CDCl₃) δ 1.82-1.86 (m, 2H), 1.87 (s, 3H), 2.00-2.04 (m, 4H), 2.58-2.85 (m, 10H), 3.45 (d, 2H), 3.56-3.61 (m, 2H), 3.79-3.84 (m, 2H), 4.02 (t, 2H), 4.97 (brs, 1H), 6.91 (d, 2H), 7.19 (d, 2H). LRMS APCI⁺ m/z 393 [MH]⁺.

### Example 216: N-({4-[4-(3-thiomorpholin-4-ylpropoxy)phenyl]tetrahydro-2H-pyran-4-yl}methyl)ethanamine

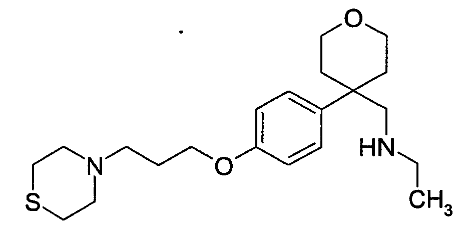

The title compound (492mg, 44%) was prepared using *N*-({4-[4-(3-thiomorpholin-4-ylpropoxy)phenyl]tetrahydro-2H-pyran-4-yl}methyl)acetamide and lithium aluminium hydride similarly to the procedure used for example 142.
¹H NMR (400MHz, CDCl₃) δ 0.96 (t, 3H), 1.88-1.98 (m, 4H), 2.11-2.15 (m, 2H), 2.49 (q, 2H), 2.55 (t, 2H), 2.68-2.74 (m, 10H), 3.55 (t, 2H), 3.74-3.79 (m, 2H), 4.00 (t, 2H), 6.89 (d, 2H), 7.22 (d, 2H).
LRMS APCI⁺ m/z 379 [MH]⁺.

### Intermediate 69: 4-(4-methoxyphenyl)tetrahydro-2H-pyran-4-carboxylic acid

4-(4-Methoxyphenyl)-tetrahydro-2H-pyran-4-carbonitrile (10g, 46mmol) was heated at reflux for 18 hours in concentrated hydrochloric acid (100mL). The reaction mixture was extracted with dichloromethane (2x100mL). The combined organic layers were dried over sodium sulphate, filtered and concentrated *in vacuo* to a gummy solid. This was partitioned between 2M sodium hydroxide (300ml) and ethyl acetate (300ml). The aqueous layer was then acidified with concentrated hydrochloric acid (a white precipitate formed which was dissolved by adding dichloromethane (250ml)). The layers were separated and the aqueous layer was further extracted with dichloromethane (250ml). These dichloromethane organic layers were combined, dried over sodium sulphate, filtered and concentrated *in vacuo* to provide the title compound (3.4g, 31%) as a white solid.
LRMS APCI⁻ m/z 235 [M-H]⁻.

### Intermediate 70: 1-{[4-(4-methoxyphenyl)tetrahydro-2H-pyran-4-yl]carbonyl}-4-methylpiperazine

The title compound (515mg, 77%) was prepared using 4-(4-methoxyphenyl)tetrahydro-2H-pyran-4-carboxylic acid, N-methyl-piperazine and O-(1*H*-benzotriazol-1-yl)-*N,N,N',N'*-tetramethyluronium tetrafluoroborate (TBTU) similarly to the procedure used for example 166.
HRMS ESI⁺ m/z 319.2009 [MH]⁺.

### Intermediate 71: 1-{[4-(4-methoxyphenyl)tetrahydro-2H-pyran-4-yl]methyl}-4-methylpiperazine

The title compound (428mg, 93%) was prepared using 1-{[4-(4-methoxyphenyl)tetrahydro-2H-pyran-4-yl]carbonyl}-4-methylpiperazine and lithium aluminium hydride similarly to the procedure used for example 142.
HRMS ESI⁺ m/z 305.2220 [MH]⁺.

### Intermediate 72: 4-{4-[(4-methylpiperazin-1-yl)methyl]tetrahydro-2H-pyran-4-yl}phenol

To a suspension of sodium thiomethoxide (660mg, 9.4mmol) in N,N-dimethylformamide (2ml) was added 1-{[4-(4-methoxyphenyl)tetrahydro-2H-pyran-4-yl]methyl}-4-methylpiperazine (410mg, 1.3mmol) in N,N-dimethylformamide (3ml). The reaction mixture was heated to 130°C under nitrogen for 18 hours. The mixture was then allowed to cool down to room temperature and saturated aqueous ammonium chloride was added. The mixture was extracted with ethyl acetate (2x30ml). The combined organic extracts were dried over sodium sulphate, filtered and concentrated *in vacuo* to provide a pale brown oil. This was azeotroped with toluene and dried under high vacuum to provide the title compound (390mg, 100%) as an off-white solid.
HRMS ESI⁺ m/z 291.2062 [MH]⁺.

### Example 217: 1-[(4-{4-[(1-isopropylpiperidin-4-yl)oxy]phenyl}tetrahydro-2H-pyran-4-yl)methyl]-4-methylpiperazine

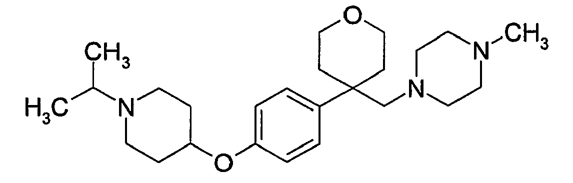

The title compound (72mg, 14%) was prepared using 4-{4-[(4-methylpiperazin-1-yl)methyl]tetrahydro-2*H-*pyran-4-yl}phenol, 1-isopropyl-4-hydroxypiperidine (step 1 of intermediate 21), PPh₃ and DIAD similarly to the procedure used for intermediate 55.
¹H NMR (400MHz, CDCl₃) δ 1.04 (d, 6H), 1.76-1.90 (m, 4H), 1.96-2.08 (m, 4H), 2.17-2.26 (m, 11H), 2.33-2.38 (m, 4H), 2.69-2.80 (m, 3H), 3.51 (t, 2H), 3.69-3.74 (m, 2H), 4.24 (m, 1H), 6.84 (d, 2H), 7.18 (d, 2H). HRMS ESI⁺ m/z 416.3268 [MH]⁺.

### Intermediate 73: 4-{[4-(4-methoxyphenyl)tetrahydro-2H-pyran-4-yl]carbonyl}morpholine

The title compound (3.1g, 87%) was prepared using 4-(4-methoxyphenyl)tetrahydro-2H-pyran-4-carboxylic acid, morpholine and O-(1*H*-benzotriazol-1-yl)-*N,N,N',N'*-tetramethyluronium tetrafluoroborate (TBTU) similarly to the procedure used for example 166.
HRMS ESI⁺ m/z 306.1696 [MH]⁺.

### Intermediate 74: 4-{[4-(4-methoxyphenyl)tetrahydro-2H-pyran-4-yl]methyl}-morpholine

The title compound (2.48g, 87%) was prepared using 4-{[4-(4-methoxyphenyl)tetrahydro-2H-pyran-4-yl]carbonyl}morpholine and lithium aluminium hydride similarly to the procedure used for example 142. HRMS ESI⁺ m/z 292.1897 [MH]⁺.

### Intermediate 75: 4-[4-(morpholin-4-ylmethyl)tetrahydro-2H-pyran-4-yl]phenol

The title compound (1.70g, 75%) was prepared using 4-{[4-(4-methoxyphenyl)tetrahydro-2*H*-pyran-4-yl]methyl}-morpholine and sodium thiomethoxide similarly to the procedure used for intermediate 72. HRMS ESI⁺ m/z 278.1740 [MH]⁺.

### Example 218: 4-[(4-{4-[(1-isopropylpiperidin-4-yl)oxy]phenyl}tetrahydro-2H-pyran-4-yl)methyl]-morpholine

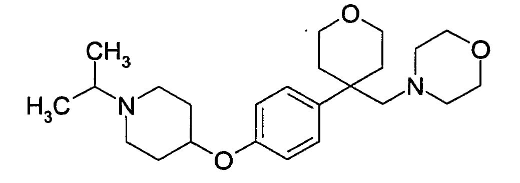

The title compound (52mg, 12%) was prepared using 4-[4-(morpholin-4-ylmethyl)tetrahydro-2H-pyran-4-yl]phenol, 1-isopropyl-4-hydroxypiperidine (step1 of intermediate 21), PPh₃ and DIAD similarly to the procedure used for intermediate 55.
¹H NMR (400MHz, CD₃OD) δ 1.09 (d, 6H), 1.74-1.80 (m, 2H), 1.83-1.90 (m, 2H), 1.98-2.04 (m, 2H), 2.12-2.18 (m, 6H), 2.40 (s, 2H), 2.47 (t, 2H), 2.75 (m, 1H), 2.80-2.85 (m, 2H), 3.47-3.54 (m, 6H), 3.71-3.77 (m, 2H), 4.36 (m, 1H), 6.90 (d, 2H), 7.28 (d, 2H).
HRMS ESI⁺ m/z 403.2951 [MH]⁺.

### Intermediate 76: 4-{[4-(4-{[1-(benzhydryl)azetidin-3-yl]methoxy}phenyl) tetrahydro-2H-pyran-4-yl]methyl}-morpholine

The title compound (1.09g, 66%) was prepared using 4-[4-(morpholin-4-ylmethyl)tetrahydro-2H-pyran-4-yl]phenol, (1-benzhydryl-azetidin-3-yl)-methanol, PPh₃ and DIAD similarly to the procedure used for intermediate 55.
HRMS ESI⁺ m/z 513.3103 [MH]⁺.

### Intermediate 77: 4-({4-[4-(azetidin-3-ylmethoxy)phenyl]tetrahydro-2H-pyran-4-yl}methyl)-morpholine

A solution of 4-{[4-(4-{[1-(benzhydryl)azetidin-3-yl]methoxy}phenyl)tetrahydro-2H-pyran-4-yl]methyl}-morpholine (1.0g, 1.95 mmol) in ethanol (10mL) was hydrogenated for 16 hours at room temperature at 60 psi in the presence of Pd(OH)₂/C (150mg, 15% w/w). 2N Hydrochloric acid (few drops) was added and the reaction mixture hydrogenated for 16 hours at 60°C at 60 psi. The reaction mixture was basified with aqueous sodium carbonate and the mixture filtered over Arbocel® and rinsed with ethanol. The filtrate was concentrated *in vacuo*. The residue was purified by flash chromatography on silica gel eluting with dichloromethane:methanol:ammonia (96:4:0.4 to 90:10:1 by volume) to provide the title compound (210mg, 31%).
HRMS ESI⁺ m/z 347.2323 [MH]⁺.

### Examples 219-221:

The compounds of the following tabulated examples of the general formula 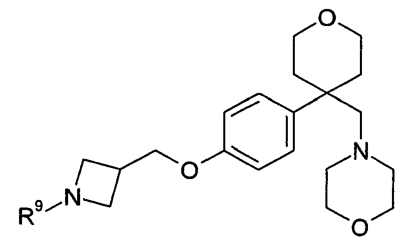 were prepared using 4-({4-[4-(azetidin-3-ylmethoxy)phenyl]tetrahydro-2H-pyran-4-yl}methyl)-morpholine, the appropriate ketones and sodium triacetoxyborohydride similarly to the procedure used for example 54.

| Ex No | R⁹ | Analytical Data |
|---|---|---|
| **219** | cyclopentyl | 4-[(4-{4-[(1-cyclopentylazetidin-3-yl)methoxy]phenyl}tetrahydro-2H-pyran-4-yl)methyl]morpholine |
| | | ¹H NMR (400MHz, CDCl₃) δ 1.32-1.37 (m, 2H), 1.49-1.53 (m, 2H), 1.58-1.69 (m, 4H), 1.83-1.89 (m, 2H), 2.08-2.15 (m, 6H), 2.38 (s, 2H), 2.74 (m, 1H), 2.91 (m, 1H), 3.03 (t, 2H), 3.45 (t, 2H), 3.49-3.54 (m, 6H), 3.72-3.76 (m, 2H), 4.04 (d, 2H), 6.84 (d, 2H), 7.22 (d, 2H). |
| | | HRMS ESI⁺ m/z 415.2946 [MH]⁺. |
| | | 92% yield |
| **220** | isopropyl | 4-[(4-{4-[(1-isopropylazetidin-3-yl)methoxy]phenyl}tetrahydro-2H-pyran-4-yl)methyl]morpholine |
| | | ¹H NMR (400MHz, CDCl₃) δ 0.92 (d, 6H), 1.83-1.90 (m, 2H), 2.09-2.14 (m, 6H), 2.31 (m, 1H), 2.38 (s, 2H), 2.85 (m, 1H), 3.03 (t, 2H), 3.41 (t, 2H), 3.49-3.54 (m, 6H), 3.72-3.76 (m, 2H), 4.06 (d, 2H), 6.85 (d, 2H), 7.22 (d, 2H). |
| | | HRMS ESI⁺ m/z 389.2791 [MH]⁺. |
| | | 61% yield |
| **221** | cyclobutyl | 4-[(4-{4-[(1-cyclobutylazetidin-3-yl)methoxy]phenyl}tetrahydro-2H-pyran-4-yl)methyl]morpholine |
| | | ¹H NMR (400MHz, CDCl₃) δ 1.62-1.75 (m, 2H), 1.82-1.90 (m, 4H), 1.93-1.99 (m, 2H), 2.09-2.15 (m, 6H), 2.38 (s, 2H), 2.88 (m, 1H), 3.07 (t, 2H), 3.14 (m, 1H), 3.40 (t, 2H), 3.49-3.55 (m, 6H), 3.72-3.77 (m, 2H), 4.07 (d, 2H), 6.85 (d, 2H), 7.22 (d, 2H). |
| | | HRMS ESI⁺ m/z 401.2791 [MH]⁺. |
| | | HRMS ESI⁺ m/z 401.2791 [MH]⁺. |

### Intermediate 78: 4-[4-(methylthio)phenyl]tetrahydro-2H-pyran-4-carbonitrile

The title compound (5.5g, 77%, solid after trituration in pentane) was prepared using 4-(methylthio)phenylacetonitrile, bis(2-bromoethyl)ether, sodium hydride and potassium iodide similarly to the procedure used for intermediate 56.
Microanalysis: Found: C, 66.62; H, 6.46; N, 5.97%. C₁₃H₁₅NOS requires C, 66.92; H, 6.48; N, 6.00%.

### Intermediate 79: 4-[4-(methylsulfinyl)phenyl]tetrahydro-2H-pyran-4-carbonitrile

A solution of meta-chloroperbenzoic acid (5.08g, 22.66mmol) in dichloromethane (30ml) was added dropwise over 15 minutes at 0°C to a solution of 4-[4-(methylthio)phenyl]tetrahydro-2*H*-pyran-4-carbonitrile (4.8g, 20.6mmol) in dichloromethane (40ml). The reaction mixture was warmed to room temperature over 4 hours. The mixture was then diluted with dichloromethane (180ml), washed with sodium sulphite solution and 10% aqueous sodium carbonate. The organic layer was separated, dried over sodium sulphate and concentrated *in vacuo.* The crude product was purified by flash chromatography on silica gel eluting with dichloromethane:methanol:ammonia (100:0:0 to 95:5:0.5 by volume) to provide the title compound (4.55g, 89%) as an off-white solid.
LRMS APCI⁺ m/z 250 [MH]⁺.

### Example 222: 4-{4-[(3-pyrrolidin-1-ylpropyl)thio]phenyl}tetrahydro-2H-pyran-4-carbonitrile

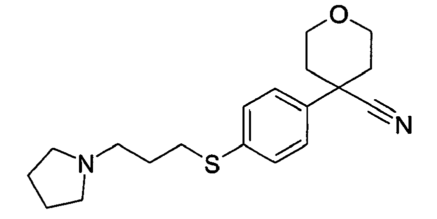

2,6-Lutidine (3.33ml, 28.6mmol) was added to a solution of 4-[4-(methylsulfinyl)phenyl]tetrahydro-2H-pyran-4-carbonitrile (2.3g, 9.23mmol) in acetonitrile (70ml). The mixture was cooled in an ice-acetone bath and trifluoroacetic anhydride (3.87ml, 27.69mmol) added slowly. The solution was stirred at this temperature under nitrogen for 3 hours. The reaction mixture was then concentrated *in vacuo* and the residue taken up in methanol (7ml). Triethylamine (7ml) was added at 0°C and the mixture stirred at 0°C for 30 minutes. N,N-Dimethylformamide (10ml) was added at 0°C, followed by potassium carbonate (2.8g, 20.31mmol) and 1-(3-chloro-propyl)-pyrrolidine (2.72g, 18.46mmol) in N,N-dimethylformamide (10ml). The mixture was then warmed to room temperature and stirred for 18 hours. The reaction mixture was concentrated *in vacuo.* The residue was taken up in water (50ml) and extracted with ethyl acetate (2x150ml). The organic layers were combined, dried over sodium sulphate, filtered and concentrated *in vacuo.* The crude product was purified by flash chromatography on silica gel eluting with dichloromethane:methanol:ammonia (97:3:0.3 to 95:5:0.5 by volume) to provide the title compound (2.62g, 86%) as a solid.
¹H NMR (400MHz, CDCl₃) δ 1.77-1.80 (m, 4H), 1.84-1.91 (m, 2H), 2.01-2.14 (m, 4H), 2.48-2.52 (m, 4H), 2.58 (t, 2H), 2.99 (t, 2H), 3.89 (t, 2H), 4.06-4.10 (m, 2H), 7.34-7.39 (m, 4H).
LRMS APCI⁺ m/z 331 [MH]⁺.

### Intermediate 80: 1-(4-{4-[(3-pyrrolidin-1-ylpropyl)thio]phenyl}tetrahydro-2H-pyran-4-yl)methanamine

The title compound (2.42g, 91%) was prepared using 4-{4-[(3-pyrrolidin-1-ylpropyl)thio]phenyl}tetrahydro-2H-pyran-4-carbonitrile and lithium aluminium hydride similarly to the procedure used for intermediate 51. LRMS APCI⁺ m/z 335 [MH]⁺.
Microanalysis: Found: C, 64.08; H, 8.79; N, 7.447%. C₁₉H₃₀N₂OS.0.33DCM requires C, 64.04; H, 8.52; N, 7.73%.

### Example 223: N,N-dimethyl-1-(4-{4-[(3-pyrrolidin-1-ylpropyl)thio]phenyl}tetrahydro-2H-pyran-4-yl)methanamine

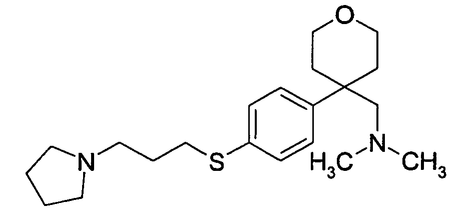

Formaldehyde (37%w/w solution in water, 650µl, 7.58mmol) was added to a solution of 1-(4-{4-[(3-pyrrolidin-1-ylpropyl)thio]phenyl}tetrahydro-2H-pyran-4-yl)methanamine (1.0g, 3.03mmol) in tetrahydrofuran (10ml). Acetic acid (173µl, 3.03mmol) was then added and the mixture stirred at room temperature for 10 minutes. The mixture was cooled to 0°C and sodium triacetoxyborohydride (1.6g, 7.57mmol) was added in 2 portions. The mixture was then warmed to room temperature and stirred for 18 hours. The reaction was quenched with water and concentrated *in vacuo*. The residue was taken up in sodium bicarbonate until the aqueous layer pH reached 8. The aqueous layer was then extracted with dichloromethane (2x100ml). The organic extracts were combined, dried over sodium sulphate, filtered and concentrated *in vacuo*. The crude product was purified by flash chromatography on silica gel eluting with dichloromethane:methanol:ammonia (97:3:0.3 to 90:10:1 by volume) to provide the title compound (139mg, 13%) as an oil which crystallised on standing.
¹H NMR (400MHz, CD₃OD) δ 1.77-1.91 (m, 8H), 1.95 (s, 6H), 2.13-2.18 (m, 2H), 2.49 (s, 2H), 2.51-2.54 (m, 4H), 2.60 (t, 2H), 2.96 (t, 2H), 3.50 (t, 2H), 3.73-3.78 (m, 2H), 7.31-7.36 (m, 4H).
HRMS ESI⁺ m/z 363.2462 [MH]⁺.

### Example 224: N,N-dimethyl-1-(4-{4-[(3-pyrrolidin-1-ylpropyl)sulphonyl]phenyl}ltetrahydro-2H-pyran-4-yl)methanamine

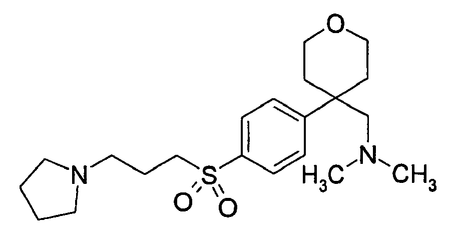

*N,N*-Dimethyl-1-(4-{4-[(3-pyrrolidin-1-ylpropyl)thio]phenyl}tetrahydro-2*H*-pyran-4-yl)methanamine (70mg, 0.19mmol) was dissolved in acetone (2ml) and water (0.5ml). The mixture was cooled to 0°C and a solution of Oxone® (131mg, 0.21mmol) in water (0.5ml) added dropwise. The reaction mixture was stirred at room temperature for 18 hours. The mixture was then diluted with more water, basified with 10% aqueous sodium carbonate and extracted with dichloromethane (2x20ml). The organic layers were combined, dried over sodium sulphate, filtered and concentrated *in vacuo.* The crude product was purified by flash chromatography on silica gel eluting with dichloromethane:methanol:ammonia (96:4:0.4 by volume) to provide the title compound (23mg, 30%) as an oil.
¹H NMR (400MHz, CDCl₃) δ 1.72-1.75 (m, 4H), 1.90-1.99 (m, 10H), 2.10-2.14 (m, 2H), 2.40-2.44 (m, 4H), 2.48-2.52 (m, 4H), 3.20 (t, 2H), 3.52 (t, 2H), 3.75-3.80 (m, 2H), 7.52 (d, 2H), 7.86 (d, 2H).
HRMS ESI⁺ m/z 395.2363 [MH]⁺.

### Intermediate 81: 4-{4-[(3-pyrrolidin-1-ylpropyl)thio]phenyl}tetrahydro-2H-pyran-4-carboxylic acid

4-{4-[(3-Pyrrolidin-1-ylpropyl)thio]phenyl}tetrahydro-2H-pyran-4-carbonitrile (1g, 3.03mmol) was heated at reflux in concentrated hydrochloric acid (10ml) for 18 hours. More hydrochloric acid (11ml) was added and the mixture heated at reflux for a further 18 hours. The reaction mixture was then concentrated *in vacuo* to a solid. This was taken up in dichloromethane and triethylamine (4ml) added. The reaction mixture was then concentrated *in vacuo.* This was repeated twice to provide the title compound (2.03g) as a solid containing triethylamine hydrochloride.
LRMS APCI⁺ m/z 350 [MH]⁺.

### Intermediate 82: 1-[3-({4-[4-(pyrrolidin-1-ylcarbonyl)tetrahydro-2H-pyran-4-yl]phenyl}thio)propyl]pyrrolidine

The title compound (228mg, 40%) was prepared using 4-{4-[(3-pyrrolidin-1-ylpropyl)thio]phenyl}tetrahydro-2H-pyran-4-carboxylic acid and pyrrolidine similarly to the procedure used for example 149.
HRMS ESI⁺ m/z 403.2403 [MH]⁺.

### Example 225: 1-[3-({4-[4-(pyrrolidin-1-ylmethyl)tetrahydro-2H-pyran-4-yl]phenyl}thio)propyl]pyrrolidine

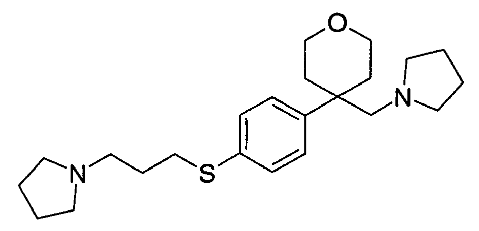

The title compound (58mg, 27%) was prepared using 1-[3-({4-[4-(pyrrolidin-1-ylcarbonyl)tetrahydro-2*H-*pyran-4-yl]phenyl}thio)propyl]pyrrolidine and lithium aluminium hydride similarly to the procedure used for example 142.
¹H NMR (400MHz, CDCl₃) δ 1.54-1.57 (m, 4H), 1.75-1.79 (m, 4H), 1.84-1.94 (m, 4H), 2.06-2.10 (m, 2H), 2.18-2.22 (m, 4H), 2.47-2.50 (m, 4H), 2.57 (t, 2H), 2.63 (s, 2H), 2.96 (t, 2H), 3.54 (t, 2H), 3.73-3.78 (m, 2H), 7.22 (d, 2H), 7.29 (d, 2H).
HRMS ESI⁺ m/z 389.2615 [MH]⁺.

### Intermediate 83: 1-[4-(3-pyrrolidin-1-ylpropoxy)phenyl] cyclohexanecarboxylic acid

The title compound (contaminated with ammonium chloride) was prepared using 1-[4-(3-pyrrolidin-1-ylpropoxy)phenyl]cyclohexanecarbonitrile and concentrated hydrochloric acid similarly to the procedure used for intermediate 80.
LRMS ESI⁺ m/z 332 [MH]⁺.

### Intermediate 84: 1-({1-[4-(3-pyrrolidin-1-ylpropoxy)phenyl]cyclohexyl} carbonyl)piperidin-4-ol

The title compound (200mg, 16%) was prepared using 1-[4-(3-pyrrolidin-1-ylpropoxy)phenyl]cyclohexanecarboxylic acid and 4-hydroxypiperidine similarly to the procedure used for example 149.
HRMS ESI⁺ m/z 415.2943 [MH]⁺.

### Example 226: 1-({1-[4-(3-pyrrolidin-1-ylpropoxy)phenyl]cyclohexyl}methyl) piperidin-4-ol

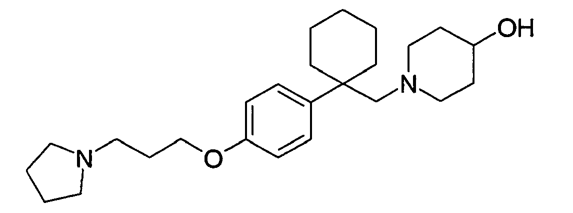

The title compound (90mg, 46%) was prepared using 1-({1-[4-(3-pyrrolidin-1-ylpropoxy)phenyl]cyclohexyl}carbonyl)piperidin-4-ol and lithium aluminium hydride similarly to the procedure used for example 142.
¹H NMR (400MHz, CDCl₃) δ 1.29-1.55 (m, 10H), 1.64-1.69 (m, 2H), 1.77-1.81 (m, 4H), 1.90-2.06 (m, 4H), 2.10-2.13 (m, 2H), 2.23 (s, 2H), 2.26-2.30 (m, 2H), 2.51-2.54 (m, 4H), 2.62 (t, 2H), 3.51 (m, 1H), 4.01 (t, 2H), 6.83 (d, 2H), 7.25 (d, 2H).
HRMS ESI⁺ m/z 401.3154 [MH]⁺.

### Example 227: 1-methyl-4-({1-[4-(3-pyrrolidin-1-ylpropoxy)phenyl]cyclohexyl} carbonyl)piperazine

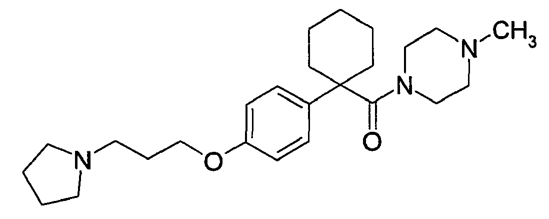

The title compound (10mg, 8%) was prepared using 1-[4-(3-pyrrolidin-1-ylpropoxy)phenyl]cyclohexanecarboxylic acid and 1-methylpiperazine similarly to the procedure used for example 149.
¹H NMR (400MHz, CD₃OD) δ 1.32 (m, 1H), 1.64-1.69 (m, 7H), 1.81-1.85 (m, 4H), 1.98-2.03 (m, 2H), 2.05-2.13 (m, 4H), 2.15 (s, 3H), 2.25-2.28 (m, 2H), 2.59-2.62 (m, 4H), 2.69 (t, 2H), 3.33-3.42 (m, 4H), 4.01 (t, 2H), 6.90 (d, 2H), 7.16 (d, 2H).
HRMS ESI⁺ m/z 414.3107 [MH]⁺.

### Intermediate 85: 1-({1-[4-(3-pyrrolidin-1-ylpropoxy)pheny]cyclohexyl} carbonyl)piperazine

The title compound (351mg, 19%) was prepared using 1-[4-(3-pyrrolidin-1-ylpropoxy)phenyl]cyclohexanecarboxylic acid and piperazine similarly to the procedure used for example 149.
LRMS APCI⁺ m/z 400 [MH]⁺.

### Example 228: 1-({1-[4-(3-pyrrolidin-1-ylpropoxy)phenyl]cyclohexy}methyl)piperazine

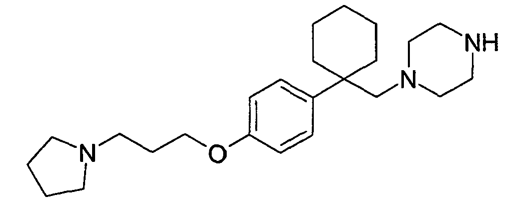

The title compound (187mg, 59%) was prepared using 1-({1-[4-(3-pyrrolidin-1-ylpropoxy)phenyl]cyclohexyl}carbonyl)piperazine and lithium aluminium hydride similarly to the procedure used for example 142.
¹H NMR (400MHz, CDCl₃) δ 1.30-1.33 (m, 3H), 1.46-1.54 (m, 4H), 1.78-1.81 (m, 4H), 1.98-2.05 (m, 4H), 2.09-2.12 (m, 6H), 2.23 (s, 2H), 2.53-2.57 (m, 4H), 2.62-2.71 (m, 6H), 4.01 (t, 2H), 6.83 (d, 2H), 7.26 (d, 2H).
LRMS APCI⁺ m/z 386 [MH]⁺.

### Example 229: 1-acetyl-4-({1-[4-(3-pyrrolidin-1-ylpropoxy)phenyl]cyclohexyl} methyl)piperazine

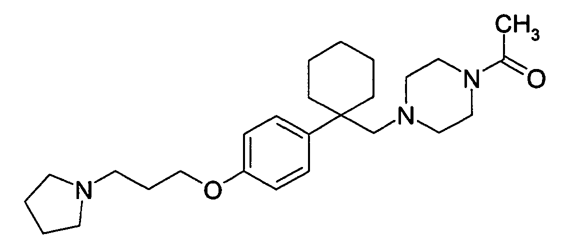

The title compound (73mg, 73%) was prepared using 1-({1-[4-(3-pyrrolidin-1-ylpropoxy)phenyl]cyclohexyl}methyl) piperazine and acetic anhydride similarly to the procedure used for example 166.
¹H NMR (400MHz, CDCl₃) δ 1.31-1.34 (m, 3H), 1.48-1.53 (m, 5H), 1.82-1.85 (m, 4H), 1.99 (s, 3H), 2.02-2.07 (m, 4H), 2.13-2.17 (m, 4H), 2.28 (s, 2H), 2.58-2.64 (m, 4H), 2.70 (t, 2H), 3.20 (t, 2H), 3.41-3.43 (m, 2H), 4.01 (t, 2H), 6.83 (d, 2H), 7.25 (d, 2H).
HRMS ESI⁺ m/z 428.3264 [MH]⁺.

### Example 230: 1-(methylsulfonyl)-4-({1-[4-(3-pyrrolidin-1-ylpropoxy)phenyl] cyclohexyl}methyl)piperazine

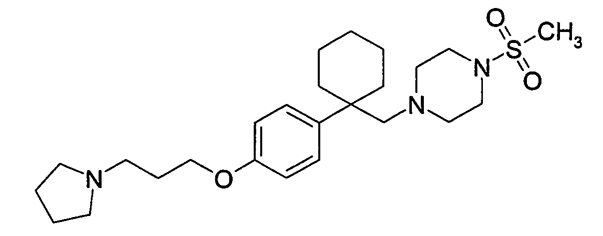

Methanesulphonic acid anhydride (49mg, 0.28mmol) was added at 0°C to a solution of 1-({1-[4-(3-pyrrolidin-1-ylpropoxy)phenyl]cyclohexyl}methyl) piperazine (90mg, 0.23mmol) and pyridine (47µl, 0.58mmol) in dichloromethane (1 ml). The reaction was stirred at 0 °C for 10 minutes, then allowed to warm up to room temperature and stirred for 18 hours. The reaction mixture was concentrated under reduced pressure and partitioned between dichloromethane (20ml) and aqueous sodium carbonate (20ml). The layers were separated. The organic layer was dried over sodium sulphate, filtered and concentrated *in vacuo.* The crude product was purified by flash chromatography on silica gel eluting with dichloromethane:methanol:ammonia (96:4:0.4 by volume) to provide the title compound (61 mg, 56%).
¹H NMR (400MHz, CDCl₃) δ 1.29-1.36 (m, 3H), 1.45-1.52 (m, 5H), 1.85-1.90 (m, 4H), 2.08-2.15 (m, 4H), 2.22 (t, 4H), 2.32 (s, 2H), 2.67-2.79 (m, 9H), 3.00-3.03 (m, 4H), 4.02 (t, 2H), 6.83 (d, 2H), 7.24 (d, 2H). HRMS ESI⁺ m/z 464.2931 [MH]⁺.

### Intermediate 86: tert-butyl bis(2-chloroethyl)carbamate

Bis-(2-chloroethyl)amine hydrochloride (10g, 56mmol) was stirred vigourously in a mixture of dichloromethane (150ml) and aqueous 10% sodium hydroxide (50ml). Di-*tert*-butyldicarbonate (12.2g, 56mmol) as a solution in dichloromethane (75ml) was then added dropwise. The reaction mixture was stirred at room temperature for 5 hours. TLC showed the reaction was not complete so more di-*tert-*butyldicarbonate (4g, 18.3mmol) was added. The mixture was stirred vigourously for 18 hours at room temperature. The layers were then separated and the aqueous layer extracted with more dichloromethane (2x50ml). The combined organic layers were dried over Na₂SO₄, filtered and concentrated under reduced pressure. The crude product was purified by flash chromatography on silica gel eluting with pentane:ethyl acetate (99:1 to 80:20 by volume) to provide the title compound (7.9g, 58%).

### Intermediate 87: tert-butyl 4-cyano-4-[4-(3-pyrrolidin-1-ylpropoxy)phenyl] piperidine-1-carboxylate

The title compound (1.8g, 53%) was prepared using [4-(3-pyrrolidin-1-ylpropoxy)phenyl]acetonitrile, *tert-*butyl bis(2-chloroethyl)carbamate, sodium hydride and potassium iodide similarly to the procedure used for intermediate 56.
LRMS APCI⁺ m/z 414 [MH]⁺ and m/z 245 [SMH]⁺.

### Example 231: 4-[4-(3-pyrrolidin-1-ylpropoxy)phenyl]piperidine-4-carbonitrile

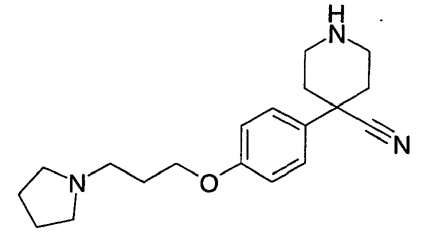

*tert*-Butyl 4-cyano-4-[4-(3-pyrrolidin-1-ylpropoxy)phenyl] piperidine-1-carboxylate (500mg, 1.24mmol) was stirred for 1 hour in trifluoroacetic acid (1.91ml, 24.8mmol) and dichloromethane (30ml). More trifluoroacetic acid (3ml) was added and the reaction stirred at room temperature for another hour. The reaction mixture was quenched with saturated aqueous sodium carbonate solution (15ml) and the layers separated. The organic layer was dried over sodium sulphate and concentrated *in vacuo.* The crude product was purified by flash chromatography on silica gel eluting with dichloromethane:methanol:ammonia (95:5:0.5 to 90:10:1 by volume) to provide the title compound (450mg, 18% over 2 steps).
¹H NMR (400MHz, CD₃OD) δ 1.81-1.85 (m, 4H), 1.91-2.09 (m, 6H), 2.59-2.62 (m, 4H), 2.69 (t, 2H), 2.99 (t, 2H), 3.12-3.15 (m, 2H), 4.04 (t, 2H), 6.96 (d, 2H), 7.42 (d, 2H).
HRMS ESI⁺ m/z 314.2227 [MH]⁺.

### Example 232: 1-acetyl-4-[4-(3-pyrrolidin-1-ylpropoxy)phenyl]piperidine-4-carbonitrile

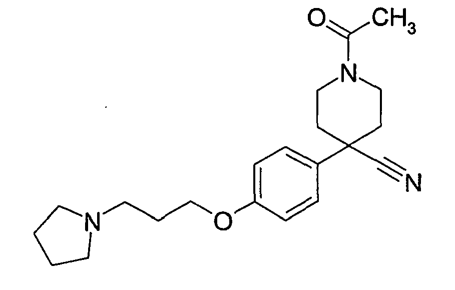

Acetyl chloride (27µl, 0.37mmol) was added dropwise to a solution of 4-[4-(3-pyrrolidin-1-ylpropoxy)phenyl]piperidine-4-carbonitrile (90mg, 0.28mmol) and triethylamine (78µl, 0.56mmol) in dichloromethane (3ml). The reaction mixture was stirred at room temperature for 1 hour. The reaction mixture was diluted with dichloromethane (15ml) and washed with water (15ml). The layers were separated and the aqueous layer was further extracted with dichloromethane (2x10ml). The organics were combined, washed with brine, dried over sodium sulphate, filtered and concentrated *in vacuo*. The crude product was purified by flash chromatography on silica gel eluting with ethyl acetate:methanol:ammonia (95:5:0.5 by volume) to provide the title compound (28mg, 28%).
¹H NMR (400MHz, CD₃OD) δ 1.82-1.85 (m, 4H), 1.89-2.18 (m, 9H), 2.60-2.63 (m, 4H), 2.69 (t, 2H), 3.00 (t, 1H), 3.50 (t, 1 ), 4.03-4.12 (m, 3H), 4.70 (d, 1H), 6.97 (d, 2H), 7.44 (d, 2H).
HRMS ESI⁺ m/z 356.2329 [MH]⁺.

### Example 233: 1-methyl-4-[4-(3-pyrrolidin-1-ylpropoxy)phenyl]piperidine-4-carbonitrile

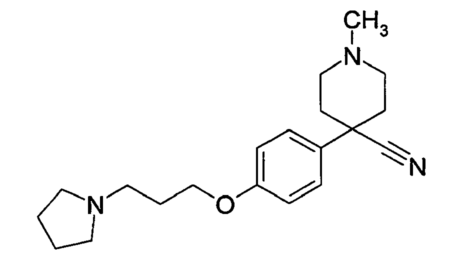

The title compound (80mg, 76%) was prepared from 4-[4-(3-pyrrolidin-1-ylpropoxy)phenyl]piperidine-4-carbonitrile, formaldehyde and sodium triacetoxyborohydride similarly to the procedure used for example 54.
¹H NMR (400MHz, CD₃OD) δ 1.81-1.85 (m, 4H), 1.97-2.15 (m, 6H), 2.37 (s, 3H), 2.45 (t, 2H), 2.58-2.62 (m, 4H), 2.68 (t, 2H), 2.99 (d, 2H), 4.04 (t, 2H), 6.96 (d, 2H), 7.42 (d, 2H).
LRMS APCI⁺ m/z 328 [MH]⁺.
Microanalysis: Found: C, 73.01; H, 8.94; N, 12.81%. C₂₀H₂₉N₃O requires C, 73.36; H, 8.93; N, 12.83%.

### Example 234: 1-isopropyl-4-[4-(3-pyrrolidin-1-ylpropoxy)phenyl]piperidine-4-carbonitrile

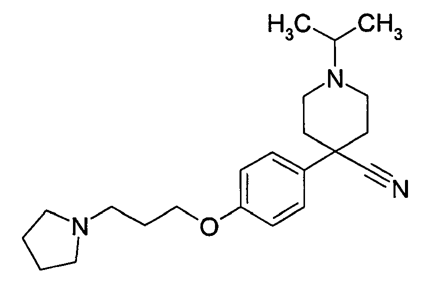

The title compound (45mg, 58%) was prepared from 4-[4-(3-pyrrolidin-1-ylpropoxy)phenyl]piperidine-4-carbonitrile, acetone and sodium triacetoxyborohydride similarly to the procedure used for example 54.
¹H NMR (400MHz, CD₃OD) δ 1.12 (d, 6H), 1.81-1.84 (m, 4H), 1.99-2.14 (m, 6H), 2.58-2.69 (m, 8H), 2.82 (m, 1H), 3.02 (d, 2H), 4.04 (t, 2H), 6.96 (d, 2H), 7.42 (d, 2H).
HRMS ESI⁺ m/z 356.2691 [MH]⁺.

### Example 235: 1-(2-methoxyethyl)-4-[4-(3-pyrrolidin-1-ylpropoxy)phenyl] piperidine-4-carbonitrile

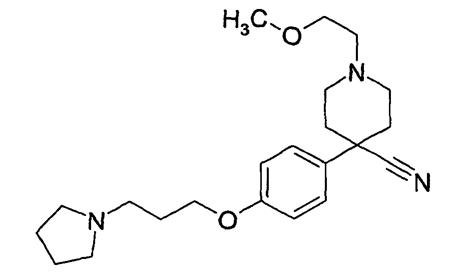

A mixture of 4-[4-(3-pyrrolidin-1-ylpropoxy)phenyl]piperidine-4-carbonitrile (68mg, 0.22mmol), 2-bromoethylmethylether (21 µl, 0.22mmol), solid sodium bicarbonate (84mg, 1.0mmol) and potassium iodide (5mg, catalytic) was heated at 50°C in acetonitrile (1ml) for 18 hours. The reaction mixture was concentrated *in vacuo*. The residue was partitioned between dichloromethane (2x50ml) and 10% aqueous sodium carbonate (20ml). The organic layers were combined, dried over sodium sulphate, filtered and concentrated *in vacuo.* The crude product was purified by flash chromatography on silica gel eluting with dichloromethane:methanol:ammonia (97:3:0.3 to 95:5:0.5 by volume) to provide the title compound (46mg, 61 %).
¹H NMR (400MHz, CD₃OD) δ 1.83-1.87 (m, 4H), 2.01-2.10 (m, 6H), 2.45-2.52 (m, 2H), 2.65-2.69 (m, 6H), 2.75 (t, 2H), 3.09 (d, 2H), 3.35 (s, 3H), 3.56 (t, 2H), 4.04 (t, 2H), 6.96 (d, 2H), 7.42 (d, 2H).
HRMS ESI⁺ m/z 372.2639 [MH]⁺.

### Example 236: 2-[methyl({4-[4-(3-pyrrolidin-1-ylpropoxy)phenyl]tetrahydro-2H-pyran-4-yl}methyl)amino]ethanol

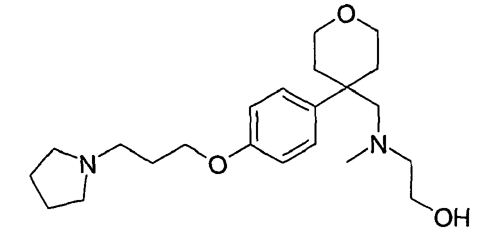

A solution of methyl-{4-[4-(3-pyrrolidin-1-ylpropoxy)phenyl]-tetrahydropyran-4-ylmethyl}amine (350 mg, 1.05 mmol), glycolaldehyde dimer (130 mg, 1.05 mmol) and AcOH (0.15 ml, 2.1 mmol) in DCM (5 ml) was stirred at room temperature for 18 hours. Saturated aqueous sodium carbonate (10 ml) and DCM (10 ml) were added and the mixture was shaken and partitioned. The aqueous phase was extracted with DCM (2x10 ml) and the combined organics dried (K₂CO₃), filtered and concentrated *in vacuo*. The crude product was purified by flash chromatography on silica gel eluting with dichloromethane:methanol:ammonia (92:8:1) to provide the title compound as a clear colourless oil (223 mg, 0.59 mmol, 56%).
¹H NMR (400MHz, CDCl₃) δ 1.70-1.90 (m, 6H), 1.96 (s, 3H), 2.00 (quintet, 2H), 2.14 (m, 2H), 2.37 (t, 2H), 2.40-2.60 (m, 6H), 2.65 (t, 2H), 3.35 (t, 2H), 3.50 (m, 2H), 3.76 (m, 2H), 4.01 (t, 2H), 6.89 (d, 2H), 7.19 (d, 2H).
HRMS ESI⁺ m/z 377.2796 [MH]⁺.

### Example 237: N-methyl-N-({4-[4-(3-pyrrolidin-1-ylpropoxy)phenyl]tetrahydro-2H-pyran-4-yl}methyl)cyclopropanamine

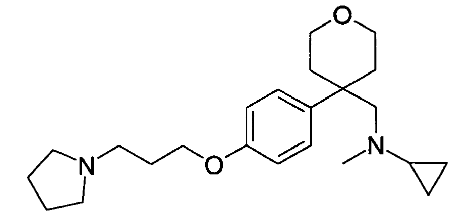

A mixture of methyl-{4-[4-(3-pyrrolidin-1-ylpropoxy)phenyl]-tetrahydropyran-4-ylmethyl}amine (115 mg, 0.35 mmol), (1-Ethoxy-cyclopropoxy)-trimethylsilane (360 mg, 2.0 mmol), AcOH (0.2 ml, 3.5 mmol), NaCNBH₃ (110 mg, 1.75 mmol) and 4A molecular sieves (100 mg) in MeOH (5 ml) was heated to reflux for 18 hours. The mixture was cooled, concentrated *in vacuo* and partitioned between 2M NaOH (10 ml) and DCM (10 ml). The organic phase was separated and the aqueous phase extracted with DCM (2x10 ml). The combined organic phases were concentrated *in vacuo* and purified by flash chromatography on silica gel eluting with dichloromethane:methanol:ammonia (92:8:1) to provide the title compound as a clear colourless oil (20 mg, 0.054 mmol, 15%).

¹H NMR (400MHz, CDCl₃) δ 0.15 (m, 2H), 0.29 (m, 2H), 1.68 (m, 1H), 1.70-1.90 (m, 6H), 1.85 (s, 3H), 2.00-2.10 (m, 4H), 2.60 (m, 4H), 2.65 (s, 2H), 2.68 (m, 2H), 3.50 (t, 2H), 3.76 (m, 2H), 4.01 (t, 2H), 6.85 (d, 2H), 7.16 (d, 2H).
HRMS ESI⁺ m/z 373.2844 [MH]⁺.

### Example 238: 1-(3-{4-[4-(aziridin-1-ylmethyl)tetrahydro-2H-pyran-4-yl]phenoxy}propyl)pyrrolidine

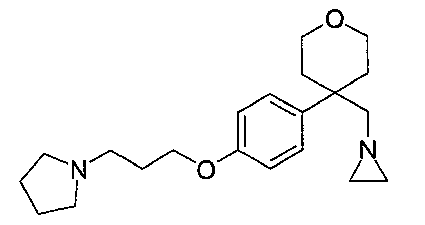

A mixture of {4-[4-(3-Pyrrolidin-1-ylpropoxy)phenyl]tetrahydropyran-4-yl} methylamine (482 mg, 1.50 mmol), 1,2-dibromoethane (0.13 ml, 1.50 mmol) and K₂CO₃ (420 mg, 3.0 mmol) in acetonitrile (50 ml) was heated to 70°C for 3 days. The mixture was concentrated *in vacuo* and purified by flash chromatography on silica gel eluting with dichloromethane:methanol:ammonia (93:7:1 to 91:9:1) to provide the title compound as a clear colourless oil (51 mg, 0.15 mmol, 10%).
¹H NMR (400MHz, CDCl₃) δ 0.71 (m, 2H), 1.48 (m, 2H), 1.78 (m, 4H), 2.00 (m, 4H), 2.19 (m, 2H), 2.30 (s, 2H), 2.54 (m, 4H), 2.63 (t, 2H), 3.54 (m, 2H), 3.78 (m, 2H), 4.01 (t, 2H), 6.86 (d, 2H), 7.21 (d, 2H). HRMS ESI⁺ m/z 345.2534 [MH]⁺.

### Example 239: 2-(methylthio)-1-({4-[4-(3-pyrrolidin-1-ylpropoxy)phenyl]tetrahydro-2H-pyran-4 yl}methyl)-1H-imidazole

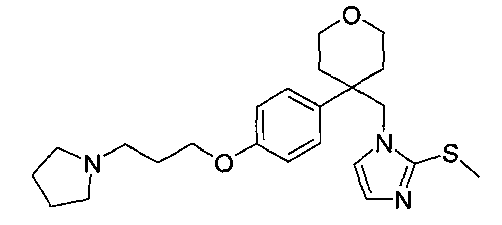

A solution of {4-[4-(3-Pyrrolidin-1-ylpropoxy)phenyl]tetrahydropyran-4-yl} methylamine (159 mg, 0.50 mmol) and dimethyl 2,2-diethoxyethyldithioimido-carbonate (120 mg, 0.50 mmol, ARKIVOC 2001, viii, 34-39) in AcOH (5 ml) was heated at reflux for 8 h. The mixture was concentrated *in vacuo* and partitioned between saturated aqueous sodium bicarbonate (10 ml) and DCM (10 ml). The aqueous phase was extracted with DCM (2x10 ml) and the combined organics dried (K₂CO₃), filtered and concentrated *in vacuo.* The crude product was purified by flash chromatography on silica gel eluting with dichloromethane:methanol:ammonia (90:10:1) to provide the title compound as a clear colourless gum (90 mg, 0.22 mmol, 43%).
¹H NMR (400MHz, CDCl₃) δ 1.80-2.00 (m, 6H), 2.12 (m, 2H), 2.21 (quintet, 2H), 2.48 (s, 3H), 2.80-3.00 (m, 6H), 3.45 (m, 2H), 3.83 (m, 2H), 3.98 (s, 2H), 4.06 (t, 2H), 6.10 (s, 1H), 6.85 (d, 2H), 6.87 (s, 1H), 7.01 (d, 2H).
LRMS APCI⁺ m/z 416 [MH]⁺.

### Example 240: 1-{4-[4-(3-Pyrrolidin-1-yl-propoxy)-phenyl]-tetrahydro-pyran-4-ylmethyl}-1H-imidazole

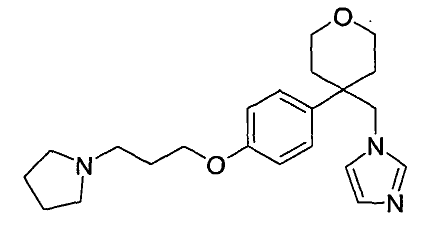

To a solution of 2-(methylthio)-1-({4-[4-(3-pyrrolidin-1-ylpropoxy)phenyl]tetrahydro-2H-pyran-4-yl}methyl)-1H-imidazole (90 mg, 0.22 mmol) in EtOH (4 ml) and water (1ml) was added Raney Nickel (50% slurry in water) in 200 mg aliquots every 30 min. After 3 h all starting material had been consumed. The mixture was filtered through Arbocel® with EtOH (200 ml) and concentrated *in vacuo.* The crude product was purified by flash chromatography on silica gel eluting with dichloromethane:methanol:ammonia (90:10:1) to provide the title compound as a yellow oil (29 mg, 0.078 mmol, 36%).
¹H NMR (400MHz, CDCl₃) δ 1.80-2.00 (m, 6H), 2.10 (m, 4H), 2.70-2.90 (m, 6H), 3.50 (t, 2H), 3.81 (m, 2H), 3.97 (s, 2H), 4.04 (t, 2H), 6.32 (s, 1H), 6.80-6.90 (m, 4H), 6.98 (d, 2H).
HRMS ESI⁺ m/z 370.2485 [MH]⁺.

### Intermediate 88: 4-(4-{[tert-butyl(dimethyl)silyl]oxy}phenyl)tetrahydro-2H-pyran-4-carbonitrile

A solution of 4-(4-hydroxy-phenyl)-tetrahydro-pyran-4-carbonitrile (5g, 25mmol), t-butyldimethylsilyl chloride (4.46g, 29mmol) and imidazole (2.34g, 34mmol) were stirred in DMF (15ml) at room temperature. After 30 minutes, a yellow suspension was observed. The reaction was partitioned between ether (75ml) and water (100ml). The ether was then washed further with water (4x50ml), dried over sodium sulphate, filtered and concentrated *in vacuo.* The title compound was isolated as a pale orange waxy solid (7g, 90%).

### Intermediate 89: 1-[4-(4-{[tert-butyl(dimethyl)silyl]oxy}phenyl)tetrahydro-2H-pyran-4-yl]methanamine

To a stirred solution of 4-(4-{[tert-butyl(dimethyl)silyl]oxy}phenyl)tetrahydro-2H-pyran-4-carbonitrile (3.5g, 11mmol) in THF (35ml) at 0°C was added dropwise lithium aluminium hydride (1M solution in ether, 44ml, 44mmol). The reaction was allowed to warm up to room temperature and stirred for 4 hours until complete. The reaction mixture was cooled to 0°C, water (1.66 mL) was added followed by NaOH (2.0M, 1.66 mL) and water (4.97 mL). The mixture was filtered through a short pad of celite, eluting with dichloromethane:methanol (97:3, 150ml) and concentrated *in vacuo.* Solid obtained was partitioned between ethyl acetate (50ml) and sodium bicarbonate (50ml). The organic layer was then dried over sodium sulphate, filtered and concentrated *in vacuo* to give the title compound (2.04g, 58%).
LRMS APCI⁺ m/z 322 [MH]⁺.

### Intermediate 90: N-{[4-(4-{[tert-butyl(dimethyl)silyl]oxy}phenyl)tetrahydro-2H-pyran-4-yl]methyl}pyridin-2-amine

1-[4-(4-{[tert-butyl(dimethyl)silyl]oxy}phenyl)tetrahydro-2H-pyran-4-yl]methanamine (3.65g, 11.4mmol), 2-bromopyridine (910µl, 9.48mmol), tris(dibenzylideneacetone)dipalladium(0) (471mg, 0.455mmol), 2,2'-bis(diphenylphosphino)1,1'-binaphyl (567mg, 0.910mmol) and sodium *tert*-butoxide (1.29g, 13.0mmol) were stirred in toluene (50ml) at 70°C under nitrogen for 24 hours. The toluene was removed *in vacuo* and the residue partitioned between dichloromethane (50ml) and saturated sodium bicarbonate solution (50ml). The organic layer was separated and dried over sodium sulphate, filtered and concentrated *in vacuo*. The crude product was purified by flash chromatography on silica gel eluting with ether:pentane (80:20 by volume) to provide the title compound (2.10g, 46%) as a yellow solid.
LRMS APCI⁺m/z 399 [MH]⁺.

### Intermediate 91: 4-{4-[(pyridin-2-ylamino)methyl]tetrahydro-2H-pyran-4-yl}phenol

N-{[4-(4-{[tert-butyl(dimethyl)silyl]oxy}phenyl)tetrahydro-2H-pyran-4-yl]methyl}pyridin-2-amine (2.099g, 5.27mmol) and tetrabutylammonium fluoride (1M solution in THF, 5.80ml, 5.80mmol) were stirred in THF (20ml) at room temperature for 24 hours until complete. THF was removed *in vacuo* and the residue partitioned between dichloromethane (75ml) and sodium bicarbonate (50ml). The organic layer was washed further with sodium bicarbonate (2x50ml), then separated and dried over sodium sulphate, filtered and concentrated *in vacuo*. The crude product was purified by column chromatography on Biotage® silica gel eluting with dichloromethane:methanol:ammonia (100:0:0 to 90:10:1 by volume) to give the title compound (862mg, 58%) as a solid.
LRMS APCI⁺ m/z 285 [MH]⁺.

### Intermediate 92: N-({4-[4-(4-chlorobutoxy)phenyl]tetrahydro-2H-pyran-4-yl}methyl)pyridin-2-amine

4-{4-[(pyridin-2-ylamino)methyl]tetrahydro-2H-pyran-4-yl}phenol (220mg, 0.775mmol), 1-bromo-4-chlorobutane (146mg, 0.852mmol) and potassium carbonate (118mg, 0.852mmol) were stirred in DMF (1ml) at 60°C for 24 hours until complete. Reaction was partitioned between ethyl acetate (50ml) and water (75ml). The organic layer was separated and washed further with water (2x30ml), then dried over sodium sulphate, filtered and concentrated *in vacuo*. The crude compound was purified by flash chromatography on silica gel eluting with dichloromethane:methanol:ammonia (100:0:0 to 98:2:0.2 by volume) to give the title compound (184mg, 63%) as a yellow solid.
LRMS APCI⁺m/z 375 [MH]⁺.

### Example 241 : N-({4-[4-(4-pyrrolidin-1-ylbutoxy)phenyl]tetrahydro-2H-pyran-4-yl}methyl)pyridin-2-amine

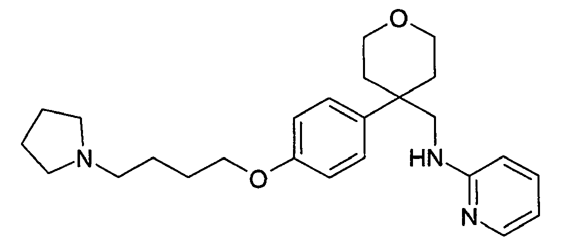

A mixture of N-({4-[4-(4-chlorobutoxy)phenyl]tetrahydro-2H-pyran-4-yl}methyl)pyridin-2-amine (180mg, 0.481mmol), pyrrolidine (80µl, 0.963mmol), sodium carbonate (102mg, 0.963mmol) and sodium iodide (4mg, 0.024mmol) were stirred in butanol (5ml) at 100°C for 24 hours until complete. Butanol was removed *in vacuo* and the residue partitioned between ethyl acetate (40ml) and water (40ml). Organic layer was dried over sodium sulphate, filtered and concentrated *in vacuo* to give a brown solid. Solid was purified by flash chromatography on silica gel eluting with dichloromethane:methanol:ammonia (98:2:02 to 96:4:0.4 by volume), followed by recrystallisation from ether (1.5ml) to give the title compound (56.6mg, 29%).
¹H NMR (400MHz, CDCl₃) δ 1.78-2.00 (m, 12H), 2.08-2.20 (m, 2H), 2.54-2.90 (m, 4H), 3.50 (d, 2H), 2.54-2.63 (m, 2H), 3.79-3.88 (m, 2H), 3.96-4.08 (m, 2H), 6.23 (d, 1H), 6.50 (t, 1H), 6.90 (d, 2H), 7.25 (d, 2H), 7.32 (t, 1H), 8.01 (d, 1H).
LRMS APCI⁺m/z 410 [MH]⁺.

### Example 242: N-({4-[4-(3-piperidin-1-ylpropoxy)phenyl]tetrahydro-2H-pyran-4-yl}methyl)pyridin-2-amine

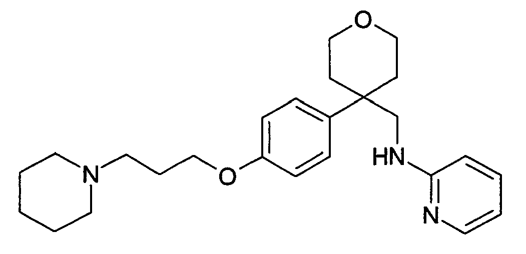

4-{4-[(pyridin-2-ylamino)methyl]tetrahydro-2H-pyran-4-yl}phenol (200mg, 0.704mmol), 3-chloropropylpiperidine hydrochloride (152mg, 0.775mmol) and potassium carbonate (204mg, 1.48mmol) were stirred in DMF (1ml) at 45°C for 24 hours until complete. The reaction was then partitioned between ethyl acetate (40ml) and water (40ml). The organic layer was separated, dried over sodium sulphate and concentrated *in vacuo* to give a solid. The solid was purified by preparative HPLC to give the title compound (220mg, 76%) as a white solid.
¹H NMR (400MHz, CDCl₃) δ 1.44-1.55 (m, 2H), 1.64-1.78 (m, 4H), 1.85-2.00 (m, 2H), 2.08-2.18 (m, 4H), 2.52-2.70 (m, 6H), 3.48 (d, 2H), 3.52-3.62 (m, 2H), 3.78-3.85 (m, 2H), 4.02 (t, 2H), 6.21 (d, 1H), 6.50 (t, 1H), 6.90 (d, 2H), 7.24 (d, 2H), 7.34 (t, 1H), 8.01 (d, 1H).
LRMS APCI⁺ m/z 410 [MH]⁺.

### Example 243: N-[(4-{4-[(1-ethylpiperidin-4-yl)oxy]phenyl}tetrahydro-2H-pyran-4-yl)methyl]pyridin-2-amine

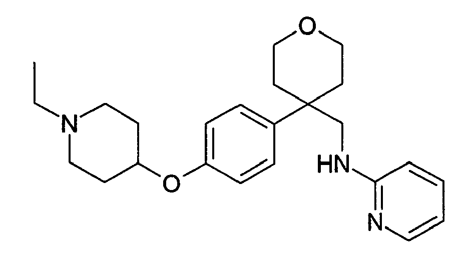

To a solution of 4-{4-[(pyridin-2-ylamino)methyl]tetrahydro-2H-pyran-4-yl}phenol (250mg, 0.881mmol), 1-ethylpiperidin-4-ol (103mg, 0.8mmol) and triphenylphosphine (231mg, 0.881mmol) in THF (5ml) at 0°C under nitrogen was added dropwise diisopropyl azodicarboxylate (95% solution, 183µl, 0.881mmol). Reaction was stirred for 10 minutes then warmed to room temperature for 72 hours until complete. THF was removed *in vacuo* and the residue partitioned between ethyl acetate (50ml) and saturated sodium bicarbonate solution (50ml). The organic layer was separated, dried over sodium sulphate, filtered and concentrated *in vacuo.* Crude product was purified by flash chromatography eluting with dichloromethane:methanol:ammonia (100:0:0 to 96:4:0.4 by volume) to give the title compound (8.2mg, 23%) as a yellow oil.
¹H NMR (400MHz, CDCl₃) δ 1.05 (t, 3H), 1.80-1.98 (m, 4H), 2.00-2.18 (m, 4H), 2.28-2.42 (m, 2H), 2.44-2.54 (q, 2H), 2.72-2.86 (m, 2H), 3.50 (d, 2H), 3.55-3.62 (m, 2H), 3.78-3.88 (m, 2H), 4.00-4.08 (m, 1H), 4.30-4.39 (m, 1H), 6.22 (d, 1H), 6.50 (t, 1H), 6.90 (d, 2H), 7.23 (d, 2H), 7.30 (t, 1H), 8.01 (d, 1H).
LRMS APCI⁺m/z 396 [MH]⁺.
HRMS ESI⁺ m/z 396.2641 [MH]⁺.

### Intermediate 93: N-(3-chloropropyl)-N-methylcyclobutanamine

To a solution of N-methylcyclobutanamine (1g, 3.89mmol) and potassium carbonate (1.18g, 8.56mmol) in acetonitrile (20ml) at 0°C was added 1-bromo-3-chloropropane (383µl, 3.89mmol). Reaction was warmed to room temperature and stirred for 24 hours. Solid removed by filtration and filtrate concentrated *in vacuo* to give the title compound (241 mg, 38%) as an oil. No purification was performed.
LRMS APCI⁺m/z 162 [MH]⁺.

### Example 244: N-{[4-(4-{3-[cyclobutyl(methyl)amino]propoxy}phenyl)tetrahydro-2H-pyran-4-yl]methyl}pyridin-2-amine

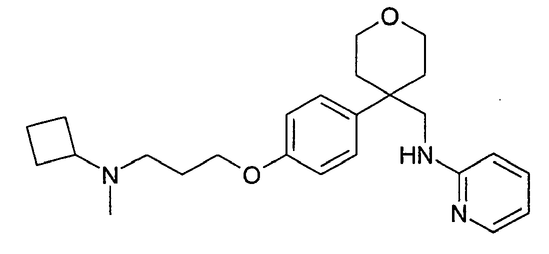

4-{4-[(pyridin-2-ylamino)methyl]tetrahydro-2H-pyran-4-yl}phenol (386mg, 1.36mmol), N-(3-chloropropyl)-N-methylcyclobutanamine (241 mg, 1.496mmol) and potassium carbonate (280mg, 2.03mmol) were stirred in DMF (2ml) at 45°C for 24 hours until complete. Reaction was partitioned between ethyl acetate (40ml) and saturated sodium bicarbonate solution (50ml), then the organic layer washed with water (50ml). Organic layer separated and dried over sodium sulphate, filtered and concentrated *in vacuo.* Crude product purified by flash chromatography on silica gel eluting with dichloromethane:methanol:ammonia (100:0:0 to 90:10:1 by volume) to give the title compound (165mg, 30%).
¹H NMR (400MHz, CDCl₃) δ 1.55-1.72 (m, 2H), 1.80-1.99 (m, 6H), 2.00-2.09 (m, 2H), 2.10-2.20 (m, 4H), 2.40 (t, 2H), 2.80 (quintet, 1H), 3.50 (d, 2H), 3.54-3.62 (m, 2H), 3.78-3.88 (m, 2H), 4.00 (m, 3H), 6.20 (d, 1H), 6.50 (t, 1H), 6.92 (d, 2H), 7.22 (d, 2H), 7.32 (t, 1H), 8.00 (d, 1H).
LRMS APCI⁺m/z 410 [MH]⁺.
HRMS ESI⁺ m/z 410.2794 [MH]⁺.

### Intermediate 94: 4-{4-[(1-isopropylpiperidin-4-yl)oxy]phenyl}tetrahydro-2H-pyran-4-carbaldehyde

To a cooled solution of 4-{4-[(1-isopropylpiperidin-4-yl)oxy]phenyl}tetrahydro-2H-pyran-4-carbonitrile (1.16g, 3.53mmol) in dichloromethane (12ml) at -78°C under nitrogen, was added dropwise diisobutylaluminium hydride (1M solution in dichloromethane, 12.38ml, 12mmol) keeping the temperature below -60°C. Reaction stirred at -78°C for 40 minutes, then at -20°C for 1 hour before allowing to warm to room temperature. After 1 hour, reaction cooled and quenched with 2M HCl (13.2ml) and basified with sodium carbonate to pH 8, and filtered though a short pad of celite eluting with dichloromethane:methanol (99:1 by volume). Reaction then partitioned between dichloromethane (50ml) and water (50ml), and organic layer washed with a further portion of water (50ml) before being separated, dried over sodium sulphate, filtered and concentrated *in vacuo.* Purified by flash chromatography eluting with dichloromethane:methanol (99:1 to 92:8 by volume) to give the title compound (318mg, 27%).
LRMS APCI⁺ m/z 332 [MH]⁺.

### Example 245: 4-{4-[4-(4,5-dimethyl-1H-imidazol-2-yl)tetrahydro-2H-pyran-4-yl]phenoxy}-1-isopropylpiperidine

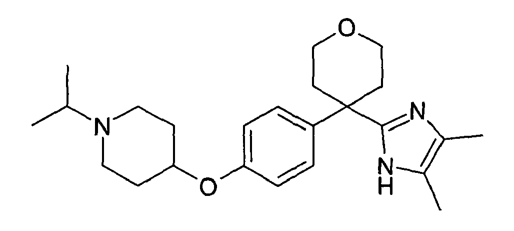

4-{4-[(1-isopropylpiperidin-4-yl)oxy]phenyl}tetrahydro-2H-pyran-4-carbaldehyde (50mg, 0.151 mmol), ammonium acetate (116mg, 1.51mmol) and 2,3-butanedione (13mg, 0.151mmol) in acetic acid (1ml) heated in a Smith Personal Synthesiser microwave for 900 seconds at 180°C. Acetic acid removed *in vacuo* and residue partitioned between ethyl acetate (20ml) and saturated sodium bicarbonate solution (20ml). Organic layer was separated, dried over sodium sulphate and concentrated *in vacuo*. Purified by flash chromatography eluting with dichloromethane:methanol:ammonia (100:0:0 to 95:5:0.5 by volume) to give the title compound (25.5mg, 43%) as a brown oil.
¹H NMR (400MHz, CDCl₃) δ 1.08 (d, 6H), 1.76-1.88 (m, 2H), 1.96-2.07 (m, 2H), 2.10 (s, 6H), 2.19-2.28 (m, 2H), 2.39-2.50 (m, 4H), 2.72-2.87 (m, 3H), 3.67-3.84 (m, 4H), 4.20-4.34 (m, 1H), 6.83 (d, 2H), 7.14 (d, 2H).
LRMS APCI⁺ m/z 398 [MH]⁺.

### Intermediate 95: (3-Nitro-pyridin-4-yl)-{4-[4-(3-pyrrolidin-1-yl-propoxy)-phenyl]-tetrahydro-pyran-4-ylmethyl}-amine

To a stirred solution of {4-[4-(3-Pyrrolidin-1-ylpropoxy)phenyl]tetrahydropyran-4-yl} methylamine (390mg, 1.2mmol) in acetonitrile (~5mL), was added Hunig's base (230µL, 1.35mmol, 1.1eq.) and 4-ethoxy-3-nitropyridine hydrogen chloride (250mg, 1.2mmol, 1eq.) and the mixture stirred at reflux for 72 hours. To this a further portion of 4-ethoxy-3-nitropyridine hydrogen chloride (50mg, 0.24mmol, 0.2eq.) was added and the mixture stirred at reflux for a further 24 hours. The reaction mixture was then concentrated *in vacuo* to give a dark yellow oily residue. This was partitioned between 2M NaOH (30mL) and DCM (2 x 30mL). The organics were combined, dried over sodium sulphate, filtered and concentrated *in vacuo* to give a dark yellow oil. The crude product was purified by flash chromatography on silica gel eluting with dichloromethane:methanol:ammonia (98:2:0.2 to 90:10:1, by volume) to provide the title compound (205mg, 38%).
HRMS ESI⁺ m/z 441.2486 [MH]⁺.

### Intermediate 96: (3-Nitro-pyridin-2-yl)-{4-[4-(3-pyrrolidin-1-yl-propoxy)-phenyl]-tetrahydro-pyran-4-ylmethyl}-amine

To a stirred solution of {4-[4-(3-Pyrrolidin-1-ylpropoxy)phenyl]tetrahydropyran-4-yl} methylamine (320mg, 1.0mmol) in acetonitrile (~5mL), was added Hunig's base (190µL, 1.1mmol, 1.1eq.) and 2-chloro-3-nitropyridine (175mg, 1.1mmol, 1.1eq.) and the mixture stirred at reflux for 4 hours. The reaction mixture was then concentrated *in vacuo* and partitioned between saturated NaHCO₃ solution (20mL) and DCM (2 x 20mL). The organics were combined, dried over sodium sulphate, filtered and concentrated *in vacuo* to give the title compound (475mg, 100%) as a yellow oil.
LRMS APCI⁺ m/z 441 [MH]⁺.

### Intermediate 97: N*4*-{4-[4-(3-Pyrrolidin-1-yl-propoxy)-phenyl]-tetrahydro-pyran-4-ylmethyl}-pyridine-3,4-diamine

A solution of (3-Nitro-pyridin-4-yl)-{4-[4-(3-pyrrolidin-1-yl-propoxy)-phenyl]-tetrahydro-pyran-4-ylmethyl}-amine (200mg, 0.45 mmol) in ethanol (~5mL) was hydrogenated for 4 hours at room temperature at 40 psi in the presence of 10% Pd/C (20mg, 10%w/w). The reaction mixture was filtered over Arbocel® and rinsed with ethanol. The filtrate was concentrated *in vacuo* to give a brown oily residue. The crude product was purified by flash chromatography on silica gel eluting with dichloromethane:methanol:ammonia (94:6:0.6, by volume) to provide the title compound (68mg, 36%).
HRMS ESI⁺ m/z 411.2750 [MH]⁺.

### Example 246: 2-Methyl-1-{4-[4-(3-pyrrolidin-1-yl-propoxy)-phenyl]-tetrahydro-pyran-4-ylmethyl}-1H-imidazo[4,5-c]pyridine

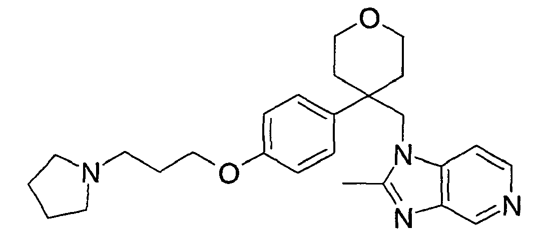

N*4*-{4-[4-(3-Pyrrolidin-1-yl-propoxy)-phenyl]-tetrahydro-pyran-4-ylmethyl}-pyridine-3,4-diamine (60mg, 0.15mmol) and acetic anhydride (~1mL) were stirred at reflux for 18 hours. The reaction mixture was quenched with ~1mL water, then basified with dilute sodium carbonate solution then extracted with DCM (2 x 20mL). The organics were combined, dried over sodium sulphate, filtered and concentrated *in vacuo* to give a brown oil. The crude product was purified by flash chromatography on silica gel eluting with dichloromethane:methanol:ammonia (95:5:0.5 to 90:10:1 by volume) to provide the title compound as a brown oil (19mg, 30%).
¹H NMR (400MHz, CDCl₃) δ 1.78-1.82 (m, 6H), 1.97-2.10 (m, 5H), 2.23 (m, 2H), 2.54 (m, 4H), 2.62 (t, 2H), 3.40 (m, 2H), 3.82 (m, 2H), 4.00 (t, 2H), 4.07 (s, 2H), 6.80-6.88 (m, 4H), 7.00 (d, 1H), 8.30 (d, 1H), 8.92 (s, 1H).
LRMS ESI⁺ m/z 435 [MH]⁺.

### Intermediate 98: N*2*-{4-[4-(3-Pyrrolidin-1-yl-propoxy)-phenyl]-tetrahydro-pyran-4-ylmethyl}-pyridine-2,3-diamine

A solution of (3-Nitro-pyridin-2-yl)-{4-[4-(3-pyrrolidin-1-yl-propoxy)-phenyl]-tetrahydro-pyran-4-ylmethyl}-amine (465mg, 1.06 mmol) in ethanol (~10mL) was hydrogenated for 4 hours at room temperature at 40 psi in the presence of 10% Pd/C (45mg, 10%w/w). The reaction mixture was filtered over Arbocel® and rinsed with ethanol (30mL), 2M HCl (20mL) and EtOH (30mL). The filtrate was concentrated *in vacuo* to give the title compound (365mg, 84%).
HRMS ESI⁺ m/z 411.2750 [MH]⁺.

### Example 247: 1-({4-[4-(3-pyrrolidin-1-ylpropoxy)phenyl]tetrahydro-2H-pyran-4-yl}methyl)-1H-imidazo[4,5-c]pyridine

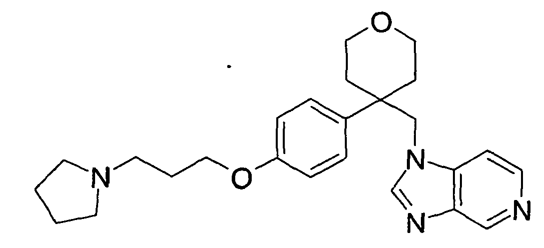

N*4*-{4-[4-(3-Pyrrolidin-1-yl-propoxy)-phenyl]-tetrahydro-pyran-4-ylmethyl}-pyridine-3,4-diamine (280mg, 0.68mmol), trimethylorthoformate (5mL) and formic acid (0.5mL) were stirred at 40°C for 18 hours then at 60°C for 24 hours, then at 80°C for 24 hours. The reaction mixture was concentrated *in vacuo* and the residue partitioned between 2M NaOH (30mL) and DCM (2 x 30mL). The organics were combined, dried over sodium sulphate, filtered and concentrated *in vacuo* to give a brown oil. The crude product was purified by flash chromatography on silica gel eluting with dichloromethane:methanol:ammonia (95:5:0.5 to 90:10:1 by volume) to provide the title compound as a clear viscous oil (185mg, 65%).
¹H NMR (400MHz, CDCl₃) δ 1.78-1.82 (m, 4H), 1.91-2.03 (m, 4H), 2.15-2.20 (m, 2H), 2.52 (m, 4H), 2.61 (t, 2H), 3.44 (m, 2H), 3.82 (m, 2H), 4.00 (t, 2H), 4.19 (s, 2H), 6.82 (d, 2H), 6.86-6.93 (m, 3H), 7.08 (s, 1H), 8.30 (d, 1H), 9.01 (s, 1H).
HRMS ESI⁺ m/z 421.2590 [MH]⁺.

### Example 248: 3-({4-[4-(3-pyrrolidin-1-ylpropoxy)phenyl]tetrahydro-2H-pyran-4-yl}methyl)-3H-imidazo[4,5-b]pyridine

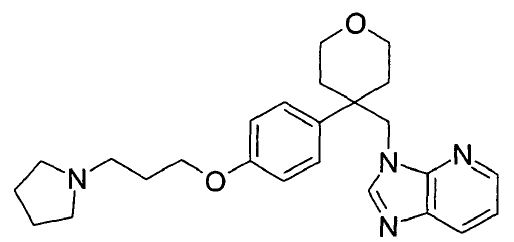

N*2*-{4-[4-(3-Pyrrolidin-1-yl-propoxy)-phenyl]-tetrahydro-pyran-4-ylmethyl}-pyridine-2,3-diamine (350mg, 0.85mmol), trimethylorthoformate (5mL) and formic acid (0.5mL) were stirred at 40°C for 3 hours. The reaction mixture was concentrated *in vacuo* and the residue partitioned between saturated NaHCO₃ solution (40mL) and DCM (2 x 40mL). The organics were combined, dried over sodium sulphate, filtered and concentrated *in vacuo* to give a brown oil. The crude product was purified by flash chromatography on silica gel eluting with dichloromethane:methanol:ammonia (97:3:0.3 to 93:7:0.7 by volume) to provide the title compound as a brown oil (255mg, 71 %).
¹H NMR (400MHz, CDCl₃) δ 1.78-1.82 (m, 4H), 1.99-2.10 (m, 6H), 2.57 (m, 4H), 2.64 (t, 2H), 3.56 (m, 2H), 3.92 (m, 2H), 4.03 (t, 2H), 4.40 (s, 2H), 6.82 (s, 1H), 6.90 (d, 2H), 7.02 (d, 2H), 7.20 (m, 1H), 7.99 (d, 1H), 8.38 (d, 1H).
HRMS ESI⁺ m/z 421.2586 [MH]⁺.

### Example 249: 1-({4-[4-(3-pyrrolidin-1-ylpropoxy)phenyl]tetrahydro-2H-pyran-4-yl}methyl)-1H-imidazo[4,5-b]pyridine

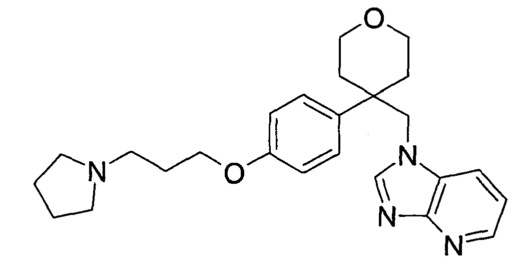

### Step 1:

Trifluoro-methanesulfonic acid 2-nitro-pyridin-3-yl ester (1.1g, 4.1mmo), {4-[4-(3-Pyrrolidin-1-ylpropoxy)phenyl]tetrahydropyran-4-yl} methylamine (1.3g, 4.1mmol) and Hunig's base (1.7mL, 9.8mmol) were stirred at 60°C for 72 hours. The mixture was diluted with DCM (30mL) and washed with water (10mL). The DCM layer was dried over sodium sulphate, filtered and concentrated *in vacuo* to give a dark brown oil. The crude product was purified by flash chromatography on silica gel eluting with dichloromethane:methanol:ammonia (98:2:0.2 to 90:10:1, by volume) to give a green oily residue after concentration *in vacuo.*

### Step 2:

To a solution of this residue in methanol (10mL) was added methyl viologen dichloride hydrate (5mg, 0.02mmol) and a solution of sodium dithionite (1.07g, 6.13mmol) and NaHCO₃ (940mg, 11.2mmol) in water (10mL). The mixture was stirred at room temperature for 10 minutes and then extracted with DCM (2 x 30mL). The organics were dried over sodium sulphate, filtered and concentrated *in vacuo* to give a dark green oil. The crude product was purified by flash chromatography on silica gel eluting with dichloromethane:methanol:ammonia (94:6:0.6 to 90:10:1, by volume) to give a dark green solid after concentration *in vacuo.*

### Step 3:

This green solid, trimethylorthoformate (5mL) and formic acid (0.5mL) were stirred at room temperature for 18 hours. The reaction mixture was concentrated *in vacuo* and the residue partitioned between saturated 2M NaOH solution (20mL) and DCM (2 x 20mL). The organics were combined, dried over sodium sulphate, filtered and concentrated *in vacuo* to give an orange oil. The crude product was purified by flash chromatography on silica gel eluting with dichloromethane:methanol:ammonia (96:4:0.4 to 90:10:1 by volume) to provide the title compound as a pale yellow oil (95mg, 6%).
¹H NMR (400 MHz, CDCl₃) δ 1.80 (m, 4H), 1.90-2.02 (m, 4H), 2.17 (m, 2H), 2.52 (m, 4H), 2.61 (t, 2H), 3.41 (t, 2H), 3.80 (m, 2H), 3.98 (t, 2H), 4.16 (s, 2H), 6.78 (d, 2H), 6.90 (d, 2H), 7.02 (m, 1H), 7.17 (d, 1H), 7.31 (s, 1H), 8.43 (m, 1H)
LRMS APCI⁺ m/z 421 [MH]⁺.

### Example 250: N-({4-[4-(3-pyrrolidin-1-ylpropoxy)phenyl]tetrahydro-2H-pyran-4-yl}methyl)pyridazin-4-amine

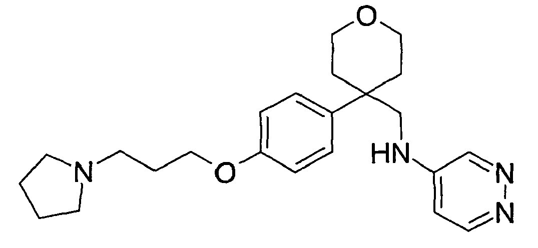

### Step 1:

A mixture of {4-[4-(3-Pyrrolidin-1-ylpropoxy)phenyl]tetrahydropyran-4-yl} methylamine (500mg, 1.57mmol), 3,4,5-Trichloro-pyridazine (403mg, 2.20mmol) and potassium carbonate (240mg, 1.73mmol) in acetonitrile (5mL) were stirred at room temperature for 72 hours. The mixture was concentrated *in vacuo* and partitioned between water (30mL) and DCM (2 x 30mL). The combined organics were dried over sodium sulphate, filtered and concentrated *in vacuo* to give a deep red oil. The crude product was purified by flash chromatography on silica gel eluting with dichloromethane:methanol:ammonia (96:4:0.4 to 93:70:7, by volume) to give a deep red oil after concentration *in vacuo.*

### Step 2:

A solution of this oil and sodium hydroxide (110mg, 2.7mmol) in ethanol (~5mL) was hydrogenated for 18 hours at room temperature at 50 psi in the presence of 10% Pd/C (60mg, 10%w/w). The reaction mixture was filtered over Arbocel® and rinsed with ethanol. The filtrate was concentrated *in vacuo* to give an oily residue. The crude product was purified by flash chromatography on silica gel eluting with dichloromethane:methanol:ammonia (94:6:0.6 to 90:10:1, by volume) to provide the title compound (230mg, 37%).
¹H NMR (400 MHz, CDCl₃) δ 1.80 (m, 4H), 1.86 (m, 2H), 2.01 (m, 2H), 2.20 (m, 2H), 2.55 (m, 4H), 2.63 (t, 2H), 3.25 (d, 2H), 3.55 (m, 2H), 3.80 (m, 2H), 3.90 (t, 1H), 4.02 (t, 2H), 6.33 (m, 1H), 6.96 (d, 2H), 7.20 (d, 2H), 8.40 (d, 1H), 8.57 (d, 1H)
HRMS ESI⁺ m/z 397.2591 [MH]⁺.

### Intermediate 99: 4-[4-(3-Morpholin-4-yl-propoxy)-phenyl]-tetrahydro-pyran-4-carboxylic acid hydrochloride

A solution of 4-[4-(3-morpholino-4-ylpropoxy)phenyl]tetrahydropyran-4-carbonitrile (2.0g, 6.05mmol) in concentrated hydrochloric acid (30mL, 10vol) was heated at reflux for 16h. The solution was cooled to room temperature affording a precipitate. This was filtered, washed with water (~5mL) and diethyl ether (~10mL) and then dried to give the title compound (1.34g, 57%) as a pale orange solid.
LRMS (APCI⁻) 348 (M-H⁺)⁻.
Microanalysis: Found: C, 58.16; H, 7.30; N, 3.45%. C₁₉H₂₈NO₅Cl. 0.3H₂O requires C, 58.32; H, 7.37; N, 3.58%.

### Example 251: N-methyl-4-[4-(3-morpholin-4-ylpropoxy)phenyl]tetrahydro-2H-pyran-4-carboxamide

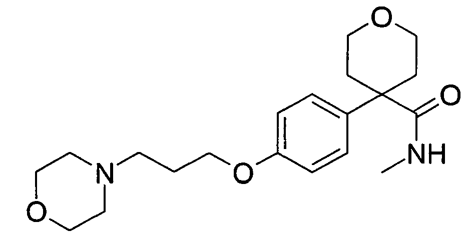

O-(1*H*-Benzotriazol-1-yl)-*N,N,N',N'*-tetramethyluronium hexafluorophosphate (640mg, 1.7mmol) was added to a solution of 4-[4-(3-Morpholin-4-yl-propoxy)-phenyl]-tetrahydro-pyran-4-carboxylic acid hydrochloride (500mg, 1.3mmol), methylamine hydrogen chloride (130mg, 1.9mmol) and Hunig's base (790µL, 4.5mmol) in *N,N*-dimethylformamide (5mL). The mixture was stirred at room temperature for 2 hours and then concentrated *in vacuo.* The residue was partitioned between 2M NaOH solution (20mL) and DCM (2x20mL). The organics were combined, dried over sodium sulphate, filtered and concentrated under reduced pressure. The crude product was purified by flash chromatography on silica gel eluting with dichloromethane:methanol:ammonia (95:5:0.5 to 90:10:1, by volume) to provide the title compound (355mg, 76%) as an off-white solid.
¹H NMR (400 MHz, CDCl₃) δ 1.93-2.00 (m, 2H), 2.01-2.08 (m, 2H), 2.37-2.40 (m, 2H), 2.48 (m, 4H), 2.50 (t, 2H), 2.70 (d, 3H), 3.67-3.80 (m, 8H), 4.02 (t, 2H), 6.88 (d, 2H), 7.22 (d, 2H).
LRMS APCI⁺ m/z 363 [MH]⁺.

### Example 252: 4-(3-{4-[4-(morpholin-4-ylcarbonyl)tetrahydro-2H-pyran-4-yl]phenoxy}propyl)morpholine

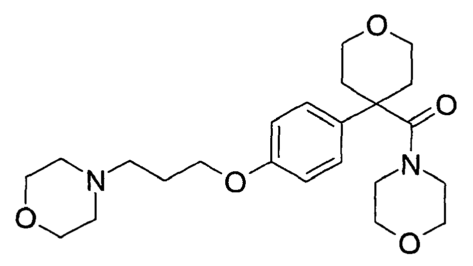

O-(1*H*-Benzotriazol-1-yl)-*N,N,N',N'*-tetramethyluronium hexafluorophosphate (200mg, 0.5mmol) was added to a solution of 4-[4-(3-Morpholin-4-yl-propoxy)-phenyl]-tetrahydro-pyran-4-carboxylic acid hydrochloride (160mg, 0.4mmol), morpholine (54 µL, 0.6mmol) and Hunig's base (180µL, 1mmol) in N,N-dimethylformamide (2mL). The mixture was stirred at room temperature for 3 hours and then concentrated *in vacuo.* The residue was partitioned between 2M NaOH solution (20mL) and DCM (2x20mL). The organics were combined, dried over sodium sulphate, filtered and concentrated under reduced pressure. The crude product was purified by flash chromatography on silica gel eluting with dichloromethane:methanol:ammonia (95:5:0.5 to 90:10:1, by volume) to provide the title compound (182mg, 100%) as a pale brown solid.
¹H NMR (400 MHz, CDCl₃) δ 1.93-2.04 (m, 4H), 2.20 (m, 2H), 2.42-2.55 (m, 6H), 3.22-3.43 (br m, 8H), 3.72 (t, 4H), 3.79 (m, 2H), 3.90 (m, 2H), 4.00 (t, 2H), 6.88 (d, 2H), 7.17 (d, 2H).
LRMS APCI⁺ m/z 419 [MH]⁺.

### Example 253: 4-(3-{4-[4-(morpholin-4-ylmethyl)tetrahydro-2H-pyran-4-yl]phenoxy}propyl)morpholine

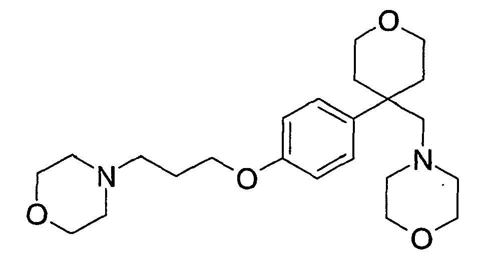

To a stirred solution of 4-(3-{4-[4-(morpholin-4-ylcarbonyl)tetrahydro-2H-pyran-4-yl]phenoxy}propyl)morpholine (160mg, 0.38 mmol) in THF (4 mL) at 0°C was added dropwise a solution of LiAlH₄ (1.0M solution in Et₂O, 1.15 mL, 1.15 mmol). The reaction mixture was stirred at 0°C for 20mins then heated at reflux for 1.5 hours. The reaction was then cooled to 0°C, water (0.10 ml) was added dropwise followed by sodium hydroxide (2.0M, 0.10 ml) and water (0.30 ml). The mixture was filtered through a short pad of celite, eluting with 1% methanol in dichloromethane (15 ml) and the organic washings concentrated *in vacuo.* The crude compound was purified by column chromatography on silica gel, eluting with dichloromethane:methanol:ammonia, 97:3:0.3 to 92:8:0.8, to give the title compound as a clear oil (125 mg, 81%).
¹H NMR (400MHz, CDCl₃) δ 1.83 (m, 2H), 1.95 (m, 2H), 2.07-2.16 (m, 6H), 2.40 (s, 2H), 2.43-2.50 (m, 4H), 2.52 (t, 2H), 3.51 (m, 6H), 3.76 (m, 6H), 4.01(t, 2H), 6.82 (d, 2H), 7.21 (d, 2H).
HRMS ESI⁺ m/z 405.2742 [MH]⁺.

### Example 254: N-methyl-1-{4-[4-(3-morpholin-4-ylpropoxy)phenyl]tetrahydro-2H-pyran-4-yl}methanamine

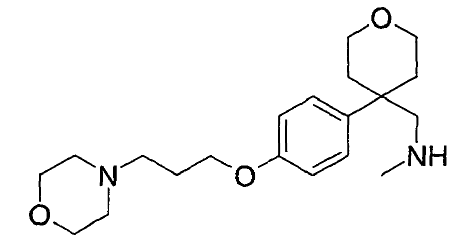

The title compound (252mg, 77%) was prepared from N-methyl-4-[4-(3-morpholin-4-ylpropoxy)phenyl]tetrahydro-2H-pyran-4-carboxamide and a solution of LiAlH₄ (1.0M solution in Et₂O) similarly to the procedure used for 4-(3-{4-[4-(morpholin-4-y(methyl)tetrahydro-2H-pyran-4-yl]phenoxy}propyl)morpholine.
¹H NMR (400 MHz, CD₃OD) δ 1.85-2.00 (m, 6H), 2.12 (m, 2H), 2.24 (s, 3H), 2.45 (m, 4H), 2.53 (t, 2H), 2.63 (s, 2H), 3.57 (m, 2H), 3.70-3.80 (m, 6H), 4.01 (t, 1H), 6.88 (d, 2H), 7.20 (d, 2H).
HRMS ESI⁺ m/z 349.2477 [MH]⁺.

### Example 255: N-methyl-2-(methylsulfonyl-N-({4-[4-(3-morpholin-4-ylpropoxy)phenyl]tetrahydro-2H-pyran-4-yl}methyl)ethanamine

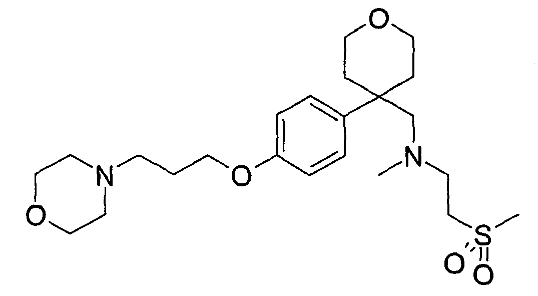

To a stirred solution of N-methyl-1-{4-[4-(3-morpholin-4-ylpropoxy)phenyl]tetrahydro-2H-pyran-4-yl}methanamine (230mg, 0.66mmol) in methanol (2mL) was added methanesulfonyl-ethene (64µL, 0.72mmol) and the mixture stirred at room temperature for 18 hours. The mixture was concentrated in *vacuo* and triturated with diethyl ether to give the title compound as a white solid (274mg, 91%).
¹H NMR (400MHz, CDCl₃) δ 1.81 (m, 2H), 1.97 (m, 2H), 2.04 (s, 3H), 2.13 (m, 2H), 2.42-2.58 (m, 8H), 2.62 (t, 2H), 2.74 (s, 3H), 2.81 (t, 2H), 3.50 (m, 2H), 3.70-3.80 (m, 6H), 4.00 (t, 2H), 6.86 (d, 2H), 7.20 (d, 2H).
HRMS ESI⁺ m/z 455.2570 [MH]⁺.
Microanalysis: Found: C, 60.61; H, 8.47; N, 6.09%. C₂₃H₃₈N₂O₅S requires C, 60.76; H, 8.42; N, 6.16%.

### Example 256: 6-{4-[4-(3-pyrrolidin-1-ylpropoxy)phenyl]tetrahydro-2H-pyran-4-yl}-5H-pyrrolo[2,3-b]pyrazine

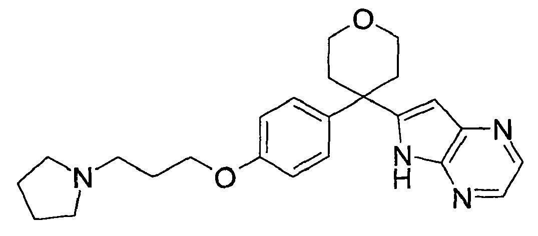

A solution of 2-methyl-pyrazine (580µL, 6.4mmol) in THF (4mL) was added to 4.7mL of 1.5M lithium diisopropylamide : THF complex in hexanes at -40°C and the resulting deep red solution stirred at -40°C for 0.5 hours. A solution of 4-[4-(3-Pyrrolidin-1-yl-propoxy)-phenyl]-tetrahydro-pyran-4-carbonitrile (1.00g, 3.2mmol) in THF (5mL) was added at -40°C and the reaction mixture stirred at this temperature for 0.5 hours, then at room temperature for 18 hours. A further 1 equivalent of lithium diisopropylamide complex was then added and the reaction mixture stirred at reflux for 24 hours.. The mixture was cooled to room temperature, water (10mL) added and the mixture concentrated *in vacuo.* The residue was partitioned between water (60mL) and DCM (2 x 60mL). The organics were combined, dried over sodium sulphate, filtered and concentrated under reduced pressure. The crude product was purified by flash chromatography on silica gel eluting with dichloromethane:methanol:ammonia (95:5:0.5 to 90:10:1, by volume) to provide the title compound (65mg, 6%) as a brown oil.
¹H NMR (400 MHz, CDCl₃) δ 1.74-1.80 (m, 6H), 2.00 (m, 2H), 2.42-2.55 (m, 6H), 2.60 (t, 2H), 3.80 (m, 4H), 4.00 (t, 2H), 6.60 (s, 1H), 6.86 (d, 2H), 7.21 (d, 2H), 8.05 (d, 1H), 8.39 (d, 1H).
HRMS ESI⁺ m/z 407.2437 [MH]⁺.

### Example 257: 4-[4-(3-azetidin-1-ylpropoxy)phenyl]tetrahydro-2H-pyran-4-carbonitrile

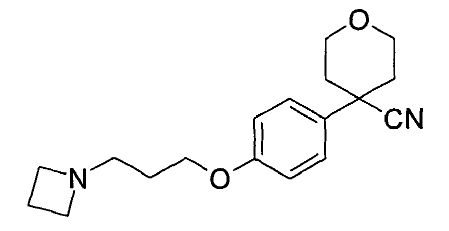

A mixture of 4-[4-(3-chloro-propoxy)-phenyl]-tetrahydropyran-4-carbonitrile and 4-[4-(3-bromo-propoxy)-phenyl]-tetrahydropyran-4-carbonitrile (7.5g, 27mmol), K₂CO₃ (9.6g, 70mmol) and azetidine hydrochloride (4.0g, 43mmol) in acetonitrile (80mL) was heated at 40°C for 18 hours. The mixture was concentrated *in vacuo* then partitioned between water (150mL) and ethyl acetate (150mL). The organic layer was extracted with 2M HCl solution (2 x 100mL) and the combined acid layers basified with 40% potassium hydroxide solution, then extracted with DCM (2 x 150mL). The DCM layers were combined, dried over sodium sulphate, filtered and concentrated under reduced pressure to give the title compound (1.1g, 57%) as a clear, viscous oil.
¹H NMR (400 MHz, DMSO) δ 1.80 (quintet, 2H), 2.00-2.14 (m, 6H), 2.58 (t, 2H), 3.20 (t, 4H), 3.88 (m, 2H), 4.00-4.11 (m, 4H), 6.90 (d, 2H), 7.38 (d, 2H);
LRMS (APCl⁺) m/z 301 (MH)⁺.

### Example 258: 4-({4-[4-(3-azetidin-1-ylpropoxy)phenyl]tetrahydro-2H-pyran-4-yl}carbonyl)morpholine

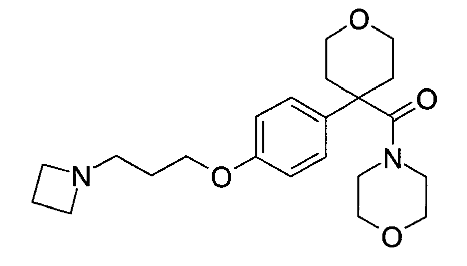

### Step 1:

A solution of 4-[4-(3-azetidin-1-yipropoxy)phenyl]tetrahydro-2H-pyran-4-carbonitrile (1.1g, 3.7mmol) in concentrated hydrochloric acid (15mL, 10vol) was heated and stirred at reflux for 24 hours. The solution was cooled to room temperature and then concentrated *in vacuo* affording a brown oily solid. Hunig's base (6mL) was added and the mixture concentrated *in vacuo* to give a brown oily residue.

### Step2:

O-(1*H*-Benzotriazol-1-yl)-*N,N,N',N'*-tetramethyluronium hexafluorophosphate (1.7g, 4.5mmol) was added to a solution of this residue, morpholine (600µL, 6.9mmol) and Hunig's base (780µL, 4.5mmol) in *N,N-*dimethylformamide (5mL). The mixture was stirred at room temperature for 72 hours and then concentrated *in vacuo*. The residue was partitioned between water (50mL) and DCM (2 x 50mL). The organics were combined, dried over sodium sulphate, filtered and concentrated under reduced pressure. The crude product was purified by flash chromatography on silica gel eluting with dichloromethane:methanol:ammonia (95:5:0.5 to 90:10:1, by volume) to provide the title compound (128mg, 9%) as a pale pink foam after trituration with diethyl ether.
¹H NMR (400 MHz, CDCl₃) δ 1.93-2.04 (m, 4H), 2.20 (m, 2H), 2.51-2.60 (m, 2H), 3.22-3.45 (br m, 10H), 3.78 (m, 2H), 3.86 (m, 2H), 4.02 (t, 2H), 4.13 (t, 4H), 6.88 (d, 2H), 7.18 (d, 2H).
HRMS ESI⁺ m/z 389.2426 [MH]⁺.

### Example 259: 4-({4-[4-(3-azetidin-1-ylpropoxy)phenyl]tetrahydro-2H-pyran-4-yl}methyl)morpholine

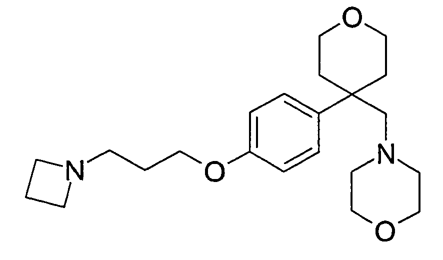

To a stirred solution of 4-({4-[4-(3-azetidin-1-ylpropoxy)phenyl]tetrahydro-2H-pyran-4-yl}carbonyl)morpholine (120mg, 0.31mmol) in THF (1 mL) at 0°C was added dropwise a solution of LiAlH₄ (1.0M solution in Et₂O, 1.00mL, 1.00mmol). The reaction mixture was stirred at 0°C for 20mins then heated at reflux for 3 hours. The reaction was then cooled to 0°C, water (0.10 ml) was added dropwise followed by sodium hydroxide (2.0M, 0.10 ml) and water (0.30 ml). The mixture was filtered through a short pad of celite, eluting with 1% methanol in dichloromethane (15 ml) and the organic washings concentrated *in vacuo.* The crude compound was purified by column chromatography on silica gel, eluting with dichloromethane:methanol:ammonia, 97:3:0.3 to 94:6:0.6, to give the title compound as a clear oil (25 mg, 22%).
¹H NMR (400MHz, CDCl₃) δ 1.78-1.90 (m, 8H), 2.08 (m, 2H), 2.13 (m, 4H), 2.38 (s, 2H), 2.59 (t, 2H), 3.20 (t, 2H), 3.51 (m, 6H), 3.76 (m, 2H), 4.00 (t, 2H), 6.82 (d, 2H), 7.21 (d, 2H).
LRMS APCI⁺ m/z 375 [MH]⁺.

### Intermediate 100: 4-(4-{[1-(diphenylmethyl)azetidin-3-yl]methoxy})phenyl)tetrahydro-2H-pyran-4-carbonitrile

4-(4-Hydroxy-phenyl)-tetrahydro-pyran-4-carbonitrile (6.18g, 30.4mmol), (1-benzhydryl-azetidin-3-yl)-methanol (7g, 28mmol), PPh₃ (8g, 30.4mmol), and DIAD (69mL, 30.4mmol), were reacted together similarly to general procedure C. The crude material was purified by flash chromatography on silica gel eluting with pentane:ethyl acetate:diethylamine (50:45:5 to 0:95:5) to provide the title compound (5.03g, 41%) as a yellow solid.
LRMS APCI⁺ m/z 439 [MH]⁺.

### Intermediate 101: 4-[4-(azetidin-3-ylmethoxy)phenyl]tetrahydro-2H-pyran-4-carbonitrile

4-(4-{[1-(diphenylmethyl)azetidin-3-yl]methoxy}phenyl)tetrahydro-2H-pyran-4-carbonitrile (0.605g, 1.38mmol), palladium hydroxide (0.08g), concentrated hydrochloric acid (0.115mL, 1.38mmol), and ethanol (8mL) were combined and hydrogenated for 18 hours at 40°C at 40psi. The mixture was filtered through Arbocel® and rinsed with ethanol. The filtrate was concentrated *in vacuo.* The residue was dissolved in DCM (50mL) and washed with 10% aqueous sodium carbonate (20mL). The organics were dried over sodium sulphate, filtered and concentrated *in vacuo* to provide a green oil. This was purified by flash chromatography on silica gel eluting with dichloromethane:methanol:ammonia (95:5:0.5 to 90:10:1) to provide the title compound (0.145g, 39%) as a yellow oil.
LRMS APCI⁺ m/z 273 [MH]⁺.

### Example 260: 4-{4-[(1-cyclobutylazetidin-3-yl)methoxy]phenyl}tetrahydro-2H-pyran-4-carbonitrile

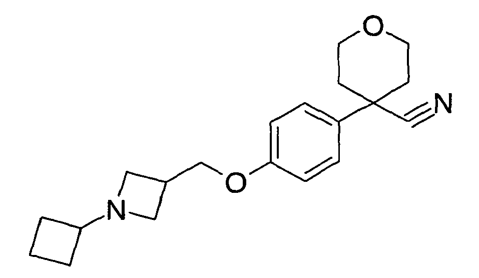

4-[4-(azetidin-3-ylmethoxy)phenyl]tetrahydro-2H-pyran-4-carbonitrile (0.2g, 0.73mmol) was dissolved in THF (2mL). Cyclobutanone (0.06mL, 0.80mmol) and acetic acid (0.042mL, 0.73mmol) were added. The mixture was stirred at room temperature for 30 minutes. Sodium triacetoxyborohydride (0.311g, 1.47mmol) was added. The mixture was stirred at room temperature for 18 hours. The reaction mixture was diluted with 10% aqueous sodium carbonate (5mL) then extracted with DCM (2 x 50mL). The organics were combined, dried over sodium sulphate, filtered and concentrated *in vacuo* to provide a clear oil. This was purified by flash chromatography on silica gel eluting with dichloromethane:methanol:ammonia (96:4:0.4 to 95:5:0.5) to provide the title compound (0.194g, 81%) as a clear oil.
¹H NMR (400MHz, CDCl₃) δ 1.60-1.70 (m, 2H), 1.70-1.80 (m, 2H), 1.80-1.90 (m, 2H), 1.95-2.00 (m, 2H), 2.0-2.15 (m, 2H), 2.9 (m, 1H), 3.1 (t, 2H), 3.16 (m, 1H), 3.4 (t, 2H), 3.9 (m, 2H), 4.10 (m, 4H), 6.90 (d, 2H), 7.40 (d, 2H)
HRMS ESI⁺ m/z 327.2059 [MH]⁺.

### Example 261: 1-(4-{4-[(1-cyclobutylazetidin-3-yl)methoxy]phenyl}tetrahydro-2H-pyran-4-yl)methanamine

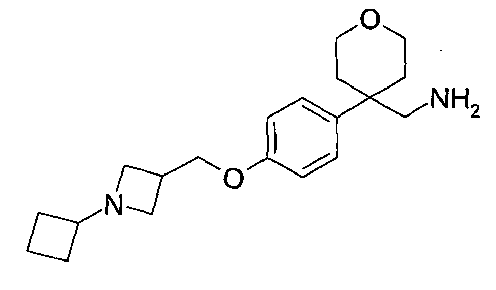

4-{4-[(1-cyclobutylazetidin-3-yl)methoxy]phenyl}tetrahydro-2H-pyran-4-carbonitrile (0.29g, 0.89mmol), was dissolved in THF (5mL). The solution was cooled to 0°C under nitrogen. Lithium aluminiumhydride (2.7mL, 1.0M solution in diethyl ether) was added dropwise. The reaction was stirred at 0°C for 30 minutes, then warmed up to ambient temperature for 4 hours under a nitrogen atmosphere until complete. The reaction was cooled to 0°C, water (0.1mL) was added dropwise followed by sodium hydroxide (2.0M, 0.1mL) and water (0.3mL). The mixture was filtered (using dichloromethane:methanol (90:10) ) through celite. The organics were dried over sodium sulphate, filtered and concentrated *in vacuo* to provide a clear oil. This was purified by flash chromatography on silica gel eluting with dichloromethane:methanol:ammonia (96:4:0.4 to 90:10:1) to provide the title compound (0.175g, 59%) as a clear colourless oil.
¹H NMR (400MHz, CDCl₃) δ 1.60-1.70 (m, 2H), 1.70-1.85 (m, 2H), 1.85-1.95 (m, 2H), 1.95-2.06 (m, 2H), 2.1-2.18 (m, 2H), 2.79 (s, 2H), 2.9 (m, 1H), 3.1 (t, 2H), 3.2 (m, 1H), 3.48 (m, 2H), 3.54 (m, 2H), 3.8 (m, 2H), 4.10 (d, 2H), 6.9 (d, 2H), 7.2 (d, 2H)
HRMS ESI⁺ m/z 331.2374 [MH]⁺.

### Example 262: N-[(4-{4-[(1-cyclobutylazetidin-3-yl)methoxy]phenyl}tetrahydro-2H-pyran-4-yl)methyl]pyridin-2-amine

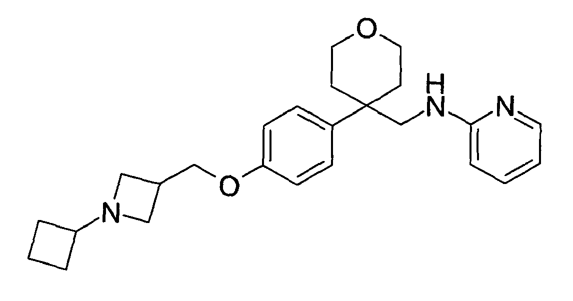

1-(4-(4-[(1-cyclobutylazetidin-3-yl)methoxy]phenyl)tetrahydro-2H-pyran-4-yl)methanamine (0.175g, 0.53mmol) was dissolved in toluene (5mL), 2-bromopyridine (0.051mL, 0.53mmol), 2,2'-bis(diphenylphosphino)-1,1'binapthyl (0.013g, 0.02mmol) and sodium tert-butoxide (0.076g, 0.79mmol) were added. The reaction mixture was purged with nitrogen, tris(dibenzylideneacetone)dipalladium(0) (0.011g, 0.01mmol) was added. The mixture was heated under nitrogen at 70°C for 18 hours. The reaction mixture was diluted with water (20mL) and extracted with DCM (2 x 30mL). The combined organics were dried over sodium sulphate, filtered and concentrated *in vacuo* to provide a yellow oil. This was purified by HPLC eluting with methanol: 10nM ammonium formate (aq) (1:1), fractions were combined and concentrated *in vacuo* to leave a clear oil. This was dissolved in DCM (50mL) and washed with saturated sodium hydrogen carbonate solution in water (10mL). The organic was dried over sodium sulphate, filtered and concentrated *in vacuo* to provide the title compound (0.016g, 7%) as a clear colourless oil.
¹H NMR (400MHz, CDCl₃) δ 1.60-1.70 (m, 2H), 1.70-1.80 (m, 2H), 1.80-1.92 (m, 2H), 1.92-2.0 (m, 2H), 2.18 (m, 2H), 2.90 (m, 1H), 3.10 (t, 2H), 3.15 (m, 1H), 3.40 (t, 2H), 3.50 (d, 2H), 3.60 (m, 2H), 3.80 (m, 2H), 4.0 (m, 1H), 4.10 (d, 2H), 6.22 (d, 1H), 6.50 (d, 1H), 6.90 (d, 2H), 7.28 (d, 2H), 7.32 (t, 1H), 8.0 (d, 1H)
HRMS ESI⁺ m/z 408.2641 [MH]⁺.

### Intermediate 102: N-[(dimethylamino)methylene]-4-[4-(3-pyrrolidin-1-ylpropoxy)phenyl]tetrahydro-2H-pyran-4-carboxamide

4-[4-(3-Pyrrolidin-1-ylpropoxy)phenyl]tetrahydro-2H-pyran-4-carboxylic acid amide (0.561g, 1.69mmol) and *N,N*-dimethylformamide dimethyl acetal (5mL) were combined and heated at reflux for 4 hours. The reaction mixture was concentrated *in vacuo* and azeotroped with toluene (2 x 10mL) to provide the title compound (0.655g, 100%) as a brown solid.
¹H NMR (400MHz, CDCl₃) δ 1.80-1.85 (m, 4H), 1.85-1.95 (m, 2H), 2.0-2.10 (m, 2H), 2.6-2.78 (m, 8H), 3.0 (s, 3H), 3.05 (s, 3H), 3.60 (m, 2H), 3.90 (m, 2H), 4.0 (t, 2H), 6.80 (d, 2H), 7.35 (d, 2H), 8.30 (s, 1H)
LRMS APCI⁺ m/z 388 [MH]⁺.

### Example 263: 3-{4-[4-(3-pyrrolidin-1-ylpropoxy)phenyl]tetrahydro-2H-pyran-4-yl}-1H-1,2,4-triazole

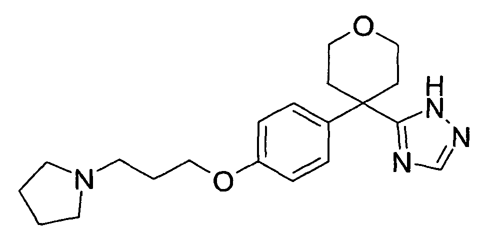

N-[(dimethylamino)methylene]-4-[4-(3-pyrrolidin-1-ylpropoxy)phenyl]tetrahydro-2H-pyran-4-carboxamide (0.15g, 0.39mmol), hydrazine hydrate (0.048mL, 0.98mmol) and acetic acid (1mL) were combined and heated at 100°C for 1.5 hours. The reaction mixture was concentrated *in vacuo.* The residue was dissolved in dichloromethane (100mL) and washed with 10% aqueous sodium carbonate (20mL). The organic was dried over sodium sulphate and evaporated to provide a clear oil. This was purified by flash chromatography on silica gel eluting with dichloromethane:methanol:ammonia (92:8:0.8 to 90:10:1) to produce a clear oil which was triturated with diethyl ether (3mL), a white solid was isolated as the title compound (0.034g, 24%).
¹H NMR (400MHz, CDCl₃) δ 1.82-1.95 (m, 4H), 2.0-2.10 (m, 2H), 2.30 (m, 2H), 2.60-2.70 (m, 2H), 2.70-2.80 (m, 6H), 3.62 (t, 2H), 3.89 (m, 2H), 3.95 (t, 2H), 6.80 (d, 2H), 7.20 (d, 2H), 7.95 (d, 1H)
HRMS ESI⁺ m/z 357.2279 [MH]⁺.

### Example 264: 1-methyl-5-{4-[4-(3-pyrrolidin-1-ylpropoxy)phenyl]tetrahydro-2H-pyran-4-yl}-1H-1,2,4-triazole

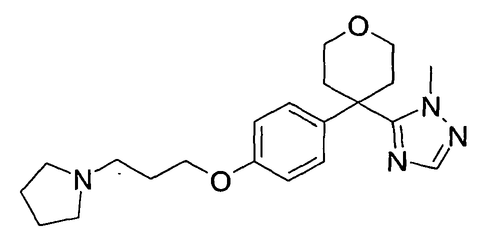

N-[(dimethylamino)methylene]-4-[4-(3-pyrrolidin-1-ylpropoxy)phenyl]tetrahydro-2H-pyran-4-carboxamide (0.34g, 0.88mmol), methyl hydrazine (0.116mL, 2.19mmol) and acetic acid (1mL) were combined and heated at 100°C for 2 hours. The reaction mixture was diluted with water (10mL), solid sodium carbonate was added until the mixture was pH9. Dichloromethane (2 x 50mL) was used to extract. The combined organics were dried over sodium sulphate, filtered and concentrated *in vacuo* to provide a yellow oil. This was purified by flash chromatography on silica gel eluting with dichloromethane:methanol:ammonia (96:4:0.4 to 90:10:1) to produce the title compound (0.065g, 20%) as a white solid.
¹H NMR (400MHz, CDCl₃) δ 1.83-1.93 (m, 4H), 2.05-2.16 (m, 2H), 2.30 (m, 2H), 2.48 (m, 2H), 2.60-2.85 (m, 6H), 3.37 (s, 3H), 3.80 (m, 2H), 3.90 (m, 2H), 4.0 (t, 2H), 6.85 (d, 2H), 7.07 (d, 2H), 7.88 (s, 1H)
HRMS ESI⁺ m/z 371.2434 [MH]⁺.

### Intermediate 103: N'-formyl-4-[4-(3-pyrrolidin-1-ylpropoxy)phenyl]tetrahydro-2H-pyran-4-carbohydrazide

4-[4-(3-Pyrrolidin-1-ylpropoxy)phenyl]tetrahydro-2*H*-pyran-4-carboxylic acid (0.75g, 0.225mmol), formic hydrazide (0.162g, 0.27mmol), 1-hydroxybenzotriazole (0.304g, 0.225mmol), diisopropylethylamine (1.18mL, 0.68mmol), 1-(3-dimethylaminopropyl)-3-ethylcarbodiimide hydrochloride (0.431g, 0.225mmol) in dichloromethane (10mL) was stirred at room temperature for 18 hours. The reaction mixture was diluted with dichloromethane (100mL) and washed with water (2 x 20mL) The organics were dried over sodium sulphate, filtered and concentrated *in vacuo* to provide a cream solid. This was purified by flash chromatography on silica gel eluting with dichloromethane:methanol:ammonia (95:5:0.5 to 90:10:1) to provide the title compound (0.145g, 17%) as a clear oil.
LRMS ESI⁺ m/z 376 [MH]⁺, ESI⁻ m/z 374 [M-H⁺]⁻

### Example 265: 2-{4-[4-(3-pyrrolidin-1-ylpropoxy)phenyl]tetrahydro-2H-pyran-4-yl}-1,3,4-oxadiazole

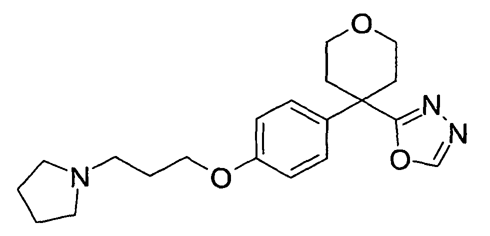

Triphenylphosphine (0.203g, 0.78mmol), iodine (0.198g, 0.78mmol) and triethylamine (0.217mL, 1.56mmol) were dissolved in dichloromethane (5mL). The mixture was stirred at room temperature for 10 minutes. N'-formyl-4-[4-(3-pyrrolidin-1-ylpropoxy)phenyl]tetrahydro-2H-pyran-4-carbohydrazide (0.145g, 0.39mmol) in dichloromethane (5mL) was added. The mixture was stirred at room temperature for 18 hours. The reaction mixture was diluted with dichloromethane (100mL) and washed with water (25mL). The organic was dried over sodium sulphate, filtered and concentrated *in vacuo* to provide a yellow oil. This was purified using preparative HPLC on Fraction-Lynx® eluting with water:acetonitrile:trifluoroacetic acid (95:5:0.1) to provide the title compound (0.028g, 20%) as a clear oil.
¹H NMR (400MHz, CDCl₃) δ1.78-1.85 (m, 4H), 2.05 (m, 2H), 2.30 (m, 2H), 2.56-2.60 (m, 4H), 2.60-2.70 (m, 4H), 3.59 (m, 2H), 3.90-4.05 (m, 4H), 6.90 (d, 2H), 7.20 (d, 2H), 8.30 (s, 1H).
HRMS ESI⁺ m/z 358.2117 [MH]⁺.

### Intermediate 104: 2-{4-[4-(3-pyrrolidin-1-ylpropoxy)phenyl]tetrahydro-2H-pyran-4-yl}-4,5-dihydro 1H-imidazole

4-[4-(3-Pyrrolidin-1-yl-propoxy)-phenyl]-tetrahydro-pyran-4-carbonitrile (0.5g, 3.1mmol), ethylenediamine (1.5mL, 22mmol), copper (II) acetate (0.07g, 0.3mmol) and N-methylpyrrolidinone (1.5mL) were combined and subjected to microwave (Smith Personal Synthesiser) irradiation at 220°C for 1 hour. The reaction mixture was concentrated *in vacuo* to provide a brown oil. This was columned by flash chromatography on silica gel eluting with dichlromethane:methanol:ammonia (94:6:0.6 to 85:15:1.5) to provide the title compound (0.157g, 31%) as a yellow oil.
LRMS ESI⁺ m/z 358 [MH]⁺.

### Example 266: 2-{4-[4-(3-pyrrolidin-1-ylpropoxy)phenyl]tetrahydro-2H-pyran-4-yl}-1H-imidazole

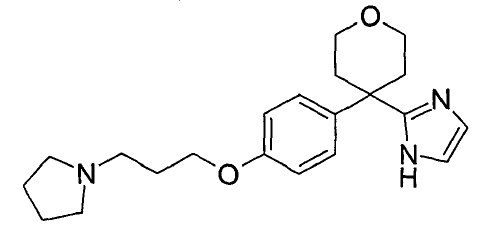

Dimethylsulphoxide (0.156mL, 2.19mmol) and dichloromethane (15mL) were combined and cooled to - 78°C under nitrogen, oxalyl chloride (0.191 mL, 2.19mmol) was added and the reaction mixture was stirred at -78°C under nitrogen for 1 hour. A solution of 2-{4-[4-(3-pyrrolidin-1-ylpropoxy)phenyl]tetrahydro-2H-pyran-4-yl}-4,5-dihydro-1H-imidazole (0.314g, 0.88mol) in dichloromethane (15mL) was added slowly. The reaction was stirred at -78°C for 1 hour. Triethylamine (0.611mL, 4.4mmol) was then added and the reaction was stirred for a further hour. The reaction mixture allowed to warm to room temperature for 1 hour. The reaction mixture was diluted with dichloromethane (100mL) and washed with an aqueous ammonia solution (20mL). The organic was dried over sodium sulphate, filtered and concentrated *in vacuo* to provide a yellow oil. This was purified by flash chromatography on silica gel eluting with dichloromethane:methanol:ammonia (96:4:0.4 to 90:10:1) to provide a yellow oil, which was triturated with diethyl ether. A cream solid was filtered to provide the title compound (0.07g, 22%)
¹H NMR (400MHz, CDCl₃) δ 1.80 (m, 4H), 2.05 (m, 2H), 2.30 (m, 2H), 2.50 (m, 2H), 2.60-2.78 (m, 6H), 3.73 (m, 2H), 3.83 (m, 2H) (4.0 (t, 2H), 6.85 (d, 2H), 6.95 (s, 2H), 7.15 (d, 2H)
HRMS ESI⁺ m/z 356.2327 [MH]⁺.

### Example 267: N-[(4-{4-[(1-isopropylpiperidin-4-yl)oxy]phenyl}tetrahydro-2H-pyran-4-yl)methyl]pyridazin-4-amine

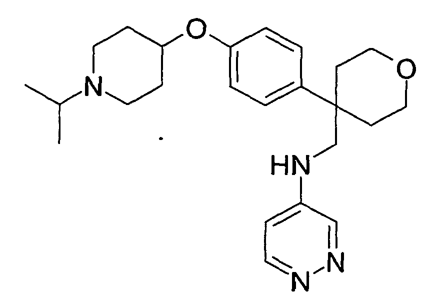

### Step 1:

(4-{4-[(1-isopropylpiperidin-4-yl)oxy]phenyl}tetrahydro-2*H*-pyran-4-yl)methylamine (0.5g, 1.5mmol), 3,4,5-trichloropyridazine (0.275g, 1.5mmol), potassium carbonate (0.690g, 5mmol) and acetonitrile (5mL) were combined and stirred at room temperature for 20 hours. The reaction mixture was partitioned between water (20mL) and dichloromethane (2 x 50 mL). The combined organics were dried over sodium sulphate, filtered and concentrated *in vacuo* to provide an orange oil. This was purified by flash chromatography eluting with dichloromethane:methanol:ammonia (100:0:0 to 90:10:1) to provide a brown oil.

### Step 2:

The product from step 1 (0.162g, 0.34mmol) and sodium hydroxide (0.033g, 0.82mmol) were dissolved in ethanol (3mL). 10% Palladium on charcoal (0.02g) was added. The mixture was hydrogenated for 18 hours at room temperature at 50 psi. The reaction mixture was filtered through Arbocel®, the filtrate was concentrated *in vacuo* to provide a yellow oil. This was purified by flash chromatography on silica gel eluting with dichloromethane:methanol:ammonia (94:6:0.6 to 90:10:1) to provide the title compound (0.08g, 60%) as a yellow oil.
¹H NMR (400MHz, CDCl₃) δ 1.08 (d, 6H), 1.78-1.90 (m, 4H), 2.05 (m, 2H), 2.20 (m, 2H), 2.43 (m, 2H), 2.72-2.82 (m, 3H), 3.28 (d, 2H), 3.55 (m, 2H), 3.78 (m, 2H), 4.30 (m, 1H), 4.35 (m, 1H), 6.25 (d, 1H), 6.90 (d, 2H), 7.20 (d, 2H), 8.40 (s, 1H), 8.48 (d, 1H)
HRMS ESI⁺ m/z 411.2749 [MH]⁺.

### Intermediate 105: 4-[4-(3-[1,4]Oxazepan-4-yl-propoxy)-phenyl]-tetrahydro-pyran-4-carbonitrile

4-[4-(3-Chloro-propoxy)-phenyl]-tetrahydro-pyran-4-carbonitrile (5.3 g, 19 mmol) was taken in acetonitrile (30 ml), to which was added Hunig's base (6 g, 47 mmol) and homo-morpholine hydrogen chloride (2.8 g, 20 mmol). This solution was heated at reflux for 18 hours. The solvent was removed *in vacuo* and the residue purified by silica gel column chromatography, eluting with a gradient of DCM:MeOH:NH₃ (from 98:2:0.2 to 94:6:0.6) to afford the title compound as a yellow oil (3.35 g, 51 %).
HRMS ESI⁺ m/z 345.2166 [MH]⁺

### Intermediate 106: C-{4-[4-(3-[1,4]Oxazepan-4-yl-propoxy)-phenyl]-tetrahydro-pyran-4-yl}-methylamine

4-[4-(3-[1,4]Oxazepan-4-yl-propoxy)-phenyl]-tetrahydro-pyran-4-carbonitrile (900 mg, 26 mmol) was dissolved in THF (5ml) and cooled to 0°C under nitrogen. Lithium aluminium hydride (1M in EtO₂, 7.8 ml, 78mmol) was added dropwise and left to stir for 10 minutes before heating to reflux for 18 hours. The solution was cooled to 0°C before quenching with water (0.78 ml), followed by sodium hydroxide (2M, 0.78ml) and finally water (2.4 ml). The resulting slurry was filtered through Arbocel®, washing through with DCM (2 x 10ml). The filtrate was concentrated *in vacuo* to afford the title compound as a colourless oil (650 mg, 72%).
HRMS ESI⁺ m/z 349.2480 [MH]⁺

### Example 268: 4-(3-{4-[4-(morpholin-4-ylmethyl)tetrahydro-2H-pyran-4-yl]phenoxy}propyl)-1,4-oxazepane

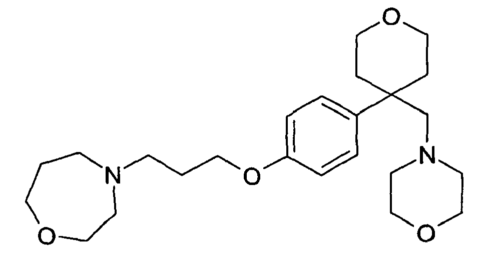

C-{4-[4-(3-[1,4]Oxazepan-4-yi-propoxy)-phenyl]-tetrahydro-pyran-4-yl}-methylamine (150 mg, 0.43 mmol) and 1-Bromo-2-(2-bromo-ethoxy)-ethane (110 mg, 0.47 mmol) were dissolved in acetonitrile (10 ml) and K₂CO₃ (130 mg, 0.95 mmol) was added. The solution was heated at 60°C for 18 hours before filtering the solution to remove to the solid K₂CO₃. The filtrate was concentrated *in vacuo* before purifying using column chromatography, eluting with a gradient of DCM:MeOH:NH₃ (from 99:1:0.1 to 90:10:1) to afford the title compound as a colourless oil (75 mg, 42%).
¹H NMR (400MHz, CD₃OD) δ 1.80-2.00 (m, 6H), 2.11-2.20 (m, 6H), 2.41 (s, 2H), 2.69-2.79 (m, 6H), 3.48 (m, 6H), 3.72-3.80 (m, 6H), 4.02 (t, 2H)6.87 (d, 2H), 7.15 (d, 2H)
HRMS ESI⁺ m/z 419.2896 [MH]⁺

### Example 269: N-[(4-{4-[3-(1,4-oxazepan-4-yl)propoxy]phenyl}tetrahydro-2H-pyran-4-*yl)methyl]pyridin-2-amine

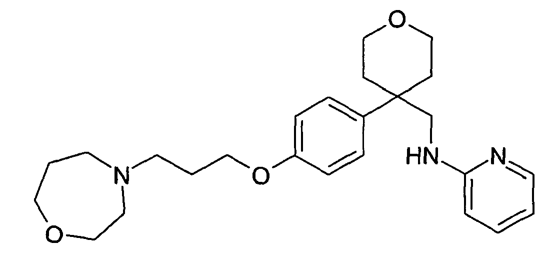

C-{4-[4-(3-[1,4]Oxazepan-4-yl-propoxy)-phenyl]-tetrahydro-pyran-4-yl}methylamine (210 mg, 0.48 mmol) was added to a solution of 2-bromopyridine (70 mg, 0.42 mmol), Pd₂(dba)₃ (91 mg, 0.1 mmol), BINAP (126 mg, 2 mmol) and sodium *tert*-butoxide (56 mg, 5.7 mmol) in toluene (3 ml). This solution was heated in the microwave (Smith Personal Synthesiser) at 110°C for 15 minutes. The solution was filtered through Celite and the filtrate was concentrated *in vacuo*. The residue was purified by column chromatography, eluting with a gradient of DCM:MeOH:NH₃ (from 99:1:0.1 to 95:5:0.5) to afford the title compound as a yellow oil (120 mg, 59%).
¹H NMR (400MHz, CD₃OD) δ 1.88-2.00 (m, 6H), 2.17 (m, 2H), 2.68-2.79 (m, 6H), 3.43 (s, 2H), 3.50 (m, 2H), 3.76 (m, 2H), 3.80 (m, 4H), 4.03 (t, 2H), 6.39 (d, 1H), 6.44 (t, 1H), 6.91 (d, 2H), 7.33 (m, 3H), 7.81 (d, 1H)
HRMS ESI⁺ m/z 426.2741 [MH]⁺

### Example 270: 4-(3-{4-[4-(piperazin-1-ylcarbonyl)tetrahydro-2H-pyran-4-yl]phenoxy}propyl)-1,4-oxazepane

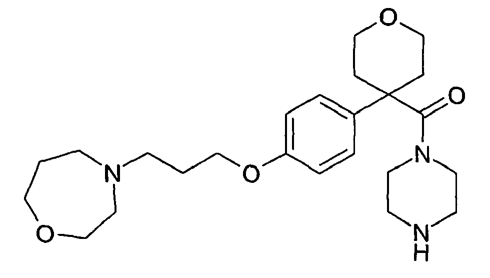

### Step1:

4-[4-(3-[1,4]Oxazepan-4-yl-propoxy)-phenyl]-tetrahydro-pyran-4-carbonitrile (1 g, 29 mmol) was dissolved in concentrated hydrochloric acid (15ml) and heated at 90°C for 24 hours, and the solvent was then removed *in vacuo*. 2-tert butyl 1,1,2,2 tetramethyl guanidine (2 ml) and water (1ml) was then added to the residue and concentrated *in vacuo* and azeotroped with toluene (2 x 4ml) to afford crude 4-[4-(3-[1,4]Oxazepan-4-yl-propoxy)-phenyl]-tetrahydro-pyran-4-carboxylic acid guanidine salt (3 g) an orange oil.

### Step 2:

4-[4-(3-[1,4]Oxazepan-4-yl-propoxy)-phenyl]-tetrahydro-pyran-4-carboxylic acid guanidine (1.2 g, 0.88 mmol of free acid) was dissolved in DMF (5 ml), to which was added N-BOC piperidine (200 mg, 1.06 mmol), HBTU (400 mg, 1.06 mmol) and Hunig's base (129 mg, 1.06 mmol). The reaction was stirred at room temperature for 3 hours before adding saturated sodium bicarbonate solution (15ml) and extracting the product into EtOAc (15 ml). The organic layer was washed with brine (15 ml), dried over Na₂SO₄ and concentrated *in vacuo* before purifying the residue by column chromatography, eluting with a gradient of DCM:MeOH:NH₃ (from 99:1:0.1 to 92:8:0.8). This afforded 4-{4-[4-(3-[1,4]Oxazepan-4-yl-propoxy)-phenyl]-tetrahydro-pyran-4-carbonyl}piperazine-1-carboxylic acid tert-butyl ester as a colourless oil that became a white solid (260 mg, 55%) on standing overnight.

### Step 3:

4-{4-[4-(3-[1,4]Oxazepan-4-yl-propoxy)-phenyl]-tetrahydro-pyran-4-carbonyl}-piperazine-1-carboxylic acid tert-butyl ester (260 mg, 0.48 mmol) was dissolved in a solution of hydrogen choride in dioxane (4M, 5 ml) and was stirred at room temperature for 2 hours. The solvent was removed *in vacuo* and the residue dissolved in DCM (10 ml) and washed with saturated sodium bicarbonate solution (15 ml). The organic layer was dried over Na₂SO₄ and concentrated *in vacuo* before purfying the residue using column chromatography, eluting with a gradient of DCM:MeOH:NH₃ (from 99:1:0.1 to 92:8:0.8) to afford the title compound as a white solid (80 mg, 37%).
¹H NMR (400MHz, d₆-DMSO) δ 1.70-1.88 (m, 6H), 2.08 (m, 2H), 2.52-2.63 (m, 6H), 3.04-3.35 (m, 8H), 3.58 (m, 4H), 3.64 (m, 2H), 3.72 (m, 2H), 3.98 (t, 2H), 6.92 (d, 2H), 7.12 (d, 2H)
LRMS ESI⁺ m/z 432 [MH]⁺

### Example 271: 4-[3-(4-{4-[(4-methylpiperazin-1-yl)carbonyl]tetrahydro-2H-pyran-4-yl}phenoxy)propyl]-1,4-oxazepane

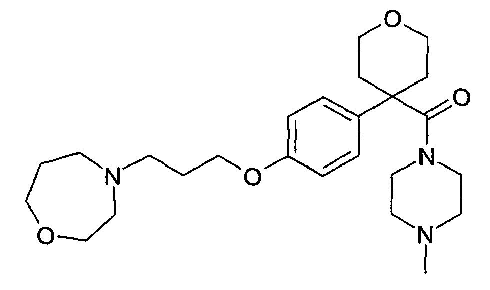

4-(3-{4-[4-(piperazin-1-ylcarbonyl)tetrahydro-2H-pyran-4-yl]phenoxy}propyl)-1,4-oxazepane (45 mg, 0.1 mmol) was dissolved in DCM (3 ml), to which was added formaldyhyde (37% w/w in H₂O, 12 mg, 0.12 mmol) and acetic acid (8 mg, 0.12 mmol) and was stirred at room temperature for 30 minutes before adding STAB (25 mg, 0.12 mmol). The solution was allowed to stir for a further 45 minutes before washing the solution with saturated sodium bicarbonate (10 ml), followed by brine (10 ml). The organic layer was dried over Na₂SO₄ and concentrated *in vacuo* to afford the title compound as a white solid (25 mg, 56%).
¹H NMR (400MHz, d₆-DMSO) δ 1.76-2.13 (m, 11H), 2.55-2.78 (m, 6H), 3.16-3.39 (m, 8H), 3.50-3.62 (m, 4H), 3.63 (m, 2H), 3.72 (M, 2H), 3.99 (t, 2H), 6.92 (d, 2H), 7.12 (d, 2H).
LRMS ESI⁺ m/z 446 [MH]⁺

### Example 272: 4-({4-[4-(3-pyrrolidin-1-ylpropoxy)phenyl]tetrahydro-2H-pyran-4-yl}methyl)-4H-1,2,4-triazole

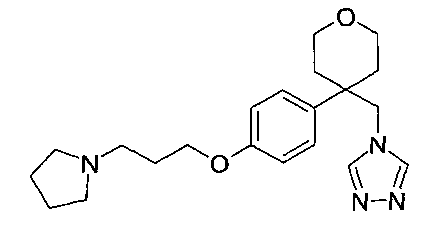

A mixture of {4-[4-(3-pyrrolidin-1-ylpropoxy)phenyl]tetrahydropyran-4-yl} methylamine (200mg, 0.63mmol), *N'*-[(1*E*)-(dimethylamino)methylene]-*N,N*-dimethylhydrazonoformamide (300mg, 2.11mmol) and p-toluenesulphonic acid monohydrate (10mg, 0.05mmol), was heated at reflux in toluene (2ml) for 24 hours. The reaction mixture was concentrated *in vacuo.* The crude product was purified by flash chromatography on silica gel eluting with dichloromethane:methanol:ammonia (95:5:0.5 to 80:20:2 by volume) to provide 160mg of beige gum. This gum was triturated with diethyl ether (3ml) to give the title compound as a white powder (120mg, 51 %).
¹H NMR (400MHz, CDCl₃) *δ* 7.46 (s, 2H), 6.97 (d, 2H), 6.89 (d, 2H), 4.02 (m, 4H), 3.82 (m, 2H), 3.43-3.52 (m, 2H), 2.62 (t, 2H), 2.51 (m, 4H), 2.12 (m, 2H), 2.02 (m, 2H), 1.84 (m, 2H), 1.80 (m, 4H).
LRMS ESI⁺ m/z 371 [M+H]⁺
HRMS ESI⁺ m/z 371.2433 [M+H]⁺

### Intermediate 107: (1-isopropylazetidin-3-yl)methanol hydrochloride

A mixture of (1-benzhydrylazetidin-3-yl)methanol (4.0g, 15.8mmol), acetone (20ml), 2-propanol (80ml), c.HCl (1.5ml) and Pd(OH)₂ was hydrogenated at 50psi/50° for 18 hours. The reaction mixture was filtered through Arbocel® and concentrated *in vacuo,* the residue was triturated with diisopropyl ether (50ml) to give the title compound as a pale green hygroscopic powder (2.6g, 99%)
LRMS ESI⁺ m/z 130 [M+H]⁺

### Intermediate 108: 3-(chloromethyl)-1-isopropylazetidine hydrochloride

(1-isopropylazetidin-3-yl)methanol hydrochloride (2.6g, 15.7mmol) was dissolved in dichloromethane (50ml) and cooled to 0°C. Thionyl chloride (1.4ml, 19.2mmol) was added dropwise over 5 min. The reaction mixture was then stirred whilst warming to ambient temperature. Stirred at ambient temperature for 48 hours. The reaction mixture was concentrated *in vacuo* and azeotroped with toluene (40ml) to give the title compound as a biege hygroscopic powder (2.8g, 99%).
LRMS ESI⁺ m/z 148/150 [M+H]⁺(Cl isotopes)

### Example 273: 4-{4-[(1-isopropylazetidin-3-ylmethoxy]phenyl}tetrahydro-2H-pyran-4-carbonitrile

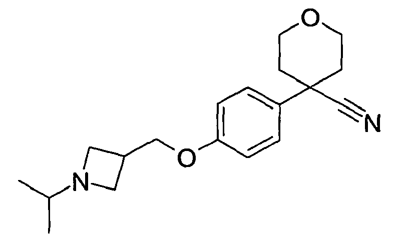

3-(chloromethyl)-1-isopropylazetidine hydrochloride (2.8g, 15.2mmol), 4-(4-hydroxyphenyl)tetrahydropyran-4-carbonitrile (3.1g, 15.2mmol) and potassium carbonate (8.5g, 61.6mmol) were combined in DMF (40ml) and stirred at 50° for 2 hours. Potassium iodide (0.5g 3.0mmol) was added and the mixture was stirred at 50° for 18 hours then at 90-95° for a further 5 hours. The reaction mixture was concentrated *in vacuo* then partitioned between ethyl acetate (80ml) and water (50ml). The ethyl acetate was washed with saturated NaCl (50ml) then dried over Na₂SO₄ and concentrated *in vacuo* to give a brown semi-crystalline material which was triturated with diisopropyl ether (25ml) to give the title compound as a beige powder (2.9g, 60%).
¹H NMR (400MHz, CDCl₃) *δ* 7.38 (d, 2H), 6.91 (d, 2H), 4.08 (m, 4H), 3.89 (m, 2H), 3.41 (t, 2H), 3.02 (t, 2H), 2.87 (m, 1H), 2.31 (m, 1H), 2.15-2.00 (m, 4H), 0.94 (d, 6H).

### Example 274: 1-(4-{4-[(1-isopropylazetidin-3-ylmethoxy)phenyl}tetrahydro-2H-pyran-4-yl)methanamine

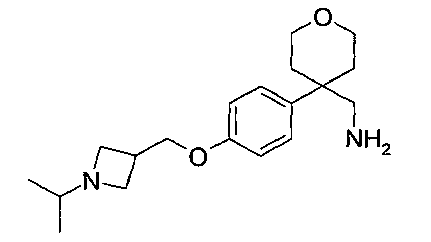

A solution of 4-{4-[(1-isopropylazetidin-3-yl)methoxy]phenyl}tetrahydro-2H-pyran-4-carbonitrile (1.8g, 5.73mmol) in THF (80ml) was cooled to 0°C under an atmosphere of nitrogen. A solution of lithium aluminium hydride (1.0M solution in Et₂O, 17ml, 17mmol) was added dropwise over 5 min. The mixture was stirred whilst warming to ambient temperature over 18 hours. The reaction was cooled to 0°C and quenched with water (0.5ml) then 2M NaOH (0.5ml) and then water (1.5ml). Celite® (3g) was added and the mixture stirred at ambient temperature for 0.5 hour. Diluted with ethyl acetate (50ml), filtered and concentrated *in vacuo* to give a colourless oil (1.8g). The crude product was purified by flash chromatography on silica gel eluting with dichloromethane:methanol:ammonia (97:3:0.3 to 88:12:1.2 by volume) to give the title compound as a white crystalline solid (1.55g, 85%).
¹H NMR (400MHz, CDCl₃) δ 7.18 (d, 2H), 6.89 (d, 2H), 4.18 (d, 2H), 3.77 (m, 2H), 3.54 (m, 2H), 3.41 (t, 2H), 3.02 (t, 2H), 2.88 (m, 1H), 2.75 (s, 2H), 2.31 (quintet, 1H), 2.11 (m, 2H), 1.80 (m, 2H), 0.95 (d, 6H).
LRMS ESI⁺ m/z 319 [M+H]⁺
HRMS ESI⁺ m/z 319.2375 [MH]⁺

### Example 275: N-[(4-{4-[(1-isopropylazetidin-3-yl)methoxy]phenyl}tetrahydro-2H-pyran-4-yl)methyl]pyridin-2-amine

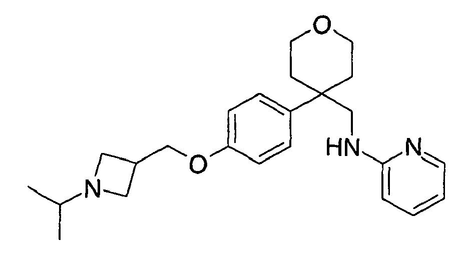

1-(4-{4-[(1-isopropylazetidin-3-yl)methoxy]phenyl}tetrahydro-2H-pyran-4-yl)methanamine was stirred with 2-bromopyridine (60µl, 0.63mmol), tris(dibenzylideneacetone)djpalladium(0) (15mg, 0.018mmol), 2,2'-bis(diphenylphosphino)-1,1'-binaphthyl (19mg, 0.036mmol) and sodium *tert*-butoxide (90mg, 0.94mmol) and heated at 80°C in toluene (5ml) for 3 hours under nitrogen. The reaction mixture was partitioned between aqueous sodium hydroxide (50ml) and ethyl acetate (80ml). The organics were dried over Na₂SO₄, filtered and concentrated *in vacuo*. The crude product was purified by flash chromatography on silica gel eluting with dichloromethane:methanol:ammonia (97:3:0.3 to 90:10:1 by volume) to give the product which required further purification by flash chromatography on silica gel eluting with dichloromethane:methanol:ammonia (96:4:0.4 to 94:6:0.6 by volume) to give a pale yellow oil which crystallized on standing, this was triturated with diethyl ether (2ml) to give the title compound as a pale yellow powder (65mg, 26%).

¹H NMR (400MHz, CDCl₃) δ 8.02 (d, 1H), 7.31 (t, 1H), 7.25 (d, 2H), 6.91 (d, 2H), 6.50 (t, 1H), 6.20 (d, 1H), 4.09 (d, 2H), 3.99 (t, 1H), 3.82 (m, 2H), 3.59 (m, 2H), 3.48 (d, 2H), 3.45 (m, 2H), 3.03 (m, 2H), 2.88 (m, 1H),2.33 (m, 1H), 2.13 (m, 2H), 1.92 (m, 2H), 0.93 (d, 6H).
LRMS ESI⁺ m/z 396 [M+H]⁺
HRMS ESI⁺ m/z 396.2639 [M+H]⁺

### Intermediate 109: 1-{4-[4-(3-pyrrolidin-1-ylpropoxy)phenyl]tetrahydro-2H-pyran-4-yl}ethanone

To a solution of 4-[4-(3-Pyrrolidin-1-ylpropoxy)-phenyl]tetrahydropyran-4-carbonitrile (2.0g, 6.37mmol) in diethyl ether (30ml) and THF (5ml) was added methyl lithium/lithium bromide complex (1.5M solution in diethyl ether, 4.5ml, 6.75mmol), the reaction mixture was stirred at ambient temperature for 18 hours. The reaction mixture was quenched by adding water (5ml) and concentrated *in vacuo*. The residue was partitioned between DCM (100ml) and water (30ml), the organics were dried over Na₂SO₄, filtered and concentrated *in vacu*o to give a crude product which was purified by flash chromatography on silica gel eluting with dichloromethane:methanol:ammonia (98:2:0.2 to 92:8:0.8 by volume) to provide a colourless gum (500mg, 24%).
LRMS ESI⁺ m/z 332 [M+H]⁺

### Example 276: 4-{4-[4-(3-pyrrolidin-1-ylpropoxy)phenyl]tetrahydro-2H-pyran-4-yl}pyrimidine

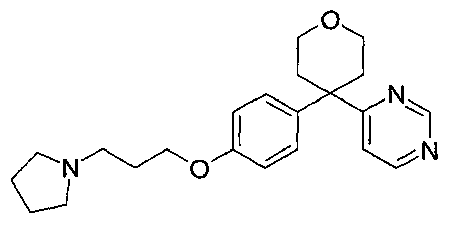

### Step 1.

1-{4-[4-(3-pyrrolidin-1-ylpropoxy)phenyl]tetrahydro-2H-pyran-4-yl}ethanone_(500mg, 1.51 mmol) was dissolved in dimethylformamide dimethylacetal (3ml) and heated in a sealed tube microwave reactor (Smiths Personal Synthesizer) at 150°C for 1800 seconds then at 170°C for 2400 seconds. The reaction mixture concentrated *in vacuo* to give a crude product (3-Dimethylamino-1-{4-[4-(3-pyrrolidin-1-ylpropoxy)phenyl]tetrahydropyran-4-yl}propenone, 500mg) which was used without purification in the next step.

### Step 2.

The crude intermediate was dissolved in ethanol (10ml), formamidine acetate (320mg, 3.07mmol) and sodium ethoxide (21% w/v solution in ethanol, 1.0ml, 3.09mmol) were added and the mixture stirred at ambient temperature for 1.5 hours then heated at 70°C for 2 hours. Formamidine acetate (500mg, 4.80mmol) was added and stirred at 70°C for 18 hours. Formamidine acetate (500mg, 4.80mmol) and potassium carbonate (2.0g, 14.5mmol) were added and stirred at reflux for 5 hours. Formamidine acetate (500mg, 4.80mmol) was added and stirred at reflux for 18 hours. The reaction mixture was partitioned between DCM (80ml) and 10% Na₂CO₃ (50ml), the organics were dried over Na₂SO₄, filtered and concentrated *in vacuo* to give a black solid. The crude product was purified by flash chromatography on silica gel eluting with dichloromethane:methanol:ammonia (100:0:0 to 94:6:0.6 by volume) to give the title compound as a brown oil (45mg, 8%).
¹H NMR (400MHz, CDCl₃) δ 9.16 (s, 1H), 8.53 (d, 1H), 7.20 (d, 2H), 7.08 (d, 1H), 6.83 (d, 2H), 3.96 (t, 2H), 3.78 (m, 2H), 3.66 (m, 2H), 2.68-2.55 (m, 2H), 2.50 (m, 4H), 2.33 (m, 2H), 1.96 (m, 4H), 1.76 (m, 4H).
LRMS ESI⁺ m/z 368 [M+H]⁺

### Intermediate 110: 4-methyl-1-({4-[4-(3-pyrrolidin-1-ylpropoxy)phenyl]tetrahydro-2H-pyran-4-yl}methyl)-1H-imidazole-2-thiol

2-Aminopropionaldehyde dimethyl acetal (0.2g, 1.7mmol), thiocarbonyldiimidazole (0.36g, 2.0mmol) and triethylamine (0.23ml, 1.7mmol) were stirred in DMF (2.0ml) at ambient temperature for 30 minutes before heating to 50°C for 18 hours. {4-[4-(3-pyrrolidin-1-ylpropoxy)phenyl]tetrahydropyran-4-yl}methylamine (540mg, 1.7mmol) was added and stirred at ambient temperature for 4 hours. 2M HCl (6ml) was added, stirred at 70°C for 18 hours, then c.HCl (1.0ml) was added and the mixture stirred at 110°C for 18 hours. The reaction mixture was cooled and partitioned between ethyl acetate (100ml) and dilute aqueous sodium carbonate (50ml). The organics were dried over Na₂SO₄ and concentrated *in vacuo* to give the title compound as a grey solid (600mg, 84%).
LRMS ESI⁺ m/z 416 [M+H]⁺

### Example 277: 4-methyl-1-({4-[4-(3-pyrrolidin-1-ylpropoxy)phenyl]tetrahydro-2H-pyran-4-yl}methyl)-1H-imidazole

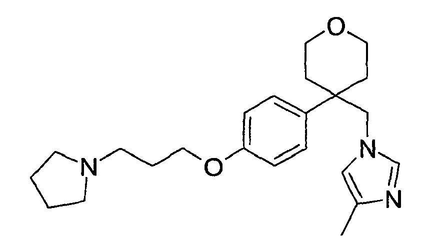

4-methyl-1-({4-[4-(3-pyrrolidin-1-ylpropoxy)phenyl]tetrahydro-2H-pyran-4-yl}methyl)-1H-imidazole-2-thiol (0.6g, 1.44mmol) was dissolved in ethanol (20mL), water (5mL) was added, and the mixture was cooled in ice. Raney nickel (4g) was added, the reaction mixture was warmed to room temperature and stirred for 2 hours. The reaction mixture was filtered though Arbocel® and concentrated *in vacuo* to provide a brown oil. This was purified by flash chromatography on silica gel eluting with ethyl acetate:diethylamine (95:5) to provide the title compound (0.086, 16%) as a clear oil.
¹H NMR (400 MHz CDCl₃) δ 1.80-1.90 (m, 6H), 2.08-2.18 (m, 7H), 2.65-2.80 (m, 6H), 3.50 (t, 2H), 3.80 (m, 2H), 3.90 (s, 2H), 4.05 (t, 2H), 6.10 (s, 1H), 6.70 (s, 1H), 6.89 (d, 2H), 7.0 (d, 2H).
LRMS ESI⁺ m/z 384 [MH]⁺

### Example 278: 4-{4-[4-(3-pyrrolidin-1-ylpropoxy)phenyl]tetrahydro-2H-pyran-4-yl}-1H-imidazole

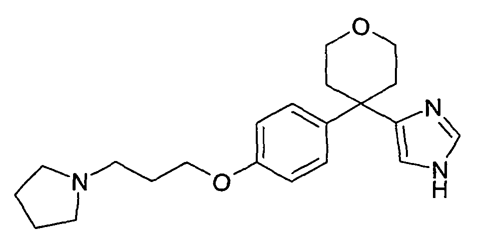

### Step 1:

4-[4-(3-pyrrolidin-1-ylpropoxy)phenyl]tetrahydro-2H-pyran-4-carbaldehyde (500 mg, 1.6 mmol) and TosMIC (340 mg, 1.7 mmol) were dissolved in EtOH (20 ml), to which NaCN (12 mg, 0.25 mmol) was added. The solution immediately turned pale yellow and was allowed to stir for 2 hours at room temperature. The solvent was removed *in vacuo* and the residue was taken up in DCM (10 ml) and washed with saturated sodium bicarbonate solution (15 ml), followed by brine (15 ml). The organic layer was dried over Na₂SO₄ and concentrated *in vacuo* to afford 5-{4-[4-(3-Pyrrolidin-1-yl-propoxy)-phenyl]-tetrahydro-pyran-4-yl}-4-(toluene-4-sulfonyl)-4,5-dihydro-oxazole as a pale brown foam (500 mg).
LRMS ESI⁺ m/z 513 [MH]⁺

### Step 2:

The 5-{4-[4-(3-Pyrrolidin-1-yl-propoxy)-phenyl]-tetrahydro-pyran-4-yl}-4-(toluene-4-sulfonyl)-4,5-dihydro-oxazole (500 mg) was dissolved in EtOH saturated with ammonia ( 7ml) before heating in a bomb at 110°C for 18 hours. The solvent was removed *in vacuo* and the residue was taken up in DCM (10 ml) and washed with saturated sodium bicarbonate solution (15 ml), followed by brine (15 ml). The organic layer was dried over Na₂SO₄ and concentrated *in vacuo,* before purifying by column chromatography, eluting with a gradient of DCM:MeOH:NH₃ (from 99:1:0.1 to 90:10:1), but this failed to separate the product from a close running impurity. The residue was therefore purified using column chromatography a second time, eluting with an isocratic solution of cyclohexane:DCM:Et₂O:MeOH:NH₃ (10:5:3:2:0.2) to afford the title compound as a colourless solid (70 mg, 12%).
¹H NMR (400MHz, CDCl₃) δ 1.8 (m, 4H), 1.93-2.05 (m, 2H), 2.24-2.39 (m, 4H), 2.57 (m, 4H), 2.63 (t, 2H), 3.67 (m, 2H), 3.79 (m, 2H), 4.00 (t, 2H), 6.77 (s, 1H), 6.81 (d, 2H), 7.18 (d, 2H), 7.38 (s, 1H)
LRMS ESI⁺ m/z 356 [MH]⁺, 354 [M-H⁺]⁻

### Example 279: 4-[4-(4-{3-[ethyl(methyl)amino]propoxy}phenyl)tetrahydro-2H-pyran-4-ylmethyl]-morpholine

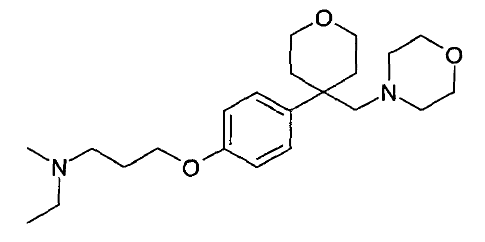

### Step 1:

A mixture of 4-[4-(3-chloro-propoxy)-phenyl]-tetrahydropyran-4-carbonitrile (3.0 g, 10.7 mmol), potassium iodide (100 mg, 0.60 mmol) and ethylmethylamine (5.0 ml, 58.3 mmol) in acetonitrile (30 mL) was heated to 60°C overnight. The mixture was cooled, concentrated *in vacuo* and partitioned between dilute Na₂CO₃ solution (40 ml) and ethyl acetate (80 ml). The organic phase was dried (Na₂SO₄), filtered and concentrated *in vacuo.* Purification of the crude material was carried out with flash chromatography (silica gel, ethyl acetate/methanol/aqueous ammonia 98:2:0.2 to 85:15:1.5) to afford 4-(4-{3-[ethyl(methyl)amino]propoxy}phenyl)tetrahydro-2H-pyran-4-carbonitrile as a pale yellow oil (2.0 g, 6.6 mmol, 62%).
¹H NMR (400 MHz CDCl₃) δ 1.06 (t, 3H), 1.91-2.00 (m, 2H), 2.00-2.14 (m, 4H), 2.25 (s, 3H), 2.40-2.50 (m, 2H), 2.53 (t, 2H), 3.89 (m, 2H), 4.00-4.11 (m, 4H), 6.91 (d, 2H), 7.38 (d, 2H).
LRMS ESI⁺ m/z 303 [MH]⁺

### Step 2:

Lithium aluminium hydride (9.93 ml of a 1 M solution in tetrahydrofuran, 9.93 mmol) was added to a solution of 4-(4-{3-[ethyl(methyl)amino]propoxy}phenyl)tetrahydro-2H-pyran-4-carbonitrile (2.0 g, 6.6 mmol) in tetrahydrofuran (10 ml) at 0°C under N₂. The reaction mixture was warmed to room temperature over 18 h and then quenched by the sequential addition of water (0.4 ml), 2M NaOH solution (0.4 ml) and water (0.4 ml). The mixture was filtered through arbocel and concentrated *in vacuo* to yield 3-{4-[4-(aminomethyl)tetrahydro-2H-pyran-4-yl]phenoxy}-N-ethyl-N-methylpropan-1-amine as a pale yellow oil (1.93 g, 6.3 mmol, 95%).
¹H NMR (400 MHz CDCl₃) δ 1.02-1.08 (t, 3H), 1.73-1.83 (m, 2H), 1.90-2.00 (m, 2H), 2.06-2.16 (m, 2H), 2.23 (s, 3H), 2.40-2.48 (q, 2H), 2.50-2.58 (t, 2H), 2.75 (s, 2H), 3.48-3.58 (m, 2H), 3.70-3.80 (m, 2H), 3.98-4.04 (t, 2H), 6.89 (d, 2H), 7.19 (d, 2H).
LRMS APCI⁺ m/z 307 [MH]⁺

### Step 3:

To a stirred solution of 3-{4-[4-(aminomethyl)tetrahydro-2H-pyran-4-yl]phenoxy}-N-ethyl-N-methylpropan-1-amine (500 mg, 1.6 mmol) and bis(2-bromoethyl)ether (345 mg, 1.5 mmol) in anhydrous acetonitrile (50 mL) was added potassium carbonate (450 mg, 3.3 mmol). The mixture was heated to 60°C overnight then concentrated *in vacuo.* The crude product was partitioned between dichloromethane (50 ml) and water (50 ml), the aqueous phase was extracted with dichloromethane (25 ml) and the combined organics were dried (Na₂SO₄), filtered and concentrated *in vacuo.* Purification of the crude material was carried out with flash chromatography (silica gel, dichloromethane/methanol/aqueous ammonia 100:0:0 to 96:4:0.4) to afford the title compound as a clear colourless oil (315 mg, 0.84 mmol, 52%).
¹H NMR (400 MHz CDCl₃) δ 1.05 (t, 3H), 1.82-1.93 (m, 2H), 1.92-2.00 (m, 2H), 2.08-2.20 (m, 6H), 2.25 (s, 3H), 2.39 (s, 2H), 2.40-2.50 (q, 2H), 2.50-2.59 (m, 2H), 3.48-3.60 (m, 6H), 3.70-3.80 (m, 2H), 4.00 (t, 2H), 6.86 (d, 2H), 7.20 (d, 2H).
LRMS APCI⁺ m/z 377 [MH]⁺
HRMS ESI⁺ m/z 377.2792 [MH]⁺

### H₃ Cell Based Functional Assay

Compounds were evaluated using a cell based functional assay measuring cAMP through β-lactamase reporter gene activity. A stable cell line was generated from HEK-293 cells expressing a CRE β-lactamase reporter gene and transfected with human histamine H₃ receptor cDNA. Cells were seeded at a density of 500,000 cells/ml, and grown overnight in MEM (Invitrogen) supplemented with 1% dialysed FBS (Sigma), 2mM glutamine (Sigma), 1mM sodium pyruvate (Sigma), 0.1mM non essential amino acids (Invitrogen) and 25mM HEPES (Sigma) in poly D lysine coated 384 well plates (BD Biosciences). H₃ receptor agonist imetit (Tocris) dose dependently inhibited 10µM forskolin (Calbiochem) stimulated synthesis of cAMP measured after 4.5hours by β-lactamase cleavage of CCF4-AM dye (Invitrogen). For IC₅₀ determination, test compounds were prepared in PBS (Sigma) and DMSO (Sigma) at a dose response of 5x10⁻¹⁰ to 5x10⁻⁵M with a final DMSO concentration in the assay of 0.5%. Cells were incubated for 15 minutes plus/minus compound and their ability to permit 10µM forskolin-stimulated cAMP synthesis in the presence of 1 nM imetit was measured as described above. Functional Kᵢ values were calculated from the IC₅₀ of compounds tested as antagonists based on an experimentally determined imetit EC₅₀ (represented in the equation as K_{d}) of 350pM, and an imetit concentration [L] of 1nM, according to the Cheng-Prussoff equation where Kᵢ = (IC₅₀)/(1+([L]/K_{d})).

All the examples were tested in the assay described above and found to have a Kᵢ value of less than 5 µM. Most of the examples have a Kᵢ value of less than 100 nM.

The data for some of said preferred compounds are given below by way of example:

| **Example Number** | **Kᵢ (H3 cell based assay - nM)** | **Example Number** | **Kᵢ (H3 cell based assay - nM)** | **Example Number** | **Kᵢ (H3 cell based assay - nM)** |
|---|---|---|---|---|---|
| 29 | 0.4 | 190 | 2.4 | 249 | 9.7 |
| 133 | 12.4 | 191 | 10.3 | 250 | 4.6 |
| 136 | 3.9 | 196 | 0.6 | 254 | 1.3 |
| 155 | 1.0 | 205 | 5.8 | 266 | 9.7 |
| 156 | 2.0 | 206 | 12.9 | 267 | 5.8 |
| 160 | 1.5 | 213 | 2.6 | 268 | 6.5 |
| 187 | 7.3 | 220 | 2.2 | 276 | 6.2 |
| 188 | 6.8 | 247 | 3.9 | 272 | 10.8 |
| 189 | 14.3 | 248 | 0.3 | | |

## Claims

1. A compound of the formula (1) : 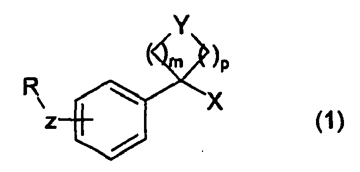 or a pharmaceutically acceptable salt and/or solvate (including hydrate) thereof wherein :
the substituent of formula -Z-R is in the meta or para position of the phenyl group;
X is selected from -CN, -CH₂OH, -CH₂-O-(C₁-C₄)alkyl, -C(O)OH, -C(O)O(C₁-C₄)alkyl, -CH₂-NR¹R², -C(O)NR³R⁴, -CH₂-O-het², -CH₂-het¹ and het¹, the group het¹ in both -CH₂-het¹ and het¹ being optionally substituted by one or two substituents independently selected from halo, cyano, (C₁-C₄)alkyl, -S-(C₁-C₄)alkyl and (C₁-C₄)alkoxy;
R¹ is hydrogen or (C₁-C₄)alkyl optionally substituted by (C₃-C₈)cycloalkyl;
R² is selected from the group consisting of :
hydrogen,
(C₁-C₆)alkyl optionally substituted by one or two substituents independently selected from (C₃-C₆)cycloalkyl, hydroxy, -S-(C₁-C₄)alkyl, -O-(C₁-C₄)alkyl, -SO₂(C₁-C₄)alkyl, -SO-(C₁-C₄)alkyl, halo, het¹, amino, (C₁-C₄)alkylamino, [(C₁-C₄)alkyl]₂amino and phenyl, said phenyl being optionally substituted by one or two substituents independently selected from halo, hydroxy, cyano, (C₁-C₄)alkyl and (C₁-C₄)alkoxy, (C₃-C₆)cycloalkyl,
het², optionally substituted by one or two substituents independently selected from halo, cyano, (C₁-C₄)alkyl, NH₂ and (C₁-C₄)alkoxy,
-SO₂-R⁵ wherein R⁵ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionally substituted by one or two substituents independently selected from halo, cyano, (C₁-C₄)alkyl and (C₁-C₄)alkoxy, and
-C(O)-R⁶ wherein R⁶ is selected from the group consisting of (C₁-C₄)alkyl, amino, (C₁-C₄)alkylamino, [(C₁-C₄)alkyl]₂amino, phenyl and -(C₁-C₄)alkyl-phenyl, said phenyl being optionally substituted by one or two substituents independently selected from halo, cyano, (C₁-C₄)alkyl and (C₁-C₄)alkoxy;
or R¹ and R² form together with the N atom to which they are attached a 3-, 4-, 5-, 6- or 7-membered saturated heterocycle wherein one C atom may be replaced by N, O, S, SO or SO₂ and wherein said saturated heterocycle is optionally substituted by one or two groups independently selected from hydroxy, halo, =O, (C₁-C₄)alkyl, -(C₁-C₄)alkyl(C₃-C₆)cycloalkyl, (C₁-C₄)alkoxy, hydroxy(C₁-C₄)alkyl, (C₁-C₄)alkoxy(C₁-C₄)alkyl, -SO₂(C₁-C₄)alkyl, -C(O)(C₁-C₄)alkyl, [(C₁-C₄)alkyl]₂amino, amino, (C₁-C₄)alkylamino, -C(O)NH₂, C(O)O(C₁-C₄)alkyl and pyrrolidinone;
R³ and R⁴ are each independently selected from hydrogen, (C₃-C₆)cycloalkyl, and (C₁-C₄)alkyl, said (C₃-C₆)cycloalkyl and (C₁-C₄)alkyl being optionally substituted by amino, (C₁-C₄)alkylamino, [(C₁-C₄)alkylhamlno or (C₃-C₆)cycloalkyl, or R³ and R⁴ form together with the N atom to which they are attached a 4-, 5-, 6- or 7-membered saturated heterocycle wherein one C atom may be replaced by N or O and wherein said saturated heterocycle is optionally substituted by (C₁-C₄)alkyl, [(C₁-C₄)alkyl]₂amino, amino, (C₁-C₄)alkylamino, or -C(O)(C₁-C₄)alkyl, said -C(O)(C₁-C₄)alkyl being optionally substituted by methoxy or ethoxy,
Y is selected from CH₂, CH(OH), O, C=O and N, said N being substituted by H, (C₁-C₄)alkyl, C(O)(C₁-C₄)alkyl or (C₁-C₄)alkoxy(C₁-C₄)alkyl;
Z is selected from O, S, SO and SO₂;
m and p are both integers which are independently 1, 2 or 3, with the condition that m+p is equal to or less than 4 so that the ring formed by : 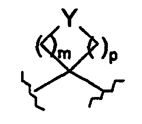 is a 4-, 5- or 6-membered ring;
and R is either a group of formula : 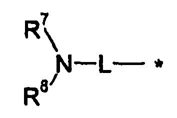 wherein * represents the attachment point to Z, L is a straight chain or branched (C₂-C₆)alkylene and R⁷ and R³ are each independently selected from hydrogen, (C₁-C₆)alkyl, (C₃-C₆)cycloalkyl, hydroxy(C₁-C₆)alkyl or R⁷ and R⁸ form together with the N atom to which they are attached a 4-, 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂, halo, amino, (C₁-C₄)alkylamino and [(C₁-C₄)alkyl]₂amino,
or R is a group of formula: 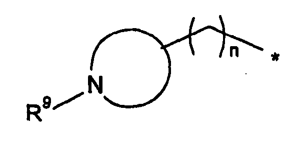 wherein * represents the attachment point to Z, the N-containing ring is a 4- to 7-membered saturated heterocycle, n is an integer equal to 0, 1 or 2, and R⁹ represents a substituent selected from hydrogen, (C₁-C₄)alkyl, hydroxy(C₁-C₆ alkyl) and (C₃-C₆)cycloalkyl;
het¹ is selected from monocyclic or bicyclic heteroaromatic groups having 5 to 10 ring members, which contain 1, 2, 3 or 4 heteroatom(s) selected from nitrogen, oxygen and sulphur and het² is selected from monocyclic or bicyclic heteroaromatic groups having 5 to 10 ring members, which contain 1, 2, 3 or 4 heteroatom(s) selected from nitrogen, oxygen and sulphur.

2. A compound of formula (1) as defined in claim 1, wherein X is selected from -CH₂-NR¹R², -C(O)NR³R⁴, -CH₂-het¹ and het¹, het¹ being optionally substituted once or twice by (C₁-C₄)alkyl, wherein R¹, R², R³, R⁴ and het¹ are as previously defined in claim 1.

3. A compound of formula (1) as defined in claim 2, wherein X is -CH₂-het¹ or het¹, and het¹ is selected from a 5 or 6 membered monocyclic heteroaromatic group or a 9 membered bicyclic heteroaromatic group, each heteroaromatic group containing 1 to 3 nitrogen atoms, or 1 to 2 nitrogen atoms and 1 oxygen atom, or 1 nitrogen atom and 1 sulphur atom, and each heteroaromatic group being optionally substituted once or twice by (C₁-C₄)alkyl.

4. A compound of formula (1) as defined in claim 3, wherein X is thiazolyl, benzimidazolylmethyl-, pyridine oxazolyl, imidazopyridinylmethyl-, pyrimidinyl, imidazolyl, imidazolylmethyl- or triazolylmethyl-, said thiazolyl, benzimidazolylmethyl-, pyridinyl, oxazolyl, imidazopyridinylmethyl-, pyrimidinyl, imidazolyl, imidazolylmethyl- and triazolylmethyl- each being optionally substituted with one methyl group.

5. A compound of formula (1) as defined in claim 1 or claim 2, wherein R¹ is hydrogen, methyl or ethyl.

6. A compound of formula (1) as defined in claim 1, claim 2 or claim 5, wherein R² is selected from the group consisting of
hydrogen,
(C₁-C₆)alkyl optionally substituted by one or two substituents independently selected from -S-(C₁-C₄)alkyl, -O-(C₁-C₄)alkyl, -SO₂-(C₁-C₄)alkyl, and phenyl, said phenyl being optionally substituted by one or two substituents independently selected from halo, hydroxy, cyano, (C₁-C₄)alkyl and (CH-C₄)alkoxy,
(C₃-C₆)cycloalkyl,
het² optionally substituted by one or two substituents independently selected from halo, cyano, (C₁-C₄)alkyl and (C₁-C₄)alkoxy, wherein het² is defined as in claim 1,
-SO₂-R⁵ wherein R⁶ is selected from the group consisting of (C₁-C₄)alkyl, [(C₁-C₄)alkyl]₂amino, phenyl, and -(C₁-C₄)alkyl-phenyl, wherein said phenyl is optionally substituted by 1 substituent independently selected from halo and cyano and
-C(O)-R⁶ wherein R⁶ Is selected from the group consisting of (C₁-C₄)alkyl, ((C₁-C₄)alkyl]₂amino, amino, and -(C₁-C₄)alkyl-phenyl, said phenyl being optionally substituted by one or two substituents independently selected from halo, cyano, (C₁-C₄)alkyl and (C₁-C₄)alkoxy.

7. A compound of formula (1) as defined in claim 6, wherein R² is selected from the group consisting of
(C₁-C₃)alkyl optionally substituted by -O-(C₁-C₃)alkyl,
(C₃-C₅)cycloalkyl,
het², wherein het² is selected from the group consisting of 5 or 6 membered monocyclic heteroaromatic groups containing 1 to 2 nitrogen atoms or 1 nitrogen atom and 1 oxygen atom or 1 nitrogen atom and 1 sulphur atom, said het² being optionally substituted by (C₁-C₄)alkyl,
SO₂-R⁵ wherein R⁶ is (C₁-C₄)alkyl and
C(O)-R⁶ wherein R⁶ is (C₁-C₄)alkyl.

8. A compound of formula (1) as defined in claim 7, wherein R² is (C₁-C₃)alkyl optionally substituted by methoxy.

9. A compound of formula (1) as defined in claim 7, wherein R² is het², and het² is selected from the group consisting of 5 or 6 membered monocyclic heteroaromatic groups containing 1 or 2 nitrogen atoms.

10. A compound of formula (1) as defined in claim 9, wherein R² is a pyridazinyl group.

11. A compound of formula (1) as defined in claim 1 or claim 2, wherein R¹ and R² form together with the N atom to which they are attached a 4-, 5-, 6- or 7-membered saturated heterocycle wherein one C atom may be replaced by N, O, S, SO or SO₂ and wherein said saturated heterocycle is optionally substituted by one or two groups independently selected from hydroxy, halo, =O, (C₁-C₄)alkyl, -(C₁-C₄)alkyl(C₃-C₆)cycloalkyl, (C₁-C₄)alkoxy, hydroxy(C₁-C₄)alkyl, (C₁-C₄)alkoxy(C₁-C₄)alkyl, -SO₂(C₁-C₄)alkyl, -C(O)(C₁-C₄)alkyl, [(C₁-C₄)alkyl]₂amino, -C(O)NH₂, C(O)O(C₁-C₄)alkyl and pyrrolidinone.

12. A compound of formula (1) as defined in claim 11, wherein R¹ and R² form together with the N atom to which they are attached a morpholinyl group.

13. A compound of formula (1) as defined in claim 1 or claim 2, wherein R³ and R⁴ are each independently selected from hydrogen and (C₁-C₄)alkyl or R³ and R⁴ form together with the N atom to which they are attached a 4, 5 or 6 membered saturated heterocycle wherein one C atom may be replaced by N or O and wherein said saturated heterocycle is optionally substituted by (C₁-C₄)alkyl.

14. A compound of formula (1) as defined in claim 13, wherein R³ and R⁴ are selected Independently from hydrogen, methyl and ethyl, or R³ and R⁴ form together with the N atom to which they are attached a pyrrolidinyl, piperidinyl, piperazinyl or azetidinyl ring, each pyrrolidinyl, piperidinyl, piperazinyl and azetidinyl ring being optionally methyl substituted.

15. A compound of formula (1) as defined in any one of the preceding claims, wherein Y is selected from CH₂, CH(OH), O and C=O.

16. A compound of formula (1) as defined in claim,15, wherein Y is O.

17. A compound of formula (1) as defined in any one of the preceding claims, wherein Z is O.

18. A compound of formula (1) as defined in any one of the preceding claims, wherein m and p are both 2.

19. A compound of formula (1) as defined in any one of the preceding claims, wherein R is a group of formula : 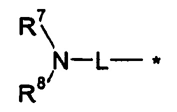 wherein * represents the attachment point to Z, L is a (C₂-C₅)alkylene and R⁷ and R⁸ are each independently selected from hydrogen, (C₁-C₈)alkyl, (C₃-C₆)cycloalkyl, hydroxy(C₁-C₆)alkyl or R⁷ and R⁸ form together with the N atom to which they are attached a 4-, 5-, 6- or 7-membered saturated heterocycle wherein one C atom is optionally replaced by N, O, S, SO or SO₂ and wherein said saturated heterocycle is optionally substituted by one or two groups independently selected from (C₁-C₄)alkyl, (C₁-C₄)alkoxy, (C₁-C₄)alkoxy(C₁-C₄)alkyl, hydroxy(C₁-C₄)alkyl, hydroxy, C(O)O(C₁-C₄)alkyl, -C(O)-(C₁-C₄)alkyl-NH₂, -C(O)NH₂ and halo.

20. A compound of formula (1) as defined in claim 19, wherein R⁷ and R⁸ form together with the N atom to which they are attached a morpholinyl or oxazepanyl group.

21. A compound of formula (1) as defined in claim 19, wherein R⁷ and R⁸ form together with the N atom to which they are attached a 4-, 5- or 6-membered saturated heterocycle, optionally substituted by one or two (C₁-C₄)alkyl groups.

22. A compound of formula (1) as defined in claim 21, wherein the saturated heterocycle is a pyrrolidinyl group, optionally substituted by one or two methyl groups.

23. A compound of formula (1) as defined in claim 19, wherein R⁷ and R⁸ are (C₁-C₃)alkyl.

24. A compound of formula (1) as defined in any one of claims 19 to 23, wherein L is propylene.

25. A compound of formula (1) as defined in any one of claims 1 to 18, wherein R is a group of formula: 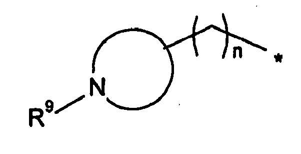 wherein * represents the attachment point to Z, the N-containing ring is a 4- or 6-membered saturated heterocycle, n is an integer equal to 0 or 1, and R⁹ represents a substituent selected from hydrogen, (C₁-C₄)alkyl and (C₃-C₅)cycloalkyl.

26. A compound of formula (1) as defined in claim 25, wherein R⁹ is isopropyl or cyclobutyl.

27. A compound of formula (1) according to claim 1 which is selected from:
N-{4-[4-(3-pyrrolidin-1-yl-propoxy)-phenyl]-tetrahydro-pyran-4-ylmethyl}-methanesulfonamide;
{4-[4-(3-pyrrolidin-1-yl-propoxy)-phenyl]-tetrahydro-pyran-4-ylmethyl}-urea;
4-[4-(3-pyrrolidin-1-yl-propoxy)-phenyl]tetrahydro-2H-pyran-4-carboxylic acid amide;
4-{4-[(-1-isopropylpiperidin-4-yl)oxy]phenyl}tetrahydro-2H-pyran-4-carboxamide;
4-{4-[(1-cyclobutylpiperidin-4-yl)oxy]phenyl}-tetrahydro-2H-pyran-4-carboxamide;
N-{[4-(4-{3-[(3R)-3-(dimethylamino)pyrrolidin-1-yl]-propoxy}phenyl)tetrahydro-2H-pyran-4-yl]-methyl}-N-methylmethanesulfonamide; and
N*4*-{4-[4-(3-pyrrolidin-1-yl-propoxy)-phenyl]-tetrahydro-pyran-4-ylmethyl}-pyridine-3,4-diamine.

28. A pharmaceutical composition including a compound of the formula (1) or a pharmaceutically acceptable salt and/or solvate thereof, as defined in any one of the preceding claims, together with a pharmaceutically acceptable excipient.

29. A compound of the formula (1) as defined in any one of claims 1 to 27 or a pharmaceutically acceptable salt and/or solvate thereof, for use as a medicament.

30. The use of a compound of the formula (1) according to any one of claims 1 to 27 or a pharmaceutically acceptable salt and/or solvate thereof, for the manufacture of a medicament for the treatment of sleep disorders, migraine, dyskinesia, stress-induced anxiety, psychotic disorders, epilepsy, Cognition deficiency diseases, depression, mood disorders, schizophrenia, anxiety disorders, attention-deficit hyperactivity disorder (ADHD), psychotic disorders, obesity, dizziness, vertigo, epilepsy, motion sickness, inflammatory diseases, adult respiratory distress syndrome, acute respiratory distress syndrome, bronchitis, chronic bronchitis, chronic obstructive pulmonary disease, cystic fibrosis, asthma, emphysema, rhinitis, chronic sinusitis, allergy, allergy-induced airway responses, allergic rhinitis, viral rhinitis, non-allergic rhinitis, perennial and seasonal rhinitis, nasal congestion, allergic congestion, male sexual dysfunction or female sexual dysfunction.

31. The use of a compound of the formula (1) according to claim 30 wherein the cognition deficiency disease is Alzheimer's disease or mild cognitive impairment.

32. A combination of a compound of formula (1) as defined in any one of claims 1 to 27, and another pharmacologically active agent

33. A combination as claimed in claim 32, wherein the other pharmacologically active agent is a histamine H₁ receptor antagonist

34. A compound of the formula (1) as defined in any one of claims 1 to 27 or a pharmaceutical acceptable salt and/or solvate thereof, for use in treating the diseases, disorders and conditions as defined in claims 30 and 31.

## Patentansprüche

1. Verbindung der Formel (1): 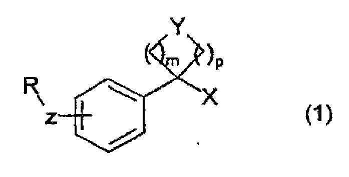 oder ein pharmazeutisch verträgliches Salz und/oder Solvat (einschließlich Hydrat) davon, wobei:
der Substituent der Formel -Z-R in der meta- oder para-Position der Phenylgruppe ist;
X ausgewählt ist aus -CN, -CH₂OH, -CH₂-O-(C₁-C₄)Alkyl, -C(O)OH, -C(O)O(C₁-C₄)Alkyl, -CH₂NR¹R², -C(O)NR³R⁴, -CH₂-O-Het², -CH₂-Het¹ und Het¹, wobei die Gruppe Het¹ sowohl in -CH₂-Het¹ als auch Het¹ gegebenenfalls mit ein oder zwei Substituenten substituiert ist, die unabhängig ausgewählt sind aus Halogen, Cyano, (C₁-C₄)Alkyl, -S-(C₁-C₄)Alkyl und (C₁-C₄)Alkoxy;
R¹ Wasserstoff oder (C₁-C₄)Alkyl, gegebenenfalls substituiert mit (C₃-C₈) Cycloalkyl, ist;
R² ausgewählt ist aus der Gruppe, bestehend aus:
Wasserstoff,
(C₁-C₆)Alkyl, gegebenenfalls substituiert mit ein oder zwei Substituenten, unabhängig ausgewählt aus (C₃-C₆)Cycloalkyl, Hydroxy, -S-(C₁-C₄)Alkyl, -O-(C₁-C₄)Alkyl, -SO₂- (C₁-C₄)Alkyl, -SO- (C₁-C₄) Alkyl, Halogen, Het¹, Amino, (C₁-C₄)Alkylamino, [(C₁-C₄)Alkyl]₂amino und Phenyl, wobei das Phenyl gegebenenfalls mit ein oder zwei Substituenten, unabhängig ausgewählt aus Halogen, Hydroxy, Cyano, (C₁-C₄)Alkyl und (C₁-C₄)Alkoxy, substituiert sein kann,
(C₃-C₆)Cycloalkyl,
Het², gegebenenfalls substituiert mit ein oder zwei Substituenten, unabhängig ausgewählt aus Halogen, Cyano, (C₁-C₄)Alkyl, NH₂ und (C₁-C₄)Alkoxy,
-SO₂R⁵, wobei R⁵ ausgewählt ist aus der Gruppe, bestehend aus (C₁-C₄)Alkyl, Amino, (C₁-C₄)Alkylamino, [(C₁-C₄)Alkyl]₂amino, Phenyl und -(C₁-C₄)Alkylphenyl, wobei das Phenyl gegebenenfalls mit ein oder zwei Substituenten, unabhängig ausgewählt aus Halogen, Cyano, (C₁-C₄)Alkyl und (C₁-C₄)Alkoxy, substituiert ist, und C(O)-R⁶, wobei R⁶ ausgewählt ist aus der Gruppe, bestehend aus (C₁-C₄)Alkyl, Amino, (C₁-C₄)Alkylamino, [(C₁-C₄)Alkyl]₂amino, Phenyl und - (C₁-C₄)Alkyl-phenyl, wobei das Phenyl gegebenenfalls mit ein oder zwei Substituenten, unabhängig ausgewählt aus Halogen, Cyano, (C₁-C₄)Alkyl und (C₁-C₄)Alkoxy, substituiert ist;
oder R¹ und R² zusammen mit dem N-Atom, an das sie gebunden sind, einen 3-, 4-, 5-, 6- oder 7-gliedrigen gesättigten Heterocyclus bilden, worin ein C-Atom durch N, 0, S, SO oder SO₂ ersetzt sein kann, und wobei der gesättigte Heterocyclus gegebenenfalls mit ein oder zwei Gruppen substituiert ist, die unabhängig ausgewählt sind aus Hydroxy, Halogen, =O, (C₁-C₄)Alkyl, -(C₁-C₄)Alkyl (C₃-C₆) cycloalkyl, (C₁-C₄)Alkoxy, Hydroxy (C₁-C₄)alkyl, (C₁-C₄) Alkoxy (C₁-C₄)alkyl, -SO₂(C₁-C₄)Alkyl, -C(O) (C₁-C₄)Alkyl, [(C₁-C₄)Alkyl]₂amino, Amino, (C₁-C₄)Alkylamino, -C(O)NH₂, C(O)O(C₁-C₄)Alkyl und Pyrrolidinon;
R³ und R⁴ jeweils unabhängig ausgewählt sind aus Wasserstoff, (C₃-C₆)Cycloalkyl und (C₁-C₄)Alkyl, wobei das (C₃-C₆)Cycloalkyl und (C₁-C₄)Alkyl gegebenenfalls mit Amino, (C₁-C₄)Alkylamino, [(C₁-C₄)Alkyl]₂amino oder (C₃-C₆)Cycloalkyl substituiert ist, oder R³ und R⁴ zusammen mit dem N-Atom, an das sie gebunden sind, einen 4-, 5-, 6- oder 7-gliedrigen gesättigten Heterocyclus bilden, in dem ein C-Atom durch N oder O ersetzt sein kann, und wobei der gesättigte Heterocyclus gegebenenfalls mit (C₁-C₄)Alkyl, [(C₁-C₄)Alkyl]₂amino, Amino, (C₁-C₄)Alkylamino oder -C(O) (C₁-C₄)Alkyl substituiert sein kann, wobei das -C(O)(C₁-C₄)Alkyl gegebenenfalls mit Methoxy oder Ethoxy substituiert ist,
Y ausgewählt ist aus CH₂, CH(OH), O, C=O und N, wobei das N mit H, (C₁-C₄) Alkyl, C(O)(C₁-C₄) Alkyl oder (C₁-C₄)Alkoxy(C₁-C₄)alkyl substituiert ist;
Z ausgewählt ist aus O, S, SO und SO₂;
m und p beide ganze Zahlen sind, die unabhängig 1, 2 oder 3 sind, mit der Maßgabe, dass m+p gleich oder kleiner als 4 ist, sodass der durch: 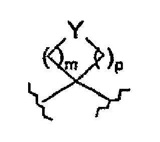 gebildete Ring ein 4-, 5- oder 6-gliedriger Ring ist;
und R entweder eine Gruppe der Formel: 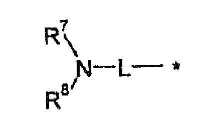 ist, worin * den Verknüpfungspunkt mit Z darstellt, L ein geradkettiges oder verzweigtes (C₂-C₆) Alkylen ist und R⁷ und R⁸ jeweils unabhängig ausgewählt sind aus Wasserstoff, (C₁-C₆) Alkyl, (C₃-C₆)Cycloalkyl, Hydroxy(C₁-C₆)alkyl oder R⁷ und R⁸ zusammen mit dem N-Atom, an das sie gebunden sind, einen 4-, 5-, 6- oder 7-gliedrigen gesättigten Heterocyclus bilden, in dem ein C-Atom gegebenenfalls N, O, S, SO oder SO₂ ersetzt ist, und wobei der gesättigte Heterocyclus gegebenenfalls mit ein oder zwei Gruppen substituiert ist, unabhängig ausgewählt aus (C₁-C₄)Alkyl, (C₁-C₄)Alkoxy, (C₁-C₄)Alkoxy (C₁-C₄) alkyl, Hydroxy(C₁-C₄) alkyl, Hydroxy, C(O)O(C₁-C₄)Alkyl, -C(O) (C₁-C₄)Alkyl-NH₂, -C(O)NH₂, Halogen, Amino, (C₁-C₄)Alkylamino und [(C₁-C₄)Alkyl]₂amino,
oder R ein Gruppe der Formel: 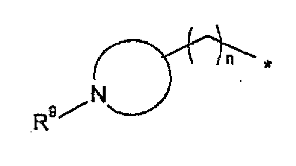 ist, worin * den Verknüpfungspunkt mit Z darstellt, der N-enthaltende Ring ein 4- bis 7-gliedriger gesättigter Heterocyclus ist, n eine ganze Zahl gleich 0, 1 oder 2 ist und R⁹ einen Substituenten darstellt, der ausgewählt ist aus Wasserstoff, (C₁-C₄)Alkyl, Hydroxy(C₁-C₆)alkyl und (C₃-C₆)Cycloalkyl;
Het¹ ausgewählt ist aus monocyclischen oder bicyclischen heteroaromatischen Gruppen, die 5 bis 10 Ringglieder haben, welche 1, 2, 3 oder 4 Heteroatom(e), ausgewählt aus Stickstoff, Sauerstoff und Schwefel, enthalten, und Het² ausgewählt ist aus monocyclischen oder bicyclischen heteroaromatischen Gruppen, die 5 bis 10 Ringglieder haben, welche 1, 2, 3 oder 4 Heteroatom(e), ausgewählt aus Stickstoff, Sauerstoff und Schwefel, enthalten.

2. Verbindung der Formel (1), wie sie in Anspruch 1 definiert ist, wobei X ausgewählt ist aus -CH₂-NR¹R², -C(O)NR³R⁴, -CH₂-Het¹ und Het¹, wobei Het¹ gegebenenfalls einmal oder zweimal mit (C₁-C₄)Alkyl substituiert ist, wobei R¹, R², R³, R⁴ und Het¹ wie vorstehend in Anspruch 1 definiert sind.

3. Verbindung der Formel (1), wie sie in Anspruch 2 definiert ist, wobei X -CH₂-Het¹ oder Het¹ ist und Het¹ ausgewählt ist aus einer 5- oder 6-gliedrigen monocyclischen heteroaromatischen Gruppe oder einer 9-gliedrigen bicyclischen heteroaromatischen Gruppe, wobei jede heteroaromatische Gruppe 1 bis 3 Stickstoffatome oder 1 bis 2 Stickstoffatome und 1 Sauerstoffatom oder 1 Stickstoffatom und 1 Schwefelatom enthält und jede heteroaromatische Gruppe gegebenenfalls einmal oder zweimal mit (C₁-C₄)Alkyl substituiert ist.

4. Verbindung der Formel (1), wie sie in Anspruch 3 definiert ist, wobei X Thiazolyl, Benzimidazolylmethyl, Pyridinyl, Oxazolyl, Imidazopyridinylmethyl, Pyrimidinyl, Imidazolyl, Imidazolylmethyl oder Triazolylmethyl ist, wobei das Thiazolyl, Benzimidazolylmethyl, Pyridinyl, Oxazolyl, Imidazopyridinylmethyl, Pyrimidinyl, Imidazolyl, Imidazolylmethyl und Triazolylmethyl jeweils unabhängig mit einer Methylgruppe substituiert ist.

5. Verbindung der Formel (1), wie sie in Anspruch 1 oder Anspruch 2 definiert ist, wobei R¹ Wasserstoff, Methyl oder Ethyl ist.

6. Verbindung der Formel (1), wie sie in Anspruch 1, Anspruch 2 oder Anspruch 5 definiert ist, wobei R² ausgewählt ist aus der Gruppe, bestehend aus
Wasserstoff,
(C₁-C₆)Alkyl, gegebenenfalls substituiert mit ein oder zwei Substituenten, unabhängig ausgewählt aus -S-(C₁-C₄)Alkyl, -O-(C₁-C₄)Alkyl, -SO₂-(C₁-C₄)Alkyl und Phenyl, wobei das Phenyl gegebenenfalls mit ein oder zwei Substituenten, unabhängig ausgewählt aus Halogen, Hydroxy, Cyano, (C₁-C₄)Alkyl und (C₁-C₄)Alkoxy, substituiert ist, (C₃-C₆)Cycloalkyl,
Het², gegebenenfalls substituiert mit ein oder zwei Substituenten, unabhängig ausgewählt aus Halogen, Cyano, (C₁-C₄)Alkyl und (C₁-C₄)Alkoxy, wobei Het² wie in Anspruch 1 definiert ist,
-SO₂-R⁵, wobei R⁵ ausgewählt ist aus der Gruppe, bestehend aus (C₁-C₄)Alkyl, [(C₁-C₄)Alkyl]₂amino, Phenyl und -(C₁-C₄)Alkylphenyl, wobei das Phenyl gegebenenfalls substituiert ist mit einem Substituenten, unabhängig ausgewählt aus Halogen und Cyano, und
-C(O)-R⁶, wobei R⁶ ausgewählt ist aus der Gruppe, bestehend aus (C₁-C₄)Alkyl, ((C₁-C₄)Alkyl]₂amino, Amino und -(C₁-C₄)Alkylphenyl, wobei das Phenyl gegebenenfalls mit ein oder zwei Substituenten, unabhängig ausgewählt aus Halogen, Cyano, (C₁-C₄)Alkyl und (C₁-C₄)Alkoxy, substituiert ist.

7. Verbindung der Formel (1), wie sie in Anspruch 6 definiert ist, wobei R² ausgewählt ist aus der Gruppe, bestehend aus
(C₁-C₃)Alkyl, gegebenenfalls substituiert mit -O-(C₁-C₃)Alkyl,
(C₃-C₆)Cycloalkyl,
Het², wobei Het² ausgewählt ist aus der Gruppe, bestehend aus 5- oder 6-gliedrigen monocyclischen heteroaromatischen Gruppen, die 1 bis 2 Stickstoffatome oder 1 Stickstoffatom und 1 Sauerstoffatom oder 1 Stickstoffatom und 1 Schwefelatom enthalten, wobei Het² gegebenenfalls mit (C₁-C₄)Alkyl substituiert ist,
SO₂-R⁵, wobei R⁵ (C₁-C₄)Alkyl ist, und
C(O)-R⁶, wobei R⁶ (C₁-C₄)Alkyl ist.

8. Verbindung der Formel (1), wie sie in Anspruch 7 definiert ist, wobei R² (C₁-C₃)Alkyl ist, das gegebenenfalls mit Methoxy substituiert ist.

9. Verbindung der Formel (1), wie sie in Anspruch 7 definiert ist, wobei R² Het² ist und Het² ausgewählt ist aus der Gruppe, bestehend aus 5- oder 6-gliedrigen monocyclischen heteroaromatischen Gruppen, die 1 oder 2 Stickstoffatome enthalten.

10. Verbindung der Formel (1), wie sie in Anspruch 9 definiert ist, wobei R² eine Pyridazinylgruppe ist.

11. Verbindung der Formel (1), wie sie in Anspruch 1 oder Anspruch 2 definiert ist, wobei R¹ und R² zusammen mit dem N-Atom, an das sie gebunden sind, einen 4-, 5-, 6- oder 7-gliedrigen gesättigten Heterocyclus bilden, in dem ein C-Atom durch N, O, S, SO oder SO₂ ersetzt sein kann, und wobei der gesättigte Heterocyclus gegebenenfalls mit 1 oder 2 Gruppen substituiert ist, unabhängig ausgewählt aus Hydroxy, Halogen, =O, (C₁-C₄)Alkyl, - (C₁-C₄) Alkyl (C₃-C₆) cycloalkyl, (C₁-C₄)Alkoxy, Hydroxy(C₁-C₄)alkyl, (C₁-C₄)Alkoxy(C₁-C₄)alkyl, -SO₂(C₁-C₄)Alkyl, -C (O) (C₁-C₄)Alkyl, [(C₁-C₄)Alkyl]₂amino, -C(O)NH₂, C(O)O(C₁-C₄)Alkyl und Pyrrolidinon.

12. Verbindung der Formel (1), wie sie in Anspruch 11 definiert ist, wobei R¹ und R² zusammen mit dem N-Atom, an das sie gebunden sind, eine Morpholinylgruppe bilden.

13. Verbindung der Formel (1), wie sie in Anspruch 1 oder Anspruch 2 definiert ist, wobei R³ und R⁴ jeweils unabhängig ausgewählt sind aus Wasserstoff und (C₁-C₄)Alkyl oder R³ und R⁴ zusammen mit dem N-Atom, an das sie gebunden sind, einen 4-, 5- oder 6-gliedrigen gesättigten Heterocyclus bilden, in dem ein C-Atom durch N oder 0 ersetzt sein kann, und wobei der gesättigte Heterocyclus gegebenenfalls mit (C₁-C₄)Alkyl substituiert ist.

14. Verbindung der Formel (1), wie sie in Anspruch 13 definiert ist, wobei R³ und R⁴ unabhängig aus Wasserstoff, Methyl und Ethyl ausgewählt sind oder R³ und R⁴ zusammen mit dem N-Atom, an das sie gebunden sind, einen Pyrrolidinyl-, Piperidinyl-, Piperazinyl- oder Azetidinylring bilden, wobei jeder Pyrrolidinyl-, Piperidinyl-, Piperazinyl- oder Azetidinylring gegebenenfalls mit Methyl substituiert ist.

15. Verbindung der Formel (1), wie sie in einem der vorangehenden Ansprüche definiert ist, wobei Y aus CH₂, CH(OH), O und C=O ausgewählt ist.

16. Verbindung der Formel (1), wie sie in Anspruch 15 definiert ist, wobei Y 0 ist.

17. Verbindung der Formel (1), wie sie in einem der vorangehenden Ansprüche definiert ist, wobei Z 0 ist.

18. Verbindung der Formel (1), wie sie in einem der vorangehenden Ansprüche definiert ist, wobei n und p beide 2 sind.

19. Verbindung der Formel (1), wie sie in einem der vorangehenden Ansprüche definiert ist, wobei R eine Gruppe der Formel: 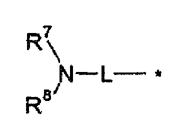 ist, worin * den Verknüpfungspunkt mit Z darstellt, L ein (C₂-C₆)Alkylen ist und R⁷ und R⁸ jeweils unabhängig ausgewählt sind aus Wasserstoff, (C₁-C₆)Alkyl, (C₃-C₆)Cycloalkyl, Hydroxy(C₁-C₆)alkyl oder R⁷ und R⁸ zusammen mit dem N-Atom, an das sie gebunden sind, einen 4-, 5-, 6- oder 7-gliedrigen gesättigten Heterocyclus bilden, in dem ein C-Atom gegebenenfalls durch N, O, S, SO oder SO₂ ersetzt ist und wobei der gesättigte Heterocyclus gegebenenfalls mit ein oder zwei Gruppen substituiert ist, unabhängig ausgewählt aus (C₁-C₄)Alkyl, (C₁-C₄)Alkoxy, (C₁-C₄)Alkoxy(C₁-C₄)alkyl, Hydroxy(C₁-C₄)alkyl, Hydroxy, C(O)O(C₁-C₄)Alkyl, -C(O)-(C₁-C₄)Alkyl-NH₂, -C(O)NH₂ und Halogen.

20. Verbindung der Formel (1), wie sie in Anspruch 19 definiert ist, wobei R⁷ und R⁸ zusammen mit dem N-Atom, an das sie gebunden sind, eine Morpholinyl- oder Oxazepanylgruppe bilden.

21. Verbindung der Formel (1), wie sie in Anspruch 19 definiert ist, wobei R⁷ und R⁸ zusammen mit dem N-Atom, an das sie gebunden sind, einen 4-, 5- oder 6-gliedrigen gesättigten Heterocyclus bilden, der gegebenenfalls mit ein oder zwei (C₁-C₄)Alkylgruppen substituiert ist.

22. Verbindung der Formel (1), wie sie in Anspruch 21 definiert ist, wobei der gesättigte Heterocyclus eine Pyrrolidinylgruppe ist, die gegebenenfalls mit ein oder zwei Methylgruppen substituiert ist.

23. Verbindung der Formel (1), wie sie in Anspruch 19 definiert ist, wobei R⁷ und R⁸ (C₁-C₃)Alkyl sind.

24. Verbindung der Formel (1), wie sie in einem der Ansprüche 19 bis 23 definiert ist, wobei L Propylen ist.

25. Verbindung der Formel (1), wie sie in einem der Ansprüche 1 bis 18 definiert ist, wobei R eine Gruppe der Formel: 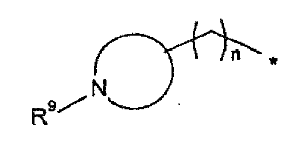 ist, worin * den Verknüpfungspunkt mit Z darstellt, der N-enthaltende Ring ein 4- oder 6-gliedriger gesättigter Heterocyclus ist, n eine ganze Zahl von 0 oder 1 ist und R⁹ einen Substituenten darstellt, ausgewählt aus Wasserstoff, (C₁-C₄)Alkyl und (C₃-C₆)Cycloalkyl.

26. Verbindung der Formel (1), wie sie in Anspruch 25 definiert ist, wobei R⁹ Isopropyl oder Cyclobutyl ist.

27. Verbindung der Formel (1) nach Anspruch 1, die ausgewählt ist aus: N-{4-[4-(3-Pyrrolidin-1-yl-propoxy)-phenyl]-tetrahydropyran-4-ylmethyl}-methansulfonamid; {4-[4-(3-Pyrrolidin-1--yl-propoxy)-phenyl]-tetrahydropyran-4-ylmethyl}-harnstoff; 4-[4-(3-Pyrrolidin-1-y1-propoxy)-phenyl]-tetrahydro-2H-pyran-4-carbonsäureamid; 4-{4-[1-Isopropylpiperidin-4-yl)oxy]phenyl}-tetrahydro-2H-pyran-4-carboxamid; 4-{4-[(1-Cyclobutylpiperidin-4-yl)oxy]phenyl}-tetrahydro-2H-pyran-4-carboxamid; N-{[4-(4-{3-[(3R)-3-(Dimethylamino)pyrrolidin-1-yl]-propoxy}phenyl)tetrahydro-2H-pyran-4-yl]-methyl}-N-methylmethansulfonamid und N*4*-{4-[4-(3-Pyrrolidin-1-yl-propoxy)-phenyl]-tetrahydropyran-4-ylmethyl}-pyridin-3,4-diamin.

28. Pharmazeutische Zusammensetzung, die eine Verbindung der Formel (1) oder ein pharmazeutisch verträgliches Salz und/oder Solvat davon, wie in einem der vorangehenden Ansprüche definiert, zusammen mit einem pharmazeutisch verträglichen Exzipiens umfasst.

29. Verbindung der Formel (1), wie sie in einem der Ansprüche 1 bis 27 definiert ist, oder ein pharmazeutisch verträgliches Salz und/oder Solvat davon zur Verwendung als Medikament.

30. Verwendung einer Verbindung der Formel (1) nach einem der Ansprüche 1 bis 27 oder eines pharmazeutisch verträglichen Salzes und/oder Solvats davon für die Herstellung eines Medikaments für die Behandlung von Schlafstörungen, Migräne, Dyskinesie, stressinduzierter Angst, psychotischen Störungen, Epilepsie, Wahrnehmungsdefizienzerkrankungen, Depression, Gemütsstörungen, Schizophrenie, Angststörungen, Aufmerksamkeits- und Hyperaktivitätsstörung (ADHS), psychotischen Störungen, Adipositas, Schwindel, Vertigo, Epilepsie, Bewegungskrankheit, Entzündungskrankheiten, Atemnotsyndrom bei Erwachsenen, akutem Atemnotsyndrom, Bronchitis, chronischer Bronchitis, chronisch-obstruktiver Lungenerkrankung, zystischer Fibrose, Asthma, Emphysem, Rhinitis, chronischer Sinusitis, Allergie, Allergie-induzierter Atemwegsreaktionen, allergischer Rhinitis, viraler Rhinitis, nichtallergischer Rhinitis, perennialer und saisonaler Rhinitis, Nasenschleimhautanschwellung, allergischer Kongestion, männlicher sexueller Dysfunktion oder weiblicher sexueller Dysfunktion.

31. Verwendung einer Verbindung der Formel (1) nach Anspruch 30, wobei die Wahrnehmungsdefizienzerkrankung Alzheimer'sche Erkrankung oder milde Wahrnehmungsbeeinträchtigung ist.

32. Kombination aus einer Verbindung der Formel (1), wie sie in einem der Ansprüche 1 bis 27 definiert ist, und einem anderen pharmakologisch aktiven Mittel.

33. Kombination, wie sie in Anspruch 32 beansprucht ist, wobei das andere pharmakologisch aktive Mittel ein Histamin-H1-Rezeptor-Antagonist ist.

34. Verbindung der Formel (1), wie sie in einem der Ansprüche 1 bis 27 definiert ist, oder ein pharmazeutisch verträgliches Salz und/oder Solvat davon zur Verwendung bei der Behandlung der Erkrankungen, Störungen und Zustände, wie sie in den Ansprüchen 30 und 31 definiert sind.

## Revendications

1. Composé de formule (1) : 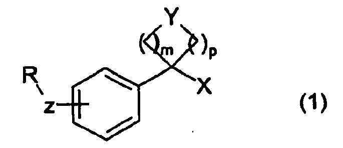 ou un de ses sels et/ou produits de solvatation (y compris hydrates) pharmaceutiquement acceptables, formule dans laquelle :
le substituant de formule -Z-R est en position méta ou para par rapport au groupe phényle ;
X est choisi entre des groupes -CN, -CH₂OH, -CH₂-O-(alkyle en C₁ à C₄), -C(O)OH, -C(O)O(alkyle en C₁ à C₄), -CH₂-NR₁R₂, -C(O)NR³R⁴, -CH₂-O-het², -CH₂-het¹ et het¹, le groupe het¹ dans les deux groupes -CH₂-het¹ et het¹ étant facultativement substitué avec un ou deux substituants choisis indépendamment entre des substituants halogéno, cyano, alkyle en C₁ à C₄, -S- (alkyle en C₁ à C₄) et alkoxy en C₁ à C₄ ;
R¹ représente un atome d'hydrogène ou un groupe alkyle en C₁ à C₄ facultativement substitué avec un substituant cycloalkyle en C₃ à C₆ ;
R² est choisi dans le groupe consistant en :
un atome d'hydrogène,
un groupe alkyle en C₁ à C₆ facultativement substitué avec un ou deux substituants choisis indépendamment entre des substituants cycloalkyle en C₃ à C₆, hydroxy, -S-(alkyle en C₁ à C₄), -0- (alkyle en C₁ à C₄), -SO₂-(alkyle en C₁ à C₄), -SO-(alkyle en C₁ à C₄), halogéno, het¹, amino, alkylamino en C₁ à C₄, (alkyle en C₁ à C₄)₂ amino et phényle, ledit substituant phényle étant facultativement substitué avec un ou deux substituants choisis indépendamment entre des substituants halogéno, hydroxy, cyano, alkyle en C₁ à C₄ et alkoxy en C₁ à C₄,
cycloalkyle en C₃ à C₆,
het², facultativement substitué avec un ou deux substituants choisis indépendamment entre des substituants halogéno, cyano, alkyle en C₁ à C₄, NH₂ et alkoxy en C₁ à C₄,
-SO₂-R⁵, dans lequel R⁵ est choisi dans le groupe consistant en des groupes alkyle en C₁ à C₄, amino, alkylamino en C₁ à C₄, (alkyle en C₁ à C₄) ₂amino, phényle et -(alkyle en C₁ à C₄)phényle, ledit groupe phényle étant facultativement substitué avec un ou deux substituants choisis indépendamment entre des substituants halogéno, cyano, alkyle en C₁ à C₄ et alkoxy en C₁ à C₄ , et
-C(O)-R⁶, dans lequel R⁶ est choisi dans le groupe consistant en des groupes alkyle en C₁ à C₄, amino, alkylamino en C₁ à C₄, (alkyle en C₁ à C₄)₂amino, phényle et -(alkyle en C₁ à C₄)phényle, ledit substituant phényle étant facultativement substitué avec un ou deux substituants choisis indépendamment entre des substituants halogéno, cyano, alkyle en C₁ à C₄ et alkoxy en C₁ à C₄ ;
ou bien R¹ et R² forment, conjointement avec l'atome de N auquel ils sont fixés, un hétérocycle tri-, tétra-, penta-, hexa- ou heptagonal saturé dans lequel un atome de C peut être remplacé par N, O, S, SO ou SO₂, et ledit hétérocycle saturé est facultativement substitué avec un ou deux groupes choisis indépendamment entre des groupes hydroxy, halogéno, =0, alkyle en C₁ à C₄, - (alkyle en C₁ à C₄) - (cycloalkyle en C₃ à C₆), alkoxy en C₁ à C₄, hydroxyalkyle en C₁ à C₄, (alkoxy en C₁ à C₄) (alkyle en C₁ à C₄), -SO₂-(alkyle en C₁ à C₄), -C(O) (alkyle en C₁ à C₄), (alkyle en C₁ à C₄)₂amino, amino, alkylamino en C₁ à C₄, -C(O)NH₂, C(O)O-(alkyle en C₁ à C₄) et pyrrolidinone ;
R³ et R⁴ sont choisis chacun indépendamment entre un atome d'hydrogène, des groupes cycloalkyle en C₃ à C₆ et alkyle en C₁ à C₄, lesdits groupes cycloalkyle en C₃ à C₆ et alkyle en C₁ à C₄ étant facultativement substitués avec des substituants amino, alkylamino en C₁ à C₄, (alkyle en C₁ à C₄)₂-amino ou cycloalkyle en C₃ à C₆, ou bien R³ et R⁴ forment, conjointement avec l'atome de N auquel ils sont fixés, un hétérocycle tétra-, penta-, hexa- ou heptagonal saturé dans lequel un atome de C peut être remplacé par N ou 0, et ledit hétérocycle saturé est facultativement substitué avec un substituant alkyle en C₁ à C₄, (alkyle en C₁ à C₄)₂amino, amino, alkylamino en C₁ à C₄ ou -C(O) (alkyle en C₁ à C₄), ledit substituant -C(O) (alkyle en C₁ à C₄) étant facultativement substitué avec un substituant méthoxy ou éthoxy,
Y est choisi entre des groupes CH₂, CH(OH), O, C=O et N, ledit atome de N étant substitué avec un substituant H, alkyle en C₁ à C₄, C(O) (alkyle en C₁ à C₄) ou (alkoxy en C₁ à C₄) (alkyle en C₁ à C₄) ;
Z est choisi entre 0, S, des groupes SO et SO₂;
m et p représentent tous deux des nombres entiers qui sont égaux indépendamment à 1, 2 ou 3, sous réserve que la somme m + p soit égale ou inférieure à 4, de telle sorte que le noyau formé par : 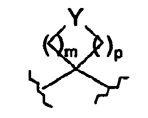 soit un noyau tétra-, penta- ou hexagonal ;
et R représente soit un groupe de formule : 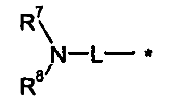 dans laquelle * représente le point de fixation à Z, L représente un groupe alkylène en C₂ à C₆ à chaîne droite ou ramifié et R⁷ et R⁸ sont choisis chacun indépendamment entre un atome d'hydrogène, des groupes alkyle en C₁ à C₆, cycloalkyle en C₃ à C₆ et hydroxyalkyle en C₁ à C₆, ou bien R⁷ et R⁸ forment, conjointement avec l'atome de N auquel ils sont fixés, un hétérocycle tétra-, penta-, hexa- ou heptagonal saturé dans lequel un atome de C est facultativement remplacé par N, O, S, SO ou SO₂, ledit hétérocycle saturé étant facultativement substitué avec un ou deux groupes choisis indépendamment entre des groupes alkyle en C₁ à C₄, alkoxy en C₁ à C₄, (alkoxy en C₁ à C₄) (alkyle en C₁ à C₄), hydroxyalkyle en C₁ à C₄, hydroxy, C(O)O(alkyle en C₁ à C₄), -C(O)(alkyle en C₁ à C₄)-NH₂, -C(O)NH₂, halogéno, amino, alkylamino en C₁ à C₄ et (alkyle en C₁ à C₄)₂amino,
soit R représente un groupe de formule : 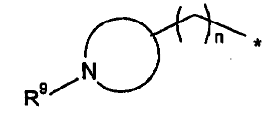 dans laquelle * représente le point de fixation à Z, le noyau contenant N est un hétérocycle tétra- à heptagonal saturé, n représente un nombre entier égal à 0, 1 ou 2 et R⁹ représente un substituant choisi entre des substituants hydrogène, alkyle en C₁ à C₄, hydroxyalkyle en C₁ à C₆ et cycloalkyle en C₃ à C₆ ;
het¹ est choisi parmi des groupes hétéroaromatiques monocycliques ou bicycliques ayant 5 à 10 membres dans le noyau, qui contiennent 1, 2, 3 ou 4 hétéroatomes choisis entre l'azote, l'oxygène et le soufre, et het² est choisi parmi des groupes hétéroaromatiques monocycliques ou bicycliques ayant 5 à 10 membres dans le noyau, qui contiennent 1, 2, 3 ou 4 hétéroatomes choisis entre l'azote, l'oxygène et le soufre.

2. Composé de formule (1) suivant la revendication 1, dans lequel X est choisi entre des groupes -CH₂-NR¹R², -C(O)NR³R⁴, -CH₂-het¹ et het¹, het¹ étant facultativement substitué une ou deux fois avec un substituant alkyle en C₁ à C₄, où R¹, R², R³, R⁴ et het¹ répondent aux définitions précitées sans la revendication 1.

3. Composé de formule (1) suivant la revendication 2, dans lequel X représente un groupe -CH₂-het¹ ou het¹, et het¹ est choisi entre un groupe hétéroaromatique penta- ou hexagonal monocyclique et un groupe hétéroaromatique nonagonal bicyclique, ledit groupe hétéroaromatique contenant 1 à 3 atomes d'azote, ou un 1 ou 2 atomes d'azote et 1 atome d'oxygène, ou bien 1 atome d'azote et un atome de soufre, et chaque groupe hétéroaromatique étant facultativement substitué une ou deux fois avec un substituant alkyle en C₁ à C₄.

4. Composé de formule (1) suivant la revendication 3, dans lequel X représente un groupe thiazolyle, benzimidazolylméthyle-, pyridinyle, oxazolyle, imidazopyridinylméthyle-, pyrimidinyle, imidazolyle, imidazolylméthyle- ou triazolylméthyle-, ledit groupe thiazolyle, benzimidazolylméthyle-, pyridinyle, oxazolyle, imidazopyridinylméthyle-, pyrimidinyle, imidazolyle, imidazolylméthyle- et triazolylméthyle- chacun étant facultativement substitué avec un groupe méthyle.

5. Composé de formule (1) suivant la revendication 1 ou la revendication 2, dans lequel R¹ représente un atome d'hydrogène, un groupe méthyle ou éthyle.

6. Composé de formule (1) suivant la revendication 1, la revendication 2 ou la revendication 5, dans lequel R² est choisi dans le groupe consistant en
un atome d'hydrogène,
un groupe alkyle en C₁ à C₆ facultativement substitué avec un ou deux substituants choisis indépendamment entre des substituants -S- (alkyle en C₁ à C₄), -O-(alkyle en C₁ à C₄), -SO₂-(alkyle en C₁ à C₄) et phényle, ledit substituant phényle étant facultativement substitué avec un ou deux substituants choisis indépendamment entre des substituants halogéno, hydroxy, cyano, alkyle en C₁ à C₄ et alkoxy en C₁ à C₄,
cycloalkyle en C₃ à C₆,
het², facultativement substitué avec un ou deux substituants choisis indépendamment entre des substituants halogéno, cyano, alkyle en C₁ à C₄ et alkoxy en C₁ à C₄, het² étant tel que défini dans la revendication 1,
-SO₂-R⁵, dans lequel R⁵ est choisi dans le groupe consistant en des groupes alkyle en C₁ à C₄, (alkyle en C₁ à C₄)₂amino, phényle et - (alkyle en C₁ à C₄)phényle, dans lesquels ledit groupe phényle est facultativement substitué avec un substituant choisi indépendamment entre des substituants halogéno et cyano, et
-C(O)-R⁶, dans lequel R⁶ est choisi dans le groupe consistant en des groupes alkyle en C₁ à C₄, (alkyle en C₁ à C₄)₂amino, amino et - (alkyle en C₁ à C₄)phényle, ledit groupe phényle étant facultativement substitué avec un ou deux substituants choisis indépendamment entre des substituants halogéno, cyano, alkyle en C₁ à C₄ et alkoxy en C₁ à C₄.

7. Composé de formule (1) suivant la revendication 6, dans lequel R² est choisi dans le groupe consistant en des groupes
alkyle en C₁ à C₃ facultativement substitué avec un substituant -O-(alkyle en C₁ à C₃),
cycloalkyle en C₃ à C₆,
het², het² étant choisi dans le groupe consistant en des groupes hétéroaromatiques penta- ou hexagonaux monocycliques contenant 1 ou 2 atomes d'azote ou un atome d'azote et un atome d'oxygène ou bien un atome d'azote et un atome de soufre, ledit groupe het² étant facultativement substitué avec un substituant alkyle en C₁ à C₄,
SO₂-R⁵, dans lequel R⁵ représente un groupe alkyle en C₁ à C₄, et
C(O)-R⁶, dans lequel R⁶ représente un groupe alkyle en C₁ à C₄.

8. Composé de formule (1) suivant la revendication 7, dans lequel R² représente un groupe alkyle en C₁ à C₃ facultativement substitué avec un substituant méthoxy.

9. Composé de formule (1) suivant la revendication 7, dans lequel R² représente un groupe het², et het² est choisi dans le groupe consistant en des groupes hétéroaromatiques penta- ou hexagonaux monocycliques contenant 1 ou 2 atomes d'azote.

10. Composé de formule (1) suivant la revendication 9, dans lequel R² représente un groupe pyridazinyle.

11. Composé de formule (1) suivant la revendication 1 ou la revendication 2, dans lequel R¹ et R² forment, conjointement avec l'atome de N auquel ils sont fixés, un hétérocycle tétra-, penta-, hexa- ou heptagonal saturé dans lequel un atome de C peut être remplacé par N, O, S, SO ou SO₂, ledit hétérocycle saturé étant facultativement substitué avec un ou deux groupes choisis indépendamment entre des groupes hydroxy, halogéno, =0, alkyle en C₁ à C₄, -(alkyle en C₁ à C₄) (cycloalkyle en C₃ à C₆), alkoxy en C₁ à C₄, hydroxyalkyle en C₁ à C₄, (alkoxy en C₁ à C₄) (alkyle en C₁ à C₄), -SO₂(alkyle en C₁ à C₄), -C(O) (alkyle en C₁ à C₄), (alkyle en C₁ à C₄)₂amino, -C(O)NH₂, C(O)O(alkyle en C₁ à C₄) et pyrrolidinone.

12. Composé de formule (1) suivant la revendication 11, dans lequel R¹ et R² forment, conjointement avec l'atome de N auquel ils sont fixés, un groupe morpholinyle.

13. Composé de formule (1) suivant la revendication 1 ou la revendication 2, dans lequel R³ et R⁴ sont choisis chacun indépendamment entre un atome d'hydrogène et un groupe alkyle en C₁ à C₄, ou bien R³ et R⁴ forment, conjointement avec l'atome de N auquel ils sont fixés, un hétérocycle tétra-, penta- ou hexagonal saturé dans lequel un atome de C peut être remplacé par N ou 0, ledit hétérocycle saturé étant facultativement substitué avec un substituant alkyle en C₁ à C₄.

14. Composé de formule (1) suivant la revendication 13, dans lequel R³ et R⁴ sont choisis indépendamment entre un atome d'hydrogène, des groupes méthyle et éthyle, ou bien R³ et R⁴ forment, conjointement avec l'atome de N auquel ils sont fixés, un noyau pyrrolidinyle, pipéridinyle, pipérazinyle ou azétidinyle, chacun des noyaux pyrrolidinyle, pipéridinyle, pipérazinyle et azétidinyle étant facultativement substitué avec un substituant méthyle.

15. Composé de formule (1) suivant l'une quelconque des revendications précédentes, dans lequel Y est choisi entre des groupes CH₂, CH(OH), 0 et C=O.

16. Composé de formule (1) suivant la revendication 15, dans lequel Y représente O.

17. Composé de formule (1) suivant l'une quelconque des revendications précédentes, dans lequel Z représente O.

18. Composé de formule (1) suivant l'une quelconque des revendications précédentes, dans lequel m et p sont tous deux égaux à 2.

19. Composé de formule (1) suivant l'une quelconque des revendications précédentes, dans lequel
R représente un groupe de formule : 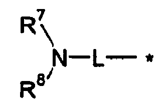 dans laquelle * représente le point de fixation à Z, L représente un groupe alkylène en C₂ à C₆ et R⁷ et R⁸ sont choisis chacun indépendamment entre un atome d'hydrogène, des groupes alkyle en C₁ à C₆, cycloalkyle en C₃ à C₆ et hydroxyalkyle en C₁ à C₆, ou bien R⁷ et R⁸ forment, conjointement avec l'atome de N auquel ils sont fixés, un hétérocycle tétra-, penta-, hexa- ou heptagonal saturé dans lequel un atome de C est facultativement remplacé par N, 0, S, SO ou SO₂, ledit hétérocycle saturé étant facultativement substitué avec un ou deux groupes choisis indépendamment entre des groupes alkyle en C₁ à C₄, alkoxy en C₁ à C₄, (alkoxy en C₁ à C₄) (alkyle en C₁ à C₄), hydroxyalkyle en C₁ à C₄, hydroxy, C(O)O(alkyle en C₁ à C₄), -C(O) (alkyle en C₁ à C₄) -NH₂, -C(O)NH₂ et halogéno.

20. Composé de formule (1) suivant la revendication 19, dans lequel R⁷ et R⁸, forment, conjointement avec l'atome de N auquel ils sont fixés, un groupe morpholinyle ou oxazépanyle.

21. Composé de formule (1) suivant la revendication 19, dans lequel R⁷ et R⁸ forment, conjointement avec l'atome de N auquel ils sont fixés, un hétérocycle tétra-, penta- ou hexagonal saturé, facultativement substitué avec un ou deux groupes alkyle en C₁ à C₄.

22. Composé de formule (1) suivant la revendication 21, dans lequel l'hétérocycle saturé est un groupe pyrrolidinyle, facultativement substitué avec un ou deux groupes méthyle.

23. Composé de formule (1) suivant la revendication 19, dans lequel R⁷ et R⁸ représentent des groupes alkyle en C₁ à C₃.

24. Composé de formule (1) suivant l'une quelconque des revendications 19 à 23, dans lequel L représente un groupe propylène.

25. Composé de formule (1) suivant l'une quelconque des revendications 1 à 18, dans lequel
R représente un groupe de formule : 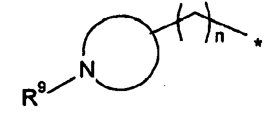 dans laquelle * représente le point de fixation à Z, le noyau contenant N est un hétérocycle tétra- ou hexagonal saturé, n représente un nombre entier égal à 0 ou 1 et R⁹ représente un substituant choisi entre des substituants hydrogène, alkyle en C₁ à C₄, et cycloalkyle en C₃ à C₆.

26. Composé de formule (1) suivant la revendication 25, dans lequel R⁹ représente un groupe isopropyle ou cyclobutyle.

27. Composé de formule (1) suivant la revendication 1, qui est choisi entre :
le N-{4-[4-(3-pyrrolidine-1-yl-propoxy)-phényl]-tétrahydropyranne-4-ylméthyl}-méthanesulfonamide ;
la {4-[4-(3-pyrrolidine-1-yl-propoxy)-phényl]-tétrahydropyranne-4-ylméthyl}-urée ;
l'amide d'acide 4-[4-(3-pyrrolidine-1-yl-propoxy)-phényl]-tétrahydro-2H-pyranne-4-carboxylique,
le 4-{4-[(1-isopropylpipéridine-4-yl)oxy]phényl}-tétrahydro-2H-pyranne-4-carboxamide ;
le 4-{4-[(1-cyclobutylpipéridine-4-yl)oxy]phényl}-tétrahydro-2H-pyranne-4-carboxamide ;
le N-{[4-(4-{3-[(3R)-3-(diméthylamino)pyrrolidine-1-yl]-propoxy}phényl)tétrahydro-2H-pyranne-4-yl]-méthyl}-N-méthyl-méthanesulfonamide ; et
la N * 4 *- {4- [4-(3-pyrrolidine-1-yl-propoxy) -phényl]-tétrahydropyranne-4-ylméthyl}-pyridine-3,4-diamine.

28. Composition pharmaceutique comprenant un composé de formule (1) ou un de ses sels et/ou produits de solvatation pharmaceutiquement acceptables, tel que défini dans l'une quelconque des revendications précédentes, conjointement avec un excipient pharmaceutiquement acceptable.

29. Composé de formule (1) suivant l'une quelconque des revendications 1 à 27 ou un de ses sels et/ou produits de solvatation pharmaceutiquement acceptables, destiné à être utilisé comme médicament.

30. Utilisation d'un composé de formule (1) suivant l'une quelconque des revendications 1 à 27 ou d'un de ses sels et/ou produits de solvatation pharmaceutiquement acceptables, pour la production d'un médicament destiné au traitement de troubles du sommeil, de la migraine, de la dyskinésie, de l'anxiété induite par le stress, de troubles psychotiques, de l'épilepsie, de maladies comportant une déficience de la cognition, de la dépression, de troubles de l'humeur, de la schizophrénie, de troubles du type de l'anxiété, du trouble d'hyperactivité avec déficit d'attention (ADHD), de troubles psychotiques, de l'obésité, des étourdissements, des vertiges, de l'épilepsie, du mal des transports, de maladies inflammatoires, du syndrome de détresse respiratoire de l'adulte, du syndrome de détresse respiratoire aiguë, de la bronchite, de la bronchite chronique, d'une maladie pulmonaire obstructive chronique, de la fibrose kystique, de l'asthme, de l'emphysème, de la rhinite, de la sinusite chronique, de l'allergie, des réponses des voies aériennes induites par une allergie, de la rhinite allergique, de la rhinite virale, de la rhinite non allergique, de la rhinite pérenne et de la rhinite saisonnière, de la congestion nasale, de la congestion allergique, d'un dysfonctionnement sexuel masculin ou d'un dysfonctionnement sexuel féminin.

31. Utilisation d'un composé de formule (1) suivant la revendication 30, dans laquelle la maladie comportant une déficience de la cognition est la maladie d'Alzheimer ou l'altération cognitive bénigne.

32. Association d'un composé de formule (1) suivant l'une quelconque des revendications 1 à 27 et d'un autre agent pharmacologiquement actif.

33. Association suivant la revendication 32, dans laquelle l'autre agent pharmacologiquement actif est un antagoniste du récepteur d'histamine H₁.

34. Composé de formule (1) suivant l'une quelconque des revendications 1 à 27 ou un de ses sels et/ou produits de solvatation pharmaceutiquement acceptables, destiné à être utilisé dans le traitement des maladies, troubles et affections définis dans les revendications 30 et 31.
